# EUROPEAN PATENT APPLICATION

(11) **EP 2 017 355 A1**
(43) Date of publication of application: **21.01.2009**
(21) Application number: 07742215.2
(22) Date of filing: 24.04.2007
(51) Int. Cl.: C12Q 1/68, A61K 31/7088, A61K 39/395, A61K 45/00, A61K 48/00, A61P 9/10, C12N 15/09, C12Q 1/02, C12Q 1/48, G01N 33/15, G01N 33/50

(54) **GENE ASSOCIATED WITH ARTERIOSCLEROTIC DISEASE, AND USE THEREOF**

(30) Priority: 25.04.2006 JP 2006121284
(71) Applicant: Kyushu University, National University Corporation, Higashi-ku Fukuoka-shi Fukuoka 812-8581 (JP); The University of Tokyo, Bunkyo-ku, Tokyo 113-8654 (JP); Hisayama Research Institute For Lifestyle Diseases, Kasuya-gun, Fukuoka 811-2501 (JP); NTT Data Corporation, Tokyo 135-6033 (JP); Hubit Genomix, Inc., Tokyo 102-0092 (JP)
(72) Inventor: KIYOHARA, Yutaka, Fukuoka-shi Fukuoka 812-8581 (JP); IIDA, Mitsuo, Fukuoka-shi Fukuoka 812-8581 (JP); IBAYASHI, Setsuro, Fukuoka-shi Fukuoka 812-8581 (JP); KUBO, Michiaki, Fukuoka-shi Fukuoka 812-8581 (JP); HATA, Jun, Fukuoka-shi Fukuoka 812-8581 (JP); NAKAMURA, Yusuke, Tokyo 113-8654 (JP); OMAE, Teruo, Kasuya-gun Fukuoka 811-2501 (JP); TANAKA, Yasuhiro, Tokyo 135-6033 (JP); ICHIEN, Go, Tokyo 102-0092 (JP)
(74) Representative: Vossius & Partner
(86) International application number: PCT/JP2007/058780
(87) International publication number: WO 2007/123233

(57) **Abstract**

Two genes implicated in arteriosclerotic diseases such as cerebral infarction were successfully identified by performing genome-wide correlation studies using SNPs by targeting the entire genome. Polymorphic mutations that can be used to examine the presence or absence of risk factors for arteriosclerotic diseases were successfully found on the genes. Subjects can be efficiently examined for the presence or absence of risk factors for arteriosclerotic diseases using the presence or absence of the polymorphic mutations as indicators. Furthermore, methods of screening for therapeutic agents for arteriosclerotic diseases are enabled by using expression or function of the genes as index.

## Description

### Technical Field

The present invention relates to methods of testing for the presence or absence of risk factors of arteriosclerotic diseases using the expression or polymorphic mutation of the AGTRL1 I gene or PRKCH gene as an index, and also relates to methods of screening for agents for treating arteriosclerotic diseases using these genes.

### Background Art

From the 1950s to 1960s, the mortality rate of cerebral apoplexy in Japan was the highest in the world. Since then, the mortality rate has declined steadily from the early 1970s, and in the 1990s the rate has become comparable to those of Europe and the United States (Non-Patent Document 1).

Nevertheless, it has not been changed and cerebral apoplexy is still the third major cause of death among Japanese. The incidence of cerebral apoplexy showed a decreasing trend during this period, but this trend has slowed down and leveled off in recent years (Non-Patent Document 2). Once cerebral apoplexy occurs, patients are often left with physical disability or cognitive dysfunction; therefore, cerebral apoplexy is a serious public health problem (Non-Patent Document 3). The incidence rate of cerebral apoplexy increases linearly with age. Since the elderly population is increasing rapidly in Japan, primary prevention of cerebral apoplexy is an important social objective worldwide.

Cerebral apoplexy can be broadly divided into cerebral infarction, intracerebral hemorrhage, and subarachnoid hemorrhage (Non-Patent Document 4). Of them, cerebral infarction has the highest incidence, and accounts for approximately 70% of all cerebral apoplexy (Non-Patent Document 5). Cerebral infarction can be further subdivided based on the size of the responsible blood vessel and mechanism of development, into the following subtypes: lacunar infarction (LA) caused by arteriosclerosis of thin perforating arteries, atherothrombotic cerebral infarction (AT) caused by atherosclerosis that affects the extracranial and major intracranial arteries, and cardiogenic embolic infarction (CE) which occurs when a thrombus produced in the cardiac cavity travels to the brain. LA and AT occur mainly due to atherosclerosis in the thin arteries or large arteries that perfuse the brain (Non-patent Document 4).

Multifactorial diseases including cerebral apoplexy implicate a plurality of genes and environmental factors, and afflict many patients, thus, elucidating those genetic factors is considered to have a major impact on medical economics and to contribute greatly to development of diagnostic and therapeutic techniques and prevention of the diseases. High blood pressure, diabetes, lipid metabolism disorders, smoking and such are known to be risk factors for cerebral infarction from past epidemiological studies (Non-Patent Documents 1 and 6). Family history of cerebral apoplexy is also a risk factor, and the risk of cerebral apoplexy is higher in monozygotic twins than in dizygotic twins (Non-Patent Document 7). While the presence of genetic factors implicated in cerebral infarction is expected from these twin studies and family history studies (Non-Patent Document 7), genetic determinants for cerebral infarction are still mostly unknown.

Several research styles have been proposed for the objective of discovering genes that provide susceptibility to common diseases that are not genetic diseases (Non-Patent Document 8). The candidate gene approach is widely used, but the lack of reproducibility of results is a problem (Non-Patent Document 9). For example, cerebral infarction-related genes have been examined using this method, and several known candidate genes implicated in atherosclerosis have been reported. However, their reproducibility has not necessarily been confirmed in other subject groups (Non-Patent Document 10). On the other hand, genome-wide study has recently been attracting attention as a highly reliable research technique for searching genes implicated in diseases that have complex mechanisms of development such as cerebral apoplexy (Non-Patent Document 14). In Japan, genome-wide correlation analyses have identified genes implicated in myocardial infarction (Non-Patent Document 11), rheumatoid arthritis (Non-Patent Document 12), or Crohn's disease (Non-Patent Document 13), and reproducibility of the results has been confirmed in different groups. Furthermore, in genome-wide linkage analyses and correlation analyses targeting Icelandic groups, phosphodiesterase 4D (PDE4D) (Non-Patent Document 15) and 5-lipoxygenase activating protein (ALOX5AP) (Non-Patent Document 16) have been reported as novel cerebral apoplexy-related genes. However, the research method for PDE4D has been criticized for having serious problems (Non-Patent Document 17).

Information on prior art literature related to the present invention is shown below.
[Patent Document 1] WO/2004/022592
[Patent Document 2] U.S. Patent No. 6,987,110
[Patent Document 3] U.S. Patent No. 6,492,324
[Patent Document 4] Japanese Patent Application No. 2003-518582
[Non-Patent Document 1] Sacco RL, et al., Stroke 1997; 28: 1507-1517.
[Non-Patent Document 2] Kubo, M. et al., Stroke 34, 2349-2354 (2003).
[Non-Patent Document 3] Kiyohara, Stroke. 34, 2343-2348 (2003).
[Non-Patent Document 4] Whisnant JP, et al., Stroke 1990; 21: 637-676.
[Non-Patent Document 5] Hata J, et al., J Neurol Neurosurg Psychiatry 2005; 76: 368-372.
[Non-Patent Document 6] Tanizaki Y, et al., Stroke 2000; 31: 2616-2622.
[Non-Patent Document 7] Flobmann E, et al., Stroke 2004; 35: 212-227.
[Non-Patent Document 8] Hirschhorn, JN. & Daly, MJ. Nat. Rev. Genet. 6, 95-108 (2005).
[Non-Patent Document 9] Tabor, HK. et al., Nat. Rev. Genet. 3,1-7 (2003).
[Non-Patent Document 10] Hassan A, Markus HS. Brain 2000; 123: 1784-1812.
[Non-Patent Document 11] Ozaki, K. et al., Nat. Genet. 32, 650-654 (2002).
[Non-Patent Document 12] Tokuhiro, S. et al., Nat. Genet. 35, 341-348 (2003).
[Non-Patent Document 13] Yamazaki, K. et al., Hum. Mol. Genet. 14, 3499-3506 (2005).
[Non-Patent Document 14] Glazier AM, et al., Science 2002; 298: 2345-2349.
[Non-Patent Document 15] Gretarsdottir S, et al., Nat Genet 2003; 35: 131-138.
[Non-Patent Document 16] Helgadottir A, et al., Nat Genet 2004; 36: 233-239.
[Non-Patent Document 17] Funalot, B. et al., Nat. Genet. 36, 3 (2004).
[Non-Patent Document 18] Bright R. et al., J Neurosci. 2004 Aug 4;24(31):6880-8.
[Non-Patent Document 19] Chintalgattu v. et al., J Pharmacol Exp Ther. 2004 Nov;311(2):691-9.
[Non-Patent Document 20] Hanlon PR. et al., FASEB J. 2005 Aug;19(10):1323-5.
[Non-Patent Document 21] Aronowski J. et al., J Cereb Blood Flow Metab. 2000 Feb;20(2):343-9.
[Non-Patent Document 22] Kleinz MJ, et al., Regul Pept 2005; 126: 233-240.
[Non-Patent Document 23] O'Carroll AM, et al., J Neuroendocrinol 2003; 15: 661-666.
[Non-Patent Document 24] Kagiyama S, et al., Regl Pept 2005; 125: 55-59.
[Non-Patent Document 25] Seyedabadi M, et al., Auton Neurosci. 2002; 101: 32-38.
[Non-Patent Document 26] Katugampola SD, et al., Br J Pharmacol 2001; 132: 1255-1260.
[Non-Patent Document 27] Tatemoto K, et al., Regul Pept 2001; 99: 87-92.
[Non-Patent Document 28] Masri B, et al., FASEB J 2004; 18: 1909-1911.
[Non-Patent Document 29] Hashimoto Y, et al., Int J Mol Med 2005; 16: 787-792.

### Disclosure of the Invention

### [Problems to be Solved by the Invention]

Cerebral infarction-related genes that have been previously reported are abnormal NOTCH3 gene in CADASIL and abnormal genes of mitochondrial DNA in MELAS, but these are causative genes of clearly hereditary cerebral infarction, and are not genes related to commonly found cerebral infarction. Research has been carried out for candidate genes related to common cerebral infarction mostly by using polymorphisms of genes predicted from the mechanism of cerebral infarction development (for example, coagulation system genes, ACE genes, and MTHFR genes), however, no definite observation has been made. Therefore, to identify new cerebral infarction-related genes, a genome-wide association study targeting the entire human genome is necessary. To date, only one genome-wide association study targeting cerebral apoplexy has been reported, and it was the PDE4D gene and ALOX5AP gene by the deCODE group in Iceland. Since the PDE4D gene showed significant relevance only in analyses when atherothrombotic cerebral infarction and cardiogenic embolism were combined, the results are questionable. In addition, there are no reports on reproducibility of the results by other groups. It has been confirmed by several groups that the ALOX5AP gene is implicated in cerebral infarction, but its implication is very weak in groups other than the Icelandic group.

The present invention was achieved in view of the above circumstances. An objective of the present invention is to provide genes implicated in arteriosclerotic diseases such as cerebral infarction, and uses that apply the characteristics of these genes. More specifically, an objective of the present invention is to provide genes implicated in arteriosclerotic diseases, methods of testing for the presence or absence of risk factors of arteriosclerotic diseases using polymorphisms of these genes, as well as methods of screening for pharmaceutical agents for treating arteriosclerotic diseases.

### [Means for Solving the Problems]

The present inventors conducted dedicated research to achieve the above-mentioned objectives. The present inventors performed case-control studies using large-scale gene-based tag-SNP markers to investigate genetic contribution to arteriosclerotic diseases such as cerebral infarction.

The present inventors discovered that PRKCH, which is a protein kinase C (PKC) family gene, is highly associated with lacunar infarction and atherothrombotic infarction (p = 4.7 x 10-⁶). In a 14-year prospective follow-up study, an SNP (1425G>A) in PRKCH affected the PKC activity and increased the risk of development of cerebral infarction (p = 0.043, relative risk 2.58). The present inventors also discovered that PKCη is expressed in the vascular endothelial cells and foamy macrophages of human atherosclerotic lesions, and that it correlates with severity of the illness. The above-mentioned results indicate that SNPs in PRKCH change the kinase activity and thereby become a novel genetic risk factor implicated in cerebral infarction.

This time, the present inventors performed a large-scale gene-based case control study using 1,112 cerebral infarction cases and the same number of age- and sex-matched control cases, and discovered that PRKCH is a gene implicated in cerebral infarction. The SNP of 1425G>A in exon 9 of PRKCH was highly relevant to the lacunar infarction group and to the group in which the lacunar infarction group and the atherothrombotic infarction group were combined. PKCη was expressed in atherosclerotic lesions and the expression level increased with the severity of atherosclerosis. Functional analysis revealed that this amino acid substitution (V374I) induces the PKC activity to 1.6-times of the original level. Furthermore, in the prospective follow-up study, the incidence of cerebral infarction was 2.58 times higher in subjects who have the AA genotype than in subjects who have the GA genotype or GG genotype. These results showed that PRKCH is a gene implication in cerebral infarction and the polymorphic mutation (SNP) of 1425G>A in PRKCH, in particular, is a site responsible for it.

Furthermore, the present inventors newly discovered that SNPs in the AGTRL1 gene are implicated in cerebral infarction. It has been shown that the APJ receptor (a receptor protein that is homologous to the angiotensin type 1 receptor) encoded by the AGTRL1 gene is expressed in the cardiovascular system (Non-Patent Document 22) and in the central nervous system (Non-Patent Document 23), and it functions in regulation of blood pressure (Non-Patent Documents 24 to 27), and migration (Non-Patent Document 28) and growth (Non-Patent Document 29) of endothelial cells. Through *in vitro* functional analysis, the present inventors discovered that the Sp1 transcription factor binds to the promoter and SNP in the intron of AGTRL1 and affects the mRNA expression level. These results indicate that a haplotype of AGTRL1 is a novel genetic determinant for susceptibility to arteriosclerotic diseases such as cerebral infarction and is involved in the pathogenic mechanism of atherosclerosis.

Of the two genes discovered this time, the AGTRL1 gene encodes AGTRL1 which is a seven-transmembrane G-protein-coupled receptor, and is known to show 30% homology with Angiotensin II receptor type 1 which is deeply connected with hypertension and arteriosclerosis. Apelin has been reported as a ligand of AGTRL1, and it has also been reported to be involved in the regulation of blood pressure in experiments using rats. There are reports that in humans, the expressions of Apelin and AGTRL1 are increased in heart failure patients, and that the administration of Apelin causes constriction of veins. Therefore, based on previous reports, AGTRL1 is predicted to be involved in blood pressure regulation. However, there have been no reports on the connection between AGTRL1 and cerebral infarction in humans. On the other hand, the PRKCH gene encodes PKC-eta, which belongs to the PKC family, and has been reported to be highly expressed in mouse skin and suggested to be implicated in cell growth and apoptosis in cancer. However, its function in human is completely unknown, and the substrates of PKC-eta and signal transduction pathways have been hardly elucidated. Accordingly, the connection between PKC-eta and cerebral infarction is not suggested from the previous findings.

As described above, the present inventors successfully identified two genes implicated in arteriosclerotic diseases such as cerebral infarction, and thereby completed the present invention.

The present invention relates to genes implicated in arteriosclerotic diseases such as cerebral infarction, methods of testing for the presence or absence of risk factors for arteriosclerotic diseases by using polymorphisms of these genes, as well as methods of screening for pharmaceutical agents for treating arteriosclerotic diseases. More specifically, the present invention relates to:
[1] a method for testing whether or not a subject has a risk factor for arteriosclerotic disease, which uses the subject's AGTRL1 gene expression as an index;
[2] a method for testing whether or not a subject has a risk factor for arteriosclerotic disease, which comprises detecting DNA mutation in the subject's AGTRL1 gene;
[3] the method of [2], wherein said mutation changes the binding with an Sp1 transcription factor;
[4] a method for testing whether or not a subject has a risk factor for arteriosclerotic disease, which uses the subject's PRKCH gene expression as an index;
[5] a method for testing whether or not a subject has a risk factor for arteriosclerotic disease, which comprises detecting DNA mutation in the subject's PRKCH gene;
[6] the method of any one of [1] to [5], wherein the mutation is a polymorphic mutation;
[7] a method for testing whether or not a subject has a risk factor for arteriosclerotic disease, wherein the method comprises determining type of nucleotide at a polymorphic site in the subject's AGTRL 1 gene;
[8] the method of [7], wherein the polymorphic site is in the AGTRL1 gene located at (1a) position 1, (2a) position 12541, (3a) position 21545, (4a) position 33051, (5a) position 35365, (6a) position 39268, (7a) position 39353, (8a) position 39370, (9a) position 39474, (10a) position 39553, (11a) position 39665, (12a) position 41786, (13a) position 42019, (14a) position 42509, (15a) position 43029, (16a) position 43406, (17a) position 43663, (18a) position 46786, (19a) position 49764, (20a) position 64276, (21a) position 74482, (22a) position 78162, (23a) position 93492, or (24a) position 102938 of the nucleotide sequence of SEQ ID NO: 1;
[9] the method of [8], wherein the subject is determined to have a risk factor for arteriosclerotic disease when the nucleotides at the polymorphic sites of (1a) to (24a) of [8] are (1b) to (24b) below, respectively:
   (1b) the type of nucleotide in the complementary strand of the AGTRL 1 gene located at position 1 of the nucleotide sequence of SEQ ID NO: 1 is T;
   (2b) the type of nucleotide in the complementary strand of the AGTRL1 gene located at position 12541 of the nucleotide sequence of SEQ ID NO: 1 is T;
   (3b) the type of nucleotide in the complementary strand of the AGTRL1 gene located at position 21545 of the nucleotide sequence of SEQ ID NO: 1 is A;
   (4b) the type of nucleotide in the complementary strand of the AGTRL1 gene located at position 33051 of the nucleotide sequence of SEQ ID NO: 1 is C;
   (5b) the type of nucleotide in the complementary strand of the AGTRL1 gene located at position 35365 of the nucleotide sequence of SEQ ID NO: 1 is T;
   (6b) the type of nucleotide in the complementary strand of the AGTRL1 gene located at position 39268 of the nucleotide sequence of SEQ ID NO: 1 is A;
   (7b) the type of nucleotide in the complementary strand of the AGTRL1 gene located at position 39353 of the nucleotide sequence of SEQ ID NO: 1 is G;
   (8b) the type of nucleotide in the complementary strand of the AGTRL1 gene located at position 39370 of the nucleotide sequence of SEQ ID NO: 1 is C;
   (9b) the type of nucleotide in the complementary strand of the AGTRL 1 gene located at position 39474 of the nucleotide sequence of SEQ ID NO: 1 is T;
   (10b) the type of nucleotide in the complementary strand of the AGTRL1 gene located at position 39553 of the nucleotide sequence of SEQ ID NO: 1 is T;
   (11b) the nucleotide in the AGTRL1 gene located at position 39665 of the nucleotide sequence of SEQ ID NO: 1 has been deleted;
   (12b) the type of nucleotide in the complementary strand of the AGTRL1 gene located at position 41786 of the nucleotide sequence of SEQ ID NO: 1 is A;
   (13b) the type of nucleotide in the complementary strand of the AGTRL1 gene located at position 42019 of the nucleotide sequence of SEQ ID NO: 1 is G;
   (14b) the type of nucleotide in the complementary strand of the AGTRL1 gene located at position 42509 of the nucleotide sequence of SEQ ID NO: 1 is G;
   (15b) the type of nucleotide in the complementary strand of the AGTRL 1 gene located at position 43029 of the nucleotide sequence of SEQ ID NO: 1 is G;
   (16b) the type of nucleotide in the complementary strand of the AGTRL1 gene located at position 43406 of the nucleotide sequence of SEQ ID NO: 1 is C;
   (17b) the type of nucleotide in the complementary strand of the AGTRL1 gene located at position 43663 of the nucleotide sequence of SEQ ID NO: 1 is T;
   (18b) the type of nucleotide in the complementary strand of the AGTRL1 gene located at position 46786 of the nucleotide sequence of SEQ ID NO: 1 is C;
   (19b) the type of nucleotide in the complementary strand of the AGTRL1 gene located at position 49764 of the nucleotide sequence of SEQ ID NO: 1 is T;
   (20b) the type of nucleotide in the complementary strand of the AGTRL1 gene located at position 64276 of the nucleotide sequence of SEQ ID NO: 1 is T;
   (21 b) the type of nucleotide in the complementary strand of the AGTRL gene located at position 74482 of the nucleotide sequence of SEQ ID NO: 1 is C;
   (22b) the type of nucleotide in the complementary strand of the AGTRL1 gene located at position 78162 of the nucleotide sequence of SEQ ID NO: 1 is G;
   (23b) the type of nucleotide in the complementary strand of the AGTRL1 gene located at position 93492 of the nucleotide sequence of SEQ ID NO: 1 is G; and
   (24b) the type of nucleotide in the complementary strand of the AGTRL1 gene located at position 102938 of the nucleotide sequence of SEQ ID NO: 1 is C;
[10] a method for testing whether or not a subject has a risk factor for arteriosclerotic disease, wherein the subject is determined to have a risk factor for arteriosclerotic disease when a DNA block showing the following haplotype is detected:
   (A) a haplotype in which the nucleotides in the complementary strand of the AGTRL1 gene at polymorphic sites located at positions 39268, 39353, 41786, 42019, and 43406 of the nucleotide sequence of SEQ ID NO: 1 are A, G, A, G, and C, respectively;
[11] a method for testing whether or not a subject has a risk factor for arteriosclerotic disease, which comprises the step of determining the type of nucleotide of a linked polymorphic site present within a DNA block showing the following haplotype:
   (A) a haplotype in which the nucleotides of the complementary strand at polymorphic sites on the AGTRL1 gene located at positions 39268, 39353, 41786, 42019, and 43406 of the nucleotide sequence of SEQ ID NO: 1 are A, Cm A, G, and C, respectively;
[12] a method for testing whether or not a subject has a risk factor for arteriosclerotic disease, which comprises the steps of:
   (a) determining the type of nucleotide at a polymorphic site in the AGTRL1 gene of the subject; and
   (b) determining that the subject has a risk factor for arteriosclerotic disease when the nucleotide determined in (a) is the same as the nucleotide at said polymorphic site in the AGTRL1 gene showing the haplotype of (A):
      (A) a haplotype in which the nucleotides of the complementary strand at polymorphic sites in the AGTRL1 gene located at positions 39268, 39353, 41786, 42019, and 43406 of the nucleotide sequence of SEQ ID NO: 1 are A, G, A, G, and C, respectively;
[13] the method of [12], wherein said polymorphic site of (a) is in the AGTRL1 gene located at any one of positions 1, 12541, 21545, 33051, 35365, 39268, 39353, 39370, 39474, 39553, 39665, 41786, 42019, 42509, 43029, 43406, 43663, 46786, 49764, 64276, 74482, 78162, 93492, or 102938 of the nucleotide sequence of SEQ ID NO: 1;
[14] a method for testing whether or not a subject has a risk factor for arteriosclerotic disease, wherein the subject is determined to have a risk factor for arteriosclerotic disease when the expression level of the subject's AGTRL1 gene is elevated compared to that of a control;
[15] a method for testing whether or not a subject has a risk factor for arteriosclerotic disease, wherein the method comprises determining the type of nucleotide at a polymorphic site in the subject's PRKCH gene;
[16] the method of [15], wherein the polymorphic site is in the PRKCH gene located at (1a) position 1, (2a) position 16212, (3a) position 30981, (4a) position 32408, (5a) position 33463, (6a) position 34446, (7a) position 39322, (8a) position 39469, (9a) position 39471, (10a) position 49248, (11a) position 49367, or (12a) position 52030 of the nucleotide sequence of SEQ ID NO: 2;
[17] the method of [16], wherein the subject is determined to have a risk factor for arteriosclerotic disease when the nucleotides in the polymorphic sites of (1a) to (12a) of [16] are the following (1b) to (12b), respectively:
   (1b) the nucleotide in the PRKCH gene located at position 1 of the nucleotide sequence of SEQ ID NO: 2 is A;
   (2b) the nucleotide in the PRKCH gene located at position 16212 of the nucleotide sequence of SEQ ID NO: 2 is G;
   (3b) the nucleotide in the PRKCH gene located at position 30981 of the nucleotide sequence of SEQ ID NO: 2 is A;
   (4b) the nucleotide in the PRKCH gene located at position 32408 of the nucleotide sequence of SEQ ID NO: 2 is G;
   (5b) the nucleotide in the PRKCH gene located at position 33463 of the nucleotide sequence of SEQ ID NO: 2 is G;
   (6b) the nucleotide in the PRKCH gene located at position 34446 of the nucleotide sequence of SEQ ID NO: 2 is T;
   (7b) the nucleotide in the PRKCH gene located at position 39322 of the nucleotide sequence of SEQ ID NO: 2 is T;
   (8b) the nucleotide in the PRKCH gene located at position 39469 of the nucleotide sequence of SEQ ID NO: 2 is A;
   (9b) the nucleotide in the PRKCH gene located at position 39471 of the nucleotide sequence of SEQ ID NO: 2 is C;
   (10b) the nucleotide in the PRKCH gene located at position 49248 of the nucleotide sequence of SEQ ID NO: 2 is C;
   (11b) the nucleotide in the PRKCH gene located at position 49367 of the nucleotide sequence of SEQ ID NO: 2 is G; and
   (12b) the nucleotide in the PRKCH gene located at position 52030 of the nucleotide sequence of SEQ ID NO: 2 is A;
[18] a method for testing whether or not a subject has a risk factor for arteriosclerotic disease, wherein the subject is determined to have a risk factor for arteriosclerotic disease when the autophosphorylation activity or kinase activity of the subject's PRKCH protein is elevated compared to that of a control;
[19] a method for testing whether or not a subject has a risk factor for arteriosclerotic disease, wherein the subject is determined to have a risk factor for arteriosclerotic disease when the subject carries a mutant protein in which valine at position 374 in the amino acid sequence of the PRKCH protein is substituted with isoleucine;
[20] the method of any one of [1] to [19], wherein a biological sample derived from the subject is subjected to the test as a test sample;
[21] a reagent for testing for the presence or absence of a risk factor for arteriosclerotic disease, which comprises an oligonucleotide that hybridizes with a DNA comprising the polymorphic sites of (1a) to (24a) of [8] or (1a) to (12a) of [16] and has a length of at least 15 nucleotides;
[22] a reagent for testing for the presence or absence of a risk factor for arteriosclerotic disease, which comprises a solid phase to which a nucleotide probe is immobilized, wherein the nucleotide probe hybridizes with a DNA comprising the polymorphic sites of (1a) to (24a) of [8] or (1a) to (12a) of [16];
[23] a reagent for testing for the presence or absence of a risk factor for arteriosclerotic disease, which comprises a primer oligonucleotide for amplifying a DNA comprising the polymorphic sites of (1a) to (24a) of [8] or (1a) to (12a) of [16];
[24] a reagent for testing for the presence or absence of a risk factor for arteriosclerotic disease, which comprises (a) or (b) as an active ingredient:
   (a) an oligonucleotide that hybridizes with a transcript of an AGTRL1 or PRKCH gene; and
   (b) an antibody that recognizes an AGTRL1 or PRKCH protein;
[25] a reagent for screening for a pharmaceutical agent for treating or preventing arteriosclerotic disease, which comprises any one of (a) to (c) as an active ingredient:
   (a) an oligonucleotide that hybridizes with a transcript of an AGTRL1 gene;
   (b) an antibody that recognizes an AGTRL1 protein; and
   (c) a polynucleotide comprising a DNA region which comprises a nucleotide site in a AGTRL1 gene located at position 39353 or 42509 of the nucleotide sequence of SEQ ID NO: 1;
[26] a reagent for screening for a pharmaceutical agent for treating or preventing arteriosclerotic disease, which comprises any one of (a) to (c) as an active ingredient:
   (a) an oligonucleotide that hybridizes with a transcript of a PRKCH gene;
   (b) an antibody that recognizes a PRKCH protein; and
   (c) a mutant PRKCH protein which has an amino acid sequence in which valine at position 374 of the amino acid sequence of a PRKCH protein is substituted with isoleucine;
[27] a pharmaceutical agent for treating or preventing arteriosclerotic disease, which comprises as an active ingredient a substance that suppresses the expression of an AGTRL1 or PRKCH gene or suppresses the function of a protein encoded by said gene;
[28] the pharmaceutical agent of [27], wherein the substance that suppresses the expression of the AGTRL1 or PRKCH gene is a compound selected from the group consisting of (a) to (c):
   (a) an antisense nucleic acid against a transcript of the AGTRL1 or PRKCH gene or a portion thereof;
   (b) a nucleic acid having a ribozyme activity of specifically cleaving a transcript of the AGTRL1 or PRKCH gene; and
   (c) a nucleic acid having an effect of inhibiting the expression of the AGTRL1 or PRKCH gene through an RNAi effect;
[29] the pharmaceutical agent of [27], wherein the substance that suppresses the function of the AGTRL1 or PRKCH protein is the compound of (a) or (b):
   (a) an antibody that binds to an AGTRL1 or PRKCH protein; or
   (b) a low-molecular-weight compound that binds to an AGTRL1 or PRKCH protein;
[30] a pharmaceutical agent for treating or preventing arteriosclerotic disease, which comprises as an active ingredient a substance that inhibits the binding of an Sp1 transcription factor with a DNA region that comprises a nucleotide site in the AGTRL1 gene located at position 39353 or 42509 of the nucleotide sequence of SEQ ID NO: 1;
[31] a pharmaceutical agent for treating or preventing arteriosclerotic disease, which comprises as an active ingredient a substance that inhibits the autophosphorylation activity of a PRKCH protein;
[32] a method of screening for a pharmaceutical agent for treating or preventing arteriosclerotic disease, which comprises selecting a compound that reduces the expression level of an AGTRL1 or PRKCH gene or reduces the activity of a protein encoded by said gene;
[33] a method of screening for a pharmaceutical agent for treating or preventing arteriosclerotic disease, which comprises the steps of:
   (a) contacting a test compound with a cell that expresses an AGTRL 1 or PRKCH gene;
   (b) measuring the expression level of said AGTRL1 or PRKCH gene; and
   (c) selecting the compound that reduces the expression level as compared with that measured in the absence of the test compound;
[34] a method of screening for a pharmaceutical agent for treating or preventing arteriosclerotic disease, which comprises the steps of:
   (a) contacting a test compound with a cell or cell extract that comprises a DNA having a structure in which a transcriptional regulatory region of an AGTRL1 or PRKCH gene and a reporter gene are operably linked with each other;
   (b) measuring the expression level of said reporter gene; and
   (c) selecting a compound that reduces said expression level as compared with that measured in the absence of the test compound;
[35] the method of any one of [32] to [34], wherein the AGTRL1 gene is a mutant AGTRL1 gene of (a) or (b) whose expression is enhanced:
   (a) a mutant AGTRL1 gene in which the nucleotide in the complementary strand of the AGTRL1 gene located at position 42509 of the nucleotide sequence of SEQ ID NO: 1 is G; or
   (b) a mutant AGTRL1 gene in which the nucleotide in the complementary strand of the AGTRL1 gene located at position 39353 of the nucleotide sequence of SEQ ID NO: 1 is G;
[36] a method of screening for a pharmaceutical agent for treating or preventing arteriosclerotic disease, which comprises the steps of:
   (a) contacting a test compound with an Sp1 transcription factor and a polynucleotide comprising a DNA region that comprises a nucleotide site in an AGTRL1 gene located at position 39353 or 42509 of the nucleotide sequence of SEQ ID NO: 1;
   (b) measuring the binding activity between said polynucleotide and the Sp1 transcription factor; and
   (c) selecting a compound that reduces said binding activity as compared with that measured in the absence of the test compound;
[37] a method of screening for a pharmaceutical agent for treating or preventing arteriosclerotic disease, which comprises the steps of:
   (a) contacting a test compound with a PRKCH protein;
   (b) measuring the autophosphorylation activity of the PRKCH protein; and
   (c) selecting a compound that reduces the autophosphorylation activity as compared with that measured in the absence of the test compound;
[38] the method of [37], wherein said PRKCH protein is a mutant protein in which valine of position 374 in the amino acid sequence of the PRKCH protein is substituted with isoleucine;
[39] a method of screening for a pharmaceutical agent for treating or preventing arteriosclerotic disease, which comprises the steps of:
   (a) contacting a test compound with a PRKCH protein;
   (b) measuring the protein kinase activity of the PRKCH protein; and
   (c) selecting a compound that reduces the protein kinase activity as compared with that measured in the absence of the test compound;
[40] Use of any one of the substances of (a) to (e) in the preparation of a reagent for testing for the presence or absence of a risk factor for arteriosclerotic disease:
   (a) an oligonucleotide that hybridizes with a DNA comprising the polymorphic sites of (1a) to (24a) of [8] or (1a) to (12a) of [16], and has a length of at least 15 nucleotides;
   (b) a solid phase to which a nucleotide probe that hybridizes with a DNA comprising the polymorphic sites of (1a) to (24a) of [8] or (1a) to (12a) of [16] is immobilized;
   (c) a primer oligonucleotide for amplifying a DNA comprising the polymorphic sites of (1a) to (24a) of [8] or (1a) to (12a) of [16];
   (d) an oligonucleotide that hybridizes with a transcript of an AGTRL1 or PRKCH gene;
   (e) an antibody that recognizes an AGTRL1 or PRKCH protein;
[41] Use of any one of the substances of (a) to (f) in the preparation of a reagent for screening for a pharmaceutical agent for treating or preventing arteriosclerotic disease:
   (a) an oligonucleotide that hybridizes with a transcript of an AGTRL1 gene;
   (b) an antibody that recognizes an AGTRL1 protein;
   (c) a polynucleotide comprising a DNA region that comprises a nucleotide site in an AGTRL1 gene located at position 39353 or 42509 in the nucleotide sequence of SEQ ID NO: 1;
   (d) an oligonucleotide that hybridizes with a transcript of a PRKCH gene;
   (e) an antibody that recognizes a PRKCH protein;
   (f) a PRKCH protein mutant that comprises an amino acid sequence in which valine of position 374 in the amino acid sequence of a PRKCH protein is substituted with isoleucine;
[42] Use of a substance that suppresses the expression of an AGTRL1 or PRKCH gene or suppresses the function of a protein encoded by said gene, in the preparation of a pharmaceutical agent for treating or preventing arteriosclerotic disease;
[43] Use of a substance of (a) or (b) in the preparation of a pharmaceutical agent for treating or preventing arteriosclerotic disease:
   (a) a substance that inhibits the binding of an Sp1 transcription factor with a DNA region that comprises a nucleotide site in an AGTRL1 gene located at position 39353 or 42509 in the nucleotide sequence of SEQ ID NO: 1;
   (b) a substance that inhibits the autophosphorylation activity of a PRKCH protein; and
[44] A method of treating or preventing arteriosclerotic disease, comprising the step of administering an agent of the present invention to a subject (human, non-human mammal, or such; preferably a patient with arteriosclerotic disease).

### Brief Description of the Drawing

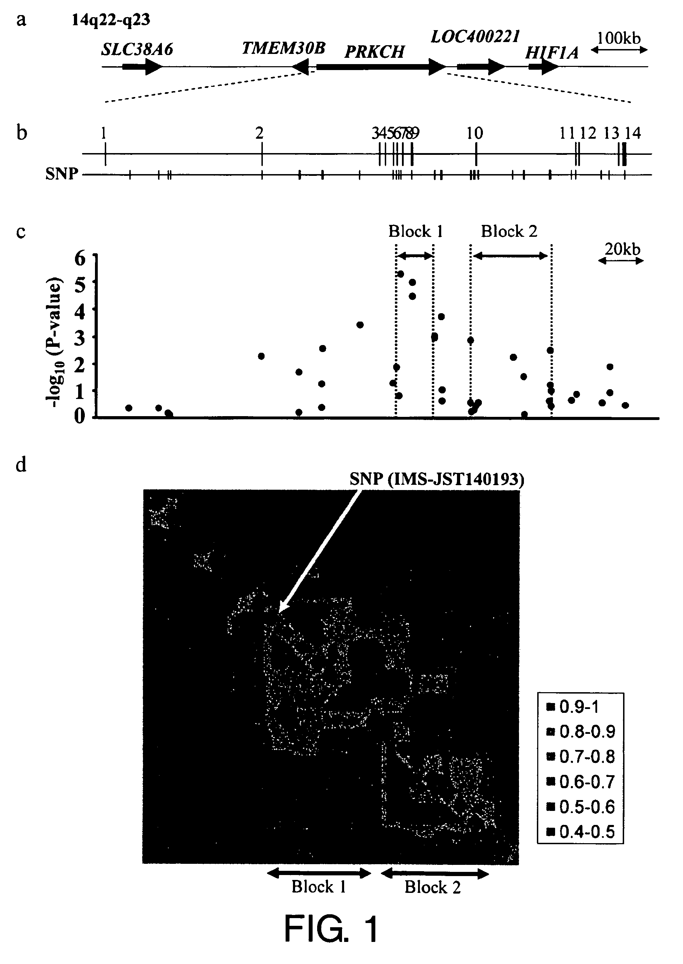

Fig. 1 depicts the genetic structure in PRKCH, case-control association, and linkage disequilibrium. a. Genome structure around PRKCH. b. Exon-intron structure of PRKCH. Genotyped SNPs in PRKCH are indicated below the gene (vertical lines). c. Case-control association analysis of lacunar infarction and atherothrombotic infarction. The log-transformed P values for allelic frequency are plotted on the y axis. d. Pair-wise linkage disequilibrium between SNPs measured by D' (lower left) and Δ (upper right).

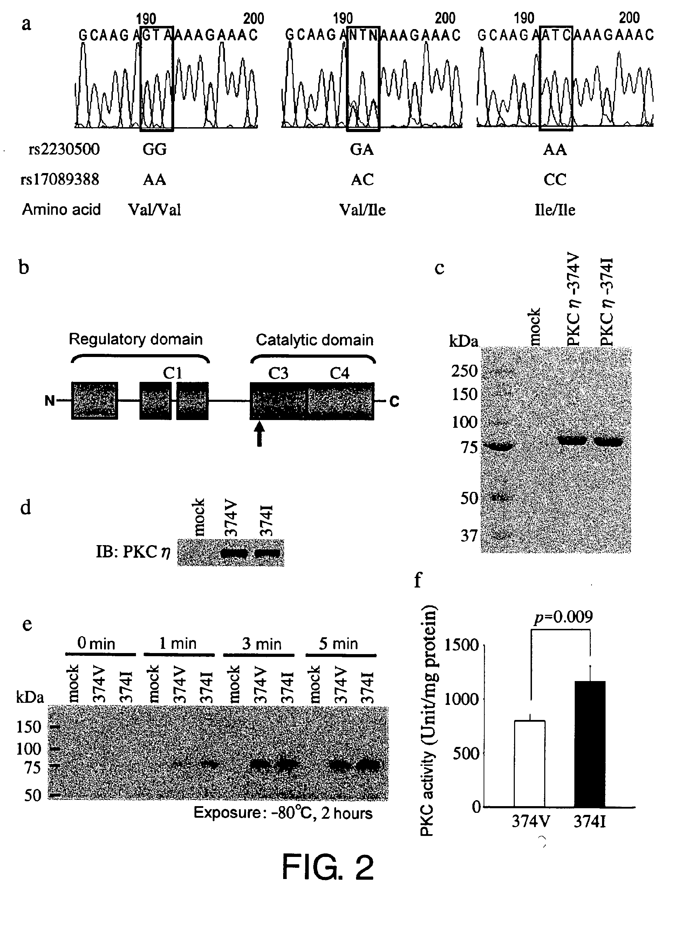

Fig. 2 presents photographs and diagrams showing a comparison of the PKC activities of 374V and 374I. a. Sequencing results of rs2230500 (1425G>A) and rs17098388 (1427A>C) are shown. b. Domain structure of PRKCH. The arrow indicates the position of rs2230500. c. A photograph showing the immunoprecipitates of mock, PRKCH-374V, and PRKCH-374I stained with Coomassie Brilliant Blue. d. A photograph showing the result of Western blotting using equal amounts of immunoprecipitates of mock, PRKCH-374V and PRKCH-3741. e. A photograph showing an autophosphorylation assay of mock, PRKCH-374V and PRKCH-374I after stimulation with 10 nM PS and 100 µM PDBu. f. The PKC activities of PRKCH-374V and PRKCH-374I after three-minute stimulation with 10 nM PS and 100 µM PDBu are shown.

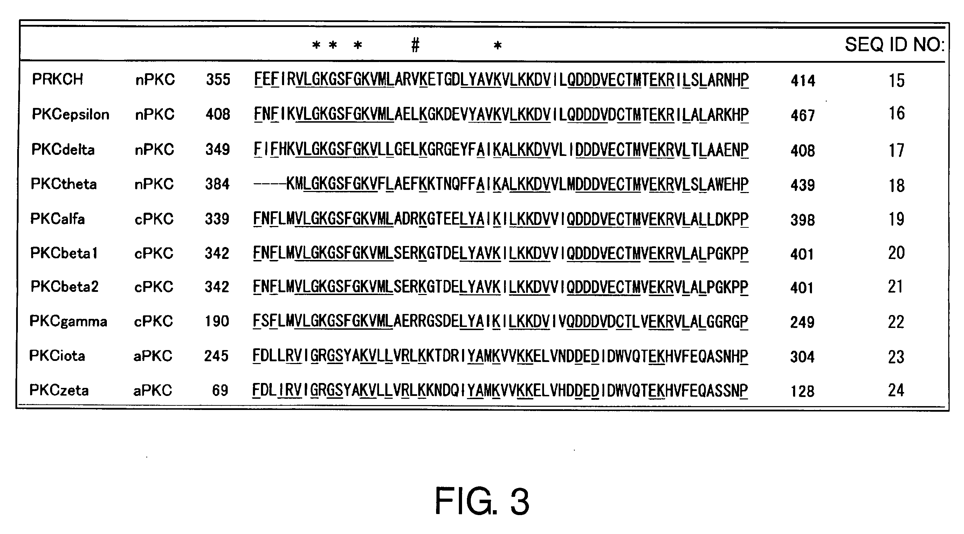

Fig. 3 shows the positions of amino acid substitutions of PRKCH. The amino acid substitution V374I of PRKCH is at a position inside the conserved ATP binding site in the PKC family. The asterisks indicate the conserved ATP binding site, and # indicates the V374I amino acid substitution.

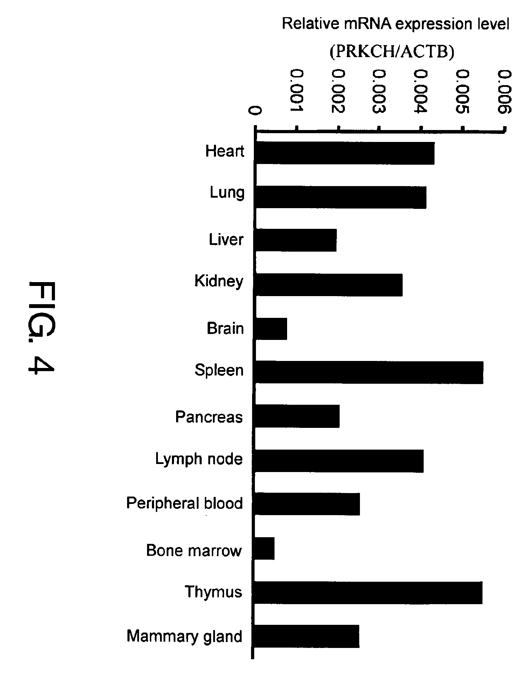

Fig. 4 shows the relative expression levels of PRKCH mRNA in various human tissues. The relative mRNA expression levels were quantified by real-time PCR and standardized by ACTB expression.

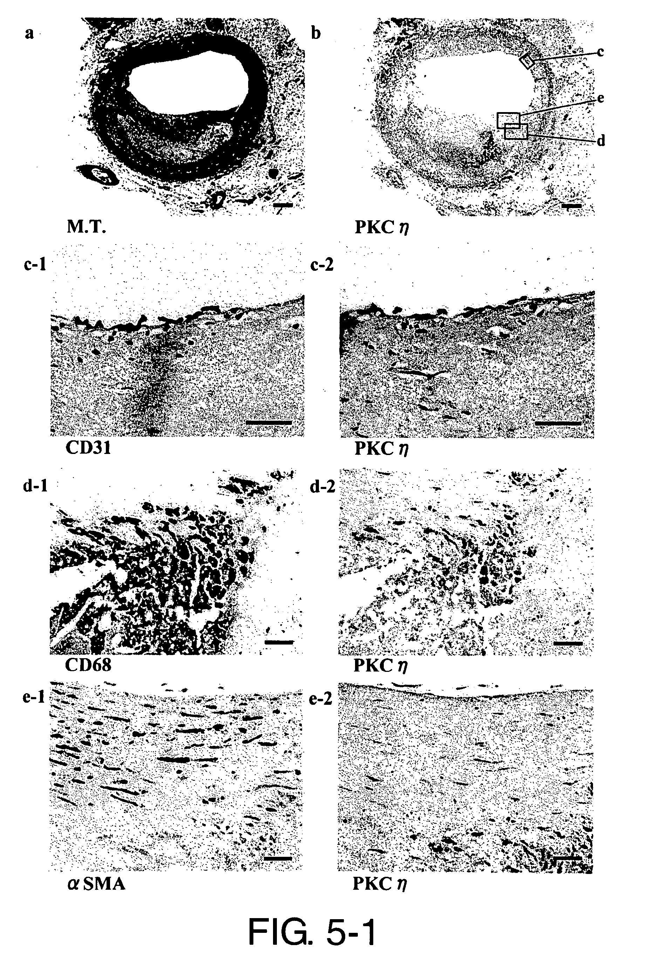

Fig. 5-1 presents photographs showing the expression of PKCη in atherosclerotic arteries. a. Low-magnification image (Masson's trichrome staining) of a case with coronary atherosclerosis (AHA type IV lesion). b. Low-magnification image of PKCη expression. c. c-1 and c-2 show lesions from serial sections of the boxed region in panel b. Each of the serial panels showed immunoreactivity towards CD31 (c-1) and PKCη (c-2). CD31-positive endothelial cells were also positive towards PKCη. d. d-1 and d-2 are lesions from serial sections of the boxed region in panel b. Each of the serial panels showed immunoreactivity towards CD68 (d-1) and PKCη (d-2). Most of the CD68-positive macrophages were also positive towards PKCη. e. e-1 and e-2 are lesions from serial sections of the boxed region in panel b. Each of the serial panels showed immunoreactivity towards α-SMA (e-1) and PKCη (e-2). A portion of the α-SMA-positive smooth muscle cells were also positive towards PKCη. The sections in panels b to e have been counterstained with hematoxylin. Scale bar: a and b = 500 µm, and c to e = 50 µm.

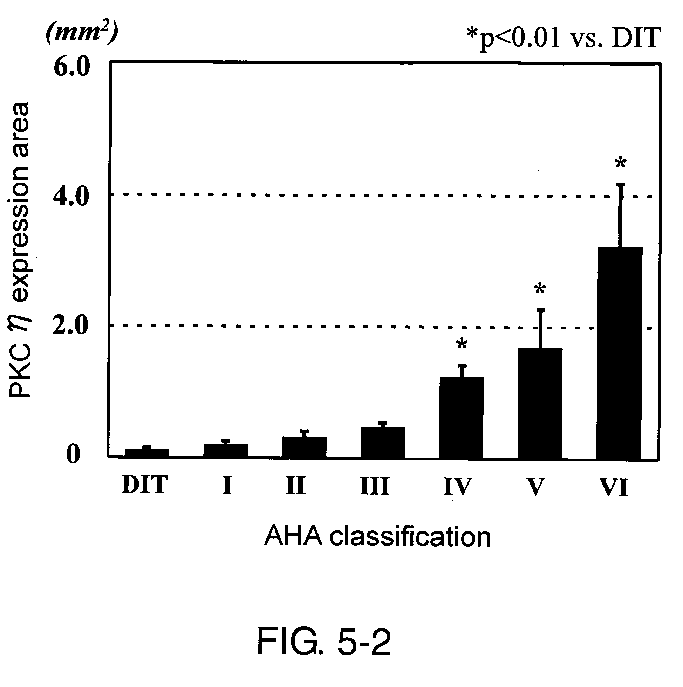

Fig. 5-2 presents a bar graph showing the relationship between the grade of atherosclerosis as defined by the AHA classification and the PKCη expression in the atherosclerotic intima. Each bar represents the mean ± s.e.m. of the positive area in all sections examined in each group. PKCη expression increased linearly with the severity of coronary atherosclerosis.

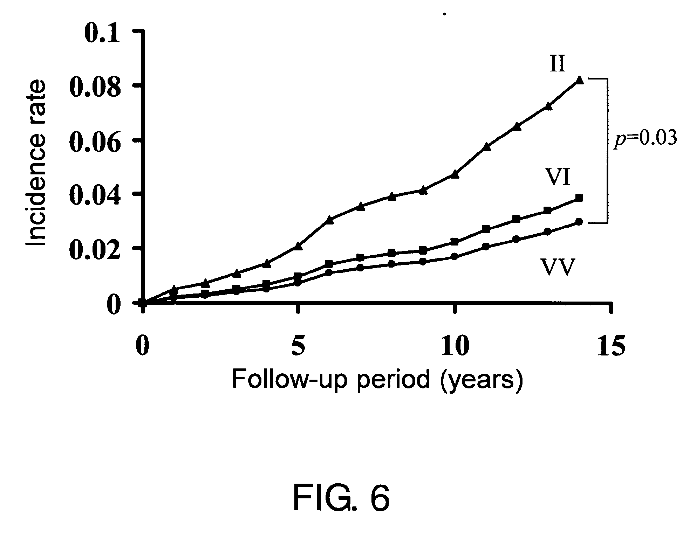

Fig. 6 shows the age- and sex-adjusted incidence rates of cerebral infarction observed according to PKCη genotypes (1425G>A corresponds to the amino acid substitution V374I) during a 14-year follow-up period in the Hisayama study.

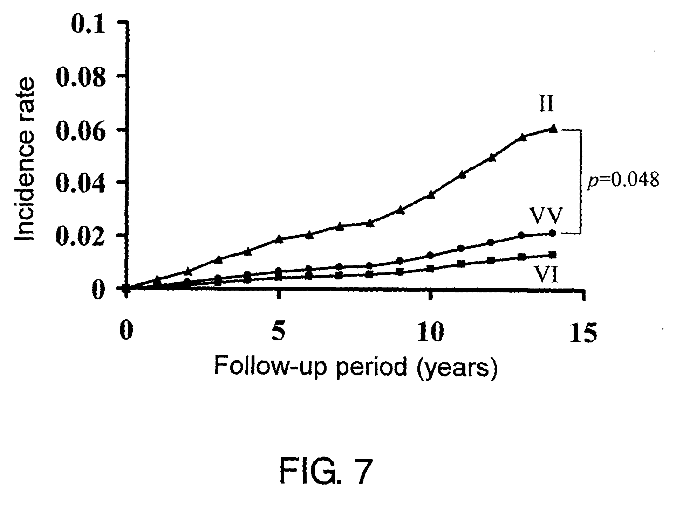

Fig. 7 shows the age- and sex-adjusted incidence rates of coronary artery diseases observed according to PKCη genotypes (1425G>A corresponds to the amino acid substitution V374I) during a 14-year follow-up period in the Hisayama study.

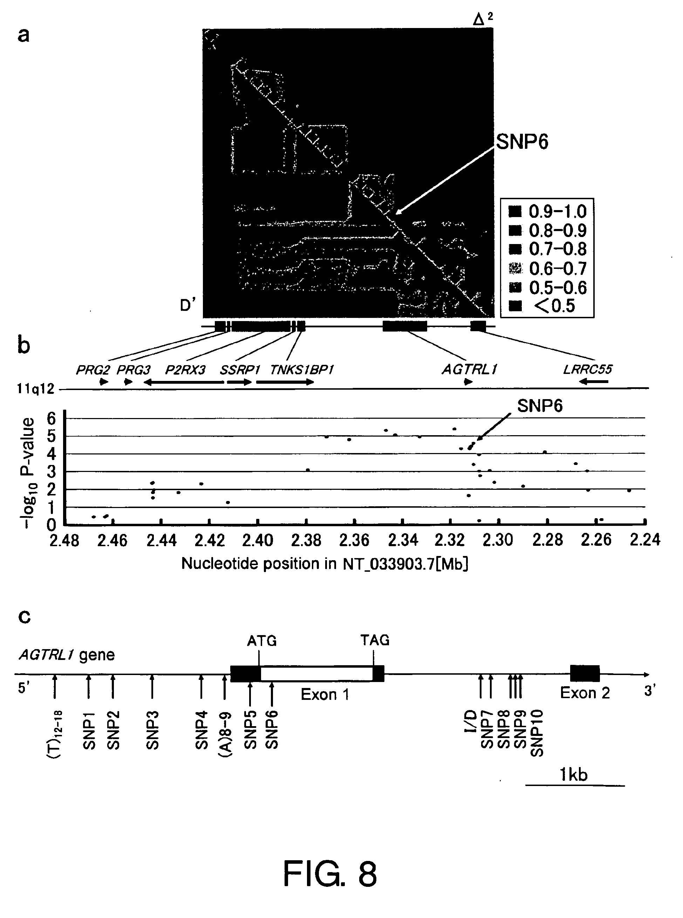

Fig. 8 shows the correlation analysis around the AGTRL1 gene. a. Pair-wise linkage disequilibrium (LD) map between SNPs around the AGTRL1 gene evaluated by D' (lower left triangle) and Δ2 (upper right triangle) in each case group. SNP6 (arrow) is a marker SNP detected in the second screening. b. Case-control plot [-log₁₀ (P value)] around the AGTRL1 gene. 860 test cases with LA and AT were compared with 860 control subject cases. SNP6 (arrow) is a marker SNP detected in the second screening. c. Gene structure and polymorphisms in the AGTRL1 gene. The exact positions of SNPs are shown in Tables 1-1 to 1-6. ATG: start codon; TAG: stop codon; I/D: insertion/deletion polymorphism.

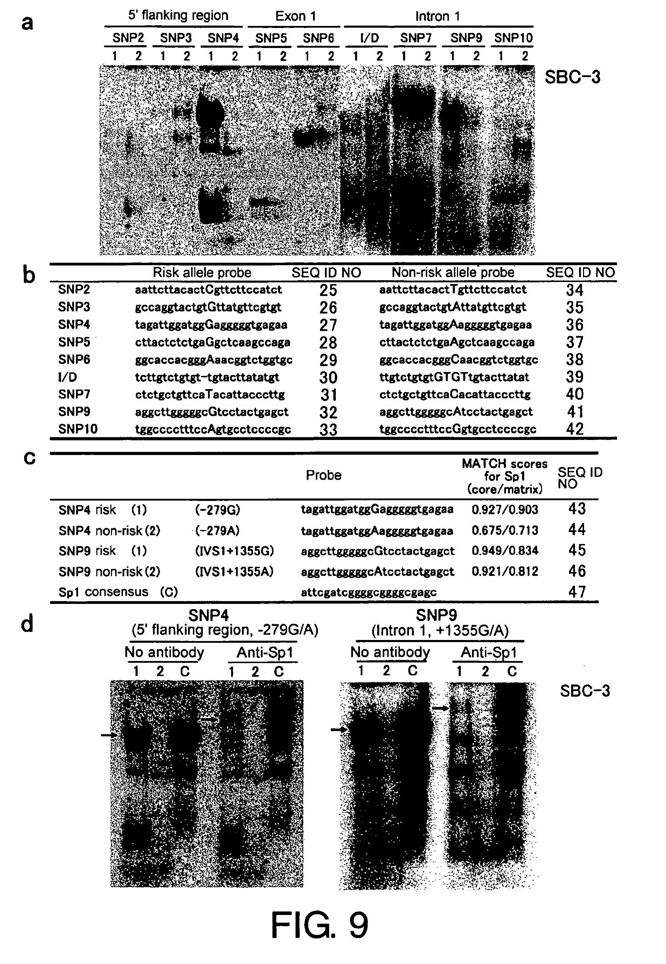

Fig. 9 presents photographs and diagrams showing the results of EMSA with AGTRL1 polymorphisms. a. EMSA using a 25-bp probe around the respective alleles of nine types of polymorphisms. Nuclear extract from SBC-3 cells was used. b. Sequences of the probes used for EMSA. Capital letters indicate the polymorphisms. 1: risk allele; 2: non-risk allele. c. Binding affinity predicted by the MATCH program between the DNA sequences around the respective alleles of SNP4 and SNP9, and Sp1. Capital letters in the sequences indicate the polymorphisms. d. Super shift assay of the SNP4 region and SNP9 region using an Sp1 antibody. Nuclear extract of SBC-3 was used. The arrows indicate the bands of the probe-Sp1 complex and supershifted bands. 1: risk allele; 2: non-risk allele; C: Sp1 consensus oligonucleotide (positive control bound to Sp1).

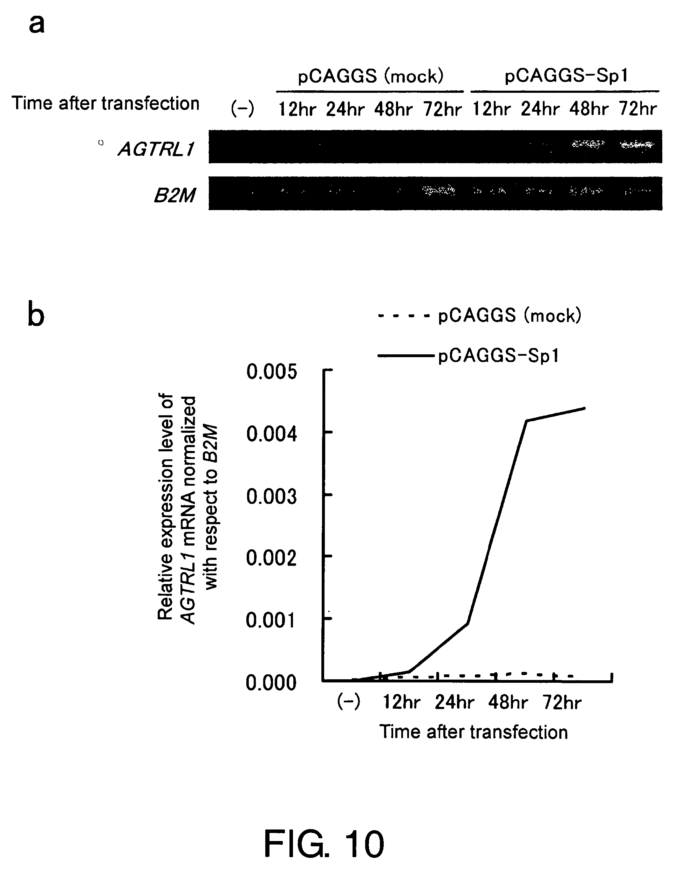

Fig. 10 presents photographs and diagram showing the results of RT-PCR with AGTRL1 mRNA in SP1-overexpressing cells. Mock pCAGGS vector or pCAGGS-Sp1 vector was transfected into 293T cells. The AGTRL1 mRNA increased in a time-dependent manner due to Sp1 overexpression. B2M was used as an internal control. a. Semi-quantitative RT-PCR. b. Quantitative real time RT-PCR.

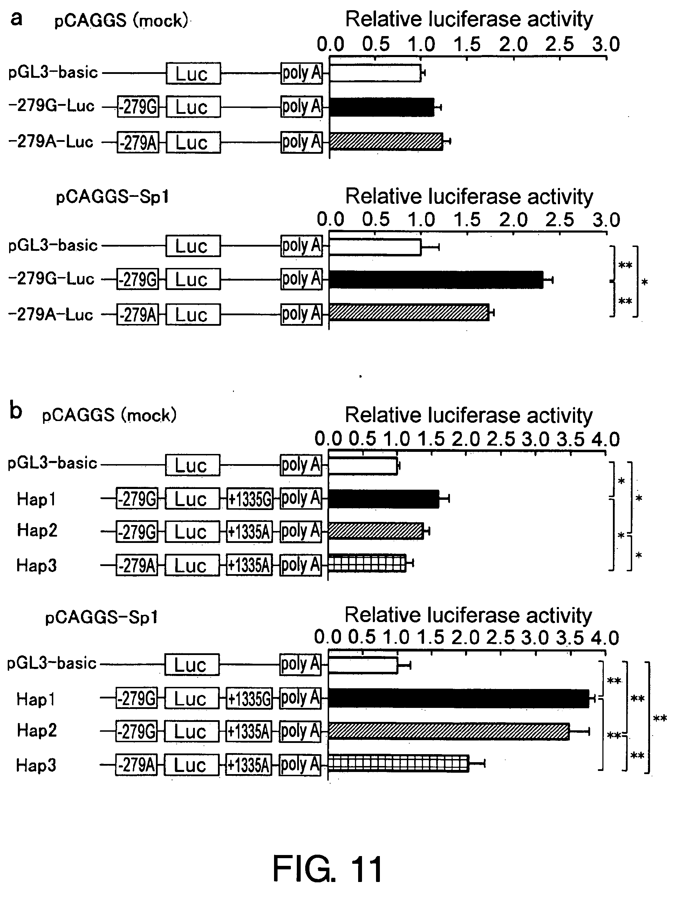

Fig. 11 shows the result of transcriptional regulatory activity affected by SNPs. a. A 44-bp fragment around each allele of SNP4 (-279G/A) in the 5' flanking region is inserted into the pGL3-basic vector. -279G and -279A represent a risk allele and non-risk allele of SNP4, respectively. b. A 44-bp fragment around SNP4 and a 53-bp fragment around SNP9 (+1355G/A) are inserted into pGL3-basic. Luciferase assay was performed using SBC-3 cells under conditions of cotransfection with mock pCAGGS and pCAGGS-Sp1. Hap1, Hap2, and Hap 3 represent a risk haplotype (-279G and +1355G), non-risk haplotype (-279G and +1355A), and intermediate haplotype (-279G and +1355G), respectively. The data shown is mean ± s.d. (n=3,^{*}P<0.05,^{**}P<0.01). Each sample was tested three times.

### Best Mode for Carrying Out the Invention

The present inventors identified the AGTRL1 and PRKCH genes as being implicated in arteriosclerotic diseases such as cerebral infarction, and also identified polymorphic mutations (SNPs) involved in the expression or function of these genes. Enhancement of the expression of the AGTRL1 or PRKCH gene, or enhancement of the function of a protein encoded by the gene was found to be deeply associated with the onset of arteriosclerotic diseases. A subject with elevated expression of the AGTRL1 or PRKCH gene can be determined to have a risk factor for arteriosclerotic diseases (a constitution that is prone to arteriosclerotic diseases).

In the present invention, the term "arteriosclerotic disease" normally refers to a disease caused by arteriosclerosis. Specific examples include cerebral infarction (including, for example, lacunar infarction and atherothrombotic infarction), myocardial infarction, arteriosclerosis (including, for example, atherosclerosis), arteriosclerosis obliterans, aortic aneurysm, and renal artery stenosis. Furthermore, lacunar infarction mentioned above is a disease caused by arteriolosclerosis, and cerebral vascular dementia (Binswanger's disease in particular), asymptomatic cerebral infarction, micromyocaridal infarction, and such are, for example, also included in the arteriosclerotic diseases of the present invention.

The present inventors were the first to discover that the presence or absence of a risk factor for arteriosclerosis can be examined using polymorphic mutations, expression, or such of the AGTRL1 or PRKCH gene as an index.

The present invention initially provides methods of testing whether or not a subject has a risk factor for arteriosclerotic diseases, wherein the expression of AGTRL1 or PRKCH gene in the subject (a biological sample derived from the subject) is used as an index.

Accordingly, it is possible to determine whether or not the subject has a risk factor for arteriosclerotic diseases by using, as an index, the expression of AGTRL1 or PRKCH gene or the activity (function) of the protein encoded by the gene.

Information on the nucleotide sequence of the AGTRL1 or PRKCH gene in the present invention and the amino acid sequence of the protein encoded by the gene is easily accessible through the above-mentioned GenBank Accession Number. One skilled in the art can readily obtain information on the nucleotide sequence of the gene, and the amino acid sequence of the protein encoded by the gene from a public gene database or document database based on the gene notation (gene name).

The accession numbers of public databases such as GenBank and SEQ ID NOs in the Sequence Listing for the nucleotide and amino acid sequences of the AGTRL1 gene and the PRKCH gene are shown below.
AGTRL1 mRNA: NM_005161 (RefSeq) (SEQ ID NO: 3), amino acid: NP_005152 (SEQ ID NO: 4)
PRKCH mRNA: NM_006255 (RefSeq) (SEQ ID NO: 5), amino acid: NP_006246 (SEQ ID NO: 6)

The nucleotide sequence of the genomic DNA region including the AGTRL1 gene is shown in SEQ ID NO: 1. The nucleotide sequence of the genomic DNA region comprising the PRKCH gene is shown in SEQ ID NO: 2. For both the AGTRL1 and PRKCH genes, the Sequence Listing shows plus strands.

The term "subject" as used in the present invention generally refers to a human; however, the testing method of the present invention is not necessarily limited to methods which utilize only a human subjects for examination. When a subject is a non-human organism (preferably a vertebrate, and more preferably a mammal such as a mouse, rat, monkey, dog, or cat), the subject may be tested using, as an index, the expression level of an endogenous gene of the subject organism corresponding to the AGTRL1 or PRKCH gene. Accordingly, the "AGTRL1 or PRKCH gene" in the present invention includes, for example, endogenous DNAs (e.g., AGTRL1 or PRKCH gene homologs) of other organisms, corresponding to the DNA having the nucleotide sequence of SEQ ID NO: 1 or 2.

Such endogenous DNAs of other organisms corresponding to the DNA having the nucleotide sequence of SEQ ID NO: 1 or 2 generally show a high homology to DNA of SEQ ID NO: 1 or 2. The term "high homology" means a homology of 50% or more, preferably 70% or more, more preferably 80% or more, furthermore preferably 90% or more (for example, 95% or more, particularly preferably 96%, 97%, 98%, or 99% or more). The homology can be determined using mBLAST algorithm (Altschul et al. (1990) Proc. Natl. Acad. Sci. USA 87:2264-8; Karlin and Altschul (1993) Proc. Natl. Acad. Sci. USA 90: 5873-7). When such DNAs are isolated from a living body, they are considered to hybridize with the DNA of SEQ ID NO: 1 or 2 under stringent conditions. The "stringent conditions" herein include, for example, "2x SSC, 0.1% SDS, 50°C", "2x SSC, 0.1% SDS, 42°C", and "1x SSC, 0.1% SDS, 37°C"; and more stringent conditions include "2x SSC, 0.1% SDS, 65°C", "0.5x SSC, 0.1% SDS, 42°C", and "0.2x SSC, 0.1% SDS, 65°C". One skilled in the art can suitably obtain endogenous genes in other organisms corresponding to the AGTRL1 or PRKCH gene, based on the nucleotide sequence of the AGTRL1 or PRKCH gene.

The present invention further provides methods of testing whether or not a subject has a risk factor for arteriosclerotic diseases, wherein the subject is determined to have a risk factor for arteriosclerotic diseases when the expression level of AGTRL1 or PRKCH gene in the subject is elevated as compared with a control.

In the above method, a biological sample derived from the subject is generally used as a test sample. The expression level of the AGTRL1 or PRKCH gene in the test sample can be suitably measured using procedures known to one skilled in the art.

The term "expression" in the context of the "gene" includes "transcription" from the gene and "translation" into a polypeptide.

When the expression level of the gene is measured using as an index the amount of a translated product (protein) of the gene, for example, a protein sample can be prepared from a test sample, and the amount of AGTRL1 or PRKCH in the protein sample can be measured. Examples of such procedures include those known to one skilled in the art, such as enzyme-linked immunosorbent assay (ELISA), double monoclonal antibody sandwich immunoassay, monoclonal polyclonal antibody sandwich assay, immunofluorescence, Western blotting, dot blotting, immunoprecipitation, protein chip analysis (Tanpakushitsu Kakusan Koso (Protein, Nucleic Acid and Enzyme) Vol. 47 No. 5 (2002); Tanpakushitsu Kakusan Koso (in Japanese; Protein, Nucleic Acid and Enzyme) Vol. 47 No. 8 (2002)), two-dimensional electrophoresis, and SDS-polyacrylamide electrophoresis, but are not limited thereto.

When the expression level of the gene is measured using the amount of a transcript (mRNA) of the gene as an index, for example, an RNA sample can be prepared from a test sample, and the amount of AGTRL 1- or PRKCH-encoding RNA contained in the RNA sample can be measured. In addition, the expression level can be evaluated by preparing a cDNA sample from the test sample and measuring the amount of AGTRL1- or PRKCH-encoding cDNA contained in the cDNA sample. The RNA sample and cDNA sample from the test sample can be prepared from a subject-derived biological sample using procedures known to one skilled in the art. Examples of such procedures include those known to one skilled in the art, such as Northern blotting, RT-PCR, and DNA array techniques.

The term "control" typically refers to the expression level of an AGTRL1 or PRKCH gene in a biological sample derived from a healthy individual. The term "expression" of the AGTRL1 or PRKCH gene as used in the present invention means both the expression of mRNA transcribed from the AGTRL1 or PRKCH gene, and the expression of a protein encoded by the AGTRL1 or PRKCH gene.

The present invention further provides methods of testing whether or not a subject has a risk factor for arteriosclerotic diseases, which include the step of detecting a mutation in the AGTRL1 or PRKCH gene of the subject.

In the present invention, "testing whether or not a subject has a risk factor for arteriosclerotic diseases" includes testing whether or not a subject has a non-risk factor for arteriosclerotic diseases, or testing to determine whether a subject is likely or less likely to develop arteriosclerotic diseases. In the method of the present invention, when a mutation is detected in the AGTRL1 or PRKCH gene, a subject is determined to have a risk factor for arteriosclerotic diseases, not to have a non-risk factor for arteriosclerotic diseases, or to have a constitution that is prone to arteriosclerotic diseases.

On the other hand, when no mutation is detected in the AGTRL1 or PRKCH gene, the subject is determined to have a non-risk factor for arteriosclerotic diseases, or have no risk factor for arteriosclerotic diseases.

The method of the present invention can determine whether a subject who is not affected by arteriosclerotic diseases is more or less likely to suffer from arteriosclerotic diseases.

The term "treatment" as used herein generally means achieving a pharmacological and/or physiological effect. The effect may be preventive in that a disease or symptom is fully or partially prevented, or may be therapeutic in that a disease or symptom is fully or partially treated. The "treatment" as used herein includes all the disease treatments in mammals, particularly in humans. Furthermore, the "treatment" also includes preventing the onset of a disease in a subject who has a risk factor for the disease but has not yet been diagnosed as being affected by the disease, suppressing the progression of the disease, alleviating the disease, and delaying its onset.

Patients who have been determined to have a risk factor for arteriosclerotic diseases by the present invention's method of testing for the presence of absence of a risk factor for arteriosclerotic diseases, can select appropriate treatments before onset and may be able to prevent the onset of arteriosclerotic diseases in advance.

Specifically, the DNA sequence of the AGTRL1 or PRKCH gene of the present invention comprises the sequence of SEQ ID NO: 1 or 2, respectively.

The "mutations" in the testing method of the present invention are generally present in ORF of the above-mentioned AGTRL1 or PRKCH gene, or in regions for regulating the expression of this gene (for example, promoter regions, enhancer regions, or introns), but the position is not limited thereto. Generally, the "mutation" preferably enhances the expression level of the above-mentioned gene, improves the stability of mRNA, or increases the activity of a protein encoded by the gene. Examples of types of mutations in the present invention include addition, deletion, substitution, and insertion of nucleotides.

In the AGTRL1 gene, the "mutation" preferably changes the binding activity of the Sp1 transcription factor. Preferred examples of the mutation include SNP4 polymorphic mutation (the type of nucleotide at the polymorphic site located at position 42509 of the nucleotide sequence of SEQ ID NO: 1 is C (and the type of nucleotide in its complementary strand is G)) and SNP9 polymorphic mutation (the type of nucleotide at the polymorphic site located at position 39353 of the nucleotide sequence of SEQ ID NO: 1 is C (and the nucleotide type of in its complementary strand is G)), which are indicated in the Examples described later.

Meanwhile, preferred embodiments in the PRKCH gene include a DNA mutation that changes the valine at position 374 to isoleucine in the amino acid sequence of the PRKCH protein encoded by the gene.

The present inventors successfully discovered polymorphic mutations in the AGTRL1 or PRKCH gene of subjects that are significantly associated with arteriosclerotic diseases. Accordingly, it is possible to test whether or not a subject has a risk factor for arteriosclerotic diseases by using, as an index, the presence or absence of a mutation (by determining the nucleotide type) at a polymorphic site on the AGTRL1 or PRKCH gene.

A preferred embodiment of the present invention relates to a method of testing whether or not a subject has a risk factor for arteriosclerotic diseases, including the step of detecting a polymorphic mutation in the AGTRL1 or PRKCH gene of the present invention.

The term "polymorphism" in genetics is generally defined as a variation of a certain nucleotide in one gene, where the variation occurs at a frequency of 1% or more in a population. However, the "polymorphism" in the present invention is not restricted by this definition. Examples of the polymorphism in the present invention include single nucleotide polymorphisms, and polymorphisms in which one to several tens of nucleotides (occasionally several thousands of nucleotides) are deleted or inserted. In addition, the number of polymorphic site is not limited to one, and two or more polymorphisms may be present.

The present invention further provides methods for testing whether or not a subject has a risk factor for arteriosclerotic diseases, including the step of determining the nucleotide type at a polymorphic site in the AGTRL1 or PRKCH gene of the present invention.

The "polymorphic site" in the present invention's method of testing for the presence or absence of a risk factor for arteriosclerotic diseases is not particularly limited so long as it is a polymorphism present in the AGTRL1 or PRKCH gene of the present invention, or in the region surrounding the gene. Information on polymorphic sites found in AGTRL1 is shown in Tables 1-1 to 1-6, and information on polymorphic sites found in the PRKCH gene is shown in Tables 2-1 to 2-6. In the "Strand" column of the Tables, "+" refers to plus strand and "-" refers to minus strand. In the "Type of polymorphism" column, "snp" refers to a single nucleotide polymorphic mutation, and "in-del" refers to an insertion/deletion mutation.

**Table 1-1**

| Chromosome No. | Chromosome position | Position in the Sequence Listing | SNP ID | Strand | Nucleotide | Polymorphism type |
|---|---|---|---|---|---|---|
| chr11 | 56719135 | 1 | rs717211 | - | C/T | snp |
| chr11 | 56719135 | 1 | SNP A-1682036 | + | A/G | snp |
| chr11 | 56719430 | 296 | rs717210 | - | C/T | snp |
| chr11 | 56719430 | 296 | SNP A-1713522 | + | A/G | snp |
| chr11 | 56719958 | 824 | rs10501364 | + | A/C | snp |
| chr11 | 56719958 | 824 | SNP A-1673288 | - | G/T | snp |
| chr11 | 56720066 | 932 | rs11228941 | + | C/T | snp |
| chr11 | 56720176 | 1042 | rs12271407 | + | C/T | snp |
| chr11 | 56720206 | 1072 | rs948849 | - | C/T | snp |
| chr11 | 56720800 | 1666 | rs10896581 | + | A/G | snp |
| chr11 | 56720974 | 1840 | rs10896582 | + | C/T | snp |
| chr11 | 56722230 | 3096 | rs11228944 | + | G/T | snp |
| chr11 | 56722315 | 3181 | rs948848 | - | A/G | snp |
| chr11 | 56722359 | 3225 | rs7103566 | + | A/G | snp |
| chr11 | 56722515 | 3381 | rs7103802 | + | C/G | snp |
| chr11 | 56722515 | 3381 | SNP A-1751467 | + | C/G | snp |
| chr11 | 56722550 | 3416 | ras10601365 | + | A/G | snp |
| chr11 | 56722550 | 3416 | SNP A-1673178 | + | A/G | snp |
| chr11 | 56722912 | 3778 | rs1815860 | - | C/T | snp |
| chr11 | 56723045 | 3911 | rs2511033 | + | A/C | snp |
| chr11 | 56723377 | 4243 | rs4939111 | + | C/T | snp |
| chr11 | 56723377 | 4243 | SNP A-1669987 | + | C/T | snp |
| chr11 | 56723391 | 4257 | rs10501366 | + | A/G | snp |
| chr11 | 56723799 | 4665 | rs11228949 | + | A/C | snp |
| chr11 | 56724191 | 5057 | rs12295419 | + | G/T | snp |
| chr11 | 56724313 | 5179 | rs11605468 | + | C/T | snp |
| chr11 | 56724551 | 5417 | rs2156459 | - | C/T | snp |
| chr11 | 56724582 | 5448 | rs17145552 | + | C/T | snp |
| chr11 | 56724664 | 5530 | rs1893937 | + | A/G | snp |
| chr11 | 56725136 | 6002 | rs17145556 | + | C/T | snp |
| chr11 | 56725535 | 6401 | rs7124474 | + | C/G | snp |
| chr11 | 56725697 | 6563 | rs10607820 | + | -/AGTG | in-del |
| chr11 | 56726012 | 6878 | rs11606597 | + | A/C | snp |
| chr11 | 56726031 | 6897 | rs17145573 | + | A/G | snp |
| chr11 | 56726095 | 6961 | rs7114596 | + | G/T | snp |
| chr11 | 56726630 | 7496 | rs12575327 | + | A/G | snp |
| chr11 | 56727824 | 8690 | rs7102922 | + | A/G | snp |
| chr11 | 56728174 | 9040 | rs2511028 | + | A/C | snp |
| chr11 | 56728293 | 9159 | rs11603531 | + | A/G | snp |
| chr11 | 56728574 | 9440 | rs7123500 | + | A/T | snp |
| chr11 | 56729484 | 10350 | rs12223141 | + | G/T | snp |
| chr11 | 56729633 | 10499 | rs11228950 | + | C/T | snp |
| chr11 | 56729669 | 10535 | rs4939112 | + | C/T | snp |
| chr11 | 56730369 | 11235 | rs1943477 | + | C/T | snp |
| chr11 | 56730424 | 11290 | rs1943478 | + | A/G | snp |
| chr11 | 56731675 | 12541 | rs2156456 | + | A/G | snp |
| chr11 | 56732238 | 13104 | rs5792032 | + | -/TAA | in-del |
| chr11 | 56732869 | 13735 | rs11602335 | + | C/T | snp |
| chr11 | 56732927 | 13793 | rs1943479 | + | C/T | snp |
| chr11 | 56732989 | 13855 | rs11602357 | + | C/G | snp |
| chr11 | 56733159 | 14025 | rs12285798 | + | A/T | snp |
| chr11 | 56734089 | 14955 | rs12806994 | + | A/G | snp |
| chr11 | 56734205 | 15071 | rs11228951 | + | C/G | snp |
| chr11 | 56736043 | 16909 | rs11603194 | + | A/G | snp |
| chr11 | 56737415 | 18281 | rs12225155 | + | A/G | snp |
| chr11 | 56738240 | 19106 | rs12283943 | + | A/T | snp |

Table 1-2 is the continuation of Table 1-1.

**Table 1-2**

| | | | | | | |
|---|---|---|---|---|---|---|
| chr11 | 56738608 | 19474 | rs2156457 | + | G/T | snp |
| chr11 | 56738788 | 19654 | rs2156458 | + | C/T | snp |
| chr11 | 56740224 | 21090 | rs7101929 | + | C/G | snp |
| chr11 | 56740679 | 21545 | rs10896586 | + | C/T | snp |
| chr11 | 56740910 | 21776 | rs11828733 | + | A/G | snp |
| chr11 | 56741024 | 21890 | rs12099289 | + | A/G | snp |
| chr11 | 56741471 | 22337 | rs12295604 | + | C/G | snp |
| chr11 | 56741508 | 22374 | rs12807301 | + | A/G | snp |
| chr11 | 56742126 | 22992 | rs11228952 | + | C/T | snp |
| chr11 | 56742382 | 23248 | rs10160780 | + | C/T | snp |
| chr11 | 56742431 | 23297 | rs7926117 | + | C/T | snp |
| chr11 | 56742777 | 23643 | rs10896587 | + | A/G | snp |
| chr11 | 56742828 | 23694 | rs4939113 | + | A/G | snp |
| chr11 | 56742979 | 23845 | rs12286248 | + | C/T | snp |
| chr11 | 56743060 | 23926 | rs10896588 | + | A/G | snp |
| chr11 | 56743194 | 24060 | rs11228954 | + | C/T | snp |
| chr11 | 56744253 | 25119 | rs11602646 | + | A/C | snp |
| chr11 | 56744582 | 25448 | rs11228955 | + | C/G | snp |
| chr11 | 56744597 | 25463 | rs957922 | - | A/G | snp |
| chr11 | 56744872 | 25738 | rs10792077 | + | A/G | snp |
| chr11 | 56744928 | 25794 | rs4939114 | + | A/G | snp |
| chr11 | 56745027 | 25893 | rs4442567 | + | A/G | snp |
| chr11 | 56745093 | 25959 | rs11228956 | + | C/T | snp |
| chr11 | 56745703 | 26569 | rs12290010 | + | C/T | snp |
| chr11 | 56745960 | 26826 | rs4939115 | + | C/T | snp |
| chr11 | 56746204 | 27070 | rs12288876 | + | A/G | snp |
| chr11 | 56746265 | 27131 | rs10736681 | + | C/T | snp |
| chr11 | 56747026 | 27892 | rs11228957 | + | C/G | snp |
| chr11 | 56747173 | 28039 | rs11604352 | + | C/T | snp |
| chr11 | 56747255 | 28121 | rs10605080 | + | -/GTTTGTTT | in-del |
| chr11 | 56747768 | 28634 | rs10896593 | + | C/T | snp |
| chr11 | 56748250 | 29116 | rs11228958 | + | A/G | snp |
| chr11 | 56748263 | 29129 | rs10896594 | + | A/G | snp |
| chr11 | 56748324 | 29190 | rs2510357 | + | C/T | snp |
| chr11 | 56748439 | 29305 | rs7928962 | + | C/T | snp |
| chr11 | 56748523 | 29389 | rs12802382 | + | G/T | snp |
| chr11 | 56748861 | 29727 | rs12225016 | + | C/T | snp |
| chr11 | 56748988 | 29854 | rs4939117 | + | A/G | snp |
| chr11 | 56749355 | 30221 | rs4939118 | + | A/T | snp |
| chr11 | 56749423 | 30289 | rs10652895 | + | -/AT | in-del |
| chr11 | 56749424 | 30290 | rs10652894 | + | -/TA | in-del |
| chr11 | 56750418 | 31284 | rs10431125 | + | A/T | snp |
| chr11 | 56750715 | 31581 | rs9667550 | + | C/T | snp |
| chr11 | 56751156 | 32022 | rs11422182 | + | -/A | in-del |
| chr11 | 56751611 | 32477 | rs7130787 | + | C/T | snp |
| chr11 | 56751745 | 32611 | rs17651297 | + | C/G | snp |
| chr11 | 56751841 | 32707 | rs1893676 | + | A/G | snp |
| chr11 | 56751910 | 32776 | rs1893677 | + | C/G | snp |
| chr11 | 56752185 | 33051 | rs1943482 | + | A/G | snp |
| chr11 | 56752529 | 33395 | rs10792078 | + | A/C | snp |
| chr11 | 56752655 | 33521 | rs17151761 | + | A/G | snp |
| chr11 | 56752790 | 33656 | rs7926885 | + | A/G | snp |
| chr11 | 56752915 | 33781 | rs7927969 | + | C/G | snp |
| chr11 | 56753784 | 34650 | rs4938853 | + | C/T | snp |
| chr11 | 56754010 | 34876 | rs4938854 | + | C/T | snp |
| chr11 | 56754225 | 35091 | rs7109894 | + | C/T | snp |
| chr11 | 56754408 | 35274 | rs721607 | + | C/T | snp |
| chr11 | 56754499 | 35365 | rs721608 | + | A/G | snp |
| chr11 | 56755401 | 36267 | rs17151767 | + | A/G | snp |
| chr11 | 56755459 | 36325 | rs1943483 | + | A/G | snp |

Table 1-3 is the continuation of Table 1-2.

**Table 1-3**

| | | | | | | | |
|---|---|---|---|---|---|---|---|
| | chr11 | 56755730 | 36596 | rs1943484 | + | C/T | snp |
| | chr11 | 56755753 | 36619 | rs5792033 | + | -/TCTC | in-del |
| | chr11 | 56755815 | 36681 | rs11228960 | + | C/T | snp |
| | chr11 | 56755816 | 36682 | rs11228961 | + | C/T | snp |
| | chr11 | 56755823 | 36689 | rs11228962 | + | C/T | snp |
| | chr11 | 56756234 | 37100 | rs10543434 | + | -/TC | in-del |
| | chr11 | 56756244 | 37110 | rs4939119 | + | -/C/CT/T | mixed |
| | chr11 | 56756246 | 37112 | rs4939120 | + | C/T | snp |
| | chr11 | 56756253 | 37119 | rs4939121 | + | C/T | snp |
| | chr11 | 56756257 | 37123 | rs4939122 | + | C/T | snp |
| | chr11 | 56757293 | 38159 | rs10667795 | + | -/TGGATGGA | in-del |
| | chr11 | 56757711 | 38577 | rs3177149 | - | C/T | snp |
| | chr11 | 56758186 | 39052 | rs1044235 | - | A/C | snp |
| *SNP10* | *chr11* | *56758402* | *39268* | *rs2282623* | + | *C*/*T* | *snp* |
| *SNP9* | *chr11* | *56758487* | *39353* | *rs2282624* | + | *C*/*T* | *snp* |
| *SNP8* | *chr11* | *56758504* | *39370* | *rs2282625* | + | *A*/*G* | *snp* |
| | chr11 | 56758608 | 39474 | rs746885 | + | A/G | snp |
| *SNP7* | *chr11* | *56758687* | *39553* | *rs746886* | + | *A*/*G* | *snp* |
| | chr11 | 56758727 | 39593 | rs948846 | + | A/G | snp |
| | chr11 | 56758823 | 39689 | rs746887 | + | A/G | snp |
| | chr11 | 56759081 | 39947 | rs12270028 | + | A/C | snp |
| | chr11 | 56760157 | 41023 | rs7943508 | + | C/T | snp |
| SNP6 | *chr11* | *56760920* | *41786* | *rs948847* | + | *G*/*T* | *snp* |
| SNP5 | *chr11* | *56761153* | *42019* | *rs11544374* | - | *A*/*G* | *snp* |
| | chr11 | 56761425 | 42291 | rs2510358 | + | G/T | snp |
| SNP4 | *chr11* | *56761643* | *42509* | *rs9943582* | + | *C*/*T* | *snp* |
| SNP3 | *chr11* | *56762163* | *43029* | *rs10501367* | + | *C*/*T* | *snp* |
| | chr11 | 56762163 | 43029 | SNP A-1660323 | + | C/T | snp |
| | chr11 | 56762184 | 43050 | rs11605847 | + | G/T | snp |
| SNP2 | *chr11* | *56762540* | *43406* | *rs7119375* | + | *A*/*G* | *snp* |
| | chr11 | 56762540 | 43406 | SNP A-1701491 | + | A/G | snp |
| SNP1 | *chr11* | *56762797* | *43663* | *rs4939123* | + | *A*/*T* | *snp* |
| | chr11 | 56763122 | 43988 | rs7120078 | + | A/G | snp |
| | chr11 | 56763174 | 44040 | rs10571462 | + | -/AAAA | in-del |
| | chr11 | 56763177 | 44043 | rs7120095 | + | A/T | snp |
| | chr11 | 56763292 | 44158 | rs12365036 | + | A/G | snp |
| | chr11 | 56763489 | 44355 | rs12365057 | + | A/G | snp |
| | chr11 | 56763502 | 44368 | rs12365058 | + | A/G | snp |
| | chr11 | 56764233 | 45099 | rs17651573 | + | A/G | snp |
| | chr11 | 56764379 | 45245 | rs7130847 | + | A/T | snp |
| | chr11 | 56764758 | 45624 | rs7128178 | + | A/G | snp |
| | chr11 | 56765112 | 45978 | rs10736682 | + | A/G | snp |
| | chr11 | 56765920 | 46786 | rs1893675 | + | G/T | snp |
| | chr11 | 56766152 | 47018 | rs12576521 | + | G/T | snp |
| | chr11 | 56766174 | 47040 | rs948844 | + | A/C | snp |
| | chr11 | 56766220 | 47086 | rs12576524 | + | A/G | snp |
| | chr11 | 56766667 | 47533 | rs11228966 | + | C/T | snp |
| | chr11 | 56766678 | 47544 | rs11228967 | + | A/G | snp |
| | chr11 | 56766990 | 47856 | rs10792079 | + | A/C | snp |
| | chr11 | 56767735 | 48601 | rs4939124 | + | G/T | snp |
| | chr11 | 56768620 | 49486 | rs17651610 | + | A/C | snp |
| | chr11 | 56768746 | 49612 | rs17151782 | + | A/C | snp |
| | chr11 | 56768851 | 49717 | rs7928905 | + | G/T | snp |
| | chr11 | 56768898 | 49764 | rs499318 | + | A/G | snp |
| | chr11 | 56769089 | 49955 | rs501160 | + | G/T | snp |
| | chr11 | 56769318 | 50184 | rs11606862 | + | A/G | snp |
| | chr11 | 56769824 | 50690 | rs599947 | - | C/T | snp |
| | chr11 | 56770404 | 51270 | rs534130 | + | A/T | snp |
| | chr11 | 56770411 | 51277 | rs694902 | - | A/G | snp |
| | chr11 | 56770479 | 51345 | rs12280902 | + | C/T | snp |

Table 1-4 is the continuation of Table 1-3.

**Table 1-4**

| | | | | | | |
|---|---|---|---|---|---|---|
| chr11 | 56771061 | 51927 | rs11228968 | + | A/T | snp |
| chr11 | 56771238 | 52104 | rs2511032 | - | A/G | snp |
| chr11 | 56771239 | 52105 | rs2511031 | - | A/T | snp |
| chr11 | 56771245 | 52111 | rs4938855 | + | G/T | snp |
| chr11 | 56771333 | 52199 | rs2511030 | - | C/T | snp |
| chr11 | 56771699 | 52565 | rs12789789 | + | C/G | snp |
| chr11 | 56771939 | 52805 | rs652016 | + | C/T | snp |
| chr11 | 56772312 | 53178 | rs7108880 | + | A/G | snp |
| chr11 | 56772338 | 53204 | rs10534300 | + | -/CAAAA | in-del |
| chr11 | 56772428 | 53294 | rs481516 | + | A/T | snp |
| chr11 | 56772478 | 53344 | rs654635 | + | C/T | snp |
| chr11 | 56772508 | 53374 | rs17151797 | + | A/G | snp |
| chr11 | 56772937 | 53803 | rs11825586 | + | C/G | snp |
| chr11 | 56773084 | 53950 | rs11228969 | + | A/C | snp |
| chr11 | 56773085 | 53951 | rs11228970 | + | A/T | snp |
| chr11 | 56773307 | 54173 | rs10896595 | + | C/T | snp |
| chr11 | 56773310 | 54176 | rs668408 | + | C/T | snp |
| chr11 | 56773637 | 54503 | rs1943469 | + | C/T | snp |
| chr11 | 56773809 | 54675 | rs1788969 | + | A/G | snp |
| chr11 | 56773893 | 54759 | rs1262323 | + | A/G | snp |
| chr11 | 56774012 | 54878 | rs1943470 | + | A/C | snp |
| chr11 | 56774133 | 54999 | rs518272 | + | C/T | snp |
| chr11 | 56774260 | 55126 | rs519277 | + | A/G | snp |
| chr11 | 56774875 | 55741 | rs2226845 | + | C/T | snp |
| chr11 | 56774978 | 55844 | rs4938856 | + | C/T | snp |
| chr11 | 56775445 | 56311 | rs12418878 | + | A/C | snp |
| chr11 | 56775819 | 56685 | rs11228971 | + | A/G | snp |
| chr11 | 56776168 | 57034 | rs578831 | + | C/T | snp |
| chr11 | 56776615 | 57481 | rs2846039 | + | G/T | snp |
| chr11 | 56776666 | 57532 | rs625565 | + | A/G | snp |
| chr11 | 56777092 | 57958 | rs10896596 | + | C/G | snp |
| chr11 | 56777280 | 58146 | rs499885 | + | A/G | snp |
| chr11 | 56777440 | 58306 | rs639248 | + | C/T | snp |
| chr11 | 56777483 | 58349 | rs522656 | + | A/G | snp |
| chr11 | 56777953 | 58819 | rs12576471 | + | C/T | snp |
| chr11 | 56777991 | 58857 | rs641550 | + | C/T | snp |
| chr11 | 56778031 | 58897 | rs1939491 | + | A/G | snp |
| chr11 | 56778039 | 58905 | rs528101 | + | A/C | snp |
| chr11 | 56778056 | 58922 | rs652353 | + | A/C | snp |
| chr11 | 56778177 | 59043 | rs4938857 | + | A/C | snp |
| chr11 | 56778375 | 59241 | rs4384399 | + | A/G | snp |
| chr11 | 56778725 | 59591 | rs655402 | + | C/G | snp |
| chr11 | 56778973 | 59839 | rs656366 | + | C/T | snp |
| chr11 | 56779036 | 59902 | rs558266 | + | C/T | snp |
| chr11 | 56779119 | 59985 | rs559168 | + | A/G | snp |
| chr11 | 56779228 | 60094 | rs1939492 | + | G/T | snp |
| chr11 | 56779548 | 60414 | rs7130252 | + | A/G | snp |
| chr11 | 56779741 | 60607 | rs12574780 | + | C/G | snp |
| chr11 | 56779921 | 60787 | rs670919 | + | A/C | snp |
| chr11 | 56780249 | 61115 | rs479949 | + | A/T | snp |
| chr11 | 56781406 | 62272 | rs1257753 | + | C/T | snp |
| chr11 | 56783090 | 63956 | rs2510359 | + | A/C | snp |
| chr11 | 56783134 | 64000 | rs7936545 | + | C/T | snp |
| chr11 | 56783410 | 64276 | rs7102963 | + | A/C | snp |
| chr11 | 56783480 | 64346 | rs2508770 | + | A/G | snp |
| chr11 | 56783481 | 64347 | rs2508771 | + | A/G | snp |
| chr11 | 56783956 | 64822 | rs11604560 | + | A/C | snp |
| chr11 | 56784334 | 65200 | rs546403 | + | A/G | snp |
| chr11 | 56784862 | 65728 | rs4939126 | + | A/G | snp |
| chr11 | 56785231 | 66097 | rs2846040 | + | G/T | snp |

Table 1-5 is the continuation of Table 1-4.

**Table 1-5**

| | | | | | | |
|---|---|---|---|---|---|---|
| chr11 | 56786530 | 67396 | rs2846041 | + | G/T | snp |
| chr11 | 56786573 | 67439 | rs2851727 | - | C/T | snp |
| chr11 | 56786755 | 67621 | rs7930138 | + | G/T | snp |
| chr11 | 56786820 | 67686 | rs11228973 | + | A/T | snp |
| chr11 | 56786869 | 67735 | rs2846042 | + | C/T | snp |
| chr11 | 56787211 | 68077 | rs11228974 | + | A/C | snp |
| chr11 | 56787499 | 68365 | rs11228975 | + | A/C | snp |
| chr11 | 56787538 | 68404 | rs11228976 | + | A/C | snp |
| chr11 | 56787703 | 68569 | rs12271448 | + | A/G | snp |
| chr11 | 56787842 | 68708 | rs12421633 | + | C/T | snp |
| chr11 | 56788241 | 69107 | rs12800196 | + | C/T | snp |
| chr11 | 56788734 | 69600 | rs11228978 | + | A/G | snp |
| chr11 | 56789364 | 70230 | rs12295518 | + | C/T | snp |
| chr11 | 56789457 | 70323 | rs7106133 | + | A/T | snp |
| chr11 | 56789982 | 70848 | rs7120644 | + | A/G | snp |
| chr11 | 56790549 | 71415 | rs2846044 | + | A/C | snp |
| chr11 | 56790795 | 71661 | rs6591413 | + | C/T | snp |
| chr11 | 56791039 | 71905 | rs6591414 | + | G/T | snp |
| chr11 | 56791325 | 72191 | rs10896597 | + | A/C | snp |
| chr11 | 56791604 | 72470 | rs2155230 | + | A/G | snp |
| chr11 | 56792179 | 73045 | rs12279206 | + | A/G | snp |
| chr11 | 56792336 | 73202 | rs12292875 | + | C/T | snp |
| chr11 | 56792454 | 73320 | rs4939127 | + | A/G | snp |
| chr11 | 56792721 | 73587 | rs12575637 | + | C/G | snp |
| chr11 | 56792930 | 73796 | rs12803405 | + | C/T | snp |
| chr11 | 56792962 | 73828 | rs2511027 | + | A/T | snp |
| chr11 | 56793014 | 73880 | rs17151809 | + | C/G | snp |
| chr11 | 56793030 | 73896 | rs17151811 | + | A/T | snp |
| chr11 | 56793141 | 74007 | rs2846045 | + | A/G | snp |
| chr11 | 56793147 | 74013 | rs2846046 | + | A/G | snp |
| chr11 | 56793206 | 74072 | rs12785770 | + | C/T | snp |
| chr11 | 56793251 | 74117 | rs12804848 | + | A/C | snp |
| chr11 | 56793280 | 74146 | rs12804170 | + | C/T | snp |
| chr11 | 56793310 | 74176 | rs2851724 | + | A/C | snp |
| chr11 | 56793337 | 74203 | rs2846047 | + | C/T | snp |
| chr11 | 56793616 | 74482 | rs1892963 | + | A/G | snp |
| chr11 | 56793824 | 74690 | rs2155231 | + | G/T | snp |
| chr11 | 56793970 | 74836 | rs11607817 | + | C/T | snp |
| chr11 | 56794028 | 74894 | rs17652078 | + | C/T | snp |
| chr11 | 56794775 | 75641 | rs1939493 | + | C/T | snp |
| chr11 | 56794826 | 75692 | rs17652103 | + | A/G | snp |
| chr11 | 56794948 | 75814 | rs11228979 | + | C/G | snp |
| chr11 | 56794989 | 75855 | rs11228980 | + | C/T | snp |
| chr11 | 56795297 | 76163 | rs10688423 | + | -/CACA | in-del |
| chr11 | 56795524 | 76390 | rs2846048 | + | C/T | snp |
| chr11 | 56795714 | 76580 | rs11410686 | + | -/C | in-del |
| chr11 | 56795939 | 76805 | rs6591415 | + | A/G | snp |
| chr11 | 56796245 | 77111 | rs11228982 | + | A/G | snp |
| chr11 | 56796246 | 77112 | rs2846049 | + | G/T | snp |
| chr11 | 56796411 | 77277 | rs4938859 | + | G/T | snp |
| chr11 | 56796446 | 77312 | rs2846050 | + | C/T | snp |
| chr11 | 56796965 | 77831 | rs1939494 | + | A/C | snp |
| chr11 | 56797296 | 78162 | rs1892964 | + | A/C | snp |
| chr11 | 56797722 | 78588 | rs12421755 | + | C/T | snp |
| chr11 | 56797954 | 78820 | rs2155232 | + | A/G | snp |
| chr11 | 56798288 | 79154 | rs4939128 | + | C/T | snp |
| chr11 | 56798341 | 79207 | rs2186679 | + | A/C | snp |
| chr11 | 56798499 | 79365 | rs12577794 | + | A/C | snp |
| chr11 | 56798568 | 79434 | rs2155233 | + | C/T | snp |
| chr11 | 56799293 | 80159 | rs10717194 | + | -/A | in-del |

Table 1-6 is the continuation of Table 1-5.

**Table 1-6**

| | | | | | | |
|---|---|---|---|---|---|---|
| chr11 | 56799584 | 80450 | rs11366848 | + | -/G | in-del |
| chr11 | 56799586 | 80452 | rs11353075 | + | -/G | in-del |
| chr11 | 56800019 | 80885 | rs1939495 | + | G/T | snp |
| chr11 | 56800122 | 80988 | rs2846051 | + | A/G | snp |
| chr11 | 56802391 | 83257 | rs7931298 | + | A/C | snp |
| chr11 | 56802859 | 83725 | rs2508769 | + | C/T | snp |
| chr11 | 56803125 | 83991 | rs12362074 | + | C/G | snp |
| chr11 | 56803412 | 84278 | rs11228984 | + | C/T | snp |
| chr11 | 56803873 | 84739 | rs12788046 | + | G/T | snp |
| chr11 | 56803971 | 84837 | rs12806978 | + | A/G | snp |
| chr11 | 56804174 | 85040 | rs12806656 | + | C/T | snp |
| chr11 | 56804760 | 85626 | rs11228985 | + | A/G | snp |
| chr11 | 56805355 | 86221 | rs6650184 | + | A/C | snp |
| chr11 | 56805680 | 86546 | rs2226613 | + | A/C | snp |
| chr11 | 56805802 | 86668 | rs4565915 | + | C/T | snp |
| chr11 | 56805827 | 86693 | rs2846052 | + | A/G | snp |
| chr11 | 56805987 | 86853 | rs1939496 | + | A/G | snp |
| chr11 | 56806163 | 87029 | rs11600878 | + | C/T | snp |
| chr11 | 56806367 | 87233 | rs12797544 | + | A/G | snp |
| chr11 | 56806481 | 87347 | rs12797769 | + | A/G | snp |
| chr11 | 56806529 | 87395 | rs12419065 | + | C/T | snp |
| chr11 | 56807458 | 88324 | rs7396193 | + | A/G | snp |
| chr11 | 56808360 | 89226 | rs7395614 | + | C/T | snp |
| chr11 | 56808411 | 89277 | rs7396611 | + | C/T | snp |
| chr11 | 56808583 | 89449 | rs10896598 | + | C/T | snp |
| chr11 | 56809073 | 89939 | rs7948723 | + | A/C | snp |
| chr11 | 56810681 | 91547 | rs4939132 | + | A/C | snp |
| chr11 | 56811694 | 92560 | rs7128371 | + | G/T | snp |
| chr11 | 56812626 | 93492 | rs4938861 | + | A/C | snp |
| chr11 | 56813068 | 93934 | rs5792034 | + | -/TG | in-del |
| chr11 | 56813428 | 94294 | rs11605053 | + | C/T | snp |
| chr11 | 56813469 | 94335 | rs10896599 | + | A/G | snp |
| chr11 | 56813606 | 94472 | rs17151820 | + | C/G | snp |
| chr11 | 56814224 | 95090 | rs5792035 | + | -/TGGA | in-del |
| chr11 | 56814626 | 95492 | rs17573746 | + | A/G | snp |
| chr11 | 56815008 | 95874 | rs6591416 | + | C/G | snp |
| chr11 | 56815075 | 95941 | rs6591417 | + | A/C | snp |
| chr11 | 56815125 | 95991 | rs12797375 | + | C/T | snp |
| chr11 | 56815329 | 96195 | rs10631051 | + | -/AG | in-del |
| chr11 | 56816391 | 97257 | rs5012051 | + | A/G | snp |
| chr11 | 56816872 | 97738 | rs12786396 | + | C/T | snp |
| chr11 | 56816887 | 97753 | rs12808788 | + | A/G | snp |
| chr11 | 56816926 | 97792 | rs6591418 | + | A/T | snp |
| chr11 | 56816946 | 97812 | rs12296071 | + | A/G | snp |
| chr11 | 56817138 | 98004 | rs12792781 | + | C/T | snp |
| chr11 | 56817985 | 98851 | rs11438573 | + | -/T | in-del |
| chr11 | 56818021 | 98887 | rs7934304 | + | C/G | snp |
| chr11 | 56818207 | 99073 | rs7935236 | + | C/G | snp |
| chr11 | 56818941 | 99807 | rs5792036 | + | -/G | in-del |
| chr11 | 56819085 | 99951 | rs1939488 | + | G/T | snp |
| chr11 | 56819147 | 100013 | rs11601123 | + | C/G | snp |
| chr11 | 56819313 | 100179 | rs11228987 | + | A/G | snp |
| chr11 | 56820462 | 101328 | rs10792083 | + | C/T | snp |
| chr11 | 56820705 | 101571 | rs12365072 | + | C/T | snp |
| chr11 | 56821452 | 102318 | rs6591419 | + | C/T | snp |
| chr11 | 56821557 | 102423 | rs6591420 | + | C/G | snp |
| chr11 | 56821796 | 102662 | rs12802096 | + | C/T | snp |
| chr11 | 56822072 | 102938 | rs1939489 | + | G/T | snp |

The shaded region in the following Tables 2-1 to 2-6 indicates the LD-block.

**Table 2-1**

| | Chromosome No. | Chromosome position | Position in the Sequence Listing | SNP ID | Strand | Nucleotide | Polymorphism type | | Risk allele |
|---|---|---|---|---|---|---|---|---|---|
| *SNP10* | *chr14* | *60954524* | *1* | *rs3783803* | + | *A*/*G* | *snp* | *IMS-JST140198* | *A* |
| | chr14 | 60955193 | 670 | rs4605077 | + | A/G | snp | | |
| | chr14 | 60955578 | 1055 | rs11850051 | + | A/G | snp | | |
| | chr14 | 60955610 | 1087 | rs11627158 | + | A/G | snp | | |
| | chr14 | 60956017 | 1494 | rs11158344 | + | A/C | snp | | |
| | chr14 | 60956092 | 1569 | rs11628122 | + | C/G | snp | | |
| | chr14 | 60956159 | 1636 | rs7155214 | + | A/G | snp | | |
| | chr14 | 60956196 | 1673 | rs7155243 | + | A/G | snp | | |
| | chr14 | 60956490 | 1967 | rs7157248 | + | C/T | snp | | |
| | chr14 | 60956499 | 1976 | rs12590371 | + | A/C | snp | | |
| | chr14 | 60956523 | 2000 | rs12590425 | + | A/G | snp | | |
| | chr14 | 60956658 | 2135 | rs12436712 | + | A/G | snp | | |
| | chr14 | 60956955 | 2432 | rs11848272 | + | A/T | snp | | |
| | chr14 | 60957054 | 2531 | rs17256219 | + | A/G | snp | | |
| | chr14 | 60957230 | 2707 | rs11852192 | + | A/G | snp | | |
| | chr14 | 60959051 | 4528 | rs17098343 | + | C/T | snp | | |
| | chr14 | 60959560 | 5037 | rs11627926 | + | C/T | snp | | |
| | chr14 | 60959879 | 5356 | rs1957890 | + | C/T | snp | | |
| | chr14 | 60960214 | 5691 | rs10144584 | + | A/G | snp | | |
| | chr14 | 60960578 | 6055 | rs7401197 | + | A/G | snp | | |
| | chr14 | 60960943 | 6420 | rs7154500 | + | C/T | snp | | |
| | chr14 | 60961680 | 7157 | rs1957891 | + | A/G | snp | | |
| | chr14 | 60961944 | 7421 | rs7159151 | + | A/G | snp | | |
| | chr14 | 60962194 | 7671 | rs13379453 | + | A/G | snp | | |
| | chr14 | 60962520 | 7997 | rs13379462 | + | A/G | snp | | |
| | chr14 | 60962568 | 8045 | rs13379465 | + | C/T | snp | | |
| | chr14 | 60962720 | 8197 | rs13379301 | + | C/T | snp | | |
| | chr14 | 60962944 | 8421 | rs11621346 | + | A/C | snp | | |
| | chr14 | 60963694 | 9171 | rs4902056 | + | A/G | snp | | |
| | chr14 | 60963697 | 9174 | rs4902057 | + | A/G | snp | | |
| | chr14 | 60963716 | 9193 | rs4899044 | + | A/T | snp | | |
| | chr14 | 60964084 | 9561 | rs8012877 | + | G/T | snp | | |
| | chr14 | 60964513 | 9990 | rs8014589 | + | G/T | snp | | |
| | chr14 | 60964549 | 10026 | rs12888208 | + | C/G | snp | | |
| | chr14 | 60964551 | 10028 | rs12888209 | + | C/T | snp | | |

Table 2-2 is the continuation of Table 2-1.

**Table 2-2**

| | | | | | | | | | |
|---|---|---|---|---|---|---|---|---|---|
| | chr14 | 60964569 | 10046 | rs12889415 | + | C/T | snp | | |
| | chr14 | 60964571 | 10048 | rs12889416 | + | C/T | snp | | |
| | chr14 | 60964579 | 10056 | rs4899045 | + | C/T | snp | | |
| | chr14 | 60964587 | 10064 | rs4902058 | + | C/T | snp | | |
| | chr14 | 60964604 | 10081 | rs12888521 | + | A/G | snp | | |
| | chr14 | 60964614 | 10091 | rs4899046 | + | C/T | snp | | |
| | chr14 | 60964798 | 10275 | rs11158345 | + | C/T | snp | | |
| | chr14 | 60965021 | 10498 | rs8018171 | + | C/T | snp | | |
| | chr14 | 60965827 | 11304 | rs1951956 | + | C/T | snp | | |
| | chr14 | 60966089 | 11566 | rs11848259 | + | C/T | snp | | |
| | chr14 | 60966250 | 11727 | rs1957892 | + | A/T | snp | | |
| | chr14 | 60966728 | 12205 | rs7492971 | + | A/C | snp | | |
| | chr14 | 60966731 | 12208 | rs7492972 | + | A/C | snp | | |
| | chr14 | 60967088 | 12565 | rs968166 | + | A/C | snp | | |
| | chr14 | 60967279 | 12756 | rs12589412 | + | G/T | snp | | |
| | chr14 | 60967371 | 12848 | rs10144460 | + | A/G | snp | | |
| | chr14 | 60967462 | 12939 | rs12435501 | + | C/T | snp | | |
| | chr14 | 60967568 | 13045 | rs13379334 | + | G/T | snp | | |
| | chr14 | 60967709 | 13186 | rs2352088 | + | C/T | snp | | |
| | chr14 | 60968333 | 13810 | rs17098351 | + | G/T | snp | | |
| | chr14 | 60968592 | 14069 | rs17098356 | + | G/T | snp | | |
| | chr14 | 60969235 | 14712 | rs12590817 | + | A/G | snp | | |
| | chr14 | 60969745 | 15222 | rs10136493 | + | C/G | snp | | |
| | chr14 | 60970204 | 15681 | rs17256226 | + | A/G | snp | | |
| *SNP11* | *chr14* | *60970735* | *16212* | *rs3783801* | + | *A*/*G* | *snp* | *IMS-JST140196* | *G* |
| | chr14 | 60970806 | 16283 | rs10139674 | + | A/G | snp | | |
| | chr14 | 60971079 | 16556 | rs4902059 | + | A/G | snp | | |
| | chr14 | 60971846 | 17323 | rs17098362 | + | A/G | snp | | |
| | chr14 | 60972183 | 17660 | rs3837626 | - | -/CATTCATT | in-del | | |
| | chr14 | 60972185 | 17662 | rs10639587 | + | -/TGAATGAA | in-del | | |
| | chr14 | 60972203 | 17680 | rs10580632 | + | -/AATGAATG | in-del | | |
| | chr14 | 60972909 | 18386 | rs2025525 | + | A/T | snp | | |
| | chr14 | 60972976 | 18453 | rs12435952 | + | C/T | snp | | |
| | chr14 | 60973292 | 18769 | rs11627389 | + | C/T | snp | | |
| | chr14 | 60973615 | 19092 | rs10146980 | + | A/G | snp | | |
| | chr14 | 60974135 | 19612 | rs17098367 | + | A/G | snp | | |

Table 2-3 is the continuation of Table 2-2.

**Table 2-3**

| | | | | | | | | | |
|---|---|---|---|---|---|---|---|---|---|
| | chr14 | 60975254 | 20731 | rs7141969 | + | A/G | snp | | |
| | chr14 | 60975695 | 21172 | rs12431773 | + | C/T | snp | | |
| | chr14 | 60975755 | 21232 | rs17098368 | + | C/T | snp | | |
| | chr14 | 60976047 | 21524 | rs8010451 | + | C/T | snp | | |
| | chr14 | 60976392 | 21869 | rs4902060 | + | A/G | snp | | |
| | chr14 | 60976738 | 22215 | rs8016759 | + | C/T | snp | | |
| | chr14 | 60976831 | 22308 | rs17098370 | + | C/T | snp | | |
| | chr14 | 60976970 | 22447 | rs10136457 | + | C/G | snp | | |
| | chr14 | 60977160 | 22637 | rs4636825 | + | C/G | snp | | |
| | chr14 | 60977829 | 23306 | rs1957893 | + | C/T | snp | | |
| | chr14 | 60977864 | 23341 | rs1957894 | + | G/T | snp | | |
| | chr14 | 60978046 | 23523 | rs7161408 | + | C/T | snp | | |
| | chr14 | 60978085 | 23562 | rs1957895 | + | G/T | snp | | |
| | chr14 | 60978864 | 24341 | rs17098373 | + | A/G | snp | | |
| | chr14 | 60978930 | 24407 | rs4899047 | + | C/T | snp | | |
| | chr14 | 60979096 | 24573 | rs4899048 | + | A/G | snp | | |
| | chr14 | 60979424 | 24901 | rs17098375 | + | A/G | snp | | |
| | chr14 | 60979669 | 25146 | rs1042559 | + | A/G | snp | | |
| | chr14 | 60980007 | 25484 | rs10151873 | + | A/T | snp | | |
| | chr14 | 60980910 | 26387 | rs10144353 | + | C/T | snp | | |
| | chr14 | 60981061 | 26538 | rs11621837 | + | C/G | snp | | |
| | chr14 | 60981100 | 26577 | rs9788451 | + | C/G | snp | | |
| | chr14 | 60981891 | 27368 | rs927686 | + | C/T | snp | | |
| | chr14 | 60981902 | 27379 | rs927687 | + | G/T | snp | | |
| | chr14 | 60983210 | 28687 | rs8020230 | + | A/C | snp | | |
| | chr14 | 60984822 | 30299 | rs1957896 | + | A/G | snp | | |
| | chr14 | 60984902 | 30379 | rs11626804 | + | C/G | snp | | |
| | chr14 | 60985158 | 30635 | rs927688 | + | A/C | snp | | |
| *SNP12* | *chr14* | *60985504* | *30981* | *rs2296273* | + | *A*/*G* | *snp* | *IMS-JST050416* | *A* |
| | chr14 | 60985754 | 31231 | rs11623862 | + | A/T | snp | | |
| | chr14 | 60985923 | 31400 | rs11850936 | + | C/T | snp | | |
| | chr14 | 60986337 | 31814 | rs5809096 | + | -/TC | in-del | | |
| | chr14 | 60986372 | 31849 | rs5809097 | + | -/CTCTCTCTCT | in-del | | |
| | chr14 | 60986371 | 31848 | rs12892468 | + | C/G | snp | | |
| | chr14 | 60986373 | 31850 | rs12436619 | + | C/G | snp | | |
| | chr14 | 60986389 | 31866 | rs5027433 | + | A/G | snp | | |

Table 2-4 is the continuation of Table 2-3.

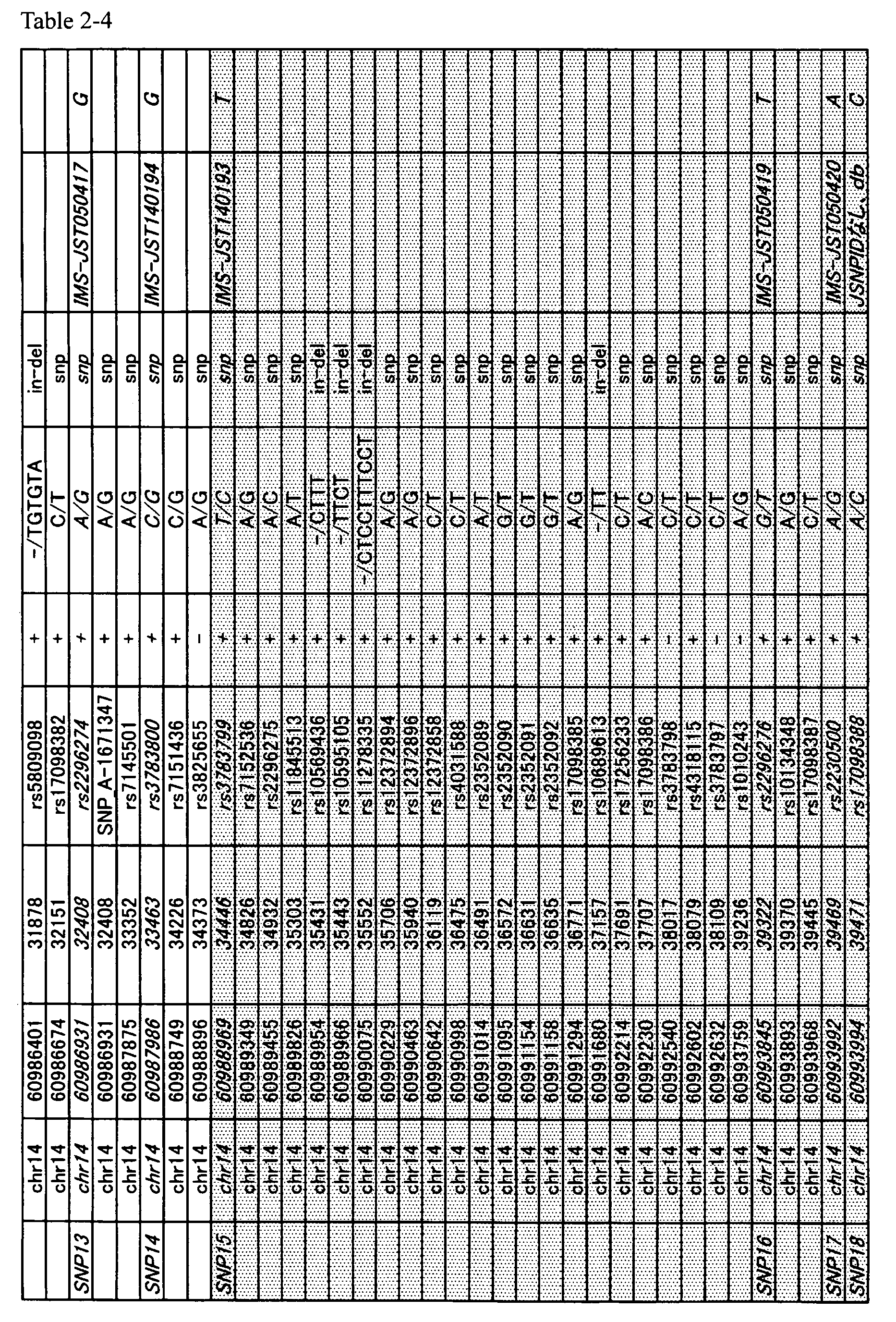

Table 2-5 is the continuation of Table2-4.

**Table 2-5**

| | | | | | | | | | |
|---|---|---|---|---|---|---|---|---|---|
| | chr14 | 60994374 | 39851 | rs10134824 | + | A/G | snp | | |
| | chr14 | 60994488 | 39965 | rs17098394 | + | C/G | snp | | |
| | chr14 | 60995039 | 40516 | rs3783796 | - | A/G | snp | | |
| | chr14 | 60995917 | 41394 | rs2031410 | + | C/T | snp | | |
| | chr14 | 60996267 | 41744 | rs10782446 | + | A/T | snp | | |
| | chr14 | 60996288 | 41765 | rs7140793 | + | A/C | snp | | |
| | chr14 | 60997024 | 42501 | rs10133302 | + | C/T | snp | | |
| | chr14 | 60997338 | 42815 | rs8010084 | + | A/G | snp | | |
| | chr14 | 60997471 | 42948 | rs10144546 | + | C/G | snp | | |
| | chr14 | 60997671 | 43148 | rs17098403 | + | C/T | snp | | |
| | chr14 | 60997702 | 43179 | rs3825654 | - | A/G | snp | | |
| | chr14 | 60997733 | 43210 | rs17098407 | + | G/T | snp | | |
| | chr14 | 60998059 | 43536 | rs11158346 | + | C/T | snp | | |
| | chr14 | 60998990 | 44467 | rs17098408 | + | A/G | snp | | |
| | chr14 | 60999107 | 44584 | rs17098409 | + | A/G | snp | | |
| | chr14 | 60999284 | 44761 | rs17098411 | + | A/G | snp | | |
| | chr14 | 60999688 | 45165 | rs17098413 | + | C/T | snp | | |
| | chr14 | 61000290 | 45767 | rs7155948 | + | A/T | snp | | |
| | chr14 | 61000431 | 45908 | rs11158347 | + | A/G | snp | | |
| | chr14 | 61000482 | 45959 | rs7155311 | + | A/G | snp | | |
| | chr14 | 61000679 | 46156 | rs7156666 | + | C/T | snp | | |
| | chr14 | 61000692 | 46169 | rs12433579 | + | C/T | snp | | |
| | chr14 | 61000905 | 46382 | rs7161353 | + | G/T | snp | | |
| | chr14 | 61000956 | 46433 | rs7160090 | + | A/G | snp | | |
| | chr14 | 61001761 | 47238 | rs12881922 | + | C/T | snp | | |
| | chr14 | 61002671 | 48148 | rs8008464 | + | A/G | snp | | |
| | chr14 | 61003047 | 48524 | rs12101078 | + | C/T | snp | | |
| | chr14 | 61003052 | 48529 | rs12147749 | + | A/G | snp | | |
| | chr14 | 61003230 | 48707 | rs912616 | + | C/T | snp | | |
| | chr14 | 61003289 | 48766 | rs912617 | + | G/T | snp | | |
| | chr14 | 61003344 | 48821 | rs912618 | + | A/G | snp | | |
| *SNP19* | *chr14* | *61003771* | *49248* | *rs959728* | + | *C*/*T* | *snp* | *IMS-JST140187* | *C* |
| *SNP20* | *chr14* | *61003890* | *49367* | *rs3783792* | + | *A*/*G* | *snp* | *IMS-JST140186* | *G* |
| | chr14 | 61003953 | 49430 | rs959729 | + | C/T | snp | | |
| | chr14 | 61004244 | 49721 | rs8019130 | + | A/C | snp | | |
| | chr14 | 61004561 | 50038 | rs8019776 | + | C/T | snp | | |

Table 2-6 is the continuation of Table 2-5.

**Table 2-6**

| | | | | | | | | | |
|---|---|---|---|---|---|---|---|---|---|
| | chr14 | 61005135 | 50612 | rs7142051 | + | G/T | snp | | |
| | chr14 | 61005150 | 50627 | rs11324300 | + | -/T | in-del | | |
| | chr14 | 61005673 | 51150 | rs11158348 | + | A/G | snp | | |
| | chr14 | 61005749 | 51226 | rs12884697 | + | A/G | snp | | |
| | chr14 | 61005927 | 51404 | rs8011286 | + | A/T | snp | | |
| | chr14 | 61005985 | 51462 | rs8010419 | + | C/G | snp | | |
| | chr14 | 61006068 | 51545 | rs8010131 | + | A/G | snp | | |
| | chr14 | 61006070 | 51547 | rs8010132 | + | A/G | snp | | |
| | chr14 | 61006296 | 51773 | rs3783791 | - | C/T | snp | | |
| | chr14 | 61006373 | 51850 | rs3783790 | - | A/G | snp | | |
| | chr14 | 61006512 | 51989 | rs17098427 | + | A/G | snp | | |
| *SNP21* | *chr14* | *61006553* | *52030* | *rs3783789* | + | *A*/*G* | *snp* | *IMS-JST140183* | *A* |

More specifically, a preferred embodiment of the present invention is a method of determining the type of nucleotides at the polymorphic sites listed in the above-mentioned Tables.

Of the above-mentioned polymorphic sites, polymorphic sites that can be used in the present invention's method of testing for the presence or absence of a risk factor for arteriosclerotic diseases are preferably those located in the AGTRL1 or PRKCH gene or in the region surrounding the gene. For example, preferred polymorphic sites in the AGTRL1 gene are located at positions 1, 296, 824, 932, 1042, 1072, 1666, 1840, 3096, 3181, 3225, 3381, 3416, 3778, 3911, 4243, 4257, 4665, 5057, 5179, 5417, 5448, 5530, 6002, 6401, 6563, 6878, 6897, 6961, 7496, 8690, 9040, 9159, 9440, 10350, 10499, 10535, 11235, 11290, 12541, 13104, 13735, 13793, 13855, 14025, 14955, 15071, 16909, 18281, 19106, 19474, 19654, 21090, 21545, 21776, 21890, 22337, 22374, 22992, 23248, 23297, 23643, 23694, 23845, 23926, 24060, 25119, 25448, 25463, 25738, 25794, 25893, 25959, 26569, 26826, 27070, 27131, 27892, 28039, 28121, 28634, 29116, 29129, 29190, 29305, 29389, 29727, 29854, 30221, 30289, 30290, 31284, 31581, 32022, 32477, 32611, 32707, 32776, 33051, 33395, 33521, 33656, 33781, 34650, 34876, 35091, 35274, 35365, 36267, 36325, 36596, 36619, 36681, 36682, 36689, 37100, 37110, 37112, 37119, 37123, 38159, 38577, 39052, 39268, 39353, 39370, 39474, 39553, 39593, 39689, 39947, 41023, 41786, 42019, 42291, 42509, 43029, 43050, 43406, 43663, 43988, 44040, 44043, 44158, 44355, 44368, 45099, 45245, 45624, 45978, 46786, 47018, 47040, 47086, 47533, 47544, 47856, 48601, 49486, 49612, 49717, 49764, 49955, 50184, 50690, 51270, 51277, 51345, 51927, 52104, 52105, 52111, 52199, 52565, 52805, 53178, 53204, 53294, 53344, 53374, 53803, 53950, 53951, 54173, 54176, 54503, 54675, 54759, 54878, 54999, 55126, 55741, 55844, 56311, 56685, 57034, 57481, 57532, 57958, 58146, 58306, 58349, 58819, 58857, 58897, 58905, 58922, 59043, 59241, 59591, 59839, 59902, 59985, 60094, 60414, 60607, 60787, 61115, 62272, 63956, 64000, 64276, 64346, 64347, 64822, 65200, 65728, 66097, 67396, 67439, 67621, 67686, 67735, 68077, 68365, 68404, 68569, 68708, 69107, 69600, 70230, 70323, 70848, 71415, 71661, 71905, 72191, 72470, 73045, 73202, 73320, 73587, 73796, 73828, 73880, 73896, 74007, 74013, 74072, 74117, 74146, 74176, 74203, 74482, 74690, 74836, 74894, 75641, 75692, 75814, 75855, 76163, 76390, 76580, 76805, 77111, 77112, 77277, 77312, 77831, 78162, 78588, 78820, 79154, 79207, 79365, 79434, 80159, 80450, 80452, 80885, 80988, 83257, 83725, 83991, 84278, 84739, 84837, 85040, 85626, 86221, 86546, 86668, 86693, 86853, 87029, 87233, 87347, 87395, 88324, 89226, 89277, 89449, 89939, 91547, 92560, 93492, 93934, 94294, 94335, 94472, 95090, 95492, 95874, 95941, 95991, 96195, 97257, 97738, 97753, 97792, 97812, 98004, 98851, 98887, 99073, 99807, 99951, 100013, 100179, 101328, 101571, 102318, 102423, 102662, and 102938 in the nucleotide sequence of SEQ ID NO: 1.

In addition, polymorphic sites in the PRKCH gene located at positions 1, 670, 1055, 1087, 1494, 1569, 1636, 1673, 1967, 1976, 2000, 2135, 2432, 2531, 2707, 4528, 5037, 5356, 5691, 6055, 6420, 7157, 7421, 7671, 7997, 8045, 8197, 8421, 9171, 9174, 9193, 9561, 9990, 10026, 10028, 10046, 10048, 10056, 10064, 10081, 10091, 10275, 10498, 11304, 11566, 11727, 12205, 12208, 12565, 12756, 12848, 12939, 13045, 13186, 13810, 14069, 14712, 15222, 15681, 16212, 16283, 16556, 17323, 17660, 17662, 17680, 18386, 18453, 18769, 19092, 19612, 20731, 21172, 21232, 21524, 21869, 22215, 22308, 22447, 22637, 23306, 23341, 23523, 23562, 24341, 24407, 24573, 24901, 25146, 25484, 26387, 26538, 26577, 27368, 27379, 28687, 30299, 30379, 30635, 30981, 31231, 31400, 31814, 31848, 31849, 31850, 31866, 31878, 32151, 32408, 33352, 33463, 34226, 34373, 34446, 34826, 34932, 35303, 35431, 35443, 35552, 35706, 35940, 36119, 36475, 36491, 36572, 36631, 36635, 36771, 37157, 37691, 37707, 38017, 38079, 38109, 39236, 39322, 39370, 39445, 39469, 39471, 39851, 39965, 40516, 41394, 41744, 41765, 42501, 42815, 42948, 43148, 43179, 43210, 43536, 44467, 44584, 44761, 45165, 45767, 45908, 45959, 46156, 46169, 46382, 46433, 47238, 48148, 48524, 48529, 48707, 48766, 48821, 49248, 49367, 49430, 49721, 50038, 50612, 50627, 51150, 51226, 51404, 51462, 51545, 51547, 51773, 51850, 51989, and 52030 are also preferred (the above polymorphic sites may be herein simply referred to as "the polymorphic sites of the present invention").

One skilled in the art can suitably obtain information regarding the particular nucleotides at the above sites based on the above-listed rs numbers for the dbSNP database. Regarding SNP IDs, those with "rs" at the head are registration IDs in the dbSNP database that have been uniquely assigned to respective single sequences by NCBI. The dbSNP database is publicly available on a web site (http://www.ncbi.nlm.nih.gov/SNP/index.html), and detailed information on SNPs in a nucleotide sequence (for example, position on a chromosome, nucleotide type at polymorphic site, and adjacent sequences) can be obtained by conducting search on the web site using a registration ID number stated in an SNP ID. By using the information, one skilled in the art can easily perform the test of the present invention.

Generally, one skilled in the art can easily find the actual genomic position, adjacent sequences and such of the polymorphic sites of the present invention using the registration IDs given to the polymorphisms disclosed herein, such as the rs numbers in the dbSNP database. If this information cannot be found by such means, one skilled in the art can readily find the actual genomic position corresponding to the polymorphic site based on the sequence of SEQ ID NO: 1 and information on the polymorphic site or such. For example, the genomic position of the polymorphic site of the present invention can be determined by consulting a public genome database or such. Specifically, even when the nucleotide sequences are slightly different between the nucleotide sequence in the sequence listing and the actual genomic nucleotide sequence, the actual genomic position of the polymorphic site of the present invention can be precisely identified by, for example, conducting a homology search of the genomic sequence based on the nucleotide sequence in the sequence listing. Even when the genomic position cannot be identified, the test of the present invention can be easily conducted based on the sequence listing and the information on the polymorphic sites disclosed herein.

Genomic DNA usually has a mutually complementary double-stranded DNA structure. Accordingly, even when the DNA sequence of one strand is disclosed herein for the sake of convenience, it will be naturally understood that the other sequence complementary to the above sequence (nucleotides) is also disclosed. When a DNA sequence (nucleotides) in one strand is known, the other sequence (nucleotides) complementary to the above sequence (nucleotides) is obvious to one skilled in the art. Regarding the human genome sequence, the International Human Genome Project build35, which is said to be an almost final version, has been published, and the sequences and the like described herein are based on the results of the International Human Genome Project build35.

In the present invention's method of testing for the presence or absence of a risk factor for arteriosclerotic diseases, the following polymorphic sites are preferably tested.

In a more preferred embodiment of the present invention, methods of testing for the presence of absence of a risk factor for arteriosclerotic diseases involve testing the polymorphic sites comprised in the region between the polymorphic sites of (1a) and (24a) listed below, or preferably the polymorphic site in the AGTRL1 gene located at (1a) position 1, (2a) position 12541, (3a) position 21545, (4a) position 33051, (5a) position 35365, (6a) position 39268, (7a) position 39353, (8a) position 39370, (9a) position 39474, (10a) position 39553, (11a) position 39665, (12a) position 41786, (13a) position 42019, (14a) position 42509, (15a) position 43029, (16a) position 43406, (17a) position 43663, (18a) position 46786, (19a) position 49764, (20a) position 64276, (21a) position 74482, (22a) position 78162, (23a) position 93492, or (24a) position 102938 of the nucleotide sequence of SEQ ID NO: 1.

In a preferred embodiment of the present invention, a subject is determined to have a risk factor for arteriosclerotic diseases when the type of nucleotide at the polymorphic sites of (1a) to (24a) described above are (1b) to (24b) listed below, respectively. The presence or absence of a risk factor for arteriosclerotic diseases can be determined regardless of whether or not a subject is suffering from an arteriosclerotic disease.
(1b) The nucleotide type of in the complementary strand of the AGTRL1 gene located at position 1 of the nucleotide sequence of SEQ ID NO: 1 is T.
(2b) The type of nucleotide in the complementary strand of the AGTRL1 gene located at position 12541 of the nucleotide sequence of SEQ ID NO: 1 is T.
(3b) The type of nucleotide in the complementary strand of the AGTRL1 gene located at position 21545 of the nucleotide sequence of SEQ ID NO: 1 is A.
(4b) The type of nucleotide in the complementary strand of the AGTRL1 gene located at position 33051 of the nucleotide sequence of SEQ ID NO: 1 is C.
(5b) The type of nucleotide in the complementary strand of the AGTRL1 gene located at position 35365 of the nucleotide sequence of SEQ ID NO: 1 is T.
(6b) The type of nucleotide in the complementary strand of the AGTRL1 gene located at position 39268 of the nucleotide sequence of SEQ ID NO: 1 is A.
(7b) The type of nucleotide in the complementary strand of the AGTRL1 gene located at position 39353 of the nucleotide sequence of SEQ ID NO: 1 is G.
(8b) The type of nucleotide in the complementary strand of the AGTRL1 gene located at position 39370 of the nucleotide sequence of SEQ ID NO: 1 is C.
(9b) The type of nucleotide in the complementary strand of the AGTRL1 gene located at position 39474 of the nucleotide sequence of SEQ ID NO: 1 is T.
(10b) The type of nucleotide in the complementary strand of the AGTRL1 gene located at position 39553 of the nucleotide sequence of SEQ ID NO: 1 is T.
(11b) A site in the AGTRL1 gene located at position 39665 of the nucleotide sequence of SEQ ID NO: 1 has been deleted.
(12b) The type of nucleotide in the complementary strand of the AGTRL1 gene located at position 41786 of the nucleotide sequence of SEQ ID NO: 1 is A.
(13b) The type of nucleotide in the complementary strand of the AGTRL1 gene located at position 42019 of the nucleotide sequence of SEQ ID NO: 1 is G.
(14b) The type of nucleotide in the complementary strand of the AGTRL1 gene located at position 42509 of the nucleotide sequence of SEQ ID NO: 1 is G.
(15b) The type of nucleotide in the complementary strand of the AGTRL1 gene located at position 43029 of the nucleotide sequence of SEQ ID NO: 1 is G.
(16b) The type of nucleotide in the complementary strand of the AGTRL1 gene located at position 43406 of the nucleotide sequence of SEQ ID NO: 1 is C.
(17b) The type of nucleotide in the complementary strand of the AGTRL1 gene located at position 43663 of the nucleotide sequence of SEQ ID NO: 1 is T.
(18b) The type of nucleotide in the complementary strand of the AGTRL1 gene located at position 46786 of the nucleotide sequence of SEQ ID NO: 1 is C.
(19b) The type of nucleotide in the complementary strand of the AGTRL1 gene located at position 49764 of the nucleotide sequence of SEQ ID NO: 1 is T.
(20b) The type of nucleotide in the complementary strand of the AGTRL1 gene located at position 64276 of the nucleotide sequence of SEQ ID NO: 1 is T.
(21b) The type of nucleotide in the complementary strand of the AGTRL1 gene located at position 74482 of the nucleotide sequence of SEQ ID NO: 1 is C.
(22b) The type of nucleotide in the complementary strand at a site on the AGTRL1 gene located at position 78162 of the nucleotide sequence of SEQ ID NO: 1 is G.
(23b) The type of nucleotide in the complementary strand of the AGTRL1 gene located at position 93492 of the nucleotide sequence of SEQ ID NO: 1 is G.
(24b) The type of nucleotide in the complementary strand of the AGTRL1 gene located at position 102938 of the nucleotide sequence of SEQ ID NO: 1 is C.

The above-mentioned nucleotides represent those either in the plus strand or the minus strand of the nucleotide sequence of a gene of the present invention. Those skilled in the art can appropriately determine the type of nucleotide that should be detected in the polymorphic sites based on the information disclosed herein.

In another embodiment, and in a more preferred embodiment of the present invention, methods of testing for the presence or absence of a risk factor for arteriosclerotic diseases involve testing the polymorphic sites comprised in the region between the polymorphic sites of (1a) and (12a) listed below, or preferably the polymorphic site on the PRKCH gene located at (1a) position 1, (2a) position 16212, (3a) position 30981, (4a) position 32408, (5a) position 33463, (6a) position 34446, (7a) position 39322, (8a) position 39469, (9a) position 39471, (10a) position 49248, (11a) position 49367, or (12a) position 52030 of the nucleotide sequence of SEQ ID NO: 2.

In a preferred embodiment of the present invention, a subject is determined to have a risk factor for arteriosclerotic diseases when the type of nucleotide at the polymorphic sites of (1a) to (12a) mentioned above are (1b) to (12b) listed below, respectively. The presence or absence of a risk factor for arteriosclerotic diseases can be determined regardless of whether or not a subject is suffering from an arteriosclerotic disease.
(1b) The type of nucleotide in the PRKCH gene located at position 1 of the nucleotide sequence of SEQ ID NO: 2 is A.
(2b) The type of nucleotide in the PRKCH gene located at position 16212 of the nucleotide sequence of SEQ ID NO: 2 is G.
(3b) The type of nucleotide in the PRKCH gene located at position 30981 of the nucleotide sequence of SEQ ID NO: 2 is A.
(4b) The type of nucleotide in the PRKCH gene located at position 32408 of the nucleotide sequence of SEQ ID NO: 2 is G.
(5b) The type of nucleotide in the PRKCH gene located at position 33463 of the nucleotide sequence of SEQ ID NO: 2 is G.
(6b) The type of nucleotide in the PRKCH gene located at position 34446 of the nucleotide sequence of SEQ ID NO: 2 is T.
(7b) The type of nucleotide in the PRKCH gene located at position 39322 of the nucleotide sequence of SEQ ID NO: 2 is T.
(8b) The type of nucleotide in the PRKCH gene located at position 39469 of the nucleotide sequence of SEQ ID NO: 2 is A.
(9b) The type of nucleotide in the PRKCH gene located at position 39471 of the nucleotide sequence of SEQ ID NO: 2 is C.
(10b) The type of nucleotide in the PRKCH gene located at position 49248 of the nucleotide sequence of SEQ ID NO: 2 is C.
(11b) The type of nucleotide in the PRKCH gene located at position 49367 of the nucleotide sequence of SEQ ID NO: 2 is G.
(12b) The type of nucleotide in the PRKCH gene located at position 52030 of the nucleotide sequence of SEQ ID NO: 2 is A.

In the methods of the present invention, the above-mentioned polymorphic mutations may be detected in one of the genomes (heterozygous); however, without particular limitation, they are preferably detected in both of the genomes (homozygous).

For example, when a subject is suitably determined to have a risk factor for arteriosclerotic diseases, the genotype in the PRKCH gene located at position 39469 of the nucleotide sequence of SEQ ID NO: 2 is AA (homozygous).

In the present invention, since the polymorphic sites may be strongly linked to their surrounding DNA regions, the testing method of the present invention can be carried out by detecting polymorphic mutations other than those described above that are present in such a strongly linked DNA block.

For the AGTRL1 gene, for example, the nucleotide of an "adjacent polymorphic site" (for example, a polymorphic site listed in Tables 1-1 to 1-6 above) is determined beforehand in a human subgroup that includes arteriosclerotic disease patients in which the nucleotide at the polymorphic sites are (1b) and (24b) as above.

Next, whether or not a subject has a risk factor for arteriosclerotic diseases can be tested by determining the nucleotides of the "adjacent polymorphic site" in the subject and comparing it with the previously-determined nucleotides. When the nucleotide of the subject is identical to the previously-determined nucleotide, the subject is determined to have a risk factor for arteriosclerotic diseases. The testing method of the present invention can determine whether or not a subject has a risk factor for arteriosclerotic diseases, and can be utilized, for example, in deciding courses of treatment and agent doses.

For example, in a human subgroups including persons suffering from arteriosclerotic diseases in which the nucleotide at a polymorphic site on the AGTRL1 gene at position 39268 in the nucleotide sequence of SEQ ID NO: 1 is A, the nucleotide at an adjacent polymorphic site, such as position 296, is determined. When the frequency that the nucleotide of this site is T is higher in the persons suffering from arteriosclerotic diseases than in persons who do not suffer from the diseases, a subject is tested for the nucleotide at the polymorphic site of position 1, and determined to have a risk factor of arteriosclerotic diseases if the nucleotide type of this site is also T.

As described above, the discovery of genetic regions associated with arteriosclerotic diseases by the present invention allows those skilled in the art to test the presence or absence of a risk factor for arteriosclerotic diseases without undue burden.

As the human genome analysis progresses, information on whole nucleotide sequences and polymorphisms such as SNPs, microsatellites, VNTRs, RFLPs has been enriched. While the genomic nucleotide sequences are now being revealed in detail, the current top concern is to analyze a relationship between a gene or a specific sequence and a function (phenotype such as disease or disease progression). One of promising solutions to this is a genetic statistical analysis using haplotypes.

Human chromosomes are present in pairs and each pair is derived from mother and father. The term "haplotype" refers to a combination of the genotypes in either one of the pair of an individual, and shows how gene loci are arranged on a paternally- or maternally-derived chromosome. A child inherits one chromosome each from the father and mother. Thus, if no recombination occurred at the time of gametogenesis, the genes on each chromosome would inevitably be transferred together to the child, namely, they would be linked to each other. However, since recombination does in fact occur upon meiosis, genes carried on one chromosome are not necessarily linked. Inversely, however, even when genetic recombination occurs, gene loci closely located in one chromosome are strongly linked.

When allele dependency is found by observing this phenomenon in a population, this is referred to as "linkage disequilibrium". For example, when three gene loci are observed and no linkage disequilibrium is seen among them, there expected to be 2³ different haplotypes, and the frequency of each haplotype is predicted from the frequencies of the gene loci. However, when a linkage disequilibrium is seen, there are only less than 2³ haplotypes, and their frequencies are different from those predicted.

It has recently been demonstrated that haplotypes are useful for analyzing linkage disequilibrium (Genetic Epidemiology 23:221-233), and further studies have been made. As a result, it has been revealed that the genome has portions susceptible or insusceptible to recombination, and that the portions that are inherited from ancestry to progeny as single regions (regions specified by haplotypes) are common to human races (Science 226, 5576:2225-2229). Specifically, there are tightly linked DNA regions, and these regions are generally called DNA blocks. In the present invention, the presence or absence of a risk factor for arteriosclerotic diseases can also be tested by detecting the presence or absence of a DNA block that includes a polymorphic site of the present invention.

Namely, a preferred embodiment of the present invention provides a method of testing whether or not a subject has a risk factor for arteriosclerotic diseases, wherein the detection of the presence of a DNA block showing the following haplotype indicates that a subject has a risk factor for arteriosclerotic diseases:
(A) a haplotype in which nucleotides of the complementary strand at polymorphic sites of positions 39268, 39353, 41786, 42019, and 43406 in the nucleotide sequence of SEQ ID NO: 1 are A, G, A, G, and C, respectively, wherein the polymorphic sites are located in the AGTRL1 gene.

In the present invention, the term "DNA block" refers to a portion (region) which shows an intense linkage disequilibrium among gene loci. If a DNA block associated with arteriosclerotic diseases is found, the presence or absence of a risk factor for arteriosclerotic diseases can be tested by detecting the DNA block.

If (a DNA block showing) a haplotype associated with arteriosclerotic diseases is found, the presence or absence of a risk factor for arteriosclerotic diseases can be tested by detecting the haplotype. As a result of dedicated research, the present inventors successfully discovered haplotypes associated with the susceptibility to arteriosclerotic diseases.

Accordingly, the present invention provides methods of testing whether or not a subject has a risk factor for arteriosclerotic diseases, which includes the step of detecting (a DNA block showing) a haplotype which is associated with arteriosclerotic diseases and is present on the AGTRL1 gene.

The present method can determine whether or not a subject has a risk factor for arteriosclerotic diseases by detecting a "haplotype associated with arteriosclerotic diseases" in the subject. These determinations can be used, for example, in deciding courses of treatment.

The above-mentioned "(DNA block showing) haplotype associated with arteriosclerotic diseases" specifically includes the following (DNA block showing) haplotype:
(A) a haplotype in which the nucleotides of the complementary strand at polymorphic sites on the AGTRL1 gene located at positions 39268, 39353, 41786, 42019, and 43406 of the nucleotide sequence of SEQ ID NO: 1 are A, G, A, G, and C, respectively.

A preferred embodiment of the above-mentioned method of the present invention is a method of testing whether or not a subject has a risk factor for arteriosclerotic diseases, which comprises the step of determining the type of nucleotides of linked polymorphic sites present in a DNA block showing the following haplotype:
(A) a haplotype in which the nucleotides in the complementary strand of the AGTRL1 gene located at polymorphic sites at positions 39268, 39353, 41786, 42019, and 43406 of the nucleotide sequence of SEQ ID NO: 1 are A, G, A, G, and C, respectively.

Specifically, even polymorphic sites other than those specifically described herein that are included in the above-mentioned DNA block and linked with the polymorphic sites of the present invention may be used for the testing method of the present invention.

A preferred embodiment of the above-mentioned method is a method for testing whether or not a subject has a risk factor for arteriosclerotic diseases, which comprises the steps of:
(a) determining the type of nucleotide at a polymorphic site in the AGTRL1 gene of the subject; and
(b) determining that the subject has a risk factor for arteriosclerotic diseases when the nucleotide determined in (a) is the same as the nucleotide at the aforementioned polymorphic site in an AGTRL1 gene that shows the haplotype of (A) described below:
   (A) a haplotype in which the nucleotides in the complementary strand of the AGTRL1 gene at polymorphic sites located at positions 39268, 39353, 41786, 42019, and 43406 of the nucleotide sequence of SEQ ID NO: 1 are A, G, A, G, and C, respectively.

Examples of the polymorphic site in step (a) include the polymorphic sites listed in Tables 1-1 to 1-6 above. Preferred polymorphic sites are those on the AGTRL1 gene located at positions 1, 12541, 21545, 33051, 35365, 39268, 39353, 39370, 39474, 39553, 39665, 41786, 42019, 42509, 43029, 43406, 43663, 46786, 49764, 64276, 74482, 78162, 93492, and 102938 in the nucleotide sequence of SEQ ID NO: 1.

Preferably, the polymorphic site in step (a) above includes, for example, the following polymorphic site (1) or (2):
(1) a polymorphic site on the GRK5 gene located at position 34799 in the nucleotide sequence of SEQ ID NO: 1; and
(2) a polymorphic site on the GRK5 gene located at position 60001 in the nucleotide sequence of SEQ ID NO: 1.

The nucleotide at a polymorphic site of the present invention can be determined by one skilled in the art using various methods. For example, it can be determined by directly determining the nucleotide sequence of a DNA having a polymorphic site of the present invention.

Typically, a test sample to be subjected to the testing methods of the present invention is preferably a biological sample collected from a subject beforehand. The biological sample may include, for example, a DNA sample. In the present invention, such DNA samples can be prepared based on chromosomal DNA or RNA extracted from, for example, the blood, skin, oral mucosa, a tissue or cell collected or excised by surgery, or a body fluid collected for testing or such, of the subject.

Thus, in the methods of the present invention, a subject-derived biological sample (a biological sample obtained from the subject beforehand) is subjected as a test sample to the test.

One skilled in the art can prepare a biological sample suitably using known techniques. For example, DNA samples can be prepared, for example, by PCR using chromosomal DNA or RNA as a template and primers that hybridize to a DNA having a polymorphic site of the present invention.

Next, the nucleotide sequence of isolated DNA is determined. The nucleotide sequence of the isolated DNA can be easily determined by one skilled in the art using, for example, a DNA sequencer.

Variations in the nucleotides at the polymorphic sites of the present invention are normally already known. The phrase "determining the nucleotide" in the present invention does not necessarily mean determining whether the nucleotide at a certain polymorphic site is A, G, T, or C. For example, when the nucleotide variations at a certain polymorphic site are known to be A or G, it is only necessary to find that the nucleotide of the site is "not A" or "not G".

There are a variety of known methods for determining the nucleotide of a polymorphic site whose nucleotide variations are known. The method for determining the nucleotide in the present invention is not particularly limited. For example, TaqMan PCR, AcycloPrime, and MALDI-TOF/MS are in practical use as analysis methods using PCR. In addition, the Invader method and RCA method are known as non-PCR-dependent methods for determining nucleotide types. The nucleotide can also be determined using DNA arrays. These methods will be briefly illustrated below. Any of these processes can be applied to the determination of the nucleotide of a polymorphic site in the present invention.

### [TaqMan PCR]

The principle of TaqMan PCR is as follows. TaqMan PCR is an analysis method using a TaqMan probe and a set of primers that can amplify a region containing an allele. The TaqMan probe is designed to hybridize with the region containing the allele, which is amplified by the set of primers.

When the TaqMan probe is allowed to hybridize with a target nucleotide sequence under a condition near the Tm of the TaqMan probe, the hybridization efficiency of the TaqMan probe is significantly lowered due to the difference in a single nucleotide. When PCR is conducted in the presence of the TaqMan probe, elongation from the primers reaches the hybridized TaqMan probe in due course. Then, the TaqMan probe is decomposed from its 5' end by the 5'-3' exonuclease activity of DNA polymerase. By labeling the TaqMan probe with a reporter dye and a quencher, the decomposition of the TaqMan probe can be traced as a change in fluorescent signal. Specifically, when the TaqMan probe is decomposed, the reporter dye is released away from the quencher and thereby generates a fluorescence signal. When the hybridization of the TaqMan probe is reduced due to the difference in a single nucleotide, the decomposition of the TaqMan probe does not proceed, and a fluorescence signal is not generated.

Multiple nucleotide types can be determined simultaneously by designing TaqMan probes corresponding to a polymorphism and further modifying them so that the decomposition of each probe produces a different signal. For example, 6-carboxy-fluorescein (FAM) and VIC are used as reporter dyes for TaqMan probes for allele A and allele B, respectively, in a given allele. The generation of fluorescence signals by the reporter dyes are inhibited by a quencher when the probes are not decomposed. When each probe hybridizes with the corresponding allele, a fluorescence signal is observed upon the hybridization. Specifically, when the signal of either FAM or VIC is more intense than the other, the allele is found to be a homozygote of allele A or allele B. On the other hand, when the allele is a heterozygote of allele A and allele B, the two signals are to be detected at substantially identical levels. By using the TaqMan PCR, PCR and the determination of nucleotide type can be simultaneously conducted using a genome as an analysis target without a time-consuming step like separation on a gel. Accordingly, the TaqMan PCR is useful as a method capable of determining nucleotide types of many subjects.

### [AcycloPrime]

AcycloPrime is also in practice use as a method of determining a nucleotide using PCR. AcycloPrime uses one pair of primers for genome amplification, and one primer for polymorphism detection. Initially, a genomic region containing a polymorphic site is amplified by PCR. This step is the same as a regular genomic PCR. Next, the resultant PCR product is annealed with a primer for detecting SNPs, and an elongation reaction is conducted. The primer for detecting SNPs is so designed as to be annealed with a region adjacent to the polymorphic site to be detected.

In this step, a nucleotide derivative (terminator), which is labeled with a fluorescence polarization dye and is blocked at its 3'-OH, is used as a nucleotide substrate for elongation. As a result, only one complementary nucleotide is incorporated at the nucleotide at a position corresponding to the polymorphic site, and the elongation reaction is terminated. The incorporation of the nucleotide derivative to the primer can be detected by an increase of fluorescence polarization (FP) due to the increase of molecular weight. When two labels having different wavelengths are used as fluorescence polarization dyes, it is possible to determine whether a particular SNP is either of two nucleotides. Since the level of fluorescence polarization can be quantified, a single analysis can also determine whether an allele is a homozygote or heterozygote.

### [MALDI-TOF/MS]

The nucleotide type can also be determined by analyzing a PCR product through MALDI-TOF/MS. The MALDI-TOF/MS can quantify molecular weights very accurately. Thus, it is used in a variety of fields as an analysis method that can distinguish a slight difference in the amino acid sequence of a protein and the nucleotide sequence of a DNA. To determine a nucleotide through MALDI-TOF/MS, a region containing an allele to be analyzed is initially amplified by PCR. Next, an amplified product is isolated, and the molecular weight thereof is measured using MALDI-TOF/MS. Since the nucleotide sequence of the allele is already known, the nucleotide sequence of the amplified product is uniquely determined based on the molecular weight.

The determination of a nucleotide using MALDI-TOF/MS requires the separation of a PCR product and such. However, this technique is expected to enable accurate determination of a nucleotide without using labeled primers and probes. This technique can also be applied to simultaneous detection of polymorphisms at multiple sites.

### [SNP-specific Labeling Method Using Type IIs Restriction Enzymes]

Methods that can determine a nucleotide type more rapidly using PCR have been reported. For example, the nucleotide at a polymorphic site can be determined using a type IIs restriction enzyme. This method uses a primer having a type IIs restriction enzyme-recognition sequence in PCR. Common restriction enzymes (type II), which are used in gene recombination, recognize a specific nucleotide sequence and cleaves a specific site in the nucleotide sequence. In contrast, type IIs restriction enzymes recognize a specific nucleotide sequence and cleaves a site away from the recognized nucleotide sequence. The number of nucleotides between the recognized sequence and the cleaving site depends on each enzyme. Accordingly, the amplified product can be cleaved exactly at the polymorphic site by a type IIs restriction enzyme when a primer that contains a recognition sequence of the type IIs restriction enzyme is allowed to anneal at a position away from the polymorphic site by the specific number of nucleotides.

A cohesive end containing a SNP nucleotide is formed at an end of the amplified product cleaved by the type IIs restriction enzyme. Then, adaptors having a nucleotide sequence corresponding to the cohesive end of the amplified product are ligated. The adaptors include different nucleotide sequences containing nucleotides corresponding to polymorphic mutations, and they can be labeled with different fluorescent dyes in advance. Finally, the amplified product is labeled with a fluorescent dye corresponding to the nucleotide of the polymorphic site.

When PCR is conducted using a primer having a type IIs restriction enzyme-recognition sequence in combination with a capture primer, the amplified product can be fluorescently labeled and then immobilized to a solid phase using the capture primer. For example, when a biotin-labeled primer is used as the capture primer, the amplified product can be captured by avidin-linked beads. The nucleotide can be determined by tracing the fluorescence dye of the amplified product thus captured.

### [Determination of Nucleotide Type at Polymorphic Site Using Magnetic Fluorescence Beads]

There are also known techniques capable of analyzing plural alleles in parallel in a single reaction system. Analyzing multiple alleles in parallel is called "multiplexing". In typing methods using fluorescent signals, fluorescent elements having different fluorescence wavelengths are necessary for multiplexing. However, not so many fluorescent elements are available in actual analyses. In contrast, when multiple fluorescent elements are mixed with resins or such, even limited kinds of fluorescent elements can yield various fluorescence signals distinguishable from each other. In addition, it is possible to prepare magnetically-separable beads that emit fluorescence by adding a magnetically-adsorbable component to the resins. Multiplex polymorphism typing using such magnetic fluorescent beads has been developed (Baiosaiensu To Baioindasutorii (Bioscience & Bioindustry), Vol. 60 No. 12, 821-824).

In the multiplex polymorphism typing using magnetic fluorescent beads, probes having at their end a nucleotide complementary to a polymorphic site of each allele are immobilized to the magnetic fluorescent beads. The two components are combined so that each allele corresponds to each magnetic fluorescent bead with a unique fluorescence signal. On the other hand, fluorescently-labeled oligoDNA having a nucleotide sequence complementary to an region on the allele that is adjacent to the complementary sequence hybridized by the probe immobilized on the magnetic fluorescent bead, is prepared.

A region containing the allele is amplified by asymmetric PCR, and hybridized with the magnetic fluorescent bead-immobilized probe and the fluorescently-labeled oligoDNA, and the two are then ligated. When the end of the magnetic fluorescent bead-immobilized probe has a nucleotide sequence complementary to the nucleotide at the polymorphic site, they are efficiently ligated. Inversely, when the terminal nucleotide is different due to a polymorphism, The efficiency of the ligation between the two is lowered. As a result, the fluorescently-labeled oligoDNA binds with each magnetic fluorescent bead only when the sample has a nucleotide complementary to that of the magnetic fluorescent bead.

The nucleotide is determined by magnetically recovering the magnetic fluorescent beads, and detecting the presence of fluorescently-labeled oligoDNA on the magnetic fluorescent beads. The fluorescence signal can be analyzed for each one of the magnetic fluorescent beads using a flow cytometer. Thus, when a number of different magnetic fluorescent beads are mixed, the signals can be easily separated. Namely, the "multiplexing", in which a number of different polymorphic sites are analyzed in parallel in a single reaction vessel, is achieved.

### [Invader Method]

Non-PCR-dependent genotyping methods have also been in practical use. For example, the invader method achieves the determination of nucleotide types using only a special nuclease called "cleavase" and three different oligonucleotides: allele probe, invader probe, and FRET probe. Of these probes, only the FRET probe needs to be labeled.

The allele probe is designed to hybridize with a region adjacent to an allele to be detected. The 5'-side of the allele probe is linked with a flap having a nucleotide sequence not involved in hybridization. The allele probe has a structure such that it is hybridized with the 3'-side of a polymorphic site and is linked to the flap on the polymorphic site.

On the other hand, the invader probe includes a nucleotide sequence which hybridizes with the 5'-side of the polymorphic site. The nucleotide sequence of the invader probe is designed so that its 3'-end corresponds to the polymorphic site as a result of hybridization. The invader probe may have an arbitrary nucleotide at the position corresponding to the polymorphic site. Namely, the nucleotide sequences of the invader probe and the allele probe are designed so that they become adjacent to each other across the polymorphic site upon hybridization.

When the polymorphic site has a nucleotide complementary to the nucleotide sequence of the allele probe, both the invader probe and the allele probe hybridize with the allele and then form a structure in which the invader probe invades the nucleotide of the allele probe corresponding to the polymorphic site. In the oligonucleotides which have formed the invasion structure as above, the cleavase cleaves the strand that has been invaded. Since the cleavage occurs on the invasion structure, it results in removal of the flap of the allele probe. On the other hand, when the nucleotide of the polymorphic site is not complementary to the nucleotide of the allele probe, no competition occurs between the invader probe and the allele probe, and no invasion structure is formed. Accordingly, the flap is not cleaved by the cleavase.

The FRET probe is for detecting the flap thus separated. The FRET probe has a self-complementary sequence on its 5'-end side and forms a hairpin loop in which a single-stranded portion is present in its 3'-end side. The single-stranded portion in the 3'-end side of the FRET probe has a nucleotide sequence complementary to the flap, and the flap can hybridize with this portion. The nucleotide sequences of the flap and the FRET probe are designed so that the flap hybridizes with the FRET probe and forms a structure in which the 3'-end of the flap invades the 5'-end portion of the self-complementary sequence of the FRET probe. The cleavase recognizes and cleaves the invasion structure. By labeling the FRET probe with a reporter dye and a quencher, which are similar to those in the TaqMan PCR, at the positions that sandwich the region to be cleaved by the cleavase, the cleavage of the FRET probe can be detected as a change in fluorescence signal.

Theoretically, uncleaved flaps should also hybridize with the FRET probe. However, in fact, the FRET-binding efficiency is largely different between cleaved flaps and flaps in the form of allele probes. Accordingly, cleaved flaps can be specifically detected by using FRET probes.

To determine nucleotides based on the invader method, two different allele probes having nucleotide sequences complementary to allele A and allele B, respectively, may be prepared. In this case, the flaps of the two have different nucleotide sequences. By preparing two different FRET probes for detecting the flaps with distinguishable reporter dyes, nucleotides can be determined in the same manner as in TaqMan PCR.

An advantage of the invader process is that the labeling is necessary only to the oligonucleotide of the FRET probe. The oligonucleotide of the FRET probe may be the same regardless of nucleotide sequences to be detected. Accordingly, mass production is possible. On the other hand, the allele probe and the invader probe do not need to be labeled. After all, the reagents for genotyping can be produced at low cost.

### [RCA]

Non-PCR-dependent methods for determining nucleotide sequences include rolling circle amplification (RCA). The rolling circle amplification (RCA) is a process of amplifying DNA based on a reaction in which a DNA polymerase having a strand displacing activity synthesizes a long complementary strand using a cyclic single-stranded DNA as a template (Lizardri PM et al., Nature Genetics 19, 225, 1998). In the RCA, an amplification reaction is constituted using a primer which anneals to a cyclic DNA to initiate complementary strand synthesis, and a second primer which anneals to a long complementary strand formed by the former primer.

The RCA uses a DNA polymerase having a strand displacing activity. Accordingly, a double-stranded portion formed by the complementary strand synthesis is displaced as a result of a complementary strand synthesis reaction initiated by another primer annealed to a further 5' region. For example, a complementary strand synthesis reaction using cyclic DNA as a template does not complete by one cycle. The complementary strand synthesis continues while displacing a previously synthesized complementary strand, and produces a long single-stranded DNA. Meanwhile, the second primer anneals to the long single-stranded DNA produced from the template cyclic DNA, and initiates a complementary strand synthesis. In the RCA method, since the single-stranded DNA is produced using a cyclic DNA as a template, it has repeated nucleotide sequence of the identical nucleotide sequence. Accordingly, the continuous production of a long single strand leads to continuous annealing of the second primer. As a result, single-stranded portions to which the primer can anneal are continuously produced without a degeneration step. DNA amplification is thus achieved.

When cyclic single-stranded DNAs necessary for RCA are prepared depending on the nucleotide types of polymorphic sites, the nucleotide types can be determined using RCA. To this end, a padlock probe, which is a single linear strand, is used. The padlock probe has at its 5'- and 3'-ends nucleotide sequences that are complementary to both sides of a polymorphic site to be detected. These nucleotide sequences are linked by a portion called "backbone", which is composed of a special nucleotide sequence. If the polymorphic site has a nucleotide sequence complementary to the ends of the padlock probe, the ends that have hybridized to the allele can be ligated by a DNA ligase. As a result, the linear padlock probe is cyclized, and an RCA reaction is triggered. The efficiency of the DNA ligase reaction is significantly reduced when the ends to be ligated is not completely complementary. Accordingly, the nucleotide can be determined by detecting the presence or absence of the ligation using the RCA method.

The RCA method can amplify DNA, but does not yield a signal as it is. In addition, when only the presence or absence of amplification is used as an index, the reaction must be conducted for every allele to determine the nucleotide type. There are known methods in which these points are improved for nucleotide determination. For example, molecular beacons can be used to determine nucleotide types in a single tube based on the RCA method. The molecular beacon is a signal-generating probe using a fluorescent dye and a quencher as in the TaqMan method. The molecular beacon includes complementary nucleotide sequences at the 5'-end and 3'-end and forms a hairpin structure by itself. When the vicinities of the two ends are labeled with a fluorescent dye and a quencher, a fluorescence signal is not detectable from the molecular beacon forming a hairpin structure. The molecular beacon in which a part thereof is designed as a nucleotide sequence complementary to an RCA amplified product hybridizes to the RCA amplified product. The hairpin structure is resolved as a result of hybridization, and a fluorescence signal is produced.

An advantage of such molecular beacons is that a common nucleotide sequence can be used in the molecular beacons, regardless of subjects to be detected, by using the nucleotide sequence of the backbone portion of the padlock probe. When different backbone nucleotide sequences are used for different alleles, and two molecular beacons having different fluorescence wavelengths are used in combination, nucleotide types can be determined in a single tube. Since the cost for synthesizing fluorescently-labeled probes is high, it is an economical advantage that a common probe can be used regardless of subjects to be assayed.

These methods have been developed for rapid genotyping of a large quantity of samples. All methods but MALDI-TOF/MS generally require the preparation of labeled probes and such in any way. In contrast, nucleotide typing methods that do not depend on labeled probes and such have long been performed. Examples of such methods include methods based on restriction fragment length polymorphisms (RFLP) and the PCR-RFLP method.

The RFLP method is based on the fact that a mutation in a recognition site of a restriction enzyme, or an insertion or deletion of nucleotides in a DNA fragment yielded by restriction enzyme treatment, can be detected as a change in the size of the fragment formed after the restriction enzyme treatment. If there is a restriction enzyme that recognizes a nucleotide sequence having a polymorphism to be detected, the nucleotide at the polymorphic site can be identified according to the principle of RFLP.

Methods of detecting a difference in nucleotides using a change in the secondary structure of a DNA as an index are also known as methods requiring no labeled probe. PCR-SSCP is based on the fact that the secondary structures of single-stranded DNAs reflect differences in their nucleotide sequence (Cloning and polymerase chain reaction-single-strand conformation polymorphism analysis of anonymous Alu repeats on chromosome 11. Genomics. 1992 Jan 1; 12(1): 139-146., Detection of p53 gene mutations in human brain tumors by single-strand conformation polymorphism analysis of polymerase chain reaction products. Oncogene. 1991 Aug 1; 6(8): 1313-1318., Multiple fluorescence-based PCR-SSCP analysis with postlabeling., PCR Methods Appl. 1995 Apr 1; 4(5): 275-282.). The PCR-SSCP method is conducted by the steps of dissociating a PCR product into single-stranded DNAs, and separating them on a non-denaturing gel. Since the mobility of DNA on the gel varies depending on the secondary structure of single-stranded DNA, a nucleotide difference at the polymorphic site can be detected as a difference in mobility.

Another example of the methods requiring no labeled probe is denaturant gradient gel electrophoresis (DGGE). DGGE is a method in which a mixture of DNA fragments is electrophoresed in a polyacrylamide gel with a gradient of denaturant concentration, and the DNA fragments are separated depending on the difference in their instability. When an unstable DNA fragment having a mismatch moves to a position at a certain denaturant concentration in the gel, a DNA sequence around the mismatch is partially dissociated to single strands due to its instability. The mobility of the partially denatured DNA fragment is much slower and differs from that of a complete double-stranded DNA having no dissociated portion. Thus, the two fragments can be separated from each other.

Specifically, a region having a polymorphic site is initially amplified by PCR or such. A probe DNA with a known nucleotide sequence is allowed to hybridize with the amplified product to form a double-strand. This is electrophoresed in a polyacrylamide gel with a gradually increasing concentration of a denaturant such as urea, and is compared with a control. A DNA fragment that has a mismatch as a result of hybridization with the probe DNA is dissociated to single strands in a portion at a lower concentration of the denaturant and then shows a markedly slow mobility. The presence or absence of the mismatch can be detected by detecting the difference in mobility thus occurred.

In addition, nucleotides can also be determined by using DNA arrays (Saibo Kogaku Bessatsu "DNA Maikuroarei To Saishin PCR-ho" (Cell Technology Suppl., "DNA Microarray and Latest PCR Techniques"), Shujunsha Co., Ltd., published on April 20, 2000, pp.97-103 "OligoDNA Chippu Niyoru SNP No Bunseki (SNP Analyses with OligoDNA Chips)", Shin-ichi Kajie). In a DNA array, a sample DNA (or RNA) is allowed to hybridize with many probes arrayed in one plate, and the plate is then scanned to detect the hybridizations with the probes. Since reactions on many probes can be observed simultaneously, such DNA arrays are useful, for example, to analyze many polymorphic sites simultaneously.

DNA arrays generally are generally composed of thousands of nucleotides densely printed on a substrate. Normally, these DNAs are printed on a surface layer of a non-porous substrate. The surface layer of the substrate is generally made of glass, but a porous membrane such as a nitrocellulose membrane can also be used.

In the present invention, an example of techniques for immobilizing (arraying) nucleotides is oligonucleotide-based arrays developed by Affymetrix, Inc. In oligonucleotide arrays, the oligonucleotides are generally synthesized *in vitro.* For example, *in situ* oligonucleotide synthesis methods are known, such as photolithographic techniques (Affymetrix, Inc.), and inkjet techniques for immobilizing chemical compounds (Rosetta Inpharmatics LLC). Any of these techniques can be used for preparing substrates used in the present invention.

The oligonucleotides are composed of nucleotide sequences complementary to regions containing SNPs to be detected. The length of nucleotide probes to bind with the substrate is, when they are oligonucleotides, generally 10 to 100 bases, preferably 10 to 50 bases, and more preferably 15 to 25 bases. In addition, the DNA array method generally uses a mismatch (MM) probe to avoid errors due to cross-hybridization (non-specific hybridization). The mismatch probe constitutes a pair with an oligonucleotide having a nucleotide sequence completely complementary to a target nucleotide sequence. The oligonucleotide that is composed of a nucleotide sequence completely complementary to a target nucleotide sequence is called a perfect match (PM) probe. The influence of cross-hybridization can be reduced by erasing signals observed with the mismatch probe in data analyzing processes.

Samples for genotyping by the DNA array method can be prepared based on biological samples collected from subjects, according to known methods to one skilled in the art. The biological samples are not particularly limited. For example, DNA samples can be prepared from chromosomal DNA extracted from tissues or cells such as blood, peripheral blood leucocytes, skin, or oral mucosa; tears, saliva, urine, faeces, or hair, of the subjects. A particular region of the chromosomal DNA is amplified using primers for amplifying a region having a polymorphic site to be determined. In this step, multiple regions can be amplified simultaneously by using multiplex PCR. The multiplex PCR is a PCR method in which multiple sets of primers are used in one reaction solution. The multiplex PCR is useful to analyze multiple polymorphic sites.

In the DNA array method, a DNA sample is amplified by PCR and the amplified product is labeled. Labeled primers are used for labeling the amplified product. For example, initially, a genomic DNA is amplified by PCR with a set of primers specific to a region containing a polymorphic site. Next, a biotin-labeled DNA is synthesized by labeling PCR using a biotin-labeled primer. The biotin-labeled DNA thus synthesized is allowed to hybridize with oligonucleotide probes on a chip. The reaction solution and conditions of hybridization can be suitably adjusted according to conditions such as the length of nucleotide probes immobilized on the substrate, and reaction temperature. One skilled in the art can appropriately design hybridization conditions. Fluorescent dye-labeled avidin is added to detect a hybridized DNA. The array is analyzed with a scanner, and the presence or absence of hybridization is detected using fluorescence as an index.

The above-mentioned process will be illustrated more specifically. Initially, DNA containing a polymorphic site of the present invention is prepared, and a solid phase to which nucleotide probes are immobilized is obtained. Next, the DNA is contacted with the solid phase. Further, DNA hybridized with the nucleotide probes immobilized on the solid phase is detected to determine the nucleotide type at the polymorphic site of the present invention.

The term "solid phase" as used in the context of the present invention refers to a material to which a nucleotide can be immobilized. The solid phase used in the present invention is not particularly limited, so long as a nucleotide can be immobilized thereto. Specific examples thereof include solid phases including microplate wells, plastic beads, magnetic particles, and substrates. In the present invention, a substrate generally used in DNA array techniques is preferably used as a solid phase. The term "substrate" in the present invention means a plate-like material to which a nucleotide can be immobilized. In the present invention, the nucleotide includes oligonucleotides and polynucleotides.

In addition to the above-mentioned methods, the allele-specific oligonucleotide (ASO) hybridization method can be used to detect a nucleotide at a specific site. The allele-specific oligonucleotide (ASO) is composed of a nucleotide sequence that hybridizes with a region containing a polymorphic site to be detected. When the ASO is hybridized with a sample DNA and a mismatch occurs at the polymorphic site due to a polymorphism, the hybridization efficiency is lowered. The mismatch can be detected by Southern blotting or a method using a special fluorescent reagent which is quenched when intercalating to a gap in a hybrid. The mismatch can also be detected by the ribonuclease A mismatch cleavage method.

Oligonucleotides that hybridize with DNA containing a polymorphic site of the present invention and have at least a 15-nucleotide chain length are usable as a reagent (testing agent) for testing whether a subject has a risk factor for arteriosclerotic diseases. These are used in tests in which the gene expression is used as an index, or tests using gene polymorphism as an index.

The oligonucleotides specifically hybridize with DNA having a polymorphic site of the present invention. The phrase "specifically hybridizes" as used herein means that significant cross-hybridizations do not occur with DNAs encoding other proteins under usual hybridization conditions, preferably under stringent hybridization conditions (for example, conditions stated by Sambrook et al., Molecular Cloning, Cold Spring Harbour Laboratory Press, New York, USA, 2nd Ed. 1989). When specific hybridization is possible, the oligonucleotide does not need to be completely complementary to the nucleotide sequence containing a polymorphic sites of the present invention in a gene to be detected or in an adjacent DNA region of the gene.

Examples of hybridization conditions in the present invention include "2x SSC, 0.1% SDS, 50°C", "2x SSC, 0.1% SDS, 42°C", and "1x SSC, 0.1% SDS, 37°C"; and as more stringent conditions, "2x SSC, 0.1% SDS, 65°C", "0.5x SSC, 0.1% SDS, 42°C", and "0.2x SSC, 0.1% SDS, 65°C". More specifically, as a process using the Rapid-hyb Buffer (Amersham Life Science), hybridization can be carried out by conducting prehybridization at 68°C for 30 minutes or more; adding probes to form hybrids while maintaining at 68°C for one hour or more; thereafter carrying out three times of washing in 2x SSC, 0.1 % SDS at room temperature for 20 minutes; subsequently carrying out three times of washing in 1x SSC, 0.1% SDS at 37°C for 20 minutes; and finally carrying out two times of washing in 1x SSC, 0.1% SDS at 50°C for 20 minutes. Hybridization can also be conducted, for example, by carrying out prehybridization in the Expresshyb Hybridization Solution (CLONTECH) at 55°C for 30 minutes or more; adding labeled probes and incubating at 37°C to 55°C for one hour or more; carrying out three times of washing in 2x SSC, 0.1% SDS at room temperature for 20 minutes; and carrying out washing once in 1x SSC, 0.1% SDS at 37°C for 20 minutes. More stringent conditions are available, for example, by setting the temperatures of prehybridization, hybridization and/or the second washing at higher levels. For example, the temperatures of prehybridization and hybridization can be set to 60°C, and, as more stringent conditions, to 68°C. One skilled in the art can set the conditions in consideration of, in addition to these conditions such as salt concentrations of buffers and temperatures, other conditions such as concentrations, lengths, and nucleotide sequence structures of probes, and reaction times.

The oligonucleotide can be used as a probe or primer in the above testing method according to the present invention. The length of the oligonucleotide, if used as a primer, is generally 15 bp to 100 bp, and preferably 17 bp to 30 bp. The primer is not particularly limited, so long as it can amplify at least a part of a DNA having a polymorphic site of the present invention.

The present invention provides a primer for amplifying a region having a polymorphic site of the present invention, and a probe that hybridizes with a DNA region containing the polymorphic site.

Such primers for amplifying a region having the polymorphic site of the present invention also include primers that can initiate complementary strand synthesis toward the polymorphic site using as a template a DNA containing the polymorphic site. The primers can be described as primers for imparting an origin of replication to the 3' side of a polymorphic site in a DNA containing the polymorphic site. The distance between the polymorphic site and the region with which the primer hybridizes is arbitrary. As the distance between them, a suitable number of nucleotides can be selected according to the technique for analyzing the nucleotide at the polymorphic site. For example, when the primer is a primer for analysis using DNA chips, it is possible to design the primer to yield an amplification product having a length of 20 to 500, generally 50 to 200 nucleotides as a region that includes the polymorphic site. One skilled in the art can design a primer according to the analysis technique based on nucleotide sequence information on an adjacent DNA region containing the polymorphic site. The nucleotide sequence constituting the primer according to the present invention can be not only a nucleotide sequence completely complementary to a genomic nucleotide sequence but also suitable modifications thereof.

The primer according to the present invention can be added with arbitrary nucleotide sequences, in addition to nucleotide sequences complementary to a genomic nucleotide sequence. For example, primers added with a type IIs restriction enzyme-recognition sequence are used in primers for a method of analyzing polymorphisms using a type IIs restriction enzyme. Such primers with modified nucleotide sequences are also included in the primers for use in the present invention. In addition, the primers for use in the present invention can be modified. For example, primers labeled with fluorescent substance or substance with binding affinity, such as biotin or digoxin, are used in various genotyping methods. These modified primers are also included within the present invention.

On the other hand, the phrase "probe that hybridizes with a region containing a polymorphic site" in the present invention refers to probes that can hybridize with a polynucleotide that has a nucleotide sequence of a region containing a polymorphic site. More specifically, a probe having a polymorphic site in its nucleotide sequence is a preferably probe for use in the present invention. Alternatively, a probe may be designed to have an end corresponding to a nucleotide (base) adjacent to a polymorphic site in some methods of analyzing the nucleotide at the polymorphic site. Accordingly, a preferred probe in the present invention includes a probe which does not contain a polymorphic site in its nucleotide sequence but contains a nucleotide sequence complementary to a neighboring region to the polymorphic site.

In other words, a probe that can hybridize with a polymorphic site of the present invention on a genomic DNA or with a neighboring site to the polymorphic site is preferable as a probe for use in the present invention. Such probes according to the present invention can be subjected to alternations in nucleotide sequence, addition of nucleotide sequence, or modification, as in the primers. For example, probes for use in the Invader process are added with a nucleotide sequence which constitutes a flap and does not relate to the genome. Such probes are also included in the probe of the present invention, so long as they hybridize with a region containing a polymorphic site. The nucleotide sequence constituting a probe according to the present invention can be designed according to the analysis method based on nucleotide sequence of a DNA region neighboring the present invention's polymorphic site on the genome.

Primers or probes according to the present invention can be synthesized by arbitrary methods based on the nucleotide sequence constituting the same. In a primer or probe according to the present invention, the length of a nucleotide sequence complementary to a genomic DNA is usually 15 to 100, generally 15 to 50, and usually 15 to 30. Procedures of synthesizing oligonucleotides having a given nucleotide sequence, based on the given nucleotide sequence, have been known. In addition, it is possible in the synthesis of oligonucleotides to introduce arbitrary modifications to the oligonucleotides using nucleotide derivatives modified with, for example, fluorescent dye or biotin. Procedures of binding synthesized oligonucleotides with, for example, fluorescent dye has also been known.

The oligonucleotides according to the present invention, when used as a probe, may be suitably labeled with, for example, a radioisotope or a nonradioactive compound. When used as a primer, it is possible, for example, to design its structure so that the 3'-end region of the oligonucleotide is complementary to a target sequence and so that a restriction enzyme-recognition sequence, a tag, or the like is added to the 5'-end of the oligonucleotide. Such a polynucleotide having a nucleotide sequence composed of at least 15 successive nucleotides can hybridize with an mRNA of the AGTRL1 or PRKCH gene.

The oligonucleotides according to the present invention may contain a nucleotide (base) other than naturally-occurring nucleotides, according to necessity, the examples of which include 4-acetylcytidine, 5-(carboxyhydroxymethyl)uridine,2'-O-methylcytidine, 5-carboxymethylaminomethyl-2-thiouridine, 5-carboxymethylaminomethyluridine, dihydrouridine, 2'-O-methylpseudouridine, β-D-galactosylqueuosine, 2'-O-methylguanosine, inosine, N6-isopentenyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, β-D-mannosylqueuosine, 5-methoxycarbonylmethyl-2-thiouridine, 5-methoxycarbonylmethyluridine, 5-methoxyuridine, 2-methylthio-N6-isopentenyladenosine, N-((9-β-D-ribofuranosyl-2-methylriopurine-6-yl)carbamoyl)threonine, N-((9-β-D-ribofuranosylpurine-6-yl)N-methylcarbamoyl)threonine, uridine-5-oxyacetic acid-methylester, uridine-5-oxyacetic acid, wybutoxosine, pseudouridine, queuosine, 2-thiocytidine, 5-methyl-2-thiouridine, 2-thiouridine, 4-thiouridine, 5-methyluridine, N-((9-β-D-ribofuranosylpurine-6-yl)carbamoyl)threonine, 2'-O-methyl-5-methyluridine, 2'-O-methyluridine, wybutosine, and 3-(3-amino-3-carboxypropyl)uridine.

The present invention also provides a reagent (testing agent) for use in a method of testing whether the subject has a risk factor for arteriosclerotic diseases. The reagent according to the present invention includes the primer and/or probe according to the present invention as described above. In testing whether the subject has a risk factor for arteriosclerotic diseases, a primer and/or a probe for amplifying a DNA containing a polymorphic site of the invention is used.

The reagent according to the present invention can be combined with, for example, various enzymes, enzyme substrates, and buffers according to the nucleotide typing process. Examples of the enzymes include enzymes necessary for the various analysis processes exemplified as the nucleotide typing process, such as DNA polymerase, DNA ligase, or IIs restriction enzyme. As the buffer, suitable buffers for maintaining the activity of the enzymes used in the analysis is suitably selected. As the enzyme substrate, for example, a substrate for complementary strand synthesis is used.

A control composed of a known nucleotide at the polymorphic site may be attached to the reagent of the present invention. The control can be a genome or a genomic fragment whose nucleotide type at the polymorphic site is already identified. The genome may be an extract from a cell; and a cell or cell fraction can also be used. When cells are used as the control, it is possible to prove that the extraction procedure of a genomic DNA is conducted suitably, based on the result of the control. Alternatively, the DNA having a nucleotide sequence that includes the polymorphic site may be used as the control. Specifically, a YAC vector and a BAC vector containing a DNA derived from a genome and having a known nucleotide type at the polymorphic site of the present invention are useful as the control. It is also possible to use, as the control, a vector prepared by splicing only several hundreds of bases corresponding to the polymorphic site and inserting the bases into a vector.

Another embodiment of the reagent of the present invention is a reagent for testing whether the subject has a risk factor for arteriosclerotic diseases, which includes a nucleotide probe immobilized on the solid phase, in which the nucleotide probe hybridizes with a DNA comprising a polymorphic site of the present invention.

These reagents find utility in the context of tests that use an index the polymorphic site of the present invention. Methods for preparing these are as mentioned above.

A preferred embodiment of the present invention relates to a method for testing whether the subject has a risk factor for arteriosclerotic diseases, which includes the step of detecting a transcription or translation product of the AGTRL1 or PRKCH gene.

Accordingly, oligonucleotides usable as a probe in the detection of a transcription product of the AGTRL1 or PRKCH gene in the testing method, such as oligonucleotides that hybridize with the transcription product of the AGTRL1 or PRKCH gene, is one example of testing reagents according to the present invention.

In addition, antibodies that recognize an AGTRL1 or PRKCH protein (anti-AGTRL1 protein antibody or anti-PRKCH protein antibody) and is usable in the detection of a translation product of the AGTRL1 or PRKCH gene in the testing method is also a preferred example of testing reagents according to the present invention.

The present inventors elucidated that enhanced expression of the AGTRL1 gene is associated with arteriosclerotic diseases. They also showed that autophosphorylation of the PRKCH protein is associated with arteriosclerotic diseases such as cerebral infarction. Therefore, substances that suppress the expression of the AGTRL1 or PRKCH gene or the function (activity) of a protein encoded by the gene may become therapeutic or preventive agents for arteriosclerotic diseases.

The present invention provides agents for treating arteriosclerotic diseases that include, as an active ingredient, substances that inhibit the expression of AGTRL1 or PRKCH protein, or substances that inhibit the function (activity) of a protein encoded by the gene (AGTRL1 or PRKCH protein).

In a preferred embodiment, the present invention initially provides agents for treating arteriosclerotic diseases (agent and/or pharmaceutical composition for treating or preventing arteriosclerotic diseases), which includes, as an active ingredient, an expression inhibitor for the expression of AGTRL1 or PRKCH gene.

The AGTRL1 or PRKCH gene expression inhibitor in the present invention includes, for example, substances that inhibit the transcription of AGTRL1 or PRKCH or the translation of the transcription product. Examples of preferred embodiments of the above expression inhibitors in the present invention include compounds (nucleic acids) selected from the group consisting of (a) to (c) below:
(a) an antisense nucleic acid to a transcription product of the AGTRL1 or PRKCH gene or a part thereof;
(b) a nucleic acid having ribozyme activity of specifically cleaving a transcription product of the AGTRL1 or PRKCH gene; and
(c) a nucleic acid having an action of inhibiting expression of the AGTRL1 or PRKCH gene through an RNA interference effect.

The term "nucleic acid" in the present invention means an RNA or a DNA. The "nucleic acid" in the present invention also includes chemically synthesized nucleic acid analogues such as a so-called PNA (peptide nucleic acid). PNA has a three-dimensional structure closely resembling that of a nucleic acid, and has a polyamide skeleton having glycine units instead of the pentose-phosphate skeleton of the nucleic acid as a basic skeleton structure.

As methods for inhibiting the expression of specific endogenous genes, a method using an antisense technique is well known to one skilled in the art. As actions for an antisense nucleic acid to inhibit the expression of a target gene, there are multiple factors as mentioned below. Specifically, examples of such actions include: inhibition of transcription initiation due to triplex formation; transcription inhibition due to hybridization with a site in which a local open loop structure is formed by RNA polymerase; transcription inhibition due to hybridization with RNA which is under synthesis; inhibition of splicing due to hybridization with an intron-exon junction; inhibition of splicing due to hybridization with a spliceosome-forming site; inhibition of translocation from the nucleus to the cytoplasm due to hybridization with mRNA; inhibition of splicing due to hybridization with a capping site or a poly(A) addition site; inhibition of translation initiation due to hybridization with translation initiation factor-binding site; inhibition of translation due to hybridization with a ribosome-binding site in the vicinity of initiation codon; inhibition of peptide chain elongation due to hybridization with a coding region or polysome-binding site of mRNA; and inhibition of gene expression due to hybridization with an interaction site between nucleic acid and protein. Thus, antisense nucleic acid inhibits the expression of a target gene by inhibiting various processes, such as transcription, splicing, or translation (Hirashima and Inoue, Shin Seikagaku Jikken Koza 2 Kakusan IV Idenshi No Fukusei To Hatsugen (Experimental Biochemistry, New Ed., 2, Nucleic Acid IV, Replication and Expression of Genes), edited by The Japanese Biochemical Society, TOKYO KAGAKU DOJIN CO., LTD., 1993, 319-347.).

The antisense nucleic acid for use in the present invention may inhibit the expression of AGTRL1 or PRKCH gene by any of the above-mentioned actions. As one embodiment, it may be effective for inhibiting gene translation to design an antisense sequence to be complementary to a noncoding region adjacent to the 5'-end of the mRNA of the AGTRL1 or PRKCH gene. In addition, sequences complementary to a coding region or to a noncoding region at the 3' side can also be used. Thus, antisense nucleic acids for use in the present invention also include nucleic acids having an antisense sequence to not only a sequence of a coding region of the AGTRL1 or PRKCH gene, but also to a sequence of a noncoding region of the AGTRL1 or PRKCH gene. The antisense nucleic acid to be used is linked downstream of a suitable promoter, and a sequence containing a transcription terminator signal is preferably linked to the 3' side. Nucleic acids thus prepared can be used to transform a desired animal according to known methods. The sequence of the antisense nucleic acid is preferably a sequence complementary to endogenous AGTRL1 or PRKCH gene of the animal to be transformed, or a part thereof. However, the sequence may not be completely complementary, so long as the gene expression can be effectively inhibited. A transcribed RNA has a complementarity of preferably 90% or more, and most preferably 95% or more to a transcription product of the target gene. To effectively inhibit the expression of the target gene (AGTRL1 or PRKCH) using an antisense nucleic acid, the length of the antisense nucleic acid is preferably at least 15 nucleotides or more and less than 25 nucleotides, but the length of the antisense nucleic acid for use in the present invention is not necessarily limited thereto.

The antisense for use in the present invention is not particularly limited; however, it can be prepared, for example, based on the nucleotide sequence of SEQ ID NO: 1 or 2.

Expression of AGTRL1 or PRKCH gene may be inhibited by using ribozymes, or the DNA encoding ribozymes. The term "ribozyme" refers to RNA molecules having catalytic activity. There are ribozymes having a variety of activities. As a result of studies focusing, of such ribozymes, on ribozymes as enzymes that cleave RNA, it becomes possible to design ribozymes that site-specifically cleave RNA. Of ribozymes, some have a size of 400 nucleotides or more, such as Group I intron-type ribozymes and M1 RNA belonging to RNase P, others have an active domain of about 40 nucleotides, called hammerhead or hairpin ribozymes (Makoto Koizumi and Eiko Otsuka, Tanpakushitsu, Kakusan Koso (PROTEIN, NUCLEIC ACID, AND ENZYME), 1990, 35, 2191.).

For example, self-cleaving domain of the hammerhead ribozyme cleaves the 3'-side of C15 in a sequence of G13U14C15; the formation of a base pair of U14 and A9 is considered to be important to its activity; and cleavage can occur at A15 or U 15 instead of C 15 (Koizumi, M. et al., FEBS Lett, 1988, 228, 228.). By designing a ribozyme having a substrate-binding site complementary to an RNA sequence adjacent to a target site, an RNA-cleaving ribozyme that recognizes UC, UU, or UA in the target RNA and acts in a restriction enzyme-like manner can be prepared (Koizumi, M. et al., FEBS Lett, 1988, 239, 285., Makoto Koizumi and Eiko Otsuka, Tanpakushitsu, Kakusan Koso (PROTEIN, NUCLEIC ACID, AND ENZYME), 1990, 35, 2191., Koizumi, M. et al., Nucl Acids Res, 1989, 17, 7059.).

The hairpin ribozyme is also useful for the objects of the present invention. This ribozyme is found, for example, in the minus strand of satellite RNA of tobacco ringspot virus (Buzayan, JM., Nature, 1986, 323, 349.). There has been shown that target-specific RNA-cleaving ribozyme can also be prepared from hairpin ribozyme (Kikuchi, Y. & Sasaki, N., Nucl Acids Res, 1991, 19, 6751., Yo Kikuchi, Kagaku To Seibutsu (Chemistry and Biology), 1992, 30, 112.). Thus, by specifically cleaving transcription products of the AGTRL1 or PRKCH gene in the present invention using ribozymes, expression of the gene can be suppressed.

In addition, suppression of the expression of endogenous gene can also be conducted through RNA interference (RNAi) using double-stranded RNA containing a sequence identical or similar to the sequence of a target gene. Nucleic acids having inhibitory action due to RNAi effect for use in the present invention is generally also called as siRNA. RNAi is a phenomenon in which a double-stranded RNA is introduced into, for example, a cell to induce the destruction of a mRNA of the target gene to thereby suppress the expression of the target gene, in which the double-stranded RNA contains a sense RNA having a sequence identical to the mRNA of the target gene and an antisense RNA having a sequence complementary thereto. Since RNAi can thus suppress the expression of the target gene, it receives attention as an easy and convenient gene knock-out method as an alternative to conventional complicated, inefficient gene destroying techniques through homologous recombination; or as a method applicable to gene therapies. RNA for use in RNAi does not necessarily need to be completely identical to AGTRL1 or PRKCH gene or a partial region of the gene, but preferably has complete homology.

A preferred embodiment of the above nucleic acid (c) in the present invention includes double-stranded RNA (siRNA) having an RNAi (RNA interference) effect to AGTRL1 or PRKCH gene. More specifically, it includes double-stranded RNA (siRNA) composed of a sense RNA and an antisense RNA to a partial sequence of the nucleotide sequence of SEQ ID NO: 1.

Although the details of the RNAi mechanism remains unrevealed, it is considered that an enzyme called DICER (one type of the RNase III nuclease family) comes in contact with the double-stranded RNA, and the double-stranded RNA is decomposed into small fragments called small interfering RNAs or siRNAs. Double-stranded RNAs having RNAi effect in the present invention also include double-stranded RNA before the decomposition by DICER. Namely, even RNAs with such a long strand as not to exhibit RNAi effect in its original length is expected to be decomposed in a cell into siRNAs having RNAi effect, and thus, the length of the double-stranded RNA in the present invention is not particularly limited.

For example, a long double-stranded RNA corresponding to a region of the full-length or substantially full-length of the mRNA of AGTRL1 or PRKCH gene of the present invention may be decomposed beforehand with DICER, and the decomposition product thereof may be used as a therapeutic agent for arteriosclerotic diseases. The decomposition product is expected to contain double-stranded RNA molecules having RNAi effect (siRNA). Following to this method, there is no need to particularly select a region on mRNA expected to have RNAi effect. Thus, it is not always necessary to precisely specify a region on the mRNA of AGTRL1 or PRKCH gene of the present invention which has RNAi effect.

Of the above described RNA molecules, those that have a conformation in which one end is closed, such as an siRNA having hairpin structure (shRNA), is also included within the present invention. Namely, single stranded RNA molecules that can intramolecularly form a double-stranded RNA structure is also included within the present invention.

The above "double-stranded RNA that can be suppressed through RNAi effect" for use in the present invention can be appropriately produced by one skilled in the art based on the nucleotide sequence of the AGTRL1 or PRKCH gene of the present invention as a target for the double-stranded RNA. For example, the double-stranded RNA for use in the present invention can be produced based on the nucleotide sequence of SEQ ID NO: 1 or 2. Namely, based on the nucleotide sequence of SEQ ID NO: 1 or 2, it is within the range of usual trials for one skilled in the art to suitably select an arbitrary consecutive RNA region of the mRNA, which is the transcription product of the sequence, and prepare a double-stranded RNA corresponding to the selected region. In addition, the selection of siRNA sequence having stronger RNAi effect from among the mRNA sequences, which are the transcription products of the sequence, can be suitably conducted by one skilled in the art according to known methods. If one of the two strands (for example, the nucleotide sequence of SEQ ID NO: 1 or 2) has been identified, one skilled in the art can easily know the nucleotide sequence of the other strand (complementary strand). The siRNA can be suitably produced by one skilled in the art using commercially available nucleic acid synthesizers. In addition, a general contract synthesis for customers can be used for the synthesis of desired RNA.

A DNA (vector) that can express the above-mentioned RNA of the present invention is also included in the preferred embodiments of the compounds that can suppress the expression of AGTRL1 or PRKCH gene of the present invention. For example, a DNA (vector) that can express the above double-stranded RNA according to the present invention is a DNA having a structure in which a DNA encoding one strand of the double-stranded RNA, and a DNA encoding the other strand of the double-stranded RNA are linked with promoters so that they can each be expressed. The above mentioned DNA of the present invention can be suitably produced by one skilled in the art according to general genetic engineering techniques. More specifically, an expression vector for use in the present invention can be prepared by suitably inserting a DNA encoding the RNA of the present invention into various known expression vectors.

The expression inhibitors of present invention further include compounds that suppress expression of the AGTRL1 or PRKCH gene by binding with, for example, the expression regulatory region (for example, a promoter region) of the AGTRL1 or PRKCH gene. The compound may be obtained, for example, by a screening method using a promoter DNA fragment of the AGTRL1 or PRKCH gene and using the binding activity with the DNA fragment as an index. One skilled in the art can suitably determine whether a desired compound suppresses expression of the AGTRL1 or PRKCH gene of the present invention according to known methods, such as a reporter assay.

In addition, the present inventors demonstrated that expression of the GTRL1 gene is enhanced by polymorphic mutations "SNP4" and "SNP9" in the AGTRL1 gene.

These polymorphic mutations exist in the DNA region that binds to Spl, which is a transcriptional regulator. These polymorphic mutations change the binding activity of the Sp1 transcription factor towards the DNA region and thereby enhance the expression of the AGTRL1 gene.

Therefore, substances that lower the binding activity between the DNA region of the AGTRL1 gene containing the above-mentioned polymorphism and the Sp1 transcription factor are considered to suppress the transcription of the AGTRL1 gene, and may be one of the preferred embodiments of the expression-suppressing substances of the present invention mentioned above. Examples of the above-mentioned DNA regions include DNA regions comprising the polymorphic site "SNP4" or "SNP9".

Furthermore, polynucleotides comprising a DNA sequence that contains a polymorphic mutation of the present invention and has an altered binding activity with the Sp1 transcription factor are useful and can be used suitably, for example, for methods of screening for pharmaceutical agents for treating or preventing atherosclerotic diseases to be described later. For example, the polynucleotides of (a) and (b) below can be used for the purpose of screening for therapeutic agents for arteriosclerotic diseases:
(a) partial polynucleotide fragments of the DNA sequence of SEQ ID NO: 1 which comprises the polymorphic sites located at position 42509 or 39353; and
(b) polynucleotides comprising a nucleotide sequence having one or more nucleotide additions, deletions, or substitutions in the sequence of the polynucleotide of (b), in which the binding ability with Sp1 transcription factor is enhanced.

Furthermore, polypeptides comprising a protein encoded by a DNA carrying a polymorphic mutation of the present invention, in which the autophosphorylation activity of the PRKCH protein has been enhanced can be suitably used, for example, in methods of screening for pharmaceutical agents for treating or preventing arteriosclerotic diseases to be described later. For example, the polypeptides of (a) and (b) below can be used for the purpose of screening for therapeutic agents for arteriosclerotic diseases:
(a) a full-length polypeptide or fragments of the PRKCH protein, in which valine at position 374 in the amino acid sequence of the PRKCH protein is substituted with isoleucine; and
(b) a polypeptide comprising a nucleotide sequence having one or more nucleotide additions, deletions, or substitutions in the sequence of the polypeptide of (b), in which the autophosphorylation activity is enhanced.

The present invention also provides therapeutic agents for arteriosclerotic diseases which comprise as an active ingredient a substance that suppresses the function of the AGTRL1 or PRKCH protein.

Substances that suppress the function of the AGTRL1 or PRKCH protein of the present invention include, for example, the compounds of (a) and (b) below:
(a) antibodies that bind to the AGTRL1 or PRKCH protein; and
(b) low-molecular weight compounds that bind to the AGTRL or PRKCH protein.

The present inventors discovered a relationship between the enhanced autophosphorylation activity of the PRKCH protein and arteriosclerotic diseases. Therefore, preferred examples of the function-suppressing substances of the present invention include antibodies or low-molecular compounds that inhibit the increase of the autophosphorylation activity of the PRKCH protein.

An antibody that binds with an AGTRL1 or PRKCH protein (anti-AGTRL1 antibody or anti-PRKCH antibody) can be prepared according to methods known to one skilled in the art. When the antibody is a polyclonal antibody, it can be obtained, for example, in the following manner. Small animals such as rabbit are immunized with natural AGTRL1 or PRKCH protein, or recombinant (recombination) AGTRL1 or PRKCH protein expressed as fusion protein with GST in microorganisms such as *Escherichia coli,* or partial peptides thereof, and the serum is collected from the small animal. Serum is purified, for example, through precipitation with ammonium sulfate, a protein A or protein G column, DEAE ion exchange chromatography, or an affinity column coupled with AGTRL1 or PRKCH protein or synthetic peptide to yield the antibody. When the antibody is a monoclonal antibody, it can be prepared, for example, in the following manner. Small animals such as mice are immunized with AGTRL1 or PRKCH protein or partial peptides thereof; the spleen is extirpated from the mice, and is ground to separate cells; the cells and mouse myeloma cells are fused using reagents such as polyethylene glycol; and clones that produce antibodies that bind with AGTRL1 or PRKCH protein are selected from the resulting fused cells (hybridomas). Next, the obtained hybridomas are intraperitoneally transplanted to a mouse; the ascites is recovered from the mouse; and the obtained monoclonal antibodies are purified, for example, through precipitation with ammonium sulfate, a protein A or protein G column, DEAE ion exchange chromatography, or an affinity column coupled with AGTRL1 or PRKCH protein or synthetic peptides to yield the antibody.

The antibody of the present invention is not particularly limited in its form and includes, in addition to the above-mentioned polyclonal antibody and monoclonal antibody, human antibody, humanized antibody obtained by gene recombination, antibody fragment, and modified antibody thereof, so long as it binds with AGTRL1 or PRKCH protein of the present invention.

The AGTRL1 or PRKCH protein for use as sensitizing antigen for obtaining the antibody in the present invention is not limited in animal species as its origin; however, it is preferably a protein derived from mammals such as mice or humans and is particularly preferably a human-derived protein. Such a human-derived protein can be suitably obtained by one skilled in the art using the gene sequence or amino acid sequence disclosed in the present specification.

The protein for use as sensitizing antigen in the present invention may be an entire protein or a partial peptide of the protein. Examples of the partial peptide of proteins include amino (N) terminal fragment, carboxy (C) terminal fragment, or kinase activity site at a center part, of a protein. The term "antibody" in the present specification means refers to antibodies that react with a full-length protein or a fragment of the protein.

Examples of the antibodies for use in the present invention include polyclonal antibodies, monoclonal antibodies, chimeric antibodies, single stranded antibodies (scFv) (Huston et al. (1988) Proc. Natl. Acad. Sci. USA 85: 5879-83; The Pharmacology of Monoclonal Antibody, Vol. 113, Rosenburg and Moore ed., Springer Verlag (1994) pp. 269-315), humanized antibodies, polyspecific antibodies (LeDoussal et al. (1992) Int. J. Cancer Suppl. 7: 58-62; Paulus (1985) Behring Inst. Mitt. 78: 118-32; Millstein and Cuello (1983) Nature 305: 537-9; Zimmermann (1986) Rev. Physiol. Biochem. Pharmacol. 105: 176-260; VanDijk et al. (1989) Int. J. Cancer 43: 944-9), and antibody fragments such as Fab, Fab', F(ab') 2, Fc, and Fv. These antibodies may be modified, for example, with PEG according to necessity. Antibodies can be configured to be detectable without using a secondary antibody, by preparing a fusion protein with, for example, β-galactosidase, maltose binding protein, glutathione S-transferase (GST), or a green fluorescent protein (GFP). Antibodies can be altered to be detectable and recoverable using, for example, avidin or streptavidin by labeling the antibody with, for example, biotin.

Besides obtaining the above described hybridomas by immunizing animals other than humans with antigens, hybridomas that produce desired human antibodies having binding activity with a protein can be obtained by sensitizing human lymphocytes, such as human lymphocytes infected by EB virus, *in vitro* with the protein, cells expressing the protein, or the lysate thereof; and fusing the sensitized lymphocytes with human-derived myeloma cells having a permanent division potential, such as U266.

Antibodies against the AGTRL1 or PRKCH protein of the present invention is expected to suppress the function of AGTRL1 or PRKCH protein by binding with said protein and to have, for example, a therapeutic or improving effect on arteriosclerotic diseases such as brain infarction. When the obtained antibody is used for administration to humans (antibody therapy), it is preferably a human antibody or a human-type antibody for lowering immunogenicity.

Substances that can inhibit the function of AGTRL1 or PRKCH protein in the present invention further include low molecular weight substances (low molecular weight compounds) that bind with the AGTRL1 or PRKCH protein. The low molecular weight substances that bind with the AGTRL1 or PRKCH protein in the present invention may be natural or artificial compounds. The compounds can be generally prepared or obtained according to methods known to one skilled in the art. The compound of the present invention can also be obtained by screening methods mentioned below.

The above-mentioned low -molecular weight compound that binds with an AGTRL1 or PRKCH protein of (b) includes, for example, compounds having a high affinity for AGTRL1 or PRKCH.

Substances that can inhibit the function of AGTRL1 or PRKCH protein of the present invention include mutant AGTRL1 or PRKCH proteins having dominant-negative property to the AGTRL1 or PRKCH protein. The "mutant AGTRL1 or PRKCH proteins having dominant-negative property to the AGTRL1 or PRKCH protein" refer to proteins having the function of causing the activity of endogenous wildtype protein to disappear or reducing the activity of endogenous wildtype protein by expressing the gene encoding the protein.

Substances (compounds) known to inhibit the function of AGTRL1 or PRKCH protein can be a suitable and specific example of "substances that can suppress the function of GRK5 protein" in the present invention.

The function inhibitor according to the present invention can be suitably obtained by screening methods according to the present invention using the AGTRL1 or PRKCH activity as an index.

Substances that inhibit (suppress) the autophosphorylation activity or protein kinase activity of the PRKCH protein are also useful as a therapeutic agent for arteriosclerotic diseases according to the present invention. Accordingly, the present invention provides a therapeutic agent for arteriosclerotic diseases, such an agent containing as an active ingredient a substance that inhibits the autophosphorylation activity or protein kinase activity of the PRKCH protein.

The present invention further provides methods of screening for an agent (candidate compound) for treating or preventing arteriosclerotic diseases, including the step of selecting compounds that lower the expression level of AGTRL1 or PRKCH gene or the activity of the protein encoded by the gene.

One embodiment of the screening methods of the present invention is a method using the expression level of AGTRL1 or PRKCH gene as an index. Compounds that lower the expression level of AGTRL1 or PRKCH gene are expected to serve as agents for the prevention and treatment of arteriosclerotic diseases.

The above described method of the present invention is, for example, a method of screening for an agent for treating or preventing arteriosclerotic diseases, which includes the following steps (a) to (c):
(a) contacting a test compound with cells that express AGTRL1 or PRKCH gene;
(b) measuring the expression level of AGTRL1 or PRKCH gene; and
(c) selecting the compound that lowers the expression level as compared to the expression level measured in the absence of the test compound.

In the present method, initially a test compound is contacted with cells that express AGTRL1 or PRKCH gene. The "cells" used herein can be derived from humans, mice, cats, dogs, cattle, sheep, birds, or other pets or livestock, but are not limited thereto. Cells expressing an endogenous AGTRL1 or PRKCH gene, or cells expressing an exogenous AGTRL1 or PRKCH gene which has been introduced thereto can be used as the "cells that express AGTRL1 or PRKCH gene". Cells expressing an exogenous AGTRL1 or PRKCH gene can be generally produced by introducing to a host cell, expression vectors into which AGTRL1 or PRKCH gene has been inserted. The expression vector can be produced according to general genetic engineering techniques.

Test compounds for use in the method are not particularly limited. Examples thereof include single compounds such as natural compounds, organic compounds, inorganic compounds, proteins, and peptides; and compound libraries, expression products of gene libraries, cell extracts, cell culture supernatants, products of fermenting microorganisms, marine organism extracts, and vegetable extracts, but are not limited thereto.

Test compounds may be "contacted" with cells that expresses AGTRL1 or PRKCH gene generally by adding the test compounds to the culture medium of the cells that express the AGTRL1 or PRKCH gene, but is not limited thereto. When the test compound is, for example, proteins, "contacting" can be carried out by introducing DNA vectors that express the protein into the cells.

In the present method, next, the expression level of the AGTRL1 or PRKCH gene is measured. The term "gene expression" as used herein includes both transcription and translation. Gene expression levels can be measured according to methods known to one skilled in the art. The transcription level of the gene can be measured, for example, by extracting mRNA from cells that express the AGTRL1 or PRKCH gene according to common procedures, and carrying out northern hybridization or RT-PCR using the extracted mRNA as the template. The gene translation level can be measured by recovering protein fractions from cells that expresses the AGTRL1 or PRKCH gene, and detecting the expression of AGTRL1 or PRKCH protein by electrophoresis such as sodium dodecyl sulfate-polyacrylamide gel electrophoresis (SDS-PAGE). In addition, the gene translation level can also be measured by carrying out Western blotting using antibodies against AGTRL1 or PRKCH protein to detect the expression of the protein. Antibodies for use in the detection of AGTRL1 or PRKCH protein is not particularly limited, so long as they are detectable antibodies, and for example, both monoclonal antibodies and polyclonal antibodies can be used.

In the present method, next, compounds that lower the expression level as compared to the expression level when the test compound is not contacted (control) is selected. Such compounds that lower the expression level can be an agent for treating or preventing arteriosclerotic diseases.

Another embodiment of the screening methods according to the present invention is a method of identifying compounds that lower the expression level of the AGTRL1 or PRKCH gene of the present invention, using the expression of a reporter gene as an index.

The above-mentioned method of the present invention is, for example, a method of screening for an agent for treating or preventing arteriosclerotic diseases, which includes the following steps (a) to (c):
(a) contacting a test compound with cells containing DNA having a structure in which a transcriptional regulatory region of AGTRL1 or PRKCH gene and a reporter gene are operably linked with each other;
(b) measuring the expression level of the reporter gene; and
(c) selecting the compound that lowers the expression level as compared to the expression level measured in the absence of the test compound.

In the present method, initially a test compound is contacted with cells or cell extracts that include a DNA having a structure in which a transcriptional regulatory region of the AGTRL1 or PRKCH gene and a reporter gene are operably linked with each other. The phrase "operably linked" herein means that a transcriptional regulatory region of the AGTRL1 or PRKCH gene and a reporter gene bind with each other so that the expression of the reporter gene is induced as a result that a transcription factor binds with the transcriptional regulatory region of the AGTRL1 or PRKCH gene. Accordingly, even when the reporter gene is linked with another gene and forms a fused protein with another gene product, one is included within the meaning of "operably linked", so long as the expression of the fused protein is induced as a result of binding of a transcription factor with a transcriptional regulatory region of the AGTRL1 or PRKCH gene. A transcriptional regulatory region of the AGTRL1 or PRKCH gene in the genome can be obtained according to known methods based on the cDNA nucleotide sequence of the AGTRL1 or PRKCH gene by one skilled in the art.

The reporter gene for use in the present method is not particularly limited, so long as its expression is detectable, and includes, for example, the CAT gene, lacZ gene, luciferase gene, and GFP gene. The "cells containing DNA having a structure in which a transcriptional regulatory region of an AGTRL1 or PRKCH gene and a reporter gene are operably linked with each other" can be, for example, cells introduced with vectors into which such structure is inserted. Such vectors can be prepared according to methods well known to one skilled in the art. The vectors can be introduced into cells according to general methods such as calcium phosphate precipitation, electroporation, a Lipofectamine method, or microinjection. The "cells containing DNA that has a structure in which a transcriptional regulatory region of an AGTRL1 or PRKCH gene and a reporter gene are operably linked with each other" further include cells in which the structure has been inserted into their chromosome. The DNA structure can be inserted to the chromosome according to methods generally used by one skilled in the art, such as gene transfer technique using homologous recombination.

The "cell extracts containing DNA that has a structure in which a transcriptional regulatory region of an AGTRL1 or PRKCH gene and a reporter gene are operably linked with each other" can be, for example, cell extracts that are contained in commercially available *in vitro* transcription/translation kit and are added with DNA that has a structure in which a transcriptional regulatory region of an AGTRL1 or PRKCH gene and a reporter gene are operably linked with each other.

The "contact" in the present method can be conducted by adding a test compound to a culture medium of the "cells containing DNA having a structure in which a transcriptional regulatory region of an AGTRL1 or PRKCH gene and a reporter gene are operably linked with each other", or by adding a test compound to the above described commercially available cell extracts containing the DNA. When the test compound is a protein, the contact can also be conducted by introducing DNA vectors that express the protein into the cells.

Next, the expression level of the reporter gene is measured in the present method. The expression level of the reporter gene can be measured by methods known to one skilled in the art according to the type of the reporter gene. When the reporter gene is, for example, the CAT gene, the expression level of the reporter gene can be measured by detecting acetylation of chloramphenicol by the gene product. The expression level of the reporter gene can be measured by detecting coloring of a dye compound catalyzed by an expression product of the lacZ gene when the reporter gene is the lacZ gene; by detecting fluorescence of a fluorescent compound catalyzed by an expression product of the luciferase gene when the reporter gene is the luciferase gene; or by detecting fluorescence of the GFP protein when the reporter gene is the GFP gene.

Next, in the present method, compounds that lower (suppress) the measured expression level of the reporter gene as compared to the expression level measured in the absence of the test compound is selected. The compounds that lower (suppress) the expression level can be an agent for treating or preventing arteriosclerotic diseases.

As the AGTRL1 or PRKCH gene for use in the above-mentioned screening method of the present invention, a wildtype gene can be generally used, and an AGTRL1 gene (mutant AGTRL1 gene) containing a polymorphic mutation ("SNP4" and/or "SNP9") which is involved in elevated expression and discovered herein can also be suitably used.

This mutant AGTRL1 gene originally shows enhanced gene expression and is suitable for screening a substance that suppresses (lowers) the expression of the gene. In addition, a substance that suppresses (lowers) the enhanced gene expression of a mutant AGTRL gene found in patients actually suffering from brain infarction is expected to be a suitable agent for preventing or treating arteriosclerotic diseases such as brain infarction.

Another embodiment of the screening method of the present invention is a method that uses as an index the activity of interaction (binding activity) between the Sp1 transcription factor and the DNA region in the AGTRL1 gene that binds to the Sp1 transcription factor.

The above-described method of the present invention is, for example, a method of screening for pharmaceutical agents for treating or preventing arteriosclerotic diseases, which comprises the steps of:
(a) contacting a test compound with the Sp1 transcription factor and a polynucleotide comprising a DNA region which comprises a nucleotide site in an AGTRL1 gene located at position 42509 or 39353 of the nucleotide sequence of SEQ ID NO: 1;
(b) measuring the binding activity between the polynucleotide and the Sp1 transcription factor; and
(c) selecting a compound that reduces the binding activity as compared with that measured in the absence of the test compound.

In the present method, a test compound is first contacted with the Sp1 transcription factor and a polynucleotide comprising a DNA region which comprises a nucleotide site in an AGTRL1 gene located at position 42509 or 39353 of the nucleotide sequence of SEQ ID NO: 1.

Next, the activity of interaction (binding activity) between the polynucleotide and the Sp1 transcription factor is measured. This interaction activity can be evaluated using various methods well known to those skilled in the art.

For example, the interaction activity can be evaluated by a shift assay. More specifically, it can be carried out appropriately by the method to be described in Example 9.

Subsequently, compounds that reduce (suppress) the interaction activity as compared with that measured in the absence of the test compound are selected. Compounds that reduce (suppress) the activity will become pharmaceutical agents for treating arteriosclerotic diseases.

A further embodiment of the screening method of the present invention is a method that uses the autophosphorylation activity or protein kinase activity of the PRKCH protein as an index. Compounds that reduce these activities are expected to show therapeutic effects for arteriosclerotic diseases.

The above-described method of the present invention is, for example, a method of screening for pharmaceutical agents for treating or preventing arteriosclerotic diseases, which comprises the steps of:
(a) contacting a test compound with a PRKCH protein;
(b) measuring the autophosphorylation activity or protein kinase activity of the PRKCH protein; and
(c) selecting a compound that reduces the activity as compared with that measured in the absence of the test compound.

In the present method, the PRKCH protein is first contacted with a test compound. This "contact" can also be carried out, for example, by contacting a test compound with cells expressing the PRKCH protein.

Next, the autophosphorylation activity or protein kinase activity of the PRKCH protein is measured. The PRKCH protein used in the above-mentioned method is preferably a mutant protein whose autophosphorylation activity is enhanced. A preferred example of such a mutant protein is a mutant protein in which valine at position 374 in the amino acid sequence of the PRKCH protein is substituted with isoleucine. Furthermore, a wild-type protein that has no mutations can also be used. Furthermore, it may be a partial polypeptide containing the part to be phosphorylated in the PRKCH protein, or a mutant polypeptide containing this part.

The amino acid site to be autophosphorylated in the PRKCH protein includes, for example, T510 (threonine at position 510), T650 (threonine at position 650), and S672 (serine at position 672). It is known that one of these positions (T510) is phosphorylated by PKD1 and the remaining two positions (T650 and S672) are autophosphorylated (B. D. Gomperts et al., translation supervised by Y. Kajiro, "Signal Transduction", Medical Science International, p 206). A preferred embodiment of the screening method of the present invention is a method that uses the autophosphorylation states of the above-mentioned amino acid sites as index.

The measurement of phosphorylation activity can be carried out by means well known to those skilled in the art. For example, it can be measured by Western blotting using phosphorylation-specific antibodies, or such. More specifically, the measurement of phosphorylation activity can be carried out appropriately by methods to be described in the Examples.

Furthermore, in the above-mentioned method, compounds that reduce the aforementioned phosphorylation activity as compared with that measured in the absence of the test compound are selected. Compounds selected in this manner are expected to exhibit therapeutic or preventive effects on arteriosclerotic diseases as a result of suppressing the autophosphorylation activity or protein kinase activity of the PRKCH protein.

Compounds used in the various screening methods described above are also useful as reagents for screening for pharmaceutical agents for treating or preventing arteriosclerotic diseases.

Specific examples of the above-mentioned reagents of the present invention include reagents for screening for pharmaceutical agents for treating or preventing arteriosclerotic diseases, which comprise any one of the following (a) to (d) as an active ingredient:
(a) an oligonucleotide that hybridizes with a transcript of the AGTRL1 or PRKCH gene;
(b) an antibody that recognizes the AGTRL1 or PRKCH protein;
(c) a polynucleotide comprising a DNA region which comprises a nucleotide site in the AGTRL1 gene located at position 39353 or 42509 of the nucleotide sequence of SEQ ID NO: 1; and
(d) a mutant PRKCH protein having an amino acid sequence in which valine at position 374 in the amino acid sequence of the PRKCH protein is substituted with isoleucine.

The present invention further provides kits which include, for example, various agents and/or reagents used for carrying out the testing methods or screening methods of the present invention.

The kit of the present invention can include, for example, a reagent suitably selected from among the reagents of the present invention, according to the testing method or screening method to be conducted. For example, the kit of the present invention may include, as a constitutional component, the genes, proteins, oligonucleotides and antibodies of the present invention. More specific examples include (1) primer oligonucleotides for use in the present invention, and PCR reaction reagents (such as Taq polymerase and buffer); (2) probe oligonucleotides for use in the present invention, and hybridization buffer; and (3) anti-AGTRL1 antibody or anti-PRKCH antibody and ELISA reagent.

The kit of the present invention may further suitably include, for example, control samples, buffer, and directions for use.

All the prior art documents cited in the present specification are incorporated herein by reference.

Furthermore, the present invention relates to methods for treating or preventing arteriosclerotic diseases comprising the step of administering a pharmaceutical agent of the present invention to a subject. A required amount of the pharmaceutical agent of the present invention is administered to a mammal, including human, within a dose range that is considered to be safe. The dose of the pharmaceutical agent of the present invention can be appropriately determined by considering the type of dosage form, method of administration, age and body weight of the patient, symptoms of the patient, and such, and ultimately by the decision of a physician or veterinarian.

### [Examples]

Herein below, the present invention will be specifically described with reference to the Examples, but it is not to be construed as being limited thereto.

### <Materials, methods, and such of various experiments relating to the PRKCH gene>

### Test groups

In a genome-wide case-control study, cerebral infarction cases from seven affiliated hospitals of Kyushu University were registered. All cases were diagnosed and classified by physicians who were experts in cerebral apoplexy using clinical information and brain imaging tests. The subtypes of cerebral infarction were determined according to the diagnostic criteria of Classification of Cerebrovascular Diseases III proposed by the National Institute of Neurological Disorders and Stroke (Whisnant JP, et al., Stroke; 21:637-676 (1990)), the Trial of Org 10172 in Acute Stroke Treatment (TOAST) (Adams, HP Jr. et al., Stroke 24, 35-41 (1993)), and Cerebral Embolism Task Force (No authors listed, Arch. Neurol. 43, 71-84 (1986)).

Control subjects were incorporated from the participants in the Hisayama Study. The Hisayama study is an ongoing prospective epidemiologic study on cardiovascular diseases using a regional population established in 1961. Details of this study have been described previously (Kubo, M. et al., Stroke 34, 2349-2354 (2003); and Kiyohara, Stroke. 34, 2343-2348 (2003)). Screening survey was performed for this study from 2002 to 2003. In short, a total of 3,328 participants who were aged 40 or older (78% participation rate) agreed to participate in a health examination and underwent a comprehensive assessment. After excluding subjects with a past medical history of cerebral apoplexy or coronary artery disease, the present inventors selected age- (within five years) and sex-matched control subjects by 1:1 matching using random numbers.

To examine the risk of rs2230500 on the onset of cerebral infarction, the present inventors used a cohort of the Hisayama study established in 1988 (Kubo, M. et al., Stroke 34, 2349-2354 (2003)). In short, 2,742 Hisayama residents aged 40 years or older participated in a health examination in 1988 (80% participation rate). After excluding subjects with a past medical history of cerebral apoplexy or coronary artery disease, a continuous follow-up was performed on 2,637 participants for the onset of cardiovascular diseases or death. Of the cohort subjects, 1,683 subjects participated in the examination in 2002.

The present inventors obtained informed consent statements signed by all the test subjects as approved by the ethics committees of the Faculty of Medical Sciences in Kyushu University, the Institute of Medical Science in the University of Tokyo, and each participating hospital.

### SNP genotyping

Genomic DNA was extracted from peripheral blood leukocytes by a standard protocol. SNPs were genotyped using the multiplex PCR-based Invader assay (Third Wave Technologies, Madison, Wis.) as described by Ohnishi *et al.* (Ohnishi Y. et al., J. Hum. Genet. 46, 471-477 (2001)), or by direct sequencing of PCR products using the ABI3700 capillary sequencer (Applied Biosystems) following a standard protocol.

### Cell culturing, transfection, and immunoprecipitation

293T cells were maintained at 37°C under 5% CO₂ in DMEM supplemented with 10% fetal calf serum and 1% antibiotics. A plasmid expressing full-length PKCη-374V was constructed by cloning human thymus cDNA into p3xFLAG-CMV-14 expression vector (Sigma). A plasmid expressing full-length PKCη-374I was constructed from p3xFLAG-CMV-14-PKCη-374V vector using the QuikChange XL Site-Directed Mutagenesis Kit (Stratagene) according to the manufacturer's instructions. For transient transfection, 293T cells were plated in 15-cm culture dishes, and when nearly confluent, p3xFLAG-CMV-14-PKCη-374V or p3xFLAG-CMV-14-PKCη-3741 was transfected using FuGENE6 (Roche) according to the manufacturer's instructions. Forty-eight hours later, the cells were collected and lysed at 4°C in lysis buffer containing 1% Nonidet P-40, 150 mM NaCl, 50 mM Tris-HCl, pH8.0, 1 mM phenylmethylsulfonyl fluoride, 1 mM dithiothreitol, and 0.1% Protease Inhibitor Cocktail Set III (Calbiochem). After a 30-minute incubation on ice, the lysates were centrifuged at 15,000 rpm at 4°C for 15 minutes. The supernatant was pretreated with rec-protein G-Sepharose 4B conjugate (Zymed) and normal mouse IgG for 30 minutes, and then incubated with anti-Flag M2 affinity gel (Sigma) for three to four hours at 4°C. After incubation, the gel was washed twice with the lysis buffer and once with 1x TBS buffer, and the Flag-labeled protein was eluted by adding 15 µg of 3x Flag peptide (Sigma). The purity and amount of the immunoprecipitates were evaluated by Coomassie Brilliant Blue staining. Protein concentration was determined by the Bradford method.

### PKC activity and autophosphorylation assay

PKC autophosphorylation activity and kinase activity were measured according to the method described by Ikuta *et al.* (Ikuta, T. et al., Cell Growth Diff. 5, 943-947 (1994)). For the PKC autophosphorylation assay, immunoprecipitates for mock, PKCη-374V, and PKCη-374I were incubated at 30°C for the indicated duration in a reaction mixture of a total volume of 50 µL containing 20 mM Tris-HCl (pH7.5), 5 mM MgSO₄, 1 mM EGTA, and 5 µCi of [γ-²P]ATP together with 10 µM phosphatidyl serine (PS, Sigma) and 100 nM phorbol-12,13-dibutyrate (PDBu, Sigma), and were then subjected to SDS-PAGE and autoradiography. Protein kinase activity was determined by the incorporation of ³²P from [γ-³²P]ATP into the myelin basic protein (MBP) peptide (Sigma). The incubation mixture (total volume of 50 µL) contained 20 mM Tris-HCl (pH7.5), 5 mM MgSO₄, 1 mM EGTA, 100 µM ATP, 1 µCi of [γ-³²P]ATP, 10 µg MBP peptide, and PKCη immunoprecipitates, as well as 10 µM PS and 100 nM PDBu. After incubation at 30°C for three minutes, the reaction was stopped by direct application to P81 phosphocellulose square (Upstate). After washing with 75 mM phosphoric acid, radioactivity was quantified. The unit activity was defined as incorporation of 1 nmol/minute of radioactive phosphoric acid from ATP to MBP.

### Quantification of PRKCH expression using real-time PCR

The present inventors performed real-time quantitative PCR using ABI 7700 (Applied Biosystems) together with SYBR Premix ExTag (TaKaRa) according to the manufacturers' instructions. Human total RNAs derived from various tissues (Clonetech) were purchased, and single-chain cDNAs were synthesized from 1 µg of total RNA using oligo d(T)₁₂₋₁₈ primer and Superscript III reverse transcriptase (Invitrogen). Relative expression of PRKCH mRNA was normalized to the beta actin expression level in the same cDNA using the standard curve method described by the manufacturer.

### Immunohistochemistry test and morphometrical analysis of the coronary artery

From 16 (eight males and eight females) Japanese patients of ages 68 to 91 (81.1 ± 6.2) who resided in Hisayama, the hearts were obtained by autopsy within 16 hours of death at Kyushu University. Cannula was inserted into the coronary artery and after washing with 0.1 mol/L phosphate buffered saline (pH7.4), perfusion with 1 L of 4% (wt/vol) paraformaldehyde (in 0.1 mol/L sodium phosphate, pH7.4) was carried out at 100 mmHg. Next, the hearts were soaked for at least 24 hours at 4°C in 4% paraformaldehyde. The right coronary artery and left anterior descending coronary artery were dissected from the surface of the heart, cut perpendicularly to the long axis at 3-mm intervals and then embedded in paraffin. Sixty blocks were obtained and 3-µm-thick continuous sections were prepared at once. The sections from each block were successively subjected to hematoxylin and eosin staining, Elastica-van Gieson staining, and Masson trichrome staining, and immunohistochemistry tests. In accordance with the definitions proposed by the Committee on Vascular Lesions of the Council on Arteriosclerosis of AHA (Stary, HC. et al., Circulation. 92, 1355-74 (1995)), each section was carefully classified into types of atherosclerotic lesion.

Immunohistochemistry tests were performed as described by Nakano *et al.* (Nakano, T. et al., Hum Pathol. 36, 330-40 (2005)). In brief, deparaffinized sections were incubated with 3% nonfat milk, and incubated with primary antibodies against human PKCη (Santa Cruz), endothelial cells (anti-human CD31, Dako), monocytes/macrophages (anti-human CD68, Dako), and smooth muscle cells (anti-human α-SMA, Sigma), and then with peroxidase-labeled secondary antibodies (Dako). The slides were incubated with 3,3'-diaminobenzidine tetrachloride (DAB), and then counterstained with hematoxylin. As a negative control, nonimmune rabbit IgG or nonimmune mouse IgG of each isotype was substituted instead of the respective primary antibodies. Tissue blocks collected from the human mammary glands were used as positive control for PKCη (Masso-Welch, PA. et al., Breast Can. Res. Treat. 68, 211-223 (2001)). A single observer who was unaware of the types of atherosclerotic lesion quantified PKCη-positive lesions, and analysis was preformed by determining the positive area in the atherosclerotic intima. All images were captured and analyzed by the image software of US National Institutes of Health.

### Statistical Analysis

Data are presented as mean ± s.d. unless otherwise stated. Association and Hardy-Weinberg equilibrium were evaluated by chi-square test and Fisher's exact test. Calculations of linkage disequilibrium index and Δ index, and creation of Figure 1d were carried out as described by Tokuhiro *et al.* (Tokuhiro, S. et al., Nat. Genet. 35, 341-348 (2003)). For adjustment of multiple testing, the present inventors repeated a random permutation test 10,000 times using the MULTTEST procedure in SAS 9.12 software. The difference in the incidence rate of cerebral infarction due to the rs2230500 genotype over 14 years was evaluated by using the Cox proportional hazard model after making adjustments for age and sex. For adjustment of clinical risk factors, a logistic regression model was used for the case-control study and Cox proportional hazards regression model was used for the prospective cohort study, using the SAS software. PKCη-positive areas were compared across grades of artery atherosclerosis using Spearman's rank correlation with Bonferroni correction.

### [Example 1] Large-scale case-control study using gene-based SNP markers

To identify genes that are susceptible and implicated in cerebral infarction, the present inventors used 1,112 cerebral infarction patients and 1,112 age- and sex-matched control subjects to perform a large-scale genome-wide case-control study. The clinical characteristics of the subject groups in this case-control study are shown in Table 3.

**Table 3**

| | Case | Control | P value |
|---|---|---|---|
| N | 1112 | 1112 | |
| Age | 70.2 ± 10.0 | 70.1 ±10.1 | 0.87 |
| Male (%) | 60.7 | 60.7 | |
| Cerebral infarction subtypes | | | |
| Lacunar (LA) | 491 | | |
| Atherothrombotic (AT) | 369 | | |
| Combined LA + AT | 860 | | |
| Cardiogenic embolism | 136 | | |
| Others | 116 | | |
| Hypertension (%) | 78.1 | 53.7 | <0.0001 |
| Hyperlipidemia (%) | 48.3 | 41.8 | 0.0024 |
| Diabetes (%) | 30.4 | 21.1 | <0.0001 |

First, the present inventors genotyped 188 Japanese cases with cerebral infarction and 188 age- and sex-matched control cases using 52,608 gene-based tag-SNPs selected from the JSNP database (Tsunoda, T. et al., Hum. Mol. Genet. 13, 1623-1632 (2004)). Allele frequencies of 48,083 successfully genotyped SNPs (overall success rate of 91.4%) were compared, and 1,098 SNPs showing p < 0.01 between the cases and controls were identified.

The present inventors subsequently genotyped the remaining cases and controls for these SNPs in the second screening. When the subjects were stratified according to the subtypes of cerebral infarction, several SNPs that highly correlated with the combined lacunar infarction and atherothrombotic infarction group were identified. Of them, SNP_15 (IMS-JST140193, rs3783799) in intron 6 of PRKCH on chromosome 14q22-23 highly correlated with the lacunar infarction group (p = 4.73 x 10⁻⁶ for allele frequency) and the combined lacunar infarction and atherothrombotic infarction group (p = 7.91 x 10⁻⁶ in the dominant model, Table 5). Even after permutation tests using all SNPs that were genotyped in the second screening, SNP_15 still showed correlation with lacunar infarction (p = 0.0036) and lacunar-atherothrombotic combined infarction (p = 0.0063). Accordingly, the present inventors considered that the susceptibility locus for lacunar infarction and atherothrombotic infarction might be located around SNP_15.

Table 4 below shows the genotyped SNP positions in the PRKCH gene (NCBI Build 35).

**Table 4**

| SNP Name | Position | jsnp_id | dbSNP rs# | ContigAcc | ContigPos |
|---|---|---|---|---|---|
| SNP_01 | Intron 1 | IMS-JST140210 | rs3783814 | NT_026437.11 | 42799673 |
| SNP_02 | Intron 1 | IMS-JST140207 | rs3783812 | NT_026437.11 | 42812443 |
| SNP_03 | Intron 1 | IMS-JST140206 | rs1033910 | NT_026437.11 | 42816593 |
| SNP_04 | Intron 1 | IMS-JST140205 | rs3783810 | NT_026437.11 | 42817442 |
| SNP_05 | Exon 2 | IMS-JST093801 | rs3742633 | NT_026437.11 | 42857722 |
| SNP_06 | Intron 2 | IMS-JST140204 | rs767757 | NT_026437.11 | 42874127 |
| SNP_07 | Intron 2 | IMS-JST140203 | rs767755 | NT_026437.11 | 42874201 |
| SNP_08 | Intron 2 | IMS-JST140202 | rs2209386 | NT_026437.11 | 42884237 |
| SNP_09 | Intron 2 | IMS-JST140201 | rs3783806 | NT_026437.11 | 42884263 |
| SNP_10 | Intron 2 | IMS-JST140198 | rs3783803 | NT_026437.11 | 42884524 |
| SNP_11 | Intron 2 | IMS-JST140196 | rs3783801 | NT_026437.11 | 42900735 |
| SNP_12 | Intron 4 | IMS-JST050416 | rs2296273 | NT_026437.11 | 42915504 |
| SNP_13 | Intron 5 | IMS-JST050417 | rs2296274 | NT_026437.11 | 42916931 |
| SNP_14 | Intron 6 | IMS-JST140194 | rs3783800 | NT_026437.11 | 42917986 |
| SNP_15 | Intron 6 | IMS-JST140193 | rs3783799 | NT_026437.11 | 42918969 |
| SNP_16 | Intron 8 | IMS-JST050419 | rs2296276 | NT_026437.11 | 42923845 |
| SNP_17 | Exon 9 | IMS-JST050420 | rs2230500 | NT_026437.11 | 42923992 |
| SNP_18 | Exon 9 | na | rs17098388 | NT_026437.11 | 42923994 |
| SNP_19 | Intron 9 | IMS-JST140187 | rs959728 | NT_026437.11 | 42933771 |
| SNP_20 | Intron 9 | IMS-JST140186 | rs3783792 | NT_026437.11 | 42933890 |
| SNP_21 | Intron 9 | IMS-JST140183 | rs3783789 | NT_026437.11 | 42936553 |
| SNP_22 | Intron 9 | IMS-JST140179 | rs3783786 | NT_026437.11 | 42936977 |
| SNP_23 | Intron 9 | IMS-JST140178 | rs3783785 | NT_026437.11 | 42937045 |
| SNP_24 | Intron 9 | IMS-JST140171 | rs3783778 | NT_026437.11 | 42949333 |
| SNP_25 | Intron 9 | IMS-JST140170 | rs3783777 | NT_026437.11 | 42949441 |
| SNP_26 | Intron 9 | IMS-JST140169 | rs3783776 | NT_026437.11 | 42949496 |
| SNP_27 | Intron 9 | IMS-JST140168 | rs912619 | NT_026437.11 | 42949686 |
| SNP_28 | Intron 9 | IMS-JST140167 | rs3783774 | NT_026437.11 | 42950854 |
| SNP_29 | Intron 9 | IMS-JST140166 | rs1536015 | NT_026437.11 | 42951558 |
| SNP_30 | Intron 10 | IMS-JST140165 | rs3783772 | NT_026437.11 | 42952680 |
| SNP_31 | Intron 10 | IMS-JST140163 | rs3783771 | NT_026437.11 | 42952898 |
| SNP_32 | Intron 10 | IMS-JST140154 | rs3783762 | NT_026437.11 | 42967956 |
| SNP_33 | Intron 10 | IMS-JST140153 | rs2245448 | NT_026437.11 | 42972568 |
| SNP_34 | Intron 10 | IMS-JST140152 | rs3783760 | NT_026437.11 | 42972779 |
| SNP_35 | Intron 10 | IMS-JST103326 | rs1091680 | NT_026437.11 | 42983840 |
| SNP_36 | Intron 10 | IMS-JST103327 | rs3751292 | NT_026437.11 | 42984053 |
| SNP_37 | Intron 10 | IMS-JST103328 | rs3751293 | NT_026437.11 | 42984152 |
| SNP_38 | Intron 10 | IMS-JST103329 | rs2252267 | NT_026437.11 | 42984437 |
| SNP_39 | Intron 10 | IMS-JST103330 | rs3751295 | Nut_026437.11 | 42984490 |
| SNP_40 | Intron 10 | IMS-JST140150 | rs3783758 | NT_026437.11 | 42993489 |
| SNP_41 | Intron 10 | IMS-JST093800 | rs2463117 | NT_026437.11 | 42995426 |
| SNP_42 | Intron 12 | IMS-JST140146 | rs3783755 | NT_026437.11 | 43006640 |
| SNP_43 | Intron 12 | IMS-JST140145 | rs3783754 | NT_026437.11 | 43009990 |
| SNP_44 | Intron 12 | IMS-JST140144 | rs3783753 | NT_026437.11 | 43010112 |
| SNP_45 | Exon 14 | IMS-JST173548 | rs3813410 | NT_026437.11 | 43016888 |

Table 5 below is a summary of the case-control correlation analysis of PRKCH_SNP_15 observed according to the subtypes of cerebral infraction.

**Table 5**

| | Case | | | | Control | | | | P-value (Adjusted_P) | | Odds ratio (95% c.i.) | |
|---|---|---|---|---|---|---|---|---|---|---|---|---|
| Phenotype | 11 | 12 | 22 | Total | 11 | 12 | 22 | Total | 1/2 | 11+12/22 | 1/2 | 11+12/22 |
| Cerebral infarction | 59 | 394 | 655 | 1108 | 41 | 336 | 731 | 1108 | 5.25E-04 (0.34) | 8.51E-04 (0.54) | 1.29 (1.12∼1.49) | 1.34 (1.13∼1.59) |
| Lacunar infarction group | 28 | 178 | 282 | 488 | 11 | 131 | 345 | 487 | 4.73E-06 (0.0036) | 2.10E-05 (0.015) | 1.69 (1.35∼2.12) | 1.77 (1.36∼2.31) |
| Atherothrombotic infarction group | 15 | 136 | 217 | 368 | 17 | 109 | 243 | 369 | 0.134 (ND) | 0.0536 (ND) | 1.21 (0.94∼1.56) | 1.34 (1.00∼1.81) |
| Combined lacunar infarction and atherothrombotic infarction group | 43 | 314 | 499 | 856 | 28 | 240 | 588 | 856 | 1.00E-05 (0.0097) | 7.91E-06 (0.0063) | 1.46 (1.23∼1.73) | 1.57 (1.29∼1.91) |

In Table 5 above, allele 1 is defined as a risk allele. P-values were adjusted by performing 10⁴ permutation tests on all SNPs examined in the screening. "c.i." refers to confidence interval.

Next, the present inventors performed high-precision mapping of PRKCH using 45 SNPs, and the extent of LD in SNP_15 was evaluated using lacunar infarction and atherothrombotic infarction cases as well as age- and sex-matched controls. These SNPs were genotyped and D' and Δ indices were calculated for 27 types of SNPs with minor allele frequency greater than 0.2 (Fig. 1d). Two LD blocks were identified in PRKCH, and SNP_15 was found to be positioned in block 1 (Fig. 1d). The present inventors also compared the allele frequencies between the cases and the controls, and discovered that the association was greatest in block 1 of PRKCH and gradually decreased in the 5' and 3' regions. From these results, the present inventors concluded that PRKCH is a susceptibility gene for lacunar and atherothrombotic cerebral infarctions.

Next, the present inventors sequenced all of the exons in PRKCH including the 5' and 3' untranslated regions from 48 cases and 48 controls. As a result, four SNPs were identified: rs3742633 (695A>G, in exon 2), rs2230500 (1425G>A, in exon 9), rs17098388 (1427A>C, in exon 9), and rs1088680 (1979C>T, in exon 12). Of them, rs3742633 and rs1088680 were positioned outside of block 1 and did not show association. rs2230500 and rs17098388 had a single-nucleotide gap between each other and were positioned in block 1. Furthermore, rs2230500 induced an amino acid substitution of isoleucine for valine (V374I, Fig. 2a), but on the other hand, rs17098388 was a synonymous SNP. The sequencing result revealed these two SNPs were completely linked.

Next, the present inventors genotyped rs2230500 by direct sequencing in all lacunar and atherothrombotic infarction cases as well as in age- and sex-matched controls. The A allele of rs2230500 which induces amino acid change into Ile was observed at high frequency in the cases, and this was highly associated with both lacunar infarction (p = 9.84 x 10⁻⁶, odds ratio (OR) of 1.66, 95% confidence interval (c.i.) of 1.33 to 2.09, allele frequency model, Table 7), and in the combined lacunar infarction and atherothrombotic infarction group (p = 4.92 x 10⁻⁵, OR of 1.42, 95% c.i. of 1.20 to 1.68). Permutation tests were applied to genotyped 45 SNPs in PRKCH, and the results obtained were p = 0.0004 for lacunar infarction and p = 0.0014 for the combined lacunar infarction and atherothrombotic infarction group. These results were both statistically significant.

To elucidate the confounding effects of the difference between the cases and the controls, the present inventors adjusted clinical risk factors using a logistic regression model. Genotype risk for cerebral infarction was substantially unchanged after adjustments of age, sex, hypertension, hyperlipidemia, and diabetes (Table 8). Therefore, the present inventors established a hypothesis that the V374I amino acid substitution in PRKCH might bring about the onset of cerebral infarction by affecting the signal transduction of PKCη.

Table 6 below shows the results of case-control correlation analysis of 45 types of SNPs in the PRKCH gene for the subgroups of the combined lacunar infarction and atherothrombotic infarction group.

**Table 6**

| | MAF | | Allele model | | Dominant model | | Allele model | | Dominant model | |
|---|---|---|---|---|---|---|---|---|---|---|
| SNP Name | Case | Control Value | P- | Adjusted_ P | P-Value | Adjusted _P | **OR** | **95%CI** | **OR** | **95%CI** |
| SNP_01 | 0.452 | 0.462 | 0.562 | 1 | 0.467 | 1 | 1.04 | (0.91∼1.19) | 1.09 | (0.86∼1.38) |
| SNP_02 | 0.389 | 0.382 | 0.654 | 1 | 0.896 | 1 | 1.03 | (0.90∼1.18) | 1.01 | (0.83∼1.23) |
| SNP_03 | 0.448 | 0.449 | 0.958 | 1 | 0.765 | 1 | 1.00 | (0.88∼1.15) | 0.97 | (0.76∼1.22) |
| SNP_04 | 0.009 | 0.008 | 0.691 | 1 | 0.690 | 1 | 1.17 | (0.54∼2.54) | 1.17 | (0.54∼2.55) |
| SNP_05 | 0.199 | 0.237 | 0.00608 | 0.16 | 0.0108 | 0.24915 | 1.26 | (1.07∼1.48) | 1.77 | (1.14∼2.77) |
| SNP_06 | 0.452 | 0.491 | 0.0234 | 0.46 | 0.0609 | 0.7696 | 1.17 | (1.02∼1.34) | 1.24 | (0.99∼1.55) |
| SNP_07 | 0.216 | 0.212 | 0.798 | 1 | 0.912 | 1 | 1.02 | (0.87∼1.20) | 1.01 | (0.83∼1.23) |
| SNP_08 | 0.362 | 0.363 | 0.940 | 1 | 0.413 | 1 | 1.01 | (0.87∼1.16) | 1.12 | (0.85∼1.49) |
| SNP_09 | 0.190 | 0.169 | 0.102 | 0.94 | 0.0734 | 0.85115 | 1.16 | (0.97∼1.38) | 1.20 | (0.98∼1.47) |
| SNP_10 | 0.138 | 0.107 | 0.00472 | 0.13 | 0.00400 | 0.1048 | 1.34 | (1.09∼1.65) | 1.39 | (1.11∼1.75) |
| SNP_11 | 0.141 | 0.102 | 0.000567 | 0.020 | 0.00100 | 0.03215 | 1.44 | (1.17∼1.77) | 1.47 | (1.17∼1.84) |
| SNP_12 | 0.264 | 0.290 | 0.0891 | 0.91 | 0.0530 | 0.72955 | 1.14 | (0.98∼1.32) | 1.41 | (0.99∼1.99) |
| SNP_13 | 0.480 | 0.437 | 0.0130 | 0.31 | 0.00691 | 0.1831 | 1.19 | (1.04∼1.37) | 1.33 | (1.08∼1.64) |
| SNP_14 | 0.024 | 0.027 | 0.595 | 1 | 0.157 | 0.99925 | 1.12 | (0.74∼1.71) | 0.00 | |
| SNP_15 | 0.234 | 0.173 | 1.00E-05 | 0.0002 | 7.90E-06 | 0.00015 | 1.46 | (1.23∼1.73) | 1.57 | (1.29∼1.91) |
| SNP_16 | 0.235 | 0.176 | 1.63E-05 | 0.00045 | 1.73E-05 | 0.00045 | 1.44 | (1.22∼1.71) | 1.54 | (1.27∼1.88) |
| SNP_17 | 0.228 | 0.173 | 4.92E-05 | 0.0016 | 4.12E-05 | 0.0014 | 1.42 | (1.20∼1.68) | 1.51 | (1.24∼1.85) |
| SNP_18 | 0.228 | 0.173 | 4.92E-05 | 0.0016 | 4.12E-05 | 0.0014 | 1.42 | (1.20∼1.68) | 1.51 | (1.24∼1.85) |
| SNP_19 | 0.266 | 0,219 | 0.00143 | 0.047 | 0.00303 | 0.087 | 1.29 | (1.10∼1.51) | 1.34 | (1.10∼1.62) |
| SNP_20 | 0.267 | 0.220 | 0.00127 | 0.042 | 0.00307 | 0.08775 | 1.29 | (1.11∼1.51) | 1.34 | (1.10∼1.62) |
| SNP_21 | 0.208 | 0.160 | 0.000272 | 0.0096 | 0.000645 | 0.01965 | 1.38 | (1.16∼1.64) | 1.42 | (1.16∼1.74) |
| SNP_22 | 0.498 | 0.478 | 0.162 | 0.99 | 0.550 | 1 | 1.10 | (0.96∼1.26) | 1.07 | (0.86∼1.32) |
| SNP_23 | 0.438 | 0.459 | 0.233 | 1 | 0.361 | 1 | 1.09 | (0.95∼1.24) | 1.11 | (0.88∼1.40) |
| SNP_24 | 0.142 | 0.153 | 0.365 | 1 | 0.275 | 1 | 1.09 | (0.90∼1.32) | 1.42 | (0.76∼2.66) |
| SNP_25 | 0.233 | 0.190 | 0.00210 | 0.063 | 0.00268 | 0.0755 | 1.29 | (1.10∼1.53) | 1.35 | (1.11∼1.64) |
| SNP_26 | 0.233 | 0.190 | 0.00207 | 0.064 | 0.00267 | 0.0783 | 1.29 | (1.10∼1.53) | 1.35 | (1.11∼1.64) |
| SNP_27 | 0.461 | 0.456 | 0.758 | 1 | 0.596 | 1 | 1.02 | (0.89∼1.17) | 1.06 | (0.86∼1.30) |
| SNP_28 | 0.463 | 0.455 | 0.662 | 1 | 0.520 | 1 | 1.03 | (0.90∼1.18) | 1.07 | (0.87∼1.32) |
| SNP_29 | 0.142 | 0.152 | 0.425 | 1 | 0.358 | 1 | 1.08 | (0.89∼1.30) | 1.33 | (0.72∼2.48) |
| SNP_30 | 0.141 | 0.152 | 0.363 | 1 | 0.275 | 1 | 1.09 | (0.90∼1.32) | 1.42 | (0.76∼2.66) |
| SNP_31 | 0.141 | 0.152 | 0.352 | 1 | 0.267 | 0.9997 | 1.09 | (0.91∼1.32) | 1.43 | (0.76∼2.67) |
| SNP_32 | 0.156 | 0.189 | 0.00952 | 0.24 | 0.323 | 1 | 1.26 | (1.06∼1.51) | 1.39 | (0.72∼2.66) |
| SNP_33 | 0.461 | 0.426 | 0.0392 | 0.66 | 0.0672 | 0.83935 | 1.15 | (1.01∼1.32) | 1.21 | (0.99∼1.49) |
| SNP_34 | 0.126 | 0.128 | 0.818 | 1 | 0.856 | 1 | 1.02 | (0.84∼1.25) | 1.07 | (0.51∼2.23) |
| SNP_35 | 0.407 | 0.424 | 0.324 | 1 | 0.685 | 1 | 1.07 | (0.93∼1.23) | 1.05 | (0.82∼1.35) |
| SNP_36 | 0.124 | 0.094 | 0.00456 | 0.136 | 0.00857 | 0.22565 | 1.37 | (1.10∼1.70) | 1.37 | (1.08∼1.74) |
| SNP_37 | 0.170 | 0.148 | 0.0736 | 0.859 | 0.146 | 0.97665 | 1.18 | (0.98∼1.42) | 1.17 | (0.95∼1.44) |
| SNP_38 | 0.414 | 0.427 | 0.414 | 1 | 0.698 | 1 | 1.06 | (0.92∼1.21) | 1.05 | (0.82∼1.34) |
| SNP_39 | 0.153 | 0.134 | 0.120 | 0.968 | 0.153 | 0.98655 | 1.16 | (0.96∼1.41) | 1.17 | (0.94∼1.45) |
| SNP_40 | 0.089 | 0.079 | 0.272 | 1 | 0.299 | 1 | 1.14 | (0.90∼1.46) | 1.15 | (0.89∼1.48) |
| SNP_41 | 0.403 | 0.422 | 0.255 | 1 | 0.147 | 0.9751 | 1.08 | (0.94∼1.24) | 1.20 | (0.94∼1.53) |
| SNP_42 | 0.463 | 0.446 | 0.320 | 1 | 0.372 | 1 | 1.07 | (0.94∼1.22) | 1.10 | (0.89∼1.35) |
| SNP_43 | 0.344 | 0.381 | 0.0219 | 0.449 | 0.0229 | 0.47195 | 1.18 | (1.02∼1.35) | 1.39 | (1.05∼1.84) |
| SNP_44 | 0.143 | 0.126 | 0.150 | 0.987 | 0.135 | 0.9722 | 1.16 | (0.95∼1.41) | 1.18 | (0.95∼1.47) |
| SNP_45 | 0.131 | 0.123 | 0.464 | 1 | 0.358 | 1 | 1.08 | (0.88∼1.32) | 1.11 | (0.89∼1.39) |

In Table 6 above, the P values were adjusted by performing 10⁴ permutation tests on all SNPs examined within the PRKCH gene. "MAF" indicates minor allele frequency. Furthermore, Table 7 below shows the results of case-control studies of rs2230500 (1425G>A, V374I).

**Table 7**

| | Case | | | | Control | | | | Minor allele frequency | | P-value (Adjusted_P) | | Odds ratio (95% c.i.) | |
|---|---|---|---|---|---|---|---|---|---|---|---|---|---|---|
| Phenotype | AA | AG | GG | Total | AA | AG | GG | Total | Case | Control | A vs. G | AA+AG vs. GG | A vs. G | AA+AG vs. GG |
| Lacunar infarction group | 27 | 178 | 286 | 491 | 11 | 130 | 344 | 485 | 0.236 | 0.157 | 9.84E-06 (0.0004) | 3.47E-05 (0.0009) | 1.66 (1.33∼2.09) | 1.75 (1.34∼2.28) |
| Combined lacunar infarction and atherothrombotic infarction group | 41 | 310 | 507 | 858 | 27 | 239 | 582 | 848 | 0.228 | 0.173 | 4.92E-05 (0.00155) | 4.12E-05 (0.0014) | 1.42 (1.20∼1.68) | 1.51 (1.24∼1.85) |

In Table 7 above, the P values were adjusted by performing 10⁴ permuts on tests on all SNPs examined within the PRKCH gene. "e.i." indicates the confidence interval. Table 8 below shows the result of multivariable logistic analysis of (1425G>A, V374I) in subgroups of the continued lacunar infarction and artherothrombotic infarction group.

**Table 8**

| | Age- and sex-adjusted | | | Multivariable-adjusted | | |
|---|---|---|---|---|---|---|
| Genotype | OR | (95%c.i.) | p-Value | HR | (95%c.i.) | p-Value |
| GG | 1.00 | | | 1.00 | | |
| GA | 1.52 | (1.22-1.88) | 0.0001 | 1.61 | (1.26-2.06) | 0.0001 |
| AA | 1.82 | (1.09-3.03) | 0.022 | 2.10 | (1.18-3.73) | 0.012 |
| | | | | | | |
| GG | 1.00 | | | 1.00 | | |
| GA+AA | 1.55 | (1.26-1.91) | <0.0001 | 1.66 | (1.31-2.11) | <0.0001 |

In Table 8 abpve, multivariable analysis were adjusted for age, sex, hypertension (presence/absence) hyperlipidemia (presence/absence), and diabetes (presence/absence). The present inventors used STRUCTURE and genomic control to examine group stratification between the cases and controls, but in both analyses, significant group stratification was not indicated in the subjects of the present inventors.

### [Example 2] Effect of V374I on PKCη activity

The V374I amino acid substitution in PKCη exists inside the ATP binding site which is conserved in the PKC family (Fig. 2b, Fig. 3) (Osada, S. et al., Cell Growth Diff. 4, 167-175 (1993)). Therefore, the present inventors investigated the effect ofV374I on PKCη kinase activity. The present inventors constructed Flag-PKCη-374V and Flag-PKCη-374I expression vectors, and transfected them into 293T cells. After transient transfection, both proteins were immunoprecipitated and purified using an anti-Flag M2 agarose gel. The quality and concentration of the immunoprecipitates were evaluated by Coomassie Brilliant Blue staining and Western blotting (Fig. 2c, d). Since PKCη has been reported to be activated by autophosphorylation (Nishizuka, Y. Science. 258, 607-614 (1992)), the present inventors examined the kinase activity of each protein by autophosphorylation assay. After stimulation with 10 nM phosphatidyl serine (PS) and 100 nM phorbol 12,13-dibutyrate (PDBu), autophosphorylation of PKCη was observed for at least one minute, and the degree of autophosphorylation was higher in PKCη-374I than in PKCη-374V (Fig. 2d). To confirm these results, PKC activity was examined using myelin basic protein as a substrate. The activity in PKCη-374I was 1.6-times greater than that of PKCη-374V (p = 0.009, Fig. 2e). These results suggest the possibility that the amino acid change from 374V to 374I in PKCη induces a higher level of autophosphorylation and kinase activation after stimulation and leads to activation of the downstream signal transduction pathway.

### [Example 3] Expression of PKCη in atherosclerosis

In mice, PKCη is expressed mainly in the epithelial tissues including the skin, digestive tract, and airway (Osada, S. et al., J. Biol. Chem. 265, 22434-22440 (1990)). The expression pattern of PKCη in humans is undetermined, and the relationship between PKCη and cerebral infarction cannot be explained from this distribution pattern in mice.

To examine the expression pattern of PKCη in human tissues, the present inventors performed quantitative real-time PCR using human cDNA in various tissues. PKCη was expressed universally in the various human tissues, and expression in the thymus and spleen was somewhat high (Fig. 4). Based on these results and the association to cerebral infarction, the present inventors examined the expression of PKCη in atherosclerotic lesions. Immunohistostaining of coronary artery preparations obtained at the time of autopsy showed that PKCη was expressed in the endothelial cells in the arterial intima, a portion of foamy macrophages, and spindle smooth muscle cells in the arterial intima and media (b to e of Fig. 5-1). In the outer membrane, PKCη was constantly expressed by a portion of vascular endothelial cells (data not shown). This expression could not be observed in preparations that had been pre-absorbed using immunogenic peptides or normal rabbit IgG.

To further examine the relationship with PKCη and atherosclerosis, 60 coronary artery preparations obtained at the time of autopsy were carefully classified according to the types of atherosclerotic lesion (AHA classification (Stary, HC. et al., Circulation. 92, 1355-74 (1995))). PKCη-positive cells were quantified by NIH imaging. PKCη expression matched the severity of coronary artery atherosclerosis, and it was observed at higher frequency in advanced lesions (p < 0.0001, f of Fig. 5-2). These results suggest that PKCη is closely related to the onset and progress of atherosclerosis in humans.

### [Example 4] Validation test of V374I using a population-based prospective cohort

In case-control studies, there is a danger of eliciting false-positive results due to selection bias of subjects and especially controls. Therefore, a candidate SNP identified in a single correlation analysis should be verified in a different population. However, the minor allele frequencies of SNP_15 (IMS-JST140193) used as a marker SNP in the correlation analysis by the present inventors were 0.239 in the Japanese group, 0.229 in the Chinese group, 0.022 in the African group, and 0.00 in the European group. These data suggest that the candidate SNP in PRKCH is specific to the Asian population and that this is difficult to reproduce in Caucasians.

To overcome this problem, the present inventors examined the association of rs2230500 in a population-based prospective cohort constructed in 1988. During a 14-year-long follow-up study, of the 1,642 subjects who had not had a medical history of cerebral apoplexy at the time of base-line examination, cerebral infarction occurred for the first time in 67 subjects. As a result of comparing the cerebral infarction incidence rates of the rs2230500 genotype, the present inventors discovered that the incidence rate increased linearly in the order of GG, GA, and AA genotypes (this corresponds to amino acid substitutions VV, VI, and II) (Fig. 6). The age- and sex-adjusted relative risk of cerebral infarction incidence rate in comparison to the GG genotype was 1.31 (95% c.i., 0.78 to 2.19) for the GA genotype and 2.83 (95% c.i., 1.11 to 7.22) for the AA genotype (Table 9). The risk for development of cerebral infarction in the AA genotype subjects was significantly higher in comparison to that of the GA genotype or GG genotype subjects (p = 0.043, relative risk 2.58, 95% c.i., 1.03 to 6.44). This relationship did not change substantially even after adjustment of baseline clinical risk factors including age, sex, hypertension, diabetes, serum cholesterol, smoking, and drinking habits. The risk of rs2230500 for development of coronary artery disease using 1,661 subjects in the same cohort yielded similar results (Fig. 7). These results indicate that rs2230500 in PRKCH is a genetic risk factor involved in the development of cerebral infarction and myocardial infarction. Although this finding needs to be reproduced in other prospective studies, the present inventors think that in the future it may become possible to predict the risk for cerebral infarction using.

Table 9 below shows the age- and sex-adjusted, multivarialbe analysis-adjusted relative risks for development of cerebral infarction according to a 14-year follow-up for the genotype in general population of 1,638 Japanese.

**Table 9**

| | | | Age- and sex-adjusted | | | Multivariable-adjusted | | |
|---|---|---|---|---|---|---|---|---|
| Genotype | Number | Event | HR | (95%c.i.) | p-Value | HR | (95%c.i.) | p-Value |
| GG | 1063 | 39 | 1.00 | | | 1.00 | | |
| AG | 518 | 23 | 1.31 | (0.78-2.19) | 0.309 | 1.31 | (0.78-2.20) | 0.317 |
| AA | 61 | 5 | 2.83 | (1.11-7.22) | 0.030 | 2.91 | (1.14-7.47) | 0.026 |
| | | | | | | | | |
| GG+AG | 1581 | 62 | 1.00 | | | 1.00 | | |
| AA | 61 | 5 | 2.58 | (1.03-6.44) | 0.043 | 2.66 | (1.06-6.68) | 0.038 |

In Table 9 above, "HR" refers to hazard ratio and "c.i." refers to confidence interval. Adjustments for multivariable analysis were made for age, sex, hypertension, diabetes, cholesterol, smoking, and drinking.

### <Materials, methods, and such for various experiments relating to the AGTRL1 gene>

### Test Population

Since 1961, the present inventors have been conducting cohort studies using a regional population on cardiovascular diseases in residents of Hisayama which is a suburban area adjacent to Fukuoka city, Japan (Hata J. et al., J. Neurol. Neurosurg. Psychiatry 2005; 76: 368-372; and Tanizaki Y., et al., Stroke 2000, 31: 2616-2622). Between 2002 and 2003, the present inventors performed screening studies on residents of Hisayama, and 3,328 residents aged of 40 or more (78% of the whole population belonging to this age group) participated in this study. Of them, 3,196 participants (96%) agreed to use their own clinical data and DNA samples for the present study.

The present inventors registered cerebral infarction patients from Kyushu University Hospital and six affiliated hospitals in the Fukuoka urban area (National Hospital Organization Kyushu Medical Center, National Hospital Organization Fukuoka-Higashi Medical Center, Japanese Red Cross Fukuoka Hospital, Hakujyuji Hospital, Imazu Red Cross Hospital, and Seiai Rehabilitation Hospital). All test cases were diagnosed as cerebral apoplexy by a neurologist using clinical information and brain imaging tests including computed tomography (CT) and/or magnetic resonance imaging (MRI), and subdivided into LA, AT, CE, and other subtypes. The subjects were all Japanese and written informed consents were obtained for participation in the study.

This study has been approved by the ethics committees of the Faculty of Medical Sciences in Kyushu University, the Institute of Medical Science in the University of Tokyo, and each participating hospital.

### Correlation analysis

The present inventors performed genome-wide correlation analysis in a stepwise manner consisting of first and second screening. In the first screening, 188 test cases affected by cerebral infarction were randomly selected. For each case, one age- and sex-matched control subject was randomly selected from Hisayama residents who had never been affected by cerebral apoplexy or coronary artery disease.

In the second screening, 860 test cases with LA and AT were incorporated as controls. Other subtypes of CE and cerebral infarction were not included in the second screening since they have mechanisms that are different from LA and AT. For each test case, one age- and sex-matched control subject was randomly selected from Hisayama residents. In both groups, the mean age ± s.d. was 70 ± 10 years old, and 60.7% of the subjects were men.

### SNP genotyping

Genomic DNA samples were extracted from whole blood by standard methods. Many genomic fragments were amplified using each polymerase chain reaction (PCR), and SNPs were genotyped using invader assay (Ohnishi Y., et al., J. Hum. Genet. 2001, 46:471-477; Lyamichev V., et al., Nat. Biotech. 1999, 17:292-296).

### Cell Culture

Human gastric adenocarcinoma SBC-3 cells were grown in RPMI 1640 medium containing 10% fetal bovine serum (FBS) and 1% antibiotic/antifungal solution (SIGMA). Human embryonic kidney fibroblast 293T cells were grown in Dulbecco's modified Eagle medium containing 10% FBS and 1% antibiotic/antifungal solution (SIGMA). Both cells were incubated at 37°C and humidified air containing 5% CO₂.

### Electrophoretic mobility shift assay (EMSA)

Probes used for EMSA were constructed as 25-bp double-stranded oligonucleotides around each polymorphism as shown in Fig. 9a. Sp1-concensus oligonucleotide (SantaCruz, sc-2502) was used as a positive control for Sp1 binding. Each probe was labeled with [γ-³²P]-ATP (Amersham) using T₄ polynucleotide kinase (TOYOBO). 10 µg of SBC-3 nuclear extract was incubated at room temperature for 30 minutes with a probe (>500,000 cpm) in a reaction mixture containing 15 mM Tris-HCl (pH7.5), 6.5% glycerol, 50 mM KCl, 0.7 mM EDTA (pH8.0), 0.2 mM dithiothreitol, 1 mg/mL bovine serum albumin, 1 µg of poly (dI-dC), and 0.1 µg of salmon sperm DNA. For the Sp1 supershift assay, 2 µg of anti-human Sp1 goat polyclonal antibody (SantaCruz, sc-59X) was added, and this was further incubated at room temperature for 30 minutes. This mixture was subjected to electrophoresis at 120 V for three hours on a 4% polyacrylamide gel containing 0.5x Tris-borate-EDTA buffer. After drying the gel, it was exposed to an X-ray film.

### Overexpression of Sp1 and reverse transcriptase polymerase chain reaction (RT-PCR)

Full-length human Sp1 cDNA was subcloned into a pCAGGS expression vector (pCAGGS-Sp1). 293T cells grown until nearly confluent in a 6-well culture plate were transfected with 1 µg of pCAGGS-Sp1 or mock pCAGGS using 3 µL of FuGENE6 (Roche) per well. Total RNA was collected at various times before or after transfection, and purified using the RNeasy Mini Kit and RNase-free DNase Set (QIAGEN). cDNA was synthesized using Superscript II reverse transcriptase (Invitrogen). Expression levels of human AGTRL1 and housekeeping gene B2M were determined using semi-quantitative RT PCR, and were quantitatively confirmed by Real Time RT-PCR.

For semi-quantitative RT-PCR, ExTaq DNA polymerase (TaKaRa) and each of the following primer pairs were used to amplify the cDNA: AGTRL1
(5'-CTGTGGGCTACCTACACGTAC-3' (SEQ ID NO: 7) and
5'-TAGGGGATGGATTTCTCGTG-3' (SEQ ID NO: 8)), and B2M
(5'-CACCCCCACTGAAAAAGATGA-3' (SEQ ID NO: 9) and
5'-TACCTGTGGAGCAACCTGC-3' (SEQ ID NO: 10)).

For real-time PCR, cDNA was amplified using SYBR Premix ExTaq (TaKaRa) and analyzed using ABI PRISM 7700 (Applied Biosystems) and each of the following primer pairs:
AGTRL1 (5'-TGCCATCTACATGTTGGTCTTC-3' (SEQ ID NO: 11) and
5'-GTCACCACGAAGGTCAGGTC-3' (SEQ ID NO: 12)), and B2M
(5'-TCTCTCTTTCTGGCCTGGAG-3' (SEQ ID NO: 13) and
5'-AATGTCGGATGGATGAAACC-3' (SEQ ID NO: 14)). mRNA was quantified by correlating the obtained PCR threshold cycle to a cDNA standard curve. Standardized AGTRL1 expression level was obtained by dividing the AGTRL1 value by the B2M value.

### Luciferase assay

A DNA fragment corresponding to nt-291 to -248 of the 5' flanking region of AGTRL1 containing any one of the alleles of SNP4 (rs9943582), and/or to nt+1329 to +1381 of the intron containing any one of the alleles of SNP9 (rs2282624) was synthesized, and cloned into the multicloning site of the pGL3-basic reporter plasmid (Promega). SBC-3 cells grown to be nearly confluent in a 24-well culture plate were transfected with 90 ng of each reporter construct, 10 ng of pRL-CMV vector used as an internal control of transfection efficiency, and 100 ng of either the pCAGGS-Sp1 or mock pCAGGS vector, using 0.6 µL of FuGENE6 (Roche) per well. The cells were collected 48 hours later and luciferase activity was measured using a Dual-Luciferase Reporter Assay System (Promega).

### Statistical Analyses

Statistical analyses for correlation analysis and Hardy-Weinberg equilibrium were performed as described by Yamada *et al.* (Yamada, R., et al., Am. J. Hum. Genet. 2001; 68: 674-685). For adjustment of multiple tests, the present inventors performed 10,000 permutation tests using the MULTITEST method of SAS software (SAS Institute). Haplotype frequency and linkage disequilibrium index (D' and Δ2) were evaluated using an expectation-maximization algorithm. Relative luciferase activities were compared by Student's t-test.

### URL

The JSNP database is available at http://snp.ims.u-tokyo.ac.jp/index.html. The International HapMap Project database is available at http://www.hapmap.org. The dbSNP database provided by the National Center of Biotechnology Information in the U.S. is available at http://www.ncbi.nlm.nih.gov/SNP/index.html. The GENSCAN program is available at http://genes.mit.edu/GENSCAN.html. The MATCH program is available at http://www.gene-regulation.com/.

### [Example 5] Genome-wide correlation analysis

The present inventors performed genome-wide correlation analysis in a stepwise manner. For the first screening, 188 Japanese patients with cerebral infarction and 188 age- and sex-matched control subjects were used, and 52,608 gene-based SNPs were selected from the JSNP database (Haga H., et al., J. Hum. Genet. 2002; 47: 605-610) by a high-throughput multiplex PCR-invader assay method (Ohnishi Y., et al., J. Hum. Genet. 2001; 46: 471-477). Genotypes and allele frequencies were compared between the cases and controls, and 1,098 types of SNPs whose p-values were shown to be less than 0.01 were identified.

Next, for second screening, the present inventors used 860 patients with LA or AT and 860 age- and sex-matched control subjects to genotype these 1,098 types of SNPs. The present inventors discovered that of them, an SNP in the AGTRL1 gene (SNP6, rs948847) showed low p-values in the allele frequency model (p = 0.000028) and the recessive model (p = 0.000057). The adjusted p-value in the recessive model after performing permutation tests for multiple tests was 0.0498. The present inventors considered that SNP6 was a candidate marker SNP associated with cerebral infarction.

### [Example 6] High precision mapping around marker SNP6

To examine the chromosome 11q12 region where SNP6 is positioned, the present inventors used all subjects to genotype 48 types of SNPs in the 240-kb region around AGTRL1. This region covered seven genes: PRG2, PRG3, P2RX3, SSRP1, TNKS1BP1, AGTRL1, and LRRC55. Of them, ten types of SNPs were in AGTRL1, and 38 types of other SNPs whose minor allele frequency was more than 20% were selected from the database of International HapMap Project (The International HapMap Consortium. Nature 2005; 437: 1299-1320) and the JSNP database (Haga H., et al., J. Hum. Genet. 2002; 47: 605-610).

The present inventors constructed a linkage disequilibrium (LD) map of this region (Fig. 8a). These D'-values showed that marker SNP6 is associated with the region covered by five genes (P2RX3, SSRP1, TNKS1BP1, AGTRL1, and LRRC55). Therefore, it was difficult to determine a single candidate gene implicated in cerebral infarction from the LD mapping alone.

Subsequently, the present inventors evaluated the P-values in correlation analyses within this region (Fig. 8b). SNP showing the most significant association was positioned between the TNKS1BP1 gene and the AGTRL1 gene, but there were no reports of genes or expression sequence tags in this region, and open reading frames could not be predicted by the GENSCAN program either. SNPs in P2RX3, SSRP1, TNKSBP1, and LRRC55 had lower degrees of significance of association compared to that of the marker SNP6 in the AGTRL1 gene. Furthermore, the present inventors selected AGTRL1 as a candidate gene implicated in cerebral infarction since the APJ receptor, which is an AGTRL1 product, has been reported to be expressed in the cardiovascular system (Kleinz MJ, et al., Regul. Pept. 2005; 126: 233-240) and the central nervous system (O'Carroll AM, et al., J. Neuroendocrinol. 2003; 15: 661-666), and has a function associated with the cardiovascular system (Kagiyama S, et al., Regl. Pept. 2005; 125: 55-59; Seyedabadi M, et al., Auton. Neurosci. 2002, 101: 32-38; Katugampola SD, et al., Br. J. Pharmacol. 2001, 132: 1255-1260; Tatemoto K, et al., Regul. Pept. 2001, 99: 87-92; Masri B, et al., FASEB J. 2004, 18: 1909-1911; Hashimoto Y, et al., Int. J. Mol. Med. 2005, 16: 787-792).

### [Example 7] SNP analysis of the AGTRL1 gene

The AGTRL1 gene consists of two exons and one intron (Fig. 8c). The protein-coding region exists only in exon 1. By the direct sequencing described in a previous report (Saito S., et al., J. Hum. Genet. 2003; 48: 461-468), the region ranging from 2 kb upstream of the transcription start site to the last exon has been screened for genetic mutations in the AGTRL1 gene. In that report, nine SNPs, two simple-repeat polymorphisms, and one insertion/deletion (I/D) polymorphism were found. The present inventors discovered another SNP (SNP5) in the 5' untranslated region (UTR) of exon 1 that had not been discovered in previous reports, but was already registered as rs11544374 in the dbSNP database. The present inventors genotyped these ten types of SNPs using 860 test cases having LA and AT, and 860 control subjects. The I/D polymorphism in the intron was also genotyped by direct sequencing, and was found to have absolute linkage with SNP7 and SNP10.

In this case-control study, there were nine polymorphisms showing significant association with cerebral infarction (SNP2, 3, 4, 5, 6, 7, 9, 10, and I/D) (Table 10).

Table 10 below shows case-control correlation analysis of the AGTRL1 gene.

**Table 10**

| SNP | dbSNP ID | SNP position (1/2) | Case (n=860) | | | | Control (n=860) | | | | Allele frequency (1/2) | | Recessive model (11/12+22) | | Dominant model (11/12+22) | |
|---|---|---|---|---|---|---|---|---|---|---|---|---|---|---|---|---|
| | | | 11 | 12 | 22 | Total | 11 | 12 | 22 | Total | OR (95%CI) | P-value | OR (95%CI) | P-value | OR (95%CI) | P-value |
| SNP1 | rs4939123 | -1433T/A | 4 | 105 | 745 | 854 | 1 | 81 | 778 | 860 | 1.40(1.04~1.87) | 0.024 | 4.04(0.45~36.2) | 0.18 | 1.39(1.02~1.88) | 0.034 |
| SNP2 | rs7119375 | -1176C/T | 450 | 347 | 59 | 858 | 373 | 391 | 91 | 855 | 1.35(1.17~1.56) | 0.000054 | 1.43(1.18~1.73) | 0.00022 | 1.61(1.14~2.27) | 0.0061 |
| SNP3 | rs10501367 | -799G/A | 451 | 345 | 59 | 855 | 373 | 391 | 91 | 855 | 1.36(1.17~1.57) | 0.000043 | 1.44(1.19~1.75) | 0.00016 | 1.61(1.14~2.26) | 0.0062 |
| SNP4 | rs9943582 | -279G/A | 450 | 346 | 59 | 855 | 376 | 389 | 94 | 859 | 1.36(1.17~1.57) | 0.000040 | 1.43(1.18~1.73) | 0.00024 | 1.66(1.18~2.33) | 0.0033 |
| SNP5 | rs11544374 | +212G/A (5'UTR) | 495 | 314 | 47 | 856 | 427 | 362 | 69 | 858 | 1.31(1.13~1.53) | 0.00043 | 1.38(1.14~1.67) | 0.00082 | 1.51(1.03~2.21) | 0.036 |
| SNP6 | rs948847 | +445A/C (Gly45Gly) | 431 | 357 | 70 | 858 | 345 | 405 | 101 | 851 | 1.36(1.18~1.57) | 0.000028 | 1.48(1.22~1.79) | 0.000057 | 1.52(1.10~2.09) | 0.011 |
| SNP7 | rs746886 | IVS1+1155T/C | 159 | 398 | 299 | 856 | 110 | 412 | 337 | 859 | 1.24(1.08~1.42) | 0.0025 | 1.55(1.19~2.02) | 0.0010 | 1.20(0.99~1.46) | 0.065 |
| SNP8 | rs2282625 | IVS1+1338C/T | 375 | 382 | 98 | 855 | 368 | 400 | 92 | 860 | 1.01(0.87~1.16) | 0.92 | 1.04(0.86~1.26) | 0.66 | 0.93(0.68~1.25) | 0.61 |
| SNP9 | rs2282624 | IVS1+1355G/A | 182 | 410 | 264 | 856 | 124 | 418 | 318 | 860 | 1.31(1.14~1.50) | 0.00012 | 1.60(1.25~2.06) | 0.00021 | 1.32(1.08~1.61) | 0.0073 |
| SNP10 | rs2282623 | IVS1+1440A/G | 160 | 399 | 300 | 859 | 112 | 411 | 331 | 854 | 1.22(1.06~1.39) | 0.0052 | 1.52(1.17~1.97) | 0.0018 | 1.18(0.97~1.44) | 0.10 |

In Table 10 above, "1" refers to risk allele, "2" refers to non-risk allele, "OR" refers to odds ratio, and "CI" refers to confidence interval.

Furthermore, the results of correlation analyses in all cerebral infarctions are shown in Tables 11-1 and 11-2 below. The results of correlation analyses in the lacunar group and atheroma group are shown in Tables 12-1 and 12-2 below.

**Table 11-1**

| | Patient group (1:risk allele) | | | | Control group (1:risk allele) | | | | 2x3 Contingency table (genotype) | (1/2) | (11/12+22) | (11+12/22) |
|---|---|---|---|---|---|---|---|---|---|---|---|---|
| dbSNP ID | 11 | 12 | 22 | Total | 11 | 12 | 22 | Total | p-Value | p-Value | p-Value | p-Value |
| rs1939489 | 583 | 445 | 78 | 1106 | 496 | 498 | 118 | 1112 | 1.15E-04 | 2.28E-05 | 1.33E-04 | 3.15E-03 |
| rs4938861 | 584 | 444 | 80 | 1108 | 496 | 498 | 118 | 1112 | 1.54E-04 | 3.01E-05 | 1.34E-04 | 5.06E-03 |
| rs1892964 | 586 | 445 | 77 | 1108 | 493 | 501 | 117 | 1111 | 5.62E-05 | 1.12E-05 | 6.02E-05 | 2.82E-03 |
| rs1892963 | 585 | 445 | 77 | 1107 | 498 | 496 | 118 | 1112 | 1.03E-04 | 2.06E-05 | 1.46E-04 | 2.36E-03 |
| rs7102963 | 581 | 447 | 78 | 1106 | 493 | 498 | 117 | 1108 | 1.39E-04 | 2.85E-05 | 1.55E-04 | 3.60E-03 |
| rs499318 | 585 | 444 | 76 | 1105 | 493 | 498 | 117 | 1108 | 5.40E-05 | 1.07E-05 | 7.04E-05 | 2.15E-03 |
| rs1893675 | 536 | 467 | 105 | 1108 | 442 | 535 | 134 | 1111 | 1.87E-04 | 7.88E-05 | 4.58E-05 | 4.96E-02 |
| rs4939123 | 9 | 133 | 964 | 1106 | 2 | 107 | 1001 | 1110 | 1.87E-02 | 9.94E-03 | 3.39E-02 | 2.49E-02 |
| rs7119375 | 576 | 447 | 85 | 1108 | 482 | 508 | 115 | 1105 | 2.31 E-04 | 6.17E-05 | 8.18E-05 | 2.48E-02 |
| rs10501367 | 577 | 445 | 84 | 1106 | 482 | 510 | 115 | 1107 | 1.38E-04 | 3.73E-05 | 4.84E-05 | 2.16E-02 |
| rs9943582 | 574 | 448 | 85 | 1107 | 486 | 507 | 118 | 1111 | 2.88E-04 | 6.62E-05 | 1.32E-04 | 1.63E-02 |
| rs11544374 | 641 | 400 | 67 | 1108 | 557 | 463 | 88 | 1108 | 1.27E-03 | 3.50E-04 | 3.43E-04 | 8.03E-02 |
| rs948847 | 551 | 455 | 99 | 1105 | 452 | 523 | 128 | 1103 | 1.12E-04 | 4.27E-05 | 2.76E-05 | 4.07E-02 |
| ss49849485 | | | | | | | | | | | | |
| rs746886 | 212 | 503 | 393 | 1108 | 145 | 543 | 423 | 1111 | 5.00E-04 | 3.10E-03 | 9.66E-05 | 2.03E-01 |
| rs746885 | 0 | | 22 1086 | 1108 | 0 | | 12 1099 | 1111 | #DIV/0! | 8.37E-02 | #DIV/0! | 8.25E-02 |
| rs2282625 | 499 | 483 | 125 | 1107 | 481 | 511 | 119 | 1111 | 5.33E-01 | 6.71E-01 | 3.98E-01 | 6.62E-01 |
| rs2282624 | 243 | 517 | 348 | 1108 | 161 | 558 | 393 | 1112 | 2.85E-05 | 1.24E-04 | 5.35E-06 | 4.94E-02 |
| rs2282623 | 213 | 505 | 391 | 1109 | 147 | 541 | 417 | 1105 | 8.38E-04 | 4.39E-03 | 1.67E-04 | 2.25E-01 |
| rs721608 | 641 | 402 | 64 | 1107 | 585 | 433 | 93 | 1111 | 1.08E-02 | 2.89E-03 | 1.29E-02 | 1.74E-02 |
| rs1943482 | 291 | 527 | 289 | 1107 | 228 | 563 | 321 | 1112 | 5.24E-03 | 4.40E-03 | 1.29E-03 | 1.45E-01 |
| rs10896586 | 204 | 517 | 387 | 1108 | 154 | 526 | 432 | 1112 | 8.54E-03 | 3.86E-03 | 3.47E-03 | 5.56E-02 |
| rs2156456 | 225 | 505 | 375 | 1105 | 151 | 527 | 429 | 1107 | 8.88E-05 | 9.19E-05 | 2.58E-05 | 1.85E-02 |
| rs71721 | 186 | 500 | 420 | 1106 | 120 | 516 | 474 | 1110 | 1.40E-04 | 2.11E-04 | 4.17E-05 | 2.33E-02 |

Table 11-2 is the continuation of Table 11-1.

**Table 11-2**

| | Position on the gene | | 1 | 2 | | |
|---|---|---|---|---|---|---|
| dbSNP ID | *AGTRL1* | | Risk | Non-risk | JSNP | NT_033903.7 |
| rs1939489 | | | C | A | | 2371291 |
| rs4938861 | | | G | T | | 2361845 |
| rs1892964 | | | G | T | | 2346515 |
| rs1892963 | | | C | T | | 2342835 |
| rs7102963 | | | T | G | | 2332629 |
| rs499318 | | | T | C | | 2318117 |
| rs1893675 | | | C | A | | 2315139 |
| rs4939123 | 5'flank. -1433 | SNP1 | T | A | ssj0009877 | 2312016 |
| rs7119375 | 5'flank, -1176 | SNP2 | C | T | ssj0009878 | 2311759 |
| rs10501367 | 5'flank. -799 | SNP3 | G | A | ssj0009879 | 2311382 |
| rs9943582 | 5'flank, -279 | SNP4 | G | A | ssj0009880 | 2310862 |
| rs11544374 | ex1(5UTR).212 | SNP5 | G | A | | 2310372 |
| rs948847 | ex1(Gly45Gly). 445 | SNP6 | A | C | IMS-JST092074 | 2310139 |
| ss49849485 | int1. 1045-1048 | I/D | del | ins | ssj0009883 | 2308013-2308016 |
| rs746886 | int1. 1155 | SNP7 | T | C | ssj0009884 | 2307906 |
| rs746885 | int1. 1234 | | T | C | | 2307827 |
| rs2282625 | int1. 1338 | SNP8 | C | T | IMS-JST031820 | 2307723 |
| rs2282624 | int1. 1355 | SNP9 | G | A | IMS-JST031819 | 2307706 |
| rs2282623 | int1. 1440 | SNP10 A | | G | IMS-JST031818 | 2307621 |
| rs721608 | | | T | C | | 2303718 |
| rs1943482 | | | C | T | | 2301404 |
| rs10896586 | | | A | G | | 2289898 |
| rs2156456 | | | T | C | | 2280894 |
| rs717211 | | | T | C | | 2268354 |

**Table 12-1**

| | Patient group (1: risk allele) | | | | Control group (1: risk allele) | | | | 2x3 Contingency table (genotype) | (1/2) | (11/12+22) | (11+12/22) |
|---|---|---|---|---|---|---|---|---|---|---|---|---|
| dbSNP ID | 11 | 12 | 22 | Total | 11 | 12 | 22 | Total | p-Value | p-Value | p-Value | p-Value |
| rs1939489 | 458 | 340 | 56 | 854 | 384 | 379 | 97 | 860 | 5.59E-05 | 1.19E-05 | 2.01E-04 | 6.08E-04 |
| rs4938861 | 459 | 339 | 58 | 856 | 384 | 379 | 97 | 860 | 8.68E-05 | 1.65E-05 | 2.02E-04 | 1.14E-03 |
| rs1892964 | 462 | 338 | 56 | 856 | 381 | 382 | 96 | 859 | 2.76E-05 | 5.12E-06 | 6.79E-05 | 7.36E-04 |
| rs1892963 | 461 | 339 | 56 | 856 | 385 | 378 | 97 | 860 | 4.71E-05 | 9.58E-06 | 1.67E-04 | 5.76E-04 |
| rs7102963 | 458 | 339 | 57 | 854 | 381 | 380 | 96 | 857 | 6.31E-05 | 1.22E-05 | 1.48E-04 | 1.03E-03 |
| rs499318 | 459 | 339 | 55 | 853 | 379 | 380 | 97 | 856 | 2.07E-05 | 4.19E-06 | 8.07E-05 | 3.91E-04 |
| rs1893675 | 417 | 364 | 75 | 856 | 340 | 412 | 108 | 860 | 2.31E-04 | 5.50E-05 | 1.28E-04 | 1.08E-02 |
| rs4939123 | 4 | 105 | 745 | 854 | 1 | 81 | 778 | 860 | 6.11E-02 | 2.40E-02 | 1.77E-01 | 3.37E-02 |
| rs7119375 | 450 | 347 | 59 | 856 | 373 | 391 | 91 | 855 | 2.42E-04 | 5.37E-05 | 2.14E-04 | 6.09E-03 |
| rs10501367 | 451 | 345 | 59 | 855 | 373 | 391 | 91 | 855 | 1.95E-04 | 4.25E-05 | 1.60E-04 | 6.23E-03 |
| rs9943582 | 450 | 346 | 59 | 855 | 376 | 389 | 94 | 859 | 1.89E-04 | 4.01E-05 | 2.42E-04 | 3.34E-03 |
| rs11544374 | 495 | 314 | 47 | 856 | 427 | 362 | 69 | 858 | 1.84E-03 | 4.32E-04 | 8.18E-04 | 3.55E-02 |
| rs948847 | 431 | 357 | 70 | 858 | 345 | 405 | 101 | 851 | 1.15E-04 | 2.80E-05 | 5.72E-05 | 1.06E-02 |
| ss49849485 | | | | | | | | | | | | |
| rs746886 | 159 | 398 | 299 | 856 | 110 | 412 | 337 | 859 | 3.29E-03 | 2.54E-03 | 1.02E-03 | 6.52E-02 |
| rs746885 | 0 | 16 | 840 | 856 | 0 | 9 | 851 | 860 | #DIV/0! | 1.57E-01 | #DIV/0! | 1.55E-01 |
| rs2282625 | 375 | 382 | 98 | 855 | 368 | 400 | 92 | 860 | 7.21E-01 | 9.25E-01 | 6.55E-01 | 6.14E-01 |
| rs2282624 | 182 | 410 | 264 | 856 | 124 | 418 | 318 | 860 | 3.24E-04 | 1.17E-04 | 2.13E-04 | 7.27E-03 |
| rs2282623 | 160 | 399 | 300 | 859 | 112 | 411 | 331 | 854 | 6.23E-03 | 5.15E-03 | 1.80E-03 | 1.00E-01 |
| rs721608 | 503 | 305 | 47 | 855 | 448 | 336 | 75 | 859 | 3.89E-03 | 9.17E-04 | 5.42E-03 | 9.22E-03 |
| rs1943482 | 218 | 417 | 220 | 855 | 176 | 422 | 262 | 860 | 1.70E-02 | 4.19E-03 | 1.33E-02 | 2.92E-02 |
| rs10896586 | 151 | 412 | 293 | 856 | 121 | 398 | 341 | 860 | 2.77E-02 | 7.04E-03 | 4.29E-02 | 2.00E-02 |
| rs2156456 | 171 | 395 | 288 | 854 | 117 | 391 | 347 | 855 | 4.04E-04 | 8.12E-05 | 4.64E-04 | 3.34E-03 |
| rs717211 | 141 | 392 | 322 | 855 | 93 | 390 | 375 | 858 | 9.70E-04 | 3.77E-04 | 6.60E-04 | 1.09E-02 |

Table 12-2 is the continuation of Table 12-1.

**Table 12-2**

| | position | | 1 | 2 | | |
|---|---|---|---|---|---|---|
| dbSNP ID | *AGTRL1* | | Risk | Non-risk | JSNP | NT_033903.7 |
| rs1939489 | | | C | A | | 2371291 |
| rs4938861 | | | G | T | | 2361845 |
| rs1892964 | | | G | T | | 2346515 |
| rs1892963 | | | C | T | | 2342835 |
| rs7102963 | | | T | G | | 2332629 |
| rs499318 | | | T | C | | 2318117 |
| rs1893675 | | | C | A | | 2315139 |
| rs4939123 | 5'flank. -1433 | SNP1 | T | A | ssj0009877 | 2312016 |
| rs7119375 | 5'flank. -1176 | SNP2 | C | T | ssj0009878 | 2311759 |
| rs10501367 | 5'flank. -799 | SNP3 | G | A | ssj0009879 | 2311382 |
| rs9943582 | 5'flank. -279 | SNP4 | G | A | ssj0009880 | 2310862 |
| rs11544374 | ex1(5UTR).212 | SNP5 | G | A | | 2310372 |
| rs948847 | ex1(Gly45Gly). 445 | SNP6 | A | C | IMS-JST092074 | 2310139 |
| ss49849485 | int1. 1045-1048 | I/D | del | ins | ssj0009883 | 2308013-2308016 |
| rs746886 | int1.1155 | SNP7 | T | C | ssj0009884 | 2307906 |
| rs746885 | int1.1234 | | T | C | | 2307827 |
| rs2282625 | int1.1338 | SNP8 | C | T | IMS-JST031820 | 2307723 |
| rs2282624 | int1.1355 | SNP9 | G | A | IMS-JST031819 | 2307706 |
| rs2282623 | int1.1440 | SNP10 | A | G | IMS-JST031818 | 2307621 |
| rs721608 | | | T | C | | 2303718 |
| rs1943482 | | | C | T | | 2301404 |
| rs10896586 | | | A | G | | 2289898 |
| rs2156456 | | | T | C | | 2280894 |
| rs717211 | | | T | C | | 2268354 |

SNPs that cause amino acid substitution were not present. Therefore, the present inventors considered that some of these polymorphisms might change the promoter activity or enhancer activity of the AGTRL1 gene.

### [Example 8] Haptotype analysis of the AGTRL1 gene

The present inventors constructed a set of haptotypes of AGTRL1. Among the nine types of SNPs significantly associated with cerebral infarction, SNP2, 3 and 4 were completely linked. Similarly, SNP7, and 10 were completely linked. Upon examination of these SNPs, the present inventors used five types of tag SNPs (SNP2, SNP5, SNP6, SNP9, and SNP10) for haptotype estimation and found three types of high-frequency haptotypes that cover more than 90%of subjects in both groups (Table 13).

Table 13 below shows the results of haptotype analysis of the AGTRL1 gene.

**Table 13**

| | | Haplotype frequency | | | | Recessive model | | Dominant model | |
|---|---|---|---|---|---|---|---|---|---|
| Haplotype | SNPs | Case (%) | Control (%) | OR (95%Cl) | P-value | OR (95%Cl) | P-value | OR (95%Cl) | P-value |
| Hap1 | C-G-A-G-A | 41.6 | 36.5 | 1.23(1.08~1.42) | 0.0024 | 1.60(1.22∼2.09) | 0.00058 | 1.20(0.98~1.45) | 0.075 |
| Hap2 | C-G-A-A-G | 26.3 | 26.0 | 1.02(0.87~1.18) | 0.83 | 1.00(0.68~1.48) | 0.99 | 1.03(0.85~1.24) | 0.79 |
| Hap3 | T-A-C-A-G | 23.4 | 28.8 | 0.76(0.65~0.88) | 0.00032 | 0.66(0.45~0.97) | 0.033 | 0.72(0.59~0.87) | 0.00062 |

In Table 13 above, five SNPs (SNP2, 5, 6, 9, and 10) were used for haplotype estimation. "OR" refers to odds ratio and "CI" refers to confidence interval.

SNPs in Hap1 were all risk alleles. Risk for cerebral infarction found in Hap1 was significantly higher than those in other haplotypes (odds ratio (OR) = 1.60 (95% confidence interval (CI), 1.22 to 2.09); p = 0.00058 in the recessive model). On the other hand, SNPs of Hap3 were all non-risk alleles, and risk for cerebral infarction found in Hap 3 was significantly lower than those in others (OR = 0.76 (95% CI, 0.65 to 0.88); p=0.00032 in an allele frequency model). As a result, Hap1, Hap2, and Hap3 were determined to be haplotypes of the risk type, intermediate type, and non-risk type, respectively.

### [Example 9] Sp1 transcription factor binds to the risk allele of SNP4 and SNP9 of the AGTRL1 gene

To select cell systems that highly express AGTRL 1 mRNA, the present inventors performed RT-PCR using cDNA from 89 types of cell systems. As a result, SBC-3 cells expressed AGTRL1 mRNA at the highest level (data not shown).

EMSA was performed to evaluate the binding between the transcription factor and DNA sequences around SNPs. ³²P-labeled double-stranded DNA probes were constructed for each allele of the nine candidate polymorphisms, and electrophoresis was performed after incubation with nuclear extract from SBC-3 cells (Fig. 9a). The present inventors found that DNA-protein binding was detected in risk allele SNP4 (-279G), but the same binding was not detected in the non-risk allele (-279A) of SNP4. The present inventors also discovered that DNA-protein binding was detected in the risk allele (+1355G) of SNP9, but not in the non-risk allele (+1355A). Similar results were obtained by using nuclear extracts from 293T cells (data not shown). The sequences of the probes used in these assays are shown in Fig. 9b. These results suggest the possibility that some kind of transcription factor binds in an allele-specific manner to transactivate AGTRL1 expression.

From the MATCH program using the TRANSFAC database, transcription factor Sp1 was predicted to bind to the DNA sequences around the risk alleles of SNP4 and SNP9 (Fig. 9c). To confirm the binding between these SNPs and Sp1 *in vitro,* the present inventors performed Sp1 supershift assays using a specific antibody (Fig. 9d). The band for the DNA-protein complex in the risk allele of SNP4 supershifted upward in the presence of an anti-Sp1 antibody. A similar supershift was also detected for the risk allele of SNP9.

According to the above, the present inventors concluded that the Sp1 transcription factor binds to the DNA sequences around the risk alleles of SNP4 and SNP9. The present inventors established a hypothesis that the interaction between Sp1 and these SNPs may affect the promoter activity or enhancer activity of the AGTRL1 gene.

### [Example 10] Sp1 induces the transcription of AGTRL1 mRNA

To confirm the hypothesis that Sp1 affects transcription of the AGTRL1 gene, the present inventors transfected 293 T cells with either the pCAGGS mock vector or the Sp1-expression vector (pCAGGS-Sp1), and compared the AGTRL1 mRNA levels by semi-quantitative RT-PCR at various time points (Fig. 10a). The present inventors found that AGTRL mRNA was not detected in endogenous 293T cells, but overexpression of Sp1 significantly induced transcription of AGTRL 1 mRNA. The same results were quantitatively confirmed by real-time RT-PCR as well (Fig. 10b).

### [Example 11] Sp1 activates the promoter function in the risk allele of SNP4

To evaluate the promoter activity in SNP4, the present inventors prepared constructs in which a 44-bp fragment around SNP4 corresponding to the risk allele (-279G-Luc) or non-risk allele (-279A-Luc) was contained in the luciferase reporter vector (pGL3-basic), and then performed luciferase assays using SBC-3 cells cotransfected with one of the constructs and mock pCAGGS vector or pCAGGS-Spl vector (Fig. 11a). In cells transfected with mock pCAGGS, increase of luciferase activity due to the SNP4 fragment was not observed. The luciferase activity of Sp1-overexpressing cells with the risk allele was at a level 2.3 times greater than that of the control (pGL3-basic). The activity of cells with the non-risk allele was at a level only 1.7 times greater than that of the control. That is, the function of the promoter around SNP4 was activated in the presence of the Sp1 transcription factor, and the risk allele showed a significantly stronger activity than the non-risk allele (p = 0.003).

### [Example 12] The combination of SNP4 and SNP9 affects the transcription ofAGTRL1

The present inventors prepared constructs containing a 44-bp fragment around SNP4 and a 53-bp fragment around SNP9 in the luciferase reporter vector (pGL3-basic) for each of the three haplotypes shown in Table 13, and then performed luciferase assays using SBC-3 cells cotransfected with one of the constructs and mock pCAGGS vector or pCAGGS-Sp1 vector (Fig. 11b). In cells transfected with mock pCAGGS, the Hap1 construct (risk haplotype) showed the highest luciferase activity, and the Hap3 construct (non-risk haplotype) showed the lowest luciferase activity. The Hap2 construct (intermediate haplotype) showed intermediate activity. As a result, an intronic enhancer that enhances the promoter activity of the AGTRL1 gene was found to be present near the risk allele of SNP9 (+1355G). These data match the results of the odds ratio in haplotype analyses (Table 11). The activity was further enhanced in Sp1-overexpressing cells.

### Industrial Applicability

The present inventors performed genome-wide association study using SNPs, which targeted the whole genome to identify cerebral infarction-related genes. The patient group consisted of 1,112 cerebral infarction patients who were making regular hospital visits to the Kyushu University Graduate School of Medical Sciences, Department of Medicine and Clinical Science and related facilities. For the control group, a sex- and age-matched control subject was randomly selected for each case in the patient group from Hisayama residents who had taken health examinations conducted from 2002 to 2003. Written informed consents were obtained after explanation by those in charge of explaining based on the "ethical guidelines for human genome/genetic analysis research" co-issued by the Ministry of Education, Culture, Sports, Science, and Technology, Ministry of Health, Labour and Welfare, and Ministry of Economy, Trade and Industry, and then blood samples and clinical information were obtained. In the first screening, 188 cases were randomly selected from each of the patient group (1,112 cases) and the control group (1,112 cases), and 52,608 SNPs distributed in the whole genome were measured. The second screening was performed using all subjects for the 1,098 SNPs which were confirmed to show association between the patient group and the control group in the first screening, and genes implicated in cerebral infarction were identified. SNPs in the whole genome were measured using a high-throughput SNP typing system that uses the invader method of the Institute of Medical Science, the University of Tokyo.

As a result, the AGTRL 1 (Angiotensin II receptor-like 1) gene and PRKCH (Protein kinase C eta) gene were identified as cerebral infarction-related genes. In the AGTRL1 gene, most of the SNPs present at the 5' side, within the gene, and at the 3' side showed significant difference of p<1x10⁻⁴ between the patient group and the control group. This relationship was also observed when the subjects were limited to patients with arteriosclerosis-related cerebral infarction (lacunar infarction and atherothrombotic infarction). Furthermore, luciferase assay and gel shift assay showed differences in the binding of the transcription factor among the SNPs of the AGTRL1 gene, and difference in the binding of the transcription factor to the region containing the SNP at the 5' side or the 3' side was found to cause change in the expression level of the AGTRL1 gene mRNA. Therefore, it was considered that in the AGTRL1 gene, change in the AGTRL1 expression level due to difference in the binding of the transcription factor to the regions containing a SNP at the 5' or 3' side is associated with the onset of arteriosclerotic diseases such as cerebral infarction.

On the other hand, in the PRKCH gene, significant difference of p<1x10⁻⁴ was observed only in the region at the central part (intron 5 to intron 10) of the gene in the group of patients with arteriosclerosis-related cerebral infarction (lacunar infarction group and atherothrombotic infarction group). Direct sequencing of this region confirmed an SNP (rs2230500) in which the amino acid at position 374 is substituted from valine (Val) to isoleucine (Ile) through change of the allele from G to A, and there were significantly more people that carry Ile (A allele) in the cerebral infarction patient group. *In vitro* autophosphorylation assay was conducted to examine the effect of this amino acid substitution on the activity of protein kinase η (Protein kinase C eta; PKC-eta), which is a gene product of PRKCH, and revealed amino acid substitution from Val to Ile enhanced the autophosphorylation reaction of PKC-eta. More specifically, the SNP (rs2230500) which substitutes the amino acid at position 374 of PKC-eta from Val to Ile affects the activity of PKC-eta, and this difference in activity was considered to be related to cerebral infarction. Furthermore, immunohistological staining of autopsy samples of human coronary arteries was conducted using a commercially available antibody against PKC-eta (rabbit polyclonal antibody, Santa Cruz). PKC-eta was expressed in the endothelial cells of the human coronary artery and in the arteriosclerotic plaques, and the degree of expression strongly correlated with the degree of arteriosclerosis. More specifically, PKC-eta was found to be deeply implicated with the onset/development of human arteriosclerosis. Furthermore, to examine whether the SNP (rs2230500) accompanying amino acid substitution of PKC-eta is implicated in the onset of human arteriosclerotic diseases, 1,683 cases were used to examine its relation to the onset of cerebral infarction and myocardial infarction. The cases used were the third population of the Hisayama study which had underwent a general Hisayama resident health examination in 1988 and were under long-term observation, and their genomic DNAs were collected from 2002 to 2003. During the 14-year follow-up period, onset of cerebral infarction was observed in 67 cases and onset of myocardial infarction was observed in 37 cases. Those who had the AA allele for rs2230500 had 2.83 times higher risk for cerebral infarction than those with the GG allele. Similarly, the risk for myocardial infarction was 2.89 times higher in those with AA than in those with GG. From these results, SNP (rs2230500) which substitutes the amino acid at position 374 existing in exon 9 of the PRKCH gene from Val to Ile is considered to increase in the activity of PKC-eta through amino acid substitution, promote arteriosclerosis in humans via various types of signal transduction, and ultimately increase the risk for cerebral infarction and myocardial infarction in ordinary residents.

In conventional search for candidate genes using genome-wide correlation studies in common diseases such as hypertension, myocardial infarction, cerebral apoplexy, diabetes, chronic rheumatoid arthritis, and such, case-control studies are used in which candidate regions are narrowed down by comparing the differences in SNP frequency between the disease group and control group. However, it is well known that the incidence rate of diseases such as cerebral infarction increases with aging, and the incidence rates differ between sexes. Therefore, when SNP differences are compared between the patient group and the control group, the age and male-female ratio of the two groups have to be taken into account. However, there are no reports so far in which the disease group and the control group completely matched. One of the reasons is that while collecting samples for the patient group can be accomplished relatively easily since it consists of patients visiting the hospital, it is difficult to collect samples for the control group which consists of ordinary residents who do not carry diseases, and in order to match the sex and age, it may be necessary to collect samples several times greater in number than the patient group. Since the epidemiological population of Hisayama consisted of ordinary residents who had taken health examinations and it included a number of those who did not have illnesses (such population was difficult to collect in previous studies), it was possible to set a control group in which the sex and age matched for each case in the patient group. Furthermore, it is known that in case-control studies, if there is a bias when establishing the control group, difference between the disease group and the control group may be different from it really should be (selection bias). Since the residents of Hisayama have a standard Japanese composition in terms of sex, age, and such according to results of previous epidemiological investigations, they have been proven to be a sample population of the entire Japanese population. A control group that has been established randomly from this population is considered to be unbiased. Therefore, it is considered that disease-related genes have been identified with greater accuracy and higher precision compared to reports made in the past by establishing the Hisayama population as the control group of this search for cerebral infarction-related genes.

The greatest advantage of using the Hisayama residents is that this population is a population used in a long-term prospective follow-up study targeting ordinary residents. To examine whether disease-related genes discovered by genome-wide association studies and their genetic polymorphisms are truly related to the diseases, at this time, a population completely different from the population from which the disease-related genes were selected is used to perform case-control studies and examine whether or not there is reproducibility. However, even if reproducibility is observed using a different population, it may be false positive since the possibility of bias due to selection of subject population cannot be denied in case-control studies. Generally, to examine the association of risk factors with diseases, the research method of a prospective follow-up study (cohort study) which performs a long-term follow-up on a population that has not developed the disease and examines the effect of risk factors on the onset of the disease is considered to be the most accurate. However, since collection of subjects and follow-up studies involve an enormous amount of time, effort, and money, there have been no reports so far of examining the relation of genetic polymorphisms to diseases using this method. Since Hisayama cohort is a population established for this purpose and previous populations have also been subjected to continued follow-up investigation, the data of a 14-year long-term prospective follow-up investigation made it possible to examine the association of genetic polymorphisms with cerebral infarction as in the PRKCH gene of this study. This result is the first case in the world that has proven at the level of ordinary residents that genetic polymorphisms are related to the onset of a disease. Furthermore, since the Hisayama cohort has a standard Japanese composition in terms of sex, age, and such, it can be regarded as a sample population of the entire Japanese population, and the results obtained this time may be applicable not only to Hisayama residents but to the entire Japanese population.

## Claims

1. A method for testing whether or not a subject has a risk factor for arteriosclerotic disease, which uses the subject's AGTRL1 gene expression as an index.

2. A method for testing whether or not a subject has a risk factor for arteriosclerotic disease, which comprises detecting DNA mutation in the subject's AGTRL1 gene.

3. The method of claim 2, wherein said mutation changes the binding with an Sp1 transcription factor.

4. A method for testing whether or not a subject has a risk factor for arteriosclerotic disease, which uses the subject's PRKCH gene expression as an index.

5. A method for testing whether or not a subject has a risk factor for arteriosclerotic disease, which comprises detecting DNA mutation in the subject's PRKCH gene.

6. The method of any one of claims 1 to 5, wherein the mutation is a polymorphic mutation.

7. A method for testing whether or not a subject has a risk factor for arteriosclerotic disease, wherein the method comprises determining type of nucleotide at a polymorphic site in the subject's AGTRL 1 gene.

8. The method of claim 7, wherein the polymorphic site is in the AGTRL1 gene located at (1a) position 1, (2a) position 12541, (3a) position 21545, (4a) position 33051, (5a) position 35365, (6a) position 39268, (7a) position 39353, (8a) position 39370, (9a) position 39474, (10a) position 39553, (11a) position 39665, (12a) position 41786, (13a) position 42019, (14a) position 42509, (15a) position 43029, (16a) position 43406, (17a) position 43663, (18a) position 46786, (19a) position 49764, (20a) position 64276, (21a) position 74482, (22a) position 78162, (23a) position 93492, or (24a) position 102938 of the nucleotide sequence of SEQ ID NO: 1.

9. The method of claim 8, wherein the subject is determined to have a risk factor for arteriosclerotic disease when the nucleotides at the polymorphic sites of (1a) to (24a) of claim 8 are (1b) to (24b) below, respectively:
(1b) the type of nucleotide in the complementary strand of the AGTRL1 gene located at position 1 of the nucleotide sequence of SEQ ID NO: 1 is T;
(2b) the type of nucleotide in the complementary strand of the AGTRL1 gene located at position 12541 of the nucleotide sequence of SEQ ID NO: 1 is T;
(3b) the type of nucleotide in the complementary strand of the AGTRL1 gene located at position 21545 of the nucleotide sequence of SEQ ID NO: 1 is A;
(4b) the type of nucleotide in the complementary strand of the AGTRL1 gene located at position 33051 of the nucleotide sequence of SEQ ID NO: 1 is C;
(5b) the type of nucleotide in the complementary strand of the AGTRL1 gene located at position 35365 of the nucleotide sequence of SEQ ID NO: 1 is T;
(6b) the type of nucleotide in the complementary strand of the AGTRL1 gene located at position 39268 of the nucleotide sequence of SEQ ID NO: 1 is A;
(7b) the type of nucleotide in the complementary strand of the AGTRL1 gene located at position 39353 of the nucleotide sequence of SEQ ID NO: 1 is G;
(8b) the type of nucleotide in the complementary strand of the AGTRL1 gene located at position 39370 of the nucleotide sequence of SEQ ID NO: 1 is C;
(9b) the type of nucleotide in the complementary strand of the AGTRL1 gene located at position 39474 of the nucleotide sequence of SEQ ID NO: 1 is T;
(10b) the type of nucleotide in the complementary strand of the AGTRL1 gene located at position 39553 of the nucleotide sequence of SEQ ID NO: 1 is T;
(11 b) the nucleotide in the AGTRL1 gene located at position 39665 of the nucleotide sequence of SEQ ID NO: 1 has been deleted;
(12b) the type of nucleotide in the complementary strand of the AGTRL1 gene located at position 41786 of the nucleotide sequence of SEQ ID NO: 1 is A;
(13b) the type of nucleotide in the complementary strand of the AGTRL1 gene located at position 42019 of the nucleotide sequence of SEQ ID NO: 1 is G;
(14b) the type of nucleotide in the complementary strand of the AGTRL1 gene located at position 42509 of the nucleotide sequence of SEQ ID NO: 1 is G;
(15b) the type of nucleotide in the complementary strand of the AGTRL1 gene located at position 43029 of the nucleotide sequence of SEQ ID NO: 1 is G;
(16b) the type of nucleotide in the complementary strand of the AGTRL1 gene located at position 43406 of the nucleotide sequence of SEQ ID NO: 1 is C;
(17b) the type of nucleotide in the complementary strand of the AGTRL1 gene located at position 43663 of the nucleotide sequence of SEQ ID NO: 1 is T;
(18b) the type of nucleotide in the complementary strand of the AGTRL1 gene located at position 46786 of the nucleotide sequence of SEQ ID NO: 1 is C;
(19b) the type of nucleotide in the complementary strand of the AGTRL1 gene located at position 49764 of the nucleotide sequence of SEQ ID NO: 1 is T;
(20b) the type of nucleotide in the complementary strand of the AGTRL1 gene located at position 64276 of the nucleotide sequence of SEQ ID NO: 1 is T;
(21 b) the type of nucleotide in the complementary strand of the AGTRL1 gene located at position 74482 of the nucleotide sequence of SEQ ID NO: 1 is C;
(22b) the type of nucleotide in the complementary strand of the AGTRL1 gene located at position 78162 of the nucleotide sequence of SEQ ID NO: 1 is G;
(23b) the type of nucleotide in the complementary strand of the AGTRL1 gene located at position 93492 of the nucleotide sequence of SEQ ID NO: 1 is G; and
(24b) the type of nucleotide in the complementary strand of the AGTRL1 gene located at position 102938 of the nucleotide sequence of SEQ ID NO: 1 is C.

10. A method for testing whether or not a subject has a risk factor for arteriosclerotic disease, wherein the subject is determined to have a risk factor for arteriosclerotic disease when a DNA block showing the following haplotype is detected:
(A) a haplotype in which the nucleotides in the complementary strand of the AGTRL1 gene at polymorphic sites located at positions 39268, 39353, 41786, 42019, and 43406 of the nucleotide sequence of SEQ ID NO: 1 are A, G, A, G, and C, respectively.

11. A method for testing whether or not a subject has a risk factor for arteriosclerotic disease, which comprises the step of determining the type of nucleotide of a linked polymorphic site present within a DNA block showing the following haplotype:
(A) a haplotype in which the nucleotides of the complementary strand at polymorphic sites on the AGTRL1 gene located at positions 39268, 39353, 41786, 42019, and 43406 of the nucleotide sequence of SEQ ID NO: 1 are A, G, A, G, and C, respectively.

12. A method for testing whether or not a subject has a risk factor for arteriosclerotic disease, which comprises the steps of:
(a) determining the type of nucleotide at a polymorphic site in the AGTRL1 gene of the subject; and
(b) determining that the subject has a risk factor for arteriosclerotic disease when the nucleotide determined in (a) is the same as the nucleotide at said polymorphic site in the AGTRL1 gene showing the haplotype of (A):
(A) a haplotype in which the nucleotides of the complementary strand at polymorphic sites in the AGTRL1 gene located at positions 39268, 39353, 41786, 42019, and 43406 of the nucleotide sequence of SEQ ID NO: 1 are A, G, A, G, and C, respectively.

13. The method of claim 12, wherein said polymorphic site of (a) is in the AGTRL1 gene located at any one of positions 1, 12541, 21545, 33051, 35365, 39268, 39353, 39370, 39474, 39553, 39665, 41786, 42019, 42509, 43029, 43406, 43663, 46786, 49764, 64276, 74482, 78162, 93492, or 102938 of the nucleotide sequence of SEQ ID NO: 1.

14. A method for testing whether or not a subject has a risk factor for arteriosclerotic disease, wherein the subject is determined to have a risk factor for arteriosclerotic disease when the expression level of the subject's AGTRL1 gene is elevated compared to that of a control.

15. A method for testing whether or not a subject has a risk factor for arteriosclerotic disease, wherein the method comprises determining the type of nucleotide at a polymorphic site in the subject's PRKCH gene.

16. The method of claim 15, wherein the polymorphic site is in the PRKCH gene located at (1a) position 1, (2a) position 16212, (3a) position 30981, (4a) position 32408, (5a) position 33463, (6a) position 34446, (7a) position 39322, (8a) position 39469, (9a) position 39471, (10a) position 49248, (11a) position 49367, or (12a) position 52030 of the nucleotide sequence of SEQ ID NO: 2.

17. The method of claim 16, wherein the subject is determined to have a risk factor for arteriosclerotic disease when the nucleotides in the polymorphic sites of (1a) to (12a) of claim 16 are the following (1b) to (12b), respectively:
(1b) the nucleotide in the PRKCH gene located at position 1 of the nucleotide sequence of SEQ ID NO: 2 is A;
(2b) the nucleotide in the PRKCH gene located at position 16212 of the nucleotide sequence of SEQ ID NO: 2 is G;
(3b) the nucleotide in the PRKCH gene located at position 30981 of the nucleotide sequence of SEQ ID NO: 2 is A;
(4b) the nucleotide in the PRKCH gene located at position 32408 of the nucleotide sequence of SEQ ID NO: 2 is G;
(5b) the nucleotide in the PRKCH gene located at position 33463 of the nucleotide sequence of SEQ ID NO: 2 is G;
(6b) the nucleotide in the PRKCH gene located at position 34446 of the nucleotide sequence of SEQ ID NO: 2 is T;
(7b) the nucleotide in the PRKCH gene located at position 39322 of the nucleotide sequence of SEQ ID NO: 2 is T;
(8b) the nucleotide in the PRKCH gene located at position 39469 of the nucleotide sequence of SEQ ID NO: 2 is A;
(9b) the nucleotide in the PRKCH gene located at position 39471 of the nucleotide sequence of SEQ ID NO: 2 is C;
(10b) the nucleotide in the PRKCH gene located at position 49248 of the nucleotide sequence of SEQ ID NO: 2 is C;
(11b) the nucleotide in the PRKCH gene located at position 49367 of the nucleotide sequence of SEQ ID NO: 2 is G; and
(12b) the nucleotide in the PRKCH gene located at position 52030 of the nucleotide sequence of SEQ ID NO: 2 is A.

18. A method for testing whether or not a subject has a risk factor for arteriosclerotic disease, wherein the subject is determined to have a risk factor for arteriosclerotic disease when the autophosphorylation activity or kinase activity of the subject's PRKCH protein is elevated compared to that of a control.

19. A method for testing whether or not a subject has a risk factor for arteriosclerotic disease, wherein the subject is determined to have a risk factor for arteriosclerotic disease when the subject carries a mutant protein in which valine at position 374 in the amino acid sequence of the PRKCH protein is substituted with isoleucine.

20. The method of any one of claims 1 to 19, wherein a biological sample derived from the subject is subjected to the test as a test sample.

21. A reagent for testing for the presence or absence of a risk factor for arteriosclerotic disease, which comprises an oligonucleotide that hybridizes with a DNA comprising the polymorphic sites of (1a) to (24a) of claim 8 or (1a) to (12a) of claim 16 and has a length of at least 15 nucleotides.

22. A reagent for testing for the presence or absence of a risk factor for arteriosclerotic disease, which comprises a solid phase to which a nucleotide probe is immobilized, wherein the nucleotide probe hybridizes with a DNA comprising the polymorphic sites of (1a) to (24a) of claim 8 or (1a) to (12a) of claim 16.

23. A reagent for testing for the presence or absence of a risk factor for arteriosclerotic disease, which comprises a primer oligonucleotide for amplifying a DNA comprising the polymorphic sites of (1 a) to (24a) of claim 8 or (1a) to (12a) of claim 16.

24. A reagent for testing for the presence or absence of a risk factor for arteriosclerotic disease, which comprises (a) or (b) as an active ingredient:
(a) an oligonucleotide that hybridizes with a transcript of an AGTRL1 or PRKCH gene; and
(b) an antibody that recognizes an AGTRL 1 or PRKCH protein.

25. A reagent for screening for a pharmaceutical agent for treating or preventing arteriosclerotic disease, which comprises any one of (a) to (c) as an active ingredient:
(a) an oligonucleotide that hybridizes with a transcript of an AGTRL gene;
(b) an antibody that recognizes anAGTRL1 protein; and
(c) a polynucleotide comprising a DNA region which comprises a nucleotide site in a AGTRL 1 gene located at position 39353 or 42509 of the nucleotide sequence of SEQ ID NO: 1.

26. A reagent for screening for a pharmaceutical agent for treating or preventing arteriosclerotic disease, which comprises any one of (a) to (c) as an active ingredient:
(a) an oligonucleotide that hybridizes with a transcript of a PRKCH gene;
(b) an antibody that recognizes a PRKCH protein; and
(c) a mutant PRKCH protein which has an amino acid sequence in which valine at position 374 of the amino acid sequence of a PRKCH protein is substituted with isoleucine.

27. A pharmaceutical agent for treating or preventing arteriosclerotic disease, which comprises as an active ingredient a substance that suppresses the expression of an AGTRL1 or PRKCH gene or suppresses the function of a protein encoded by said gene.

28. The pharmaceutical agent of claim 27, wherein the substance that suppresses the expression of the AGTRL1 or PRKCH gene is a compound selected from the group consisting of (a) to (c):
(a) an antisense nucleic acid against a transcript of the AGTRL1 or PRKCH gene or a portion thereof;
(b) a nucleic acid having a ribozyme activity of specifically cleaving a transcript of the AGTRL1 or PRKCH gene; and
(c) a nucleic acid having an effect of inhibiting the expression of the AGTRL1 or PRKCH gene through an RNAi effect.

29. The pharmaceutical agent of claim 27, wherein the substance that suppresses the function of the AGTRL1 or PRKCH protein is the compound of (a) or (b):
(a) an antibody that binds to an AGTRL1 or PRKCH protein; or
(b) a low-molecular-weight compound that binds to an AGTRL 1 or PRKCH protein.

30. A pharmaceutical agent for treating or preventing arteriosclerotic disease, which comprises as an active ingredient a substance that inhibits the binding of an Sp1 transcription factor with a DNA region that comprises a nucleotide site in the AGTRL1 gene located at position 39353 or 42509 of the nucleotide sequence of SEQ ID NO: 1.

31. A pharmaceutical agent for treating or preventing arteriosclerotic disease, which comprises as an active ingredient a substance that inhibits the autophosphorylation activity of a PRKCH protein.

32. A method of screening for a pharmaceutical agent for treating or preventing arteriosclerotic disease, which comprises selecting a compound that reduces the expression level of an AGTRL1 or PRKCH gene or reduces the activity of a protein encoded by said gene.

33. A method of screening for a pharmaceutical agent for treating or preventing arteriosclerotic disease, which comprises the steps of:
(a) contacting a test compound with a cell that expresses an AGTRL1 or PRKCH gene;
(b) measuring the expression level of said AGTRL 1 or PRKCH gene; and
(c) selecting the compound that reduces the expression level as compared with that measured in the absence of the test compound.

34. A method of screening for a pharmaceutical agent for treating or preventing arteriosclerotic disease, which comprises the steps of:
(a) contacting a test compound with a cell or cell extract that comprises a DNA having a structure in which a transcriptional regulatory region of an AGTRL 1 or PRKCH gene and a reporter gene are operably linked with each other;
(b) measuring the expression level of said reporter gene; and
(c) selecting a compound that reduces said expression level as compared with that measured in the absence of the test compound.

35. The method of any one of claims 32 to 34, wherein the AGTRL1 gene is a mutant AGTRL1 gene of (a) or (b) whose expression is enhanced:
(a) a mutant AGTRL 1 gene in which the nucleotide in the complementary strand of the AGTRL gene located at position 42509 of the nucleotide sequence of SEQ ID NO: 1 is G; or
(b) a mutant AGTRL 1 gene in which the nucleotide in the complementary strand of the AGTRL1 gene located at position 39353 of the nucleotide sequence of SEQ ID NO: 1 is G.

36. A method of screening for a pharmaceutical agent for treating or preventing arteriosclerotic disease, which comprises the steps of:
(a) contacting a test compound with an Sp1 transcription factor and a polynucleotide comprising a DNA region that comprises a nucleotide site in an AGTRL1 gene located at position 39353 or 42509 of the nucleotide sequence of SEQ ID NO: 1;
(b) measuring the binding activity between said polynucleotide and the Sp 1 transcription factor; and
(c) selecting a compound that reduces said binding activity as compared with that measured in the absence of the test compound.

37. A method of screening for a pharmaceutical agent for treating or preventing arteriosclerotic disease, which comprises the steps of:
(a) contacting a test compound with a PRKCH protein;
(b) measuring the autophosphorylation activity of the PRKCH protein; and
(c) selecting a compound that reduces the autophosphorylation activity as compared with that measured in the absence of the test compound.

38. The method of claim 37, wherein said PRKCH protein is a mutant protein in which valine of position 374 in the amino acid sequence of the PRKCH protein is substituted with isoleucine.

39. A method of screening for a pharmaceutical agent for treating or preventing arteriosclerotic disease, which comprises the steps of:
(a) contacting a test compound with a PRKCH protein;
(b) measuring the protein kinase activity of the PRKCH protein; and
(c) selecting a compound that reduces the protein kinase activity as compared with that measured in the absence of the test compound.


SEQUENCE LISTING

<110> KYUSHU UNIVERSITY, NATIONAL UNIVERSITY CORPORATION
THE UNIVERSITY OF TOKYO
HISAYAMA RESEARCH INSTITUTE FOR LIFESTYLE DISEASES
NTT DATA CORPORATION
HUBIT GENOMIX, INC.

<120> GENE ASSOCIATED WITH ARTERIOSCLEROTIC DISEASE, AND USE THEREOF

<130> K9-A0601P

<150> JP 2006-121284
<151> 2006-04-25

<160> 47

<170> PatentIn version 3.3

<210> 1
<211> 102938
<212> DNA
<213> Homo sapiens

<400> 1
gaagggaatg aaactcctca tgggaaatgg tcatcgggat gagaaggaag gaaactgaga 60

agaagaaaaa aaatgctacc aagctagaga gttcactgat gacattcaga tttcagaaag 120

tctaccagaa atcaccacag cagggatagg tctgccaggt tgctgggaaa aagttactcc 180

tagtggatgc tggtaaagag gctatctaaa gagaaaatgt tggctgtatt gtattgctgg 240

tcattattcc caaccccctc aacatggaag gcagagatca caaggaaata caaacgccac 300

tgtgacacag ttacaagaaa aataatattg ttaggtgccc ccatcctcca cccagcttta 360

cacctatctc atggaatcat tcaggagtat actgcatgac attctagaaa acacaaaata 420

atgtggctaa ttttacaaag ctaaggattc tattcctgta gctgagccag tattcaaaag 480

agatccctgg atctgcatat taaaagccaa agctgggctg ggcacagtgg ctcacgcctg 540

taatcccagc agtttgggag gcagaggcag gtggatcacc aaaggtcagg agttcgagac 600

cagcctggcc aaaatggtga aagctggttt ccactaaaaa tacaaaaaaa ttagccgagc 660

gtagcggtgc atgcctgtag tcccagctac tcaggaggga gaggcagcag aatcacttga 720

acccaagggg tggaggttgc agtgagctga gatcacgcca ttgcactcca gcctgggtga 780

aaagagcaaa acgcagtctt aaataaataa ataagccaaa gctatgaaaa tggttgatat 840

aagaagacta cttggccaag agttagattc ccatataaat aagcagttta tgcctccatt 900

catttaaaaa tatgaatccc acacctctac atgctagaca tatatttcca gggctgggta 960

tatggtgctg aagaaactaa cattcctgcc cctgcccctt ggtgtctttc tatgcagggg 1020

tcggggtgag agggtcggta atggtggtag atcagacaat aaacaagaaa aataaatagt 1080

tccagatagt gataagtgct gcaaagaaaa tacagcagga agaggtttgg ggggtgagat 1140

tacagaggta ggcctagatc tcacccgata agtaggtcct tgtgggctct ggtcagactt 1200

caaattttgt ataaatgcaa tggaaaacca ctggagggct ttcagcaggg gaataacatg 1260

atctgacgcg attttccaag accgttctag atgctgtatg ttcaaaaggt gggaaagaat 1320

atataagctg tgacatcagg gaggggcctt ctccatgagt ccaggggaaa ggcattccaa 1380
agggacacca gggctggtgg tggatgaagg aacaatcaga gtagaaattt gaaaggcaga 1440
gccaatagga cttgctgatg cattggatgt agggagacag gacaaaagaa aaatcaaaaa 1500
taactcctaa tctttttttt cacatcagct gcagcaatgc tcagccaggc ctaagtagcg 1560
gaaagactgt cttaattcta ttaaaaaaca tacacacaca acagatagtc aacaatcagt 1620
aagtcagaaa accctgatga gtaagttgga agggagaggg aggaagcaga tagaaaaaca 1680
gattcataat gtcaggaaac ggccaaaacc tgagggcccg gagatggggg tagaaaaata 1740
tgaacacaca gagttggttt caacagtctg caatatcatt cggagtgtcc cagtggacct 1800
taaatagagc aatggggcct cgggaactgg agttcaagtc taataatcag cagcattttc 1860
agtagagata gatttgtatc catagtgatg tgctagaggc ggagcatcaa tctaaagttg 1920
tttcctagaa ggagagtctt agactccctt gactgtatac caggaaacaa acccaggaaa 1980
caaattatat ttgagatggg ctcaaatatg aaatgggaat attgcacaga atcacataaa 2040
gaaaaatggg aatgagcagc ttgtactcac tcatcttaac caaggaatga aaggcaattg 2100
tgagaaacta ggtgtctcag gatggtgcta atactgagaa catccgaaac cgttataatc 2160
attatgcatt tacaatgtat tacacactgc accaatgtac ttgcaatatc tcacggatgt 2220
ctcacgattt tcctggaaga aagcacagac ttgattaaaa gcatggacta ggaaaccaca 2280
ctccctggtt tcaaatccca atactgccac cttgggcagc tgacatcacc tctttgaatc 2340
tgagtttctc tgcctgtaaa tttcagagga taatggtagc tacttcatag gacctctgag 2400
aagattaaat gagctcatgt aaataaagta cttagaacag tgcctgggac agagtgatat 2460
tcaattagtg atagcgacta ttactatcat tcctttttta cagggaaaga cttagaagct 2520
gagagatgct taagtaactt gtctaatgtc acagggctga aatgtgacaa agccagggtt 2580
ttaatcacct ctgtcctcca gacccagtat ctatcagata ccagataggt cacactggca 2640
agctgtgaaa gcgagtctcc aaacctggga ccaagtgaac tatgacctca atattcatat 2700
ccatgagtgg tccactccca cactgaatct gcgctggccc tgtgacttga tttaaccaac 2760
aaaaagtggc agaagtaatg ctgtgcctgg cagcttctgc ttttgtgctc ttggaagccc 2820
tgagccacca tggattaagt ctgatccatg gagagtgatg gagagtccat atggagagac 2880
cacgtggaga gaaaagaagc tcaacctttc cagcatgcca gctgagccca tctttctagc 2940
cattccccca aggcaccaga catattagtg gatacatatt gaacatttca gccccaacta 3000
ccctctgact gcatccccat gggacaccaa agcaagacca gcaaaacaac tctccaacta 3060
agctacagtg gtgacaattt attgttttaa gcctctatgt ttttgttatg caacaatatg 3120
caactgaaac actaatctat aagaaaaaga cttattgggg acaaaaatgt gtcagtaaca 3180
caaggctgaa acctagagtg actgttgcat cccagaagca agctgtacag aaaataatca 3240
tccatgctct ggagtcctat taatgagcat ataatagtgc caaccacaca gtaagaaatc 3300

aacaaaaaat tagctgcttt ttttattatt aacaagaata cacttattct agatgcaatt 3360
taattgcaat gcaaggaata gataaaaatc cttagttttt gcttctaact tctccaggaa 3420
taatgctgca ggaaatttgt atcactttcc tttgctttag gaaatttcag tgcacagtat 3480
tgtaaagact tgattattaa aagatttgac aaggacaaaa aaagttaaga aagagaagat 3540
gaccacaact ggtctcatac tgctcgttaa gagtagtgac ttcctggctg ggcatggtgg 3600
ctcacatctc taatcccagc actttgggag gctgaggcag gtggaccacc tgaggtcagg 3660
agttcaagac cagcctggcc aacatggtga aaccccgtct ctactgaaaa cacaatattt 3720
agctgggcac agtggtgggc gcctataatt ccagctacta gggaggctga ggcaggagaa 3780
tcacttgaac ccaggaggca gaggttgcag tgcaccgaga tcacgccatt gcactccagc 3840
ctgggtagca aaagggaaac tctgtttaaa aaaaaagaaa aaagataggg acttcccaag 3900
ttactcctgc aaaatggcat tgttacagag caatgtatca tgacaggcat tttttgacaa 3960
aaaaaaaaaa aattcatttc caattcagac acaaagtagt atgagatttt gtctatgcaa 4020
agaattttaa acctgtctca ctccacaatg gtgggagaat attgagttcc cagaaataaa 4080
gcagcagtca aaggtagcac agacaaagac gctgagccca tgagttacat agaggagtgc 4140
agctgtactg cattcatcat tttgatgaat aaatagataa gggaaaagca aggccttcca 4200
tagaaataaa ggttatttct gtagattact ttaaacgtat aataaagtcc ttcagcgaaa 4260
aaaatagaca ttccaacaga gatctcaggg aacaagagca acatggaata cccagaagac 4320
gatctagatt gaacagaata gaatcatcag cagtagaatt gagaacaatc accagacctt 4380
tggtggaggt ggagctgagc actgatcttt ggatacgtta tacatcctgt attgcttcat 4440
cgaagcaatg gtatagtcca gaggtccttg acctgcgggc cacggatggg tactagtctg 4500
tggcctgtta ggaaccaggc tgcatagcag gaggtgagca gcaggcaagc gagtgagcga 4560
agattcctct gtatttaaag cttctccgca tcattcgcat tattgcctga gctctgtctc 4620
ctgtcagatc agcggtggca ttagattctc atggcagtgc caaccctatt gcgaactgcg 4680
catgcgaggg atctaggttg cacgctcctt aagagaatct aactcctgat gatctgtcac 4740
tgtctcccat cacccccagg tgggaccctc tagttgcagg aaaacaagct cagggctccc 4800
actgatccta caatttggtg agttgtataa ttatttcatt attgttaaca atgtaataat 4860
accaaagtac aaaataaatg taatgcactt gaatcatccc aaaaccattc cccatcccag 4920
gcgtgtggaa aaattgtctt ccatgaaacc attccacggt gacaaaaagg ttggggactg 4980
ctggtttagt ccattgaaaa tcattccttg tttccataaa gatcctcctc ttctaccatt 5040
tgtgatatgt ggagttttgg atgcatgagc cctatctcat tattagagtc ttgggatttg 5100
tcaagagttg gcaaaaccaa cgatgcagcc cactctgatt tcctccttga agaatttatt 5160
tatttctggc taaaagtgct acgatttact ccaggaggct gataattata gaaggcagct 5220
gtagtaatgt agaggaagcc ctaggcttta attcaagaga catagtccct caacaaacac 5280
ttgccgaggc cctggcgtga ggcaaggcaa tgtttaggta catgaagaat tactaaaaac 5340

caaagagatc agagattatt gattcatgtt ttatccaccg tgcccagcat agggctggat 5400
gtgtagcagg ttttcagtaa atgtttgttt aaccaaatta ataatgacac tagttagcaa 5460
atcaatttcc ctctctgggg ttggtatttc acctcctatt ttaaaattta aaaaatgtaa 5520
gtgggttata gcagcacttc tcaaaattga atgtgcataa caatttcccg gggatcctgt 5580
taaggtacat attccaaacc agtaggctgg gaaggaaggc ctggaagttg cgtttctaac 5640
aaacctccag cattactggc ccatggacca cactttgagt agcaatgggg caaatgatct 5700
ctcagggaga aattttggat acaatgaggg atcctccgat tctgtttttt aattatgaaa 5760
aaatagtaga tactctaact atggaaggga gaaaaagaga ggtaaatggg gctgggcacg 5820
gtgcctgggg tggatccagg aggttggtag aggcttctcc tgggggccag caggaaaggc 5880
ccactcttcg tcacaacgca caaacaaatt ctttttggtg cctgcaaaaa gaatcagcaa 5940
taatttcgct ggctcggaag gctctgtctc ttaatgattc ttcctgtttt tctcagcctg 6000
atccaagaga atgagaaaac agccctagtg aaggtcagcg gaggtcagag aggtccctgg 6060
agagggccgc tttcagggaa atggagcaga tagaggccca ccccttctgc ccaccctcat 6120
ctctgcctaa ctctagcgct actccttgac caatccagcc caatcccagt gtatcagtac 6180
tctgccccaa gtcaattcca ttcaattaga cattcattac tactgatgtg tgtgccagca 6240
tgctagatgc cccggatgat gccaggatgt gacactgcca gggccctcca gttgcccatg 6300
gtctaatggg gaggagttcc acagtcacaa gaagacgtgt tacaatgaag aatgcaatca 6360
aggacacgag aaagagatct gcaagtacaa tatgaattta gagaagtgag agaccaaact 6420
ttacagagaa ggggacatct gaaatttaaa gatgggtata agctcttcag taaagatgga 6480
aatgagccat tccaggtgga gggaaagcag gagagacagc acagagggga aggaagagtg 6540
gatgctcacg acagaatcca gtgtgactgg aacaaagggt attttgaaag aatagcctgc 6600
aatggagggt ctgggaggtg caggggagat tttcatcttt agggtcttag tcctcccttt 6660
ctcatcttta gagtctgata attggccctc ccttcaacat gcatatgctt ccctgaccca 6720
tctttaggat catacttgca aaaacagttc aagattaacc acagcaattc atttattcca 6780
caatctttta ccaaaaccct aagacacgtg aggcactgtg caagcatagg aaatacagcc 6840
agggatgaga taggtaagtt ccagagcact ggacaaacaa tcagtaaagt gagacggtag 6900

aaagaacata ggaacaggaa agtcctgggt gcaaatatac gtcgtgtgtc atgaacaaaa 6960
tatttcatat ctctgagccc tggtttctac ctccctgatg gggataatta tacttattcc 7020
acggggttat tgtagaaatt aacctgtggg gaaagagctt catactgtgt tgggcacact 7080
gtaagcattc gttacccatg tattctttgt tcagccataa agagaggcgc ctggaatacc 7140
aaatctccag ggtccctttc agctctgata ttttgcttgg gcagttctaa gactctgcgg 7200
cacctgtaac tgtgcccact gaccagggtc ctggcacatc ccaaggagtg acgctccatc 7260
cctccctgag caagaagctt ctaccagggt ggcctgagat gaggccaatg gatcaggcag 7320
gcattaatca ggtggccgcc ctccttggct gtggcgccgg caggccaggc agcgttctgg 7380

ctgcctagct tccccgatcg gcttccacac cactccacag acccgttgcc atgacagtga 7440
accccaaggg gcacccagga gtggccagag ctggagagga agggttccat tgatcgaaat 7500
ccatctgact cagccttggt tgatttcagg atcctggagt gagaacaaac aacaatttaa 7560
atctactgaa tgcttttttc aatgccagac aacatattaa gtgtgttttt ccgattctct 7620
catttcatac tcacaatggt aacgtgaaat agggatcctc attcccactt aacagaatga 7680
gaaactgggg atcagagaag aaaataccga tgtcacccac atagtaagtg gtaaggccag 7740
gacacaactg tgccccccag ctccagcccc agggatgtat cattccaaag accctgtgct 7800
tttccacaaa ccacagtctt ccgagccacc tgagcctcca ggaaagttga tgccactttc 7860
cccacttgcc tgaatgttcc tgaggggcca agggggacag gtgcagagag ctaggtctct 7920
gagtgattcc tcagagacaa ggccaggtca cccagatgat gggctactca gacagctgcc 7980
aaaaatatgt tctgggattt tccaatgtaa tcccaatttc aaatattctg cttgtttttc 8040
taaagaatga aaattccaac atactgccac caaactaata cgtctctcaa atgagctgtt 8100
gttcatctgg aaacagcaca atatttgaca acatctgtca gaccccgtca caacatttgg 8160
ttctaaagag cacggtccct gtattttgat gcaaggtagc aatgtgcaag ctctggcctt 8220
agatttaggg ctctgacctg ccagaaggag gatatccaga aaccagagga catcaaccct 8280
ttttctaacc tcagcagctg gggaatttgc cgaaagaaca gcttggcatt ccttccctgc 8340
ctttcacccc ccttcccatg gctactgaac tattgagccc aaactctggg aacccatgac 8400
atgggaaaat ccagctggct tctgtagccc cagacatctg gattccctaa ccccaggcag 8460
ccagctgagt gaggaggcag caacacaagg atgcttggga aagccttcgg agggacgtct 8520
gccaagtcat ctccctcctg aacaaacaca tgtcccacta tctaggggtc agcagagcca 8580
tgtttatatg tgcttaggcc attcaactga ttaattaatt agctccctgg gtggcctttg 8640
gccactaaag cttcatccct tgaggcctca gttttcttat ctataaaatg ttagaattag 8700
gcaagttgat gtctaaggtt acttcctgcc ctacgatttc agctttctac cataaaagaa 8760
caggatgagc atggacagga tttggctcta atactgtccc tgctctgcca gcatgggaaa 8820
gtcccttccc atttctaagc ctcagtttcc ttggggagtt aaatgagggg cggactagat 8880
gatctctcag tccagagctc tgttatctca gaaactggaa cacatcttga catgccctct 8940
ccctgatcac ctgcatgcac tgtaacccag cagaccccat ctcgacagct ccattgccac 9000
ctactcaatc aggctgcctg ctctcacctg gaccactgca atagcctcct aactggtcaa 9060
gcaacatctg ccgcccctcc ctcatctcta tgctggagac taccagtgtg aagtttcttt 9120
tcaaaacaca aatcagctag gcacaatggc tcacgcctgt aatcaatccc agaactttga 9180
gaggctgaag caggaggatt acttgagcgt aggagtttga gaccacccgg gacaacatag 9240
caagatccca gctctaccaa aaaaaaaaaa tttaaattat ccaggcatgg gggcgtttgt 9300
gtgtagtccc agctactcaa gaggctgagg caggaagatg acttgagccc aggaggtcag 9360
ggctgcagtg agccatgctc atgccactgc actccagcct gagcaacaga gtgagaccct 9420

gtctcaaaaa aaaaaaaaaa atatatatat atatatatat aagatcatgt tagcctgctg 9480
cttgtatgga gttccattgc aattagggta aacaatagtg cagcttgtga gaccctcacg 9540
gctaacccag cccacccctc agcaccatgt ctcaccaccc tcccttcctc aatgtacaca 9600
agtcccttgg cctttcgctt ccttaataga caagcctccc aagcctcaat ctttgttcat 9660
gcaatttctt tgcctagaat gctgatctgc ttcctctcta tcccactccc ttcttcaact 9720
tgtccatttc ttcccttcct ttagatttca cctcaggcat ctctgcttag aaatcctcct 9780
gggaactcac caggctagct cagattccct gggtagactt tcccataaga ccattgcctt 9840
ccctacagag cattcctgtc catttctgag tgcgttcagg gatgacattg ctttctctca 9900
ttcctactca tcccatagtc tacactcaat aaaccttttc aagatgaggg actggatgtt 9960
ctgcccttag gaagctccta agaagaggat ggcacctttt ccaaaagccc tggcagagac 10020
caagactaaa acagatctga tctaagactg tcaggctccc gaggagacat cctgctgact 10080
gctggaaagc tctgcccaag gcaagcttga gacttagcca attgctatgg ctatgctcag 10140
agttcattcc agcagagggt gctcagaagt ccaccctaga gcgctctgag ctggctgccc 10200
tcccagccag caggcagccc ggggcaatgg ctccagagca tcagattagg aggagagctg 10260
aagtctgtac tgtggaaaga caagatgact agatagattc ttctttggaa atgtcttaaa 10320
gtgccttttc ccccaccttc tccacccact gacttgcaca gatcattcaa gtgtaaatgg 10380
gcctcctcct tcaggaagcc ttcctgtatc acccctgctg gactaaggtt cctaaatctg 10440
catttctatg cctcctgggc atcccactag cattgtatac tactacactg agagaatgtg 10500
tttccacacc tgtctcctca gccagaatag ccttcttcat ctcctatcac cagcatccag 10560
tgcctagaac acaactatca cttgggaaga ctctatcatt ttatagatga ggaaaccaag 10620
atccaggaga aagaatgatt tgtcccccag ggtcacacaa gtcactcact ggcagggcca 10680
ggaatctaac ccacgtctac cacttcaagc tgacaccttt ccctgttgca tccctgcctt 10740

caggaaggga tgggaatgaa tttgcgatag gattcattta tgatcctgtg gacaagagcc 10800
tccaatgact ctccggggat gcctaatcac ccctttataa gaaaggctcc aagagcaaga 10860
tgccggactc tcccatgggg agagcacctg tagcctgttg tgttgcctgc ccaccagctg 10920
gagaggctga ccctactcag tactgagatt aatgaagaat ccaacctcag ttccagcagg 10980
aggtgagcac acgatggcaa tccctccctt ctctgcacgc agaaagttca tcttccagga 11040
aatggagagt tatggttcct tcccaccccc tacccccagt tgatgctttc tgagagtcaa 11100
ccagcattta ttaaatgtgc cgtctcattc ggtcattcaa ccccagacca taagtttctt 11160
gaagtcagga aagtatctta agtttggatc acagaatata ttgttctaaa gggcctttag 11220
gattcaccag gttccagctt ctctgtttac acctaggaaa tggggtccag agggtgaaag 11280
agacttgcca tgtgaatgac tgcagagcca aggtctgggt cctgctccct ggtgtcccac 11340
actgatctct ctgctccatg tctctggact ctggccctga caagaagcct ccttggatcc 11400

ttccaggcct gagagcagag caggtccctg tgaaggtgtg tctgtccgtt gatgggagag 11460
agcatgtcaa gattcaaact gcaaatctga tcgcctcact tctctgttga aggactgcag 11520
tggcttcacc ccaccctcat atgaagttca tccttcagga tgggtcctcc gagaccctga 11580
ccccgatgtc gtctccagac acggccctgt cactctgagc cacagtccct cacttggcac 11640
ttcccagctc ctcccttacc ttaccagctc actcagggct gtttcccacc tcttcacttg 11700
gccaactcct gctcttcctt cagctgaatg cccagcctcc tcctctatga agccttccct 11760
catgttatcc caccccacac acaacctcca aagcaccctg gacttttgga tttccattca 11820
gctcactgtg cttgcttatc ctctctctct ctcctccacc caaccacaga ctcccctggc 11880
ctgggacagt ctctctctcc ccatcatcaa tgtacttgca cagtcaggct ctgtcatcag 11940
cactcagagg atgttcgaca aatattgtag gggtgtttga ttgacaagtg gagaccactc 12000
ccctttatgt gtcctgctcc ttgactcagt ggaaccacag aggccactga gaatggaccc 12060
agaagatagt tcctgctaag gcaggggcag tgatagaaca ggagcagctc atggagaagc 12120
ctctaaccat ctgccccaca ggagtgtctg tccaagacag ctgatgttcc agtctccata 12180
tcagtccagg atgcatgtgg agggggaagt cttcatctcc ctctgatccc cggctgctac 12240
ttcctgaacc ttcttagggc taaggggctg gcaggcaggc gggcggtggg gctgccacac 12300
gctcccactc ctagcacata tcctgtcgct acctcttctg gcaggatgtt attgcagagc 12360
ccagggctgt tattgtcagg agaatggctt tcctgagctc attaaaggac ctctgtcctt 12420
tctccaggac cgacccccct tttcctctgt ccccaatcaa cactgagaga aaggggccct 12480
gggacaactc caggtggagc atcacagctc cccttcctcc tgctacttct ctacttggct 12540
ggctgatgac tatgatcaca gatggtcatc attcctattt gaatgccttt gtgtgcctag 12600
gcattaatgg ccattgacta attcagccct cacagcactc atgtaagata aatgttatta 12660
ttaatctcat ttatacagta gagcaagctg gggctcagag aggttaaatg atgtgctcta 12720
actcacacag caagaaggaa aaggaacagt aattccaaca cagttcttcc tgactccaac 12780
accctttctc ttgcaacacc cacttgctgc catgagctag tttttgttct gtgtaagtgg 12840
agaattgcag tgaacgaggg ggaaaaggcc ttcccaggag ccagcatttc cctacaggga 12900
gtctagaggt cacaggatct tcctcaccct tgagaaagag ctagaactaa gatgtaatag 12960
tgagatagca cccatcttgt gtttgctcag gccttaacca aatcaaagca cttccacccc 13020
tgaaggagag aagccattga ctcttcatca ttcatgggac aaggcacaag aagaatgaag 13080
ataattaaaa actggaaata ataagttgaa tttctcttaa gtgattttgt gttgatggag 13140
cagaaatgta gcagctaaga gaccacagct tggttaccac tagctgggta atcctgatat 13200
atcacaccta ctagggacct cattttccta atctgggaat caggaataac tacccccacc 13260
atacagcatt tgttatatga atccaatgac tccatgtcta tgaaagtgcc ttataaaatg 13320
aagcatacaa cccctgcctc agatttgctg aaaagattaa acacattgat acataaagca 13380
cttagcacag tgcctggaat tcaagtgctc tataaatcaa tggcagctgc tactttgtca 13440

caaagtaatc atttctatta atgcatttat tgttctgctg aagttgtatt tgcagtcctg 13500
cagcacagca gactctgggt aaggaggcag attgggccta cctggtcaac agcaagaacc 13560
ctcctgactt ccacactcaa cacctagagc catgcgccat gaaagagcaa aaccagcacc 13620
tccctaccac ctaataaaac agagagacag gccaggcaca gtggctcaca tgtgtaatcc 13680
caacactttg ggaggctgag gcagaaggat cacctgagcc caggagttca agaccagcct 13740
gggcaataca gtgagatcct ttctttacaa aaaataaaaa attagccagg cacatgctta 13800
tagtcccagc tactcaagag gctgaggcag gagggtcact tgaggccagg aggtctaggc 13860
tgcagtgagc tatgatcaca ttactgcact ccagcctgaa agacagaatg agaccctgtc 13920
tcaaaaacaa aaacaaaaag aagagaaaga gagacagaca gataaacaaa agatgctctt 13980
agaacaaaga tgtgatggga agagcagagc tgtctgcagt ggcattctct ttgttaccac 14040
cagagctaag tttgctactg gacccttatc tgatgcctaa agaaccaaaa gccttttacc 14100
tacataatct caatcaatca ctaccacatc cctaggaggt agggactgtc actttccaaa 14160
gaaggagatc aactttgaga agtttagtga cttgcccaag ctcacttagt ggtgtgatct 14220
gaacctcaaa atgactctaa agcctacttt cttaaatatt caagccgggt ccaaagacca 14280
gtccatcact tactggctgt ggcttgggac aagcctttct tgaatttcca ttttccttgc 14340
ctatgacata agtacacata aaactcacct tgtaggactg ctgtggccat tagggtcttg 14400
tggctacagc cccaacgtaa tgcctgacca aactggtgcc tggttcttct catctttagg 14460
ctacatcaaa ttttcccaaa cctgcttgac cataagaatt acttggcatg ctgaataaaa 14520
acagattcct gaaacctgcc ccacacctgc tgaattcgaa tccttagaaa atagttccca 14580

agagtctgta tttcttttta gaagtctctc atgttcctaa tttttttttt tttttttttt 14640
ttttgatacg gagtcttgct ctgtcaccag gctggagtgc agtggtgcga tcctgcacca 14700
ctgcaacctc cacgtcccgg gttcaagcca ttctcctgcc tcagcctccc cagtagctag 14760
gactacaggc acatgccacc acacctagtt aatttttata tttttagtag agatgggttt 14820
tcaccgtgtt agccaggatg gtctcgatct cttgaccttg caatccacct gcctcagcct 14880
cccaaagtgc taggattaca ggcgtaaacc accacatccg gccgttcata attcttataa 14940
tgagataagc ttgagactca gtagaccagt gctggctaca cattagaatc acctggggga 15000
gcttttgaaa aatcccaatg acagacctca ctcaagaccc actgacttag agttttggag 15060
gtaagcccag ccagacatca ggacttttcc aaagtttcca gatgatacca atgtgcagcc 15120
gcaggggtat gctgaataaa aacagattgt gttatgccaa tgctgttcac aagaattttg 15180
tgccatgatg gaagtgctcc atatctgtac tgtcccgtgt ggtaagtacc aggcacatgt 15240
ggctatagag cccttgaaat gtggccagtg ctgccgaaga actaaaattt taattttatt 15300
taactttaaa taatataaat tggaatttgc atagccacat gtggctattg gctaccgtat 15360
tggaccactg cagctctaga cctaggaggg tacactaccc ctctagataa caggcaggga 15420
ctgaagaatg aaaatgaggg gctctcctcc cttcataaaa ggattgggag gcatgctaag 15480

acatgtcctt ggaaatcaaa atgtttttgt gggctctgag ataacaattg gtcctggtgg 15540
tctgttccat ttggtaaatt cccacctggc ccctttcttg gctcaactga agaaggaaaa 15600
cacccatctc caagcttcca gcaggagcct tgaagttgca ggaaaagcca ggggggcagg 15660
ctgaggagac tagcttctat tttaaagacg ttttcctctt tggaataagc tccatcctcc 15720
aacggtgcag acagggtaaa ggttgtgact gacattgtgt tgctggacat taaacagcct 15780
ccgaggtcac ccggcagctg gtgggtatgt cctgacggac ggctcttaat cgggctcacc 15840
cctgcccctg ggaaccttga ggtcctgggg gtggggcaag gaaagggagg ccagctgttg 15900
gtgatcacca tttgcttttc ccttccccct ccacattccc tgcccctcta tgtcacctca 15960
tcctgcaatg ccctgtgatg atggatgttt gtgagttaaa tggatggaca ggcaggcagg 16020
gagtgtgagt gcagggagca gaccacccgg gcaacaacac aggaagctct gttgggtttc 16080
ttctccctgg tgtgagctcg ttcctggatg acaagctgtg atttccaacc aaatgttttc 16140
ttacagtcac cacacctatt ggttttctct cccctggcaa tatcttcagt ggggcttcag 16200
agagatcatc ttacttttgc tccattaggg cgatgagact tcgagaccag ggtacagggt 16260
catggaagta atacaaagct gcagaagaag gcctgggttg gggcaaggca agcttctgag 16320
cccagtcact tcacccttaa tatgggatag caacacccac ttaacaaggt ggttgtgaaa 16380
ataaaaccag atgatgatta atagatgaat gtggctgtca agtagcaggg gtactcaagt 16440
acgattcacc cttgaataac acagcctgcc tctcccgcct ccccttccat ctcctccacc 16500
tcttctgcct ctgccatccc tgagacagca aaaccaaccc ctccttgcct ccttctttct 16560
cctgggaatg tccactcttt tggcttccct gagccacgtt ggaagaagaa gaattgtctt 16620
gggccacaca tgaaatacac taacactaac aatagctgat gagcttaaaa gaaaaaaaat 16680
cacaaaaaag tctcataatg ttttaagaaa gtttatgaat ttgtgttaga cagcattcaa 16740
agttgtcctg ggccacatgc agccgtgagc cgtaagtttg acaagcttgc tctactcaac 16800
gtgaagacaa ccaggataaa gactattatg ataatctact tacacttaat gaagagtaaa 16860
tatattttat ttccttatgg ttttcttagt aacattttct tttctctaac ttcctttatt 16920
gtaagaatat ggtataaaat acatataaca tacaaactat gtgttaatat gtatgttatc 16980
aataaggctt accatcacca gtagactatt agtagttttc ggggagtaca aagttataca 17040
cggatttttg actgtgcatt gtttaagggt caactgtagt ttaaagtctg agcttcctta 17100
actctgttcc ctaggatttc ttagccttca ccttgccctt tttcctaaat tttctcttaa 17160
tatttctcaa ccctaagctg tgacctttga cccctgtgga tttgttaccc taagaaaatc 17220
ccacaaacac cctaacaatc cccaggcctg aatgaccaca cacaatgggt atggattccc 17280
cacctagagt cagacaaccc tccagcacca ttttctctac aaaccttcag ttccactcaa 17340
gaagcaactg atgtgtgtta ctggtttggt ggctgaacct catgcatggg gtggaacaga 17400
aagaaggcta taaagtcggt aagacacgtg ccctgcattc aaagaactcc taccggtgac 17460
aataactcac aacaccaggt ttcaaagcac tgtcctgact actagctctt aaaacaaccc 17520

aatgaagaga atgtggagat tagtatgaga attcccatat tccagatgaa gacattgaag 17580
ttcagagagt tgaaatgttt tgcttaagtt cacaagtagt agatgtacaa ccaagtctca 17640
aagtcagatt ttttttactt caaatgataa tttgcatgca aagcactcat aataggaact 17700
gaattattga tgagcattct tcattattgt ttatccaaat tccatgccct ttccaacaga 17760
agggaaagag tgtgcatttt aaaaacaaac aggctgtgat taattacaaa cacaggtgtt 17820
taaagaaacc agatattaat atacacttac aagccaagga actatattca tcttttaagg 17880
aacttggtaa tgaatgtggg ttgggaaaac ttttggagac atgatagttt taaaaagggt 17940
agtatttgta tggggagaga gaagaatcag gggccttcca gacagggggt actgaagtta 18000
gcatgcacat tttaccaatt tatacaactc agctatgagg ccaagcattg aagatgcaga 18060
aataaataag acaaggtcgc tgccctggag gaaattacag tccaaagctg atgcttttct 18120
atgggactgt cagggaggat atccaggaaa gtaggcaact cgagctcaga tactgaaagg 18180
gccttgaatg gcagtccagg gaattcggag tttatcctgc cggcaagggg ttgtgaagag 18240
gacttttcct ccaacccctt tgtcctggtc ttggacaaag ggccacacat gacatagcct 18300
cacagtcccc ctttagctac ctcccccact accccgcccc aaagctcatt attctgtaat 18360
cagttcggag aaccacaaga caaggcgata aatctggtcc ttagtgacac tcctctttcc 18420

cctcccccaa gcctttgttc ctccctttgt ctggagatga agcctcccac ccacccaccg 18480
gctggagctt ccaggctccc tcccttccca gcccctcctc tctctcaagc gcccctgcaa 18540
atgggcttta tggtttccac ggaaaccaga gaggcccagg agccaggggg tcttttgcaa 18600
aaacactgtt tgtgccacca tttggggtgg cagagacagt gagaaagtgc aaaaacagct 18660
tacaaatgcc tgcccatcat gctctcgatc tggtttaagt ggggaaagag gaactgagct 18720
catttccact tcaaaggggc tgtgttgggt taagtccaaa ctcagcctgg cagccctaaa 18780
ggaacacaca tatgcacagg cgcacacaca cacacagaca cacacacaca cacagacaca 18840
cacacacaca aagacacaca cacacagaca cacacacaca caaagacaca aagacacaca 18900
cacacagata cacacactca tatacatacc cagtcacact catataaaca caaacataaa 18960
cacacacaca ctcctatacc tacccgcaca gccacactca agcacaaaca tatacacaca 19020
cacacacttt cacacataca cacaaatcca cactcaccag ccccgccttt cccagtacag 19080
ctgggctcag tccactaaga aaacaaacct tccataagac agccctgcac ttagaacacc 19140
ctctgtacat tcccaggatg caaaaaccct tccaatgacc acaccctcag aagcccccat 19200
cccccagccc cttgtgcctc agtcccctgg tcttaaaact gacagttcaa aacttgcttc 19260
ttgcctcttt aagcacctgg tgacaggaga gtcacagatg ctgcagactg gaagcccccc 19320
acccctgtcg gctttcccac gtgatcccag cagaagccat tgatcctgct caacaggaca 19380
aaggatggtg ggcaagtgat aaatgaggac aggagagaaa catttcattt cccactttga 19440
tgggttccag cctgaagatc cagaccccta agagtgtatt caggttggaa aggcaccctc 19500

caagtaccag tagtattttc agggttctgt ggatgctgga caagccatag cagtgcaggg 19560
gaatgaaagt gtgcagcctc tatctttgta acaagtaaca gcattccaga ttgtactcat 19620
cggccattac aaagcagtct ggaatctggt gggcggttct ggtgttccag cttaattttt 19680
tagtaaataa aataacctag ggattagttc ttatccagca cagtctgctt gggagaccag 19740
atatttctga gcatcagggg gtcttggtgg tggggaaaca cagaggtcaa gccccttgag 19800
tcggtgcttt atgtagggcc cacccgaagt ccttgcttag tttactctgt acctctactt 19860
tgcattaatt ctttactcct caaagggaaa ataagagacc cctcctcttc ctctgcctag 19920
atctcacccc tgaatgtgga gtggaattta aatctggtcc tgtctctgct taaatcattt 19980
atccaccacc tgagtggcaa cttggtgtag tagatttggg ttccaacttt tctgggtccc 20040
taacctgact ctttcaattg caatattgcc ttagcaaagt tcatttaact ctattttttt 20100
tttttaaaca gagtctcact ctgtcaccag gctggagtgc agtggcatga tcttggctaa 20160
ctgtaacctc cgcctcccag gttcaagtga ttctcctgca tcagcctccc aagtagctgg 20220
aattacaggt gcatgccacc acgcccagct aatttttgta ttttcagtag agacggggtt 20280
tcaccatgtt gcccaggctg gtctcgatct cttgacctcg ttatctgccc gccttggcct 20340
cccaaagtgc tgggattgca gacatgggcc accacaccca gcctatgttg ctgtaaatga 20400
caagaattca ttttttttat gagaatacat ggacacacgg aggggaacaa cacacactgt 20460
tgcctgtcag agggtggggg gtgagaggag ggagagcacc aggaagaata actagtggat 20520
gctgggctta atacctgggt gatgggatga tctgcacagc aaaccaccag gcacacattc 20580
accctatgta acaaacctgc acatgtgccc ctgaacttaa taaaagttga aaataaaaac 20640
ataaaataaa aaatcaagat ttcatttctt atagccaaat agtgttccat tgtgtatata 20700
caccatattt tctttattca tccattaata gacactcagg ttgatttcat atctttgcta 20760
tggtgaaatg tgccacaata aacatgagag taaaggtata acctgataca ctgatttcct 20820
ttctttttct tttttttttt tttttgataa atacccagca gtgggattgc ttggatcaaa 20880
tggtaattct atttttagtt ttttgagaaa tcaccatact gttttccatg gtggctgtac 20940
taatttacat ccccaccaac agtgtataag agttcccttt tctccacatc cttgccagcg 21000
tctttgactc taaattttat ctgcaaaata gatagaataa cataggaccc tctcctcaag 21060
acagttgcag ggattgcaag ataacaatag taaaatgccc tgcaaaagtg tgttcaaagc 21120
acactacttc tcttcagtgt gctggtagat gtgaaacaac attaatcatg ttctctcttg 21180
ccatgttgtt tatctcaagg aggcaataga tcttatatgt gcacattaat gcctagtgtc 21240
tctacaccag tccctcacta cccatgaaaa ttaattttga accaatcaat tttgagtgcc 21300
taatgtgtat tctcagcaca aagatccaag caaaactgta aagtgcaaaa aagaaaaatt 21360
tcattgttac ttatgtacaa acaatttact gagcaatttg accccacata ccaagcactg 21420
cagacgacca gggggtgagt aaaatgcaat cactttcaac caaacagtac aatattgtag 21480
ttaagaaatc ctcgggccct ggatcccatc caccctcgtt caaatcccaa ctatcccttg 21540

ctggctatga tttcatgttt ggcaggttac gtcatccttc tgaacatcag tttactcatc 21600
tgtataatgg gggtataatg gggctgatca caatagccct tgtgcctccc agaattaaat 21660
gaaattaaag cccatgaaat actccagctg aggaagtgtc cagcttggaa attgttcaat 21720
tgactttgct gctggtgctg ctgttgttat tattattatt agcaatgcca cttaagtcta 21780
gaacaactca atcacaaaat aatacagtag agcagagaag gatagggaga acagtgaaga 21840
gtccagggga ccctgggaac cccacttgcc ctttccaggt ctttgttaca taaagctaaa 21900
tggagcggtg agacccaatg atccttcatc gctcactgac ttcaaggagt actcaagaag 21960
taagggtcac aaacaaacaa caaaaacaaa aactaccaac gaactcataa agcttacagt 22020
tcagctggag tcctcccatt caccttggtt ccctgactgc caaatttttt tctggaaagc 22080
tccagttgct cctccctgtg ccttcccctc ctccccacca ccttcctcct cctggggaag 22140
cagaagttcc tgagggccag agaggaggtc agagatctcc accggcctgg ccaagagcca 22200
ccaggacagc ttcctgccag cagccttcag taaggagctg tccatctcag ggaaacccaa 22260

ataaagtcac ctctccaccc gagagcattt tgccctttaa ggaaagattg tatttggagc 22320
ccagaacata tttatggctt catgaagatt tcatccaacc ttttacggac agagtcagca 22380
tcaaaggggc ctgcccaggc ccatctgcta ccactttcat aggctgacct gggcctggat 22440
cctgaataaa ttatgaagtc actgaacttt gtttctgata agcccctttt cgtgctttct 22500
tcccttttct acctccaatc ccacccgtct tccacaccat ggccaagtac ccttacaatc 22560
taaccccaat cagtctttcc aaagatgagg ctccaagtaa ttattccaaa tgactatcta 22620
atatagctta atacatttcc accctagaaa agtttaaaga agatacataa taagcatttg 22680
tgtgtccaga attttacaga catctcattt tattctcaca acagcctata agtaggtaca 22740
attgccacct ctacttggct gacaactgag gctcagaggt gttttctaac tcatccatag 22800
attcaattgc tggtaaggga agagctgaca gttcacctga ggtctctgtg agtccaaagc 22860
cgtatgcatc tcacgatacc aaaatacacc aaaagacaga ggaatttctg gcattgccag 22920
gtgtctaaag ggatctgcag ggagagacag aagtttgaga aatctccctt aaggaactaa 22980
aatagatcta ccatttgatc cagcaatccc actaatgcat atctacccaa aggaaaagaa 23040
gccattatac gaaaaagata cttgcacgtg aatgtttata gcagcacaat ttgcaattgc 23100
taaaatacgg acccagccca aatgcccatc aaccaacaag tgaataaaga aaataaagaa 23160
attgtggcat atacatatat atacgatata tacatatata caccatatat atatatacac 23220
accatatata tataccatat atatacatac catatatata taccatatat atacacacac 23280
catatatata tatatataca ccatatatat aattgtggca tatatatata tatatataca 23340
caccatggaa tactactcag tcataaaaag gaaaaaaata atggcattca cggcaatctt 23400
gatggaattg cagactatta ttctaagtga agtaactcag gaatggaaaa acaaacatca 23460
tatgttctca ctcataagtg ggagctaagc tatgaggatg caaagccata agaatgatac 23520
aatggacttt ggggactcgg ggaaagaatg ggaagggggt gagggataaa agactacact 23580

ttgggtacag tatacactgc tctggtgatg ggtgcaccaa aatctcagaa atcaccacta 23640
aaggactcat tcatgtaacc aaacaccacc tgttccccca aaaacctatt aaaataaaaa 23700
agaagtttca ggaatctcta tcacactctg taaggcagct tccaaagctt ccttctcctg 23760
gaaccagatc tgggtctgtg catgactgag gtgtcaggca ggacagcctc actctaggac 23820
acttctgatg gcaacagtta gccacgtttc agcatccaaa aaaagagaat tcaccggact 23880
tcccagcaat tttaggaaag gaaggcaaga gatttcgaga cactaggtaa atttttgaca 23940
ttccattaga tagataggtg gataggtaga gagagagaga gtatgaatac agattgagag 24000
ttttcatgcc acaagtttca tgtcaattaa aaataatcca gtgggactca ggagcacggc 24060
atggtgaaac ttgcacggat tttacaatta ggcaaaccca gctctgaatc ctggcatggc 24120
cgtgtactga tcacatgacc ttgagcaaat tgcctaacat ttcctgagct tcagttactt 24180
ctgcaaaaat gagatgataa tacttccttt tacaaagtta ttcaaaggat ttgggattgt 24240
ttatgtaaaa cacctaacag tgtgctgaga gtggcagatg tccaccaacc agatacatcc 24300
ccaggctgaa cggaggccaa gaagcacttc acccattcca tccttatctc tcaaagtcca 24360
gggcagttcc aaataaaagg cagatatttt caccaaccaa agcagctgct ctggctttcc 24420
cggaacaggc ctctgcctca gagcccttca ttgaccaccc atgtttccat ggttgaacgt 24480
ttacaggaga acattcagcc tccccttgtg gttcatggag cccttaaatc cccctccagc 24540
ctcacctcct gtcattcccc tctcccactc acacacctcc agccagacac tcagtgcacc 24600
attcaccaga cacagtattc cgtggctttg atcacacagt tcccatttcc tgaagtgtcc 24660
tcctagccct ccacctaaag aactccctgt catcattagg agcccaacct aaatccatca 24720
agcggaatta accgctacct tctctgtgat cccatcacac tttacaatga atggagaaga 24780
ggaggggtgg gggcaaggtg cacaacttgc taagtgccca ctatgcgcca ctagcaactc 24840
cacagactct aaccaccaca tgagttaagc cttattatcc ccactttgca gataggaaaa 24900
ctagttcaca gagcccagcg tatgatcctg aggtcaaaag gctccaaggc ccctttcccc 24960
tacttactat actccatgga cacatggaca tggtcctgtc tgtctcccac actagactga 25020
gttttctgta aacagagttc atattttcat catctctgaa acctcacggc cccctcggct 25080
caggctcaag cccagtccct gacacctagc aagccccact aacattcagc atgcataggg 25140
agctgctggg tcatctgtct atttattata gagactggat ggaaagaatt ctggatcact 25200
gaggaagagc atttccaggt tgaatgtctc agctttcttc atggacacca gttcgctggt 25260
gggaggcttt aaccttgaca accatccact ttcgggggct tgaagaaaga tttcctgtat 25320
atattttaac tgcattcatt ttaaagggaa ctctaaattt ttaatacaaa aagcccaagt 25380
gtactgtgat ctctgagctg gctatcacac ttcgtaaaga taggccatgg gaccaggaag 25440
gaccgcagct cagccaggac tgcgcaactt ggggcagctt tcccaaattc taggaaccaa 25500
ggtccaagga aaccaacaac cttgtggcca acctttgcac atctgtggca cacaaggttt 25560
ttttaagcag actcactatg tttgaggatc ttcccacaat ctgagcacca ctttccaagt 25620

tagaagctag aattcacttt cccagattcc tttgccacta atgctagcat tgacctgggc 25680
tccaccattc accaagtgtg aaaattcact ttgaaagaaa accacctgag gatccaggag 25740
ctgcatgaag ccgattttct gtctaaggag ggaaagtagc agagaatctt caaagtctca 25800
gagacagctg cagtgagagt tctggagatc cagtcccaga cagaacttca cggtgcaagc 25860
agagaagtcc ctactagaga cagataattc tcagatgatt ccttcctgca gcccaatgct 25920
ccagagattc tgtgagctcc ctgagttcct ttatcaaatt cattttctgc ataaatagct 25980
gagaggggta ctgtttacat cttgtattgt aaagaggggt attgtttaca tctaagatcc 26040
ccgaaggatg caaacctcat gcctgccagc ctaacaaaat caaacctcat cggactgaat 26100

agacctcaca gcaatatctg actccccacc taaactggga tctcatgtgc ctggaacatg 26160
ctgagtagaa aataaatatg tgctgaaata caatgaagaa aagccacaag actccttgtt 26220
cacaggttga atcttggact ctgagaccct gcaccgaaga aggaaaagga cagttcccat 26280
cccatatgct aaataagtct ctgcagtgca gtgggctgtc tttgtgactc acaagctggt 26340
tctgaaaatc ttctttcgga aggagtttcc agaaatggtt ggaacaatgg cagcatcact 26400
agaaagaagt ctctcacctc ccacagaagc aactttgagg gacaacatat ttttgaattt 26460
gcaattttgg agtgtttgct aaaacatcac ttccattata gggtgtcaca gaaagcactg 26520
agtctcagga gagcagagtt tagttccagt tctgccccca acctacagtg tgtctgaatt 26580
atttcctcct tctttgggcc tcagtttccc tgtctgtaaa atgagaaggt tgatctttgc 26640
tccccattgc ctctttcagg ctctttctcc ttcaaaaagc tgattccttt gacacctatg 26700
ccattcaacc agggtgtcca ggcacagctg tacaggttat acaagtaggt tgcatcctgg 26760
ccaaggaagc ccacctgaga ggtaagtaga atccagccca catccccaca ggcgtcaccc 26820
ttgggtgggc ccgcttccac tggagaaaga ctactttttc ctaatttgta caaaggcatc 26880
atatgagcta gccaaggccc caatttcagc tattttatac ccttgccctc ctccttactg 26940
gggtcattct actcacctct taatcatcct tcctccttca ctgaagactt taggtcctgg 27000
ttacaatcat gttcactgcc ctcttcctac aataattctc aaaacttcaa tatctgtatg 27060
tgcaactcaa cgagccactt ggtcttgttc tttgatgttt tctaccccac caacctttgt 27120
cctcactcca ctgcagccac ccactcaaca gccacaccat agaccatgac atggccagaa 27180
atctctattt ccaaatcctt ctatttggcc ttcatttcca gtaattctag ctcatttgct 27240
ctggtaccct tcatccccac acttctggcc ccttatcttg accactaacc cattgatctc 27300
accacacacc caccctccag cagccacttc ctgcctttgt ttccttcctt acataaatta 27360
gattccattg tccttcttta taattactct cacaaacaat cccaacgccc tggagcctct 27420
ctcctctgtc acaatccctt tgcaaaactc caacatgtgc ttattacctt ctctgtgcct 27480
ttgctgcagc atctaaatgt tgccagagaa cttcacacaa cctggctaag tgatttcact 27540
ttaaattcat ggacacaaat ctcaagtgga aaattggagc tggaagcttc cctaggaatt 27600

tttgtttttt cgttgcccaa gataacattt tatcccccta aaactttcta gacccctttc 27660
cctaccccat gctcagcaga tgatctcatt ttacaattca cttagaaatg ggaggttaca 27720
aagccaggac ttcctgatct tcccaccacc aaactctacc aacttatctg cttcttaatc 27780
catcctctcc tacgtccttt ctagtgcact gaataaaatg tcctctcttc ctaggaaagg 27840
ctgaccctcc cacatgtgct cctgcctgca tcccttctgt ctttcttgaa ggcttttctc 27900
ctccagtcat ctcttctcca caaaagcatc agtctcttca tggctcattc ttgccctcac 27960
cctcacttat agttttggtc tttcttagat tgttactagc acacaaacat gcccttgtat 28020
ctttaatttt ttaaagccct tctctagcta ttgcccattt ctctccttac cttcccagca 28080
aaactgttat tgttttgggg tgtttgtttg tttgtttgtt tgtttgtttg aaacagaaac 28140
tggctgtgtt tcccaggctg gagtgtagtg acacaatctt ggctcactgc aatttccacc 28200
tcccaggtcc aagctattct cctgcctcag cctcctgagt agctgggatt acaggtgcac 28260
accaccacac ccacctaatt tttgtatttt tagtagagat ggggtttcac catgttggcc 28320
aggctggttt caaactcctg acctcaagtg atctgtctac cttggcctcc caaagtggca 28380
aaactgttga aaagagttgt tttcacaagc attctccaat cctcctatcc actcgtcagc 28440
tctctgcagt ctgtcttcta ctgtctgtct tctacaaaca caacctgtag tctatcttct 28500
atgaacacaa ttcactgtaa cccctgaccc atgtcaccaa agacctctgt gtcaccaaat 28560
ccagtggatc gttttctggt ttcctcttat ctgacctctc tgtaggattc ttcacagttc 28620
catctctatg atgttcagat ttttatcatc agtccagacc tctcttccat tacatatcaa 28680
actgccttct caacatccgc acttgaatgt cttattaaca tctcaggaat gatatggtga 28740
tgtagccaaa acctaagcta ttctttctcc agccttccct atctcactaa ttggtatcac 28800
cctcttccca gttgctcaag tcagaaagtt tggataattc cttctttctc accccctcat 28860
ccacccactc actcaaaccc aggcgatcag aagtcctatt cattctgcat caaaaatata 28920
gctcaaacct gcctctcttc tcagttgctg tggataccac cctaatgtga cccaccaaca 28980
tccctcatct tggcctctga agtagcttcc taaccggtct ctctgctttc cctcctactc 29040
cccttcaact cattccctat gcaacagctt gaggaacaat tttaaatgca aattagattg 29100
tatcattttt ctgatgattt ctcattgcat ttaataaagt cacacttctt accacagcct 29160
tcaaggtcct acatgacctg gccccttttt ggctctcttt gtctcaggcc accccctgcc 29220
tggcttcctg ggactagcct tccagttgcc ctgacaacct tcccagaacc tactgtgatc 29280
tttcttgctt cacagtctct gcatttgctg ttttctgcat ctgggactct ctgagcctcc 29340
gttgcacagg gcaggctcat tcttattctt cagatgtcaa cggaaaggtc agcttccaag 29400
atggccttcc ccaagcacct tttctgaagt tgtacttaat attattttcc acttcagatg 29460
ttatttcctt catagtatgt tccataactg gccatttcat aaaatggcca gtttatgtat 29520
ctgtgtattt tctatctttc cctacaaaaa tgctaagcta gacaaggtct atctcatcac 29580
tataatccta gtggctagct gagtgagtgg cacacagtaa atgttcaaca agtatttgat 29640

gaatgaatga gtcatcatga tttgtcccca gtctaatcct tcctccttta ttccacattc 29700
ctttgtatgt atacccaaca cacaagctag tcaacttgct cctcagacct cctatgctct 29760
tccttaccct ctttcacact gtctcctcag gctggatgcc tcctaccacc cccattccac 29820
attcaagtca ccaaaatcct agttatcctt tcaggcccat cttcaactct aatgcctgag 29880
attccaccat tagccattct tagaattata gaatgtcagc ccttatagat ggtggatcaa 29940

aattacattt atcatttcaa ttgcccagag tgctgcatgg agagggatta tgagttttgg 30000
caccacaatc ataccagaaa ggccatccat aacaatagga aggttatgac tgtcccaatc 30060
tttttcaagc tcctcatgat acagatggga aaactgaggt tgaggcaggt ggcatgactt 30120
cccagggaac acactaagta ggagacggtg atgggaacag agtttccatc tctacatcac 30180
cagtgtggaa ttatatcagg ctaccttttc tacatcccag tctgagaagc tgaggctcac 30240
atagacttga aaggtgattg agattgtgaa tttttaattt aatatatata tatatgcaag 30300
atattatttt tatctttctt tttcaacaaa gttctgacca gattaatttc atatgcaaac 30360
ttctgtcaat tttttctagt tttgttttga tactaccaag ataaaaatat tttaaataca 30420
agattaatta ccaaccacca cattcctgga attgtgcact ctcttcttcc ttgcaccctt 30480
gcaaagatcc tccaagactt ctgtagtgga aaggagcttc tcaagtcact tttctttgta 30540
acaggagtgg caaacaggag aatgggagga aattaaaacc tatagattac ttcttatgtg 30600
ctggagactg tgagagttcc ttgcaaacat tacctcaggt aaccttcaaa atagttctct 30660
gaggaaattg cttttaaagc tattttattt tgacagatga gaaaacagcc tattaatggg 30720
tgttatgccc ctagatcttt gctcccctga atgtagtcta tgaactaata gcatccacca 30780
ttgttggagg gctttaagaa acaagatact aaggacaccc tctagatctg ttgaatccaa 30840
atctgtattt taacatctcc taatgattct tctgcacaca caagtttagg aagcaatggt 30900
ctagataata caaccaaata acagatgaat taggattcta attaggtctg tctgaggact 30960
aattcccaga atctacaaca aactcaaaca agctagcaag aaaaaaacaa acaatctcat 31020
caaaaagtgg gctaaggaca tgaatagaca attctccaaa gaagatatac aaatggccaa 31080
caaacatatg aaaaaaatgc tcaacatcac taatgatgag ggaaatgcaa atcaaaaccc 31140
caatgcaata ccaccttact cctgcaagaa tggccacaat caaaaaataa aaaataatag 31200
ctgttggcat ggatgcagtg aaaagggaac acttctacac tgctggtggg aatgtaaact 31260
agtacaacca ctatggaaaa cggtgtgtag attcctcaaa gaactaaaag tagaactacc 31320
atttgatcct gatagcccac tactgggtat ctacccagag gaaaataagt catcgtatga 31380
caaggatact tgcacacaca agtttttagc agcacaattc acaattgcaa aaatgtggaa 31440
ccaacccaaa tgcccatcaa tcaaggagtg gataaagaaa ctgtgatata tatacatata 31500
tatgtataca catatacatg tatatacgta tatacacgta tacgtgtata tatacatata 31560
cacatacaca tatgtatata cacatataca catacacata tgtatataca catatacata 31620
tatacgtggg tgtatacata cacatatata tacatatata tataaaacat atatacatat 31680

atatacacac acaatggaac actactcagc cataaaaaaa gaatgaatta atggcatttg 31740
cagcaatctg gatgggatcg cagactatta ttctaagtga agtaactcag gaatggaaaa 31800
ccaaacgatg tatgttctca cttataagtg ggaaataaac tattcggata caaaggcata 31860
agaacgacac aacagacttt gggaactcag agggaaaggg tgggaagggg gtgagggata 31920
aaagactaca aatcaagttc agtgtatact gcctgggtgg tgggtgcacc aaaatctcac 31980
aaattaccac taaagaactt actcgtttaa ccaaatatca cctgttcccc aaaaacctat 32040
ggaaataaat aaataaatta ggtctgcctg actgtaccct taacagaatg gacacccaag 32100
acacaaacat cgtggtttta aaggagacag gttaagtcat cactggtata agttgtacac 32160
atcagcagca catctgatta cctgtctagt cctatgacca tcccttccaa agctgtctac 32220
aatgtagaaa ggaggaaacc tggtgatgtg gaaagtaaac aggcctgaaa atcaagagaa 32280
ccaaaccagc caaatcaacc actacttagc aatggcccag tgtcccaggg ccccattttc 32340
tttaccagtg aagtgggact ggagggacaa ttctgtgcag ctcaattatg tggcattcct 32400
cacatcagcc cactgagctg ctcctgaaac catcagagta gaaatcactt ccttctccaa 32460
cctgtaatgt ctatctctag tcaattcctt tggtaattgc tgtgttgctt tttccaaaca 32520
taaaaatagc ttattatctg cacagcaaat aaccatcacc ttgcaggggt cctgacagtg 32580
ccccagtaca gagcttgcaa ccagctgaat cctaagaatc tcaagggctt ccaggtctca 32640
tcccggaagc caactaacca ccatattggt gtatctattt tcctaagggc aagggctgca 32700
tccattatct tcattcctgg cacgttgcag tgacagtcct cagactctgg gtcactggct 32760
ggccctgcta ccaaagtttt ttttctgcta aagagaccag acccggtcca cgtagagtaa 32820
agcctgggtc cctctctctc tttctatttt tttgagactg agtctcactg tgtcgcacaa 32880
gctggagtgc aatcgcacaa tctcggttca ctgcaatctc tgcctccctg ggtccctctc 32940
ttatcatcag aggctgcttc atccctatca tacaggaaga cctagtgacc ttagggacat 33000
attccagacc cagaagaaag actgccaaat cttcctcgat gcccttaaac atggcccaga 33060
ttttctcagg gaacggaaga gaagttttcc ttgagtattt ttatacaggg ggaaatccaa 33120
gatcttattt tctattcaat cggtacatcc cccaccctca ccacaaaagt ccttcctcca 33180
tgaacagtgg atgtccagac atggattccc agaaactgtt tgactcagtg tcggctccac 33240
ttggctcatc cataacttcc ccaggaagcc aaccagcttt gcaaaccatc atccatccaa 33300
agagacctga agacctagtt tccagtatga acactgaact ggttgccaca atgccataat 33360
ttgagaaatt ctgctgagaa gatcagaaga gcccatgtac catggtccag ccagtccttt 33420
tttctgtcac tgggtcccca gagggcagcc cttaggagcc agaacatttg gtttaattgg 33480
gtcaggggtt aataatctta gtacttatca atcccagtgt gtctcttgta gcaccagtcc 33540
taacttcatc tttctgggct attttaaaag caatcaactt gtttgctgta agcaaaaacc 33600
agcttttctc accttttctc ctgcactttc tctattcagt tgttcgatta tttaagcaaa 33660
catcctttaa gcatctgcca cattccaggc actgggaaca aaaaagatgc ataaacgtag 33720

ccctagtcta gaggatctgc ccagagagca gaagagatga gaaggaatgc agagcccaga 33780

cagagtggcc agatctgggc ttttctagag acctgttccc tgtgcgcagg ggacaagggg 33840
ctgggagtac tagggtgctg gccaagtggg gtgcttcaag taggtttggg gaatccattt 33900
ctgattatcc actgtccatg taagaagctg cctcaagtag caagagatgt gccatataac 33960
taaataataa agtacagaac aatattcggg gagatgctca ctgagcacaa atagtccttt 34020
gtctcatgtc ctcaggtttc tagttattta ggcaagaaaa tgcaccctaa ggtggctttg 34080
gaagtaggag aatgtcagac atttataccc ctgtggaatt gaacgtgggc ctgtgcattg 34140
agccagcaca ctagccctgt cttgggaccc ctctgtggtg cagaccagaa tatgggtgtt 34200
tgtccccact gcatcctgta cttcctccgc aatatgctta ctgcacttta ttattgattg 34260
ttaaactcca aaagagcagg aatcacatgt atatcttgta ttgcttcatc tcatcaccaa 34320
gcatgtgcct gatatatgga catagaaaga tatttctaca tgggacaaaa tacctgagca 34380
atgttcctcc aaaccgtcaa ggtcatcaaa aacaaagcct gtgaaactgt cacagccaag 34440
agaagtctga gacatgatga ctaaatgtga tgtggtattc tgcatgagat cctggaacag 34500
agaaagaaca tgagctaaaa agtaaggaag tctgaacttc agttcatagt gaggtatcaa 34560
tagggttgga cttcagttca tagtggactt cagttcatag tgaggtatca atagggttca 34620
ttaattgtgg caagcatgtc ctgctaatct aaaatgctaa taataaagga aactgggtgt 34680
ggaatatatg aggactctct gtattctatc agcaactttt ctgtaaatct aaaactcttc 34740
tgaaacaaaa ggtttattaa caaaacctcg aatgatcctc aagcaccttt gtgccctcct 34800
acttcccagt tggtggctca tcatggatct gactctccac ttgccctgtg tgttctcaat 34860
aagagacttc ctctcctttc taagcctaga gtcacagaac acgctcttgc ctggcagtga 34920
ggaacctgag atctagtccc acttctgcca ccatcccact atatgaccat gtgtcaatca 34980
tttccccctc ccaaccataa gcctcagttt acccctctgt tcaatgagaa agtaagacta 35040
gaaggtgctt ctagtcggtc catgaaggca gggatggtgt gtctcattca cggcagcatc 35100
tccagcttct aagacagtcc caggcacaag acagacactg aaaaacaaaa aaggattcaa 35160
tgaataaata aatgagcatt gctctgatac tccaacactc taactagaaa tggaaacttc 35220
tagagtgagc tcagcatctc agaacctcct ttcttgtgta tttgatttca agctccctgg 35280
gtcacatctt tgtgtctgtc tccaacgtcg tgcatggaga ctttataagc atttgtgcaa 35340
tgattgagaa gagctggccc tcccagatca ggccctgcaa gtgttgaact ccttggaatg 35400
cagacagtgg tgtctgctga gacggtaggg aggtgtgtca catctaaagc ttattgcgct 35460
gggggtcatc ctggcttctg aagttgggga ggatcttccc acggaatcta tctgcctctt 35520
tttaacaagc tcagactgaa agggcctggg cctcccaacc cctagttagg agaccctggc 35580
agataactta gaggaagata gctcaaaccc agggagggaa gaaactggaa gtgacaaaaa 35640
gaataaaaaa gaaaagaaaa aaagaaaaaa aaaaagccct gcacaacccc aattgtctag 35700

agacctggat tcctggattt tagtcccagc ccccagtatt ctcggcatcc acgccatgag 35760
aactgggcaa gattttctct gtgcctctaa ctggccaacc tcccagagtt ggagtggggg 35820
tgagtaaaaa caaggatgag tgttcgttcc agttcctcag aggggctgtg ttctccgtgg 35880
gcaattcctc tgcccagaac attttcccca ctaaccactt acactccatg cttcagagca 35940
ggctccaggc aggcaccgtg gctgtaaagt ctcccctgac gccctgcctg actccttcca 36000
ttgtctccac gacaggccct cgaacacttc acacattccc ttagaactca ccaccctggg 36060
ctgtaatcgc ccatttcact gtctgcctcc accagccgac tgcgagccca ctggaggccg 36120
ctcaccattt ggtcctgcgt ccagcatagc gcttggcaca tacgtgataa ataaggactt 36180
gtggaacaaa cagatgaatg aatgggtgaa taaatgaagg catgtgtgaa ccagtgaatt 36240
agtaagtgct gtcacaacca agggaaggct aaaaccgtat catgttttgg atagcttacc 36300
tcagatttcc caggagactt gctgaggttt tttacagcat ctgtaaaaat aatgataata 36360
gtggctccaa tagcctgttg ttgccgtcgt aaattcccaa agggctttcc ctggtgtgtt 36420
tcagaagagg gagagcaggc actgattccc tgccctcagt ctccctccct gcctccccgt 36480
ttcttcttcc cacctatgtt ctcactctct gtctctctct gtctctattt ctctctcctc 36540
actctgcctc tctttcacgg tctctgtctc tgtctttgtc tctgtttctt tctcttccca 36600
ctgtccctgt ctctctctct ctctctctct ttcactctct ctgcctgttt ctctctcttc 36660
cctctctctc aatctctctc tcctctctct tctctccctc tccttctttc tgtctttttc 36720
tttcactatt tctgcctctg tttctctttc ctctctcttt ctgtctctct tttctctttc 36780
tctccctcca tctctgtctc ttacactatc tttgcctctg tttctctctt ctctctccct 36840
ccctctgcct ctgtctctat ctttttcact atctctgctc tgtttctctc tcttctcaat 36900
ctctctcttt ctccctgtct ctcctctctc tccctccttt gtctctatct ttctctttca 36960
ctgtctacct gattcactct ctcttccctc tccctctgtc ttttctctct caattcctct 37020
ctccctgctt ctatatctct ctctttcact atctctgcct ctgtttctct ctccccatac 37080
ttcatttctc tctctctgtc tctctctctc tttctctctc cccctctctc tcttcccttg 37140
tctctatctt tctcttcact atctcgcctc tctctcttct ttctctgtcc ctctgctttc 37200
tcctctctgc cctgtctctg tctttcgggt actctccatc atttccctcc ccatccccat 37260
tcactctcca ctgtgtgcca ggcatctcac acagaagttc tcactttcct tcccagacac 37320
ctagtgttgc ctcatttgta ctttgggaga aacatcccct taccatggtc aagaatgtgg 37380
gctttgggct tgggagccag acagattcag ctggttccaa gcccagctct tccacctgct 37440
agtctgtgat cttggtcaag aggtctaact aacctctctg agtctcagct tccttatgag 37500
caaaatgagg tgaaaatgcc ccctcttaaa ggatggctgc aaggattaaa tgagataatg 37560
cttgggaaat gcttagcaca gtggcagaac tccataaatg cgagctctct tgttactatc 37620
agttctacta tgattattat ttttttgcat tgtctgcaaa cattattaat acccaggaga 37680
tcattattga agatgacaaa tacctctgga ctgaatagcc ccaccaccac gccgacccca 37740

ggatcatttc agccccttcc cccctcctcc aaatagatga ccaccaaagc ccagttggct 37800
gtggttctga ccctctggtc ttctatgccc actttaattt gggcagaatc catctggata 37860
gagattccct aagcctggct ttcccaaaaa attatctgag tttttagtaa ttgtaaatat 37920
ctggggtgag tcaaggagag tctgactcca taggtctggg gtgataccca ggaactgggt 37980
attttcaaaa gcatccagag cgactctgat tggagaacag ggattcaagc ttctggtcta 38040
aactcagtgg ctactcaagt gatattaagt aaatcaatca ataggagatg gtaaatgaat 38100
aaaggcataa atgagtagat ggattggtgg atggatggat ggatggatgg atggatggat 38160
ggatggatag atgagtaata ggtctacctc catgggaaga aatgctggta tttttcccca 38220
gacctatgct ctggcctcca aaatatgtgt ccttttgttc tgtggctttt cagagcacaa 38280
cacttagctg ctcccctgtt tcttttcttc cttttcttct ctgcacctaa atcccatcct 38340
ttccctttcc tgatgcctat ttcctccatc ctctctccct cctcacttcg ccccctcctt 38400
ttagctccca cttctcttta cggcttcccc ctcaccatgc tgaaacattt gcaagctgga 38460
ggttatccca agacactttc atgagaagag gctttggaca caacagttgc acaaacagtt 38520
ttatttgatg aaccacagtg actaacagga tcagaagaca gtgcagatat tctgaagaag 38580
gcactggggg aggtaagggg gtatcacagc aggcagcctc ctctgcttct gtcccagttc 38640
acagatgagt tccaggcagg aagtctctgc aggtcaccca cggcggcctc agagggacaa 38700
tttcttccct tctagaagcc tcttccagtg ttcactggat gctttgagga cagctctggg 38760
cagaggaggt gactctgtga aagatgctat cttaagatgg ggagactagg ctgtgaggag 38820
ccccttcccc tctcctcctc cctctgcccc cagagctggc gtcattccag ggagggtcaa 38880
gatgtccatt cacatcaagc tgggcttttc ttatctccat cgctcatgtc ttgtccttca 38940
ctttcatagt cctccaagaa caaaacccgg aatagacact cccagcctct agctcacaaa 39000
ggccccacta ttaatttcat cctcacggtc tctctggcag tgtagacatc atgctatctg 39060
cacacttgct ccagtcccta ttcccagatc cccaatccag cagaagccaa gaggtccaat 39120
cctgagggta gggagagaaa aaacccaccc cgtgcctttt ggcccatggc aagcaggagg 39180
cctccttagg aagagggcca ctgaccctgg aacaagaaag gacgatgagg gcaatgctca 39240
taaatttgaa gccaggcggg gaggcaccgg aaaggggcca agggaggctc tcaactagcc 39300
tcttacttcc ttcatggagc ccaagaagga gcaggaaccc agctcagtag gacgccccca 39360
agccttggcg ccagctgctt tggggaggga tctccctaca ggaagcctgg tgcaaacatt 39420
aacttcgctc ctaaagcacg gaccagccgt tggagccccc aaatccagta gggggtggac 39480
tcatttttat tctacatttt tcattgcctc agtgtgtccc tgccccccac caccccctgc 39540
caagggtaat gtgtgaacag cagagttgca gtggattggg gggtgggagg aggtttgtcc 39600
aaatgaccca ggcacactgc tgttcatgcc aggctcatcc atatagacat ataagtacaa 39660
cacacacaga caagaggcag gcatctccat cccaaacaac atgattcttg gcaccaagta 39720
gcaccagtgc ccaagaaaga aatcagtatg atggggtaag atggggagat gaggaaaccc 39780

ttcagctcac agttatgcag gaagtcagaa acaggaggac tcggggagcg agccaagcag 39840
tgtctggact aaatgcagac acccctccat cctctctctc tagttgccct tcccaactcc 39900
ccactcacat aatgaaacca aggagtacat ccattcatca gccctgctgc cttccaattc 39960
tcatagaaaa tagaggaagg atgaaagggt ctgcggacaa tttggagaaa gcatatctag 40020
tcagtgtcat ggtgccatgg gggaactgca ggaatcacac tagccccaat gcccaaaatg 40080
acagggaagt gatccaattt tagggcaagc tctggagctc ccagatctaa gggctggagc 40140
actaattatc tgctgacaca cagcccaccc ccggccccag cccattccct gcctcacact 40200
cagaaaacag gatagaaagc ttaaacaggg tcagtctctg caagctttgg gatccttaac 40260
ccttagtagc ccttcagaac aagaaagtag acaggggaga aggatggatg gagggagggg 40320
gacgagctgt aggaggggat tggtgccaga aggcagggat aaggagcggc agctgcagag 40380
agtggtgggg gaaaaactac aaggaaagaa gggaggaaag acgattgcag atctaagaaa 40440
gcagcagagg gaaacaagct tttactgcgt ccagactcag gcttcaacat tttaggatca 40500
atatgacttt cccccatttc ataccaatgg agaaactgag gctgagtgag attaaatggc 40560
ttgtgcaggg tcaggtctgt agagcccagt ctctttcccc taaaccacaa acctctgaga 40620
aagaataaag cagtagagga ggacagggcg gggcagaatc aggggacagt taaaggatgt 40680
gcataggaca aggagtagcc acactacctg attttcagaa agcaagggca aaggccgggg 40740
agggccgagg gcgccaggct tctctctgct cccagcccta gtcaaccaca agggtctcct 40800
ggctgtaggg gatggatttc tcgtgcatct gttctccacc cttgcccatg ttggggccgg 40860
gcccctggct gtgccccgaa gagtagctgg ctgacttctc cccactgctg ctgtgggagg 40920
tgcctgcgca cctgctctgg ccacagcaga gcatggaggt gcaggcctgg cggaagcggg 40980
ggtcgaaaaa ggcatagagg aaggggttga ggcagctgtt gacgtagctg atgcaggtgc 41040
agtaggggaa gatgttcatg aggaagaggt caaagtcaca gggccagtgc agcaggctgc 41100
ccagcatgta cagcgtcttc accaggtggt agggcatcca gcacagggca aaggtcacca 41160
ccagcaccac gatgatgctg agcagccggc gccgcttccg caggccctcg atgcgttcct 41220
tgcggaagtg gccagcgatg gtttgggcga tgaagaagta acaggtcagc atgatggtga 41280
agggcaccac aaagcccacg gtggtggacg agaccccaag gcccacctcc caggcccact 41340
ctgagctcac agtggccacc atggagtagt ccatgtagca ctgcacctta gtggtgttct 41400
ccaagtcccc ggtggtgcgt aacaccatga caggcatggc caggagggcg gccagcaccc 41460
aaagaactgc cgtggccacg gccccgctga cccgcagcct cagccgagca ttggccactg 41520
gcctcacgat ggccaggtag cggtcgaagc tgaggccggt gaggcagaag acgctggcgt 41580
acatgttgac gaagatgagg tagctgctga gcttgcagaa gaaggtccca aagggccagt 41640
catagtcccg gtacgtgtag gtagcccaca ggggcagcgt caccacgaag gtcaggtcag 41700
ccaccgccag gctagcaatg aagatatcag ctgagcgcct cttctcccgg ctgctccgaa 41760

acacggtcca gagcaccaga ccgttgcccg tggtgcccag gaggaagacc aacatgtaga 41820
tggcagggat gagggccccc gaggatttcc agtctgtgta ctcacactca gactggttgt 41880
ctgccccata gtagttgtca aaatcaccac cttcctccat gctggggagt ggagagaaga 41940
cgggcagagg gtcccaggga caccagctcc gtggctctgt cacccactct gcaagcagcc 42000
tgaatttctg gcttgagcct cagagagtaa gaagggctga agatgcccag agtccttccc 42060
agcactcagg aggagctgcc tctgggttct ccagaccctg gaggaagcct gtctcctgca 42120
gaatttcctc caccctctct tgccttaccc ccgtctcagt taagacactt ccttgcaccc 42180
tcctgcgtcc ctgtctcctc cttgactcgt cactccctcc tcccacctcc agaccagccc 42240
ctctcttgtg agtcaaaatc tttagtgaca gcccactttt tttttctttt tgtcactgag 42300
caagtggacc aaattgaccc ctactgtgct gggctgaaca ttatctgtgg ttttgcaagt 42360
cggctttcct ggccccgcct ctcctttctg gctgccaccc accttcaccc cagcccctat 42420
ccctccactt tcctctggcc accacttcct gcctgccctt tagggcactc cctcctcagt 42480
gctctccgcc ctcctgttct cacccccttc catccaatct aaatgggaca cattatgcag 42540
attgcaatcc agcttgagct ggagccaggg ctggtaggga gcaaggaggg tgtaagattt 42600
cgcaggatgg gcagggcatc tggcaggtcc tcctggcagg caggtctggc cccctcggct 42660
cccttcctgc acccccagcc cccacccaga ccaggtgtca caggagggtc ttctcccact 42720
ttgcctctcg cttcatcttt ctctacccct tggctttgac cctccctctg agaacttttg 42780
tggtttctag agaaataccc ctacactagt gtagactgca gtttgcagtt tgcggaaggc 42840
acaggatgct ttagaaggtt gagggcagtc acaggatgga agaaacccag gacaaaatta 42900
tctctaaaca agtcattaaa tctgtaggaa acaaaggccc aacaaggtca atgttttgcc 42960
caaggtaata aagtgaatta tgctacagtt gattgtttgc ttttcttctt ctccaaacac 43020
gaacataata cagtacctgg catgtcgtag gtgaatggct gttttgtttg tttgtttgtt 43080
tgttgagaca gagtttcact cttgttgccc aggctggtct aggctcactg caacttctgc 43140
ctcctgggtt caagcgattc tcccgcctca gcctccctag tagctgggat tgcaggctaa 43200
tttttttgta tttttagtag agatggggat tgaccaggct agtctcaaac tcctgacctc 43260
agatgatcca cccacctcgg cctcccaaag tgctgaaatt acaggcatga gccaccacgc 43320
ccagccaggt gaatggctgt tgagtgagta agtggatgga tgatgtagga ggtgagtgaa 43380
aaggggaatc catagatgga agaacaagtg taagaattac tacgtggtga gaacatggat 43440
acatttcagt tctcaggttt ttactatcct ccatggacca agaggtttct gtaagaccac 43500
agggggtcag gaagttgagc tagaggaggg gggtctctag agaaagactc agtcactgga 43560
ggatactgtg gggaaagttc cgtagacgct cactttgccc cagctcatca cctctccagg 43620
acctctagga aggaaggaag atgcacgacg taagattttt tttaaaaaat acgcaccctt 43680
tccacaacct tgaagagttg gggaagccac ctgacgttgg gtaatagtga gatggaacaa 43740
aagcaggagc tgagccttag ccacttccaa ggcaaaagac ttacaaaagc tggaattggc 43800

caggcaaggt ggctcatgcc tataatccca gcactttggg aggccaaggc aggaagactg 43860
cttgagccca ggagttcaag actagcctga ataacacagc caagaccctg tctcacgata 43920
tatatttgtc ttaagttact acaaaagctg gaatccattt tctggaggga gagtggatca 43980
gaaaatcata tgcagagatc aaggaactca aactcaaata cctgcaaaaa aaaaaaaaaa 44040
aaacaggcca agagtgtagt ttgagacaac agagaatggt aaagactgtg gcaaactgga 44100
gggtgcccat tccatcatag ggaggcctct tcagctctag tctctggttg ccacgcagaa 44160
agctggaccc cagcatgact gtgtcttagg atttttccaa gataatttca aattatggat 44220
ttttgtaaga aattttcata tttgtaaatt ttggaaatca attttcattt caaaaaaaag 44280
cacagaccat gataagtaaa acatgtcttt ggcccccgag tttgtgttct ttaaacagga 44340
gcttgccaag aatagaaggc aggagaagaa ccaagaggat ggggcaggag gaggaaacaa 44400
aaaatgcaaa gatagaaaaa gccttagagt gcagtgggga gatacaggag agaaggctgc 44460
agagagagat tagaaagatc ctgtgacctt cacctaaatg caatgtggag taatgaatat 44520
gttggtgttt ctcatgagga ctacatgttg cccacttatt gtgtgccagg cagtcttcta 44580
aacattttat acattctaca tatatttatt agtatctctt ttaatcatca aaatgacatt 44640
aggaggcagg tgctctcatt agcctcaatt tacagatgag gaaactgagg cacacagagg 44700
tgaagcagtt tagccaagat cacccagcag taattgtcag agccagaatt tgaaccaaag 44760
ctgtctcgag cccatggact aaaccactat gctcccaaag aaaaacgccc aatccttttt 44820
aaactgaggg gtgtgtcatc tgttaagcat tagagaaatg tagggcgatg ccaggatacc 44880
tgtaatttac cttaaaataa gctctaccgg gtgagatttt aaatagattg actatttaga 44940
gacacacctc acattgatta gacagggtat aagaatcctg ccaggcaata atctgtacac 45000
ttagggttag caccatagac ttgatccaga aaccaccctc gctctagcca ttgccaaaga 45060
gaattcctgg ttgcctggtg ccagctagaa agctaacagg actctgcccc tggaatcttg 45120
gcattctgta cagtgggggc tgggacaccc aataggatcc agagctgggg ctccatccat 45180
ctcacagtac acagggtagg aattgcttgg ggcctcttgc tcctgcattt ttggctaata 45240
gaagttctgc gcagatggca gaatggtttt cattatatcc tgacattcat tcatcatatc 45300
atgattcatt catcatatcc tgatataata gtttcattat atcctgacat tatatcctgg 45360
gtggaaatag ctaacctttg gctacctcag acttcttcag tggtaagcta tctttaccta 45420
tcaaagggga ccttggagtt gtgcagggac cagcttggct caggtcaggt cataatagat 45480
ggccctttta cgagtaaaca ttactgcttg ggacattcca ggataaagac caagtttgag 45540
acgcctagtg atcccagttc tcccatgatt ctcccagaaa gatgcagatg tctgtgccct 45600
ttgcagctag aaatacagag agaagggagc tagcaagctt gaagcgtccc tgaaatcagg 45660
tccctgtctc cctcccctca gctgttccca ctggaatgct gagccagaca ggagcctgga 45720
gacccctgtg ggaaaggata tggatccatc gctttcatct gccgacctcc aggatgtctg 45780
ccctagaggg agtaaagagc agttaatctc actagagtta ccagactcat tgagagggga 45840

gggaattggg ggccagggct gggaagaggc caccgtttgg gaccagagag ggtagagtgc 45900
tgataagatc cggcctccaa ccaggagcca gctgtagcca gatggcctga gtgccccctg 45960
caatgacagc ctgaagtgag cagaattagc cagctcactc cttatcctgc ctgatctgat 46020
ctgtccctgt tccgcattgc accattccac acagaagaaa gactggaaaa taggaccagt 46080
agctgaagat gaaacttgtg tgtcccgggg ctcagaagta tatgggtcct gggcctcaca 46140
gactagacat atacaaggcc tgggacagat atcttctttt catttctgcc ccccacccta 46200
cttggcacct ggtaaaagtc tgttgaatta aagcaataga aacacactca ggagaagggt 46260
gtaagacttg gatcttcacc ccagagctgc tctactgcat taacaaggca acttttgcta 46320
aagtgatcca ggaaattcta ctctgagcta tctggcaaac caatagctaa ggcaagcctg 46380
ttggccaggg tgcccagagc tggcctgccc tggagaggcc tgcacaggtt tcctggggaa 46440
cttccctctc tctcctgggt gatttcttca gggcattgtg atgagaccga gatgaatgac 46500
acctggcaga aaagcactcc agacctgcta gtgccctttg aggagagccc agacttctgg 46560
gagacctctg gagtcagtgg ttcaatattc ctcacccacc ccaccaccag gtttgtgcct 46620
ggcctaaaaa gcagagcctg acttggcctg tgcccatgga catgctgact atgtgtgctt 46680
tgtgaatgca agttttctgt gtgccacgag gtcacttgga tgtgaagtct gtaggtgctt 46740
gtgagcaggc tgaacttgtg tgtgcagggg gatatgagtg tgcactgggt gcatgcattc 46800
atggatgtag aatagtgtgc ttgcttgtgc atagtgaggt aggatgtgca ttgggtgcat 46860
gcattcatgc atgtagaaca gtgtgcttgc ttgtgcatag tgggctatga tgtgcattgg 46920
gttcatgcat taacgtgtat agaagagtat gcatgtttgt gtgttgtagg catgagcgtg 46980
cactgaatgc acacattcac ccaaggaatg tacatcaggt gcgtagaaga gtgtgcttgc 47040
ttgtgtgtag tgggatcaga gatctgaata aacacactgc ttgcagtaca gggacgggtg 47100
cttaattgga tctctctaag agtttgggat gaagggggaa agctgaaccc ctctgctggg 47160
acctcaccca gaccctaccc aaagctgtgg gctggggaca tcaaagtgga agtactgcct 47220
ttctgtgctc ttcctgactt ctcacctcct ccctcctagc tctctgctcc agccccctca 47280
cttcacaggt ctgctttgaa ctccacagct tgggaactca cccagggcaa cagggccttt 47340
ttctctcctt cccgagaggc agccacagct gtgtccctaa cagctggtgg tgttatctga 47400
gggaaagggg aagggagggc agggacacag gagggatata acttgactgc ccagcccaca 47460
ctgaggaatt tcacctctcc tttctctagc acacaacacc cccaaccccc accctaggca 47520
cgcgcgcgtg cgcgcgcgca cgcgcacaca cacacacaca cacacacaca cacacacacg 47580
cccgacttta gcagcttatc tgaccaaaaa caaatggtca gccgctttct gataattaaa 47640
acagaaaccc cctcagcaac tctctccacc cccaacccca ccatttggtg ctatttcaaa 47700
ctctaaggcc atactgcaaa ctcccaaaac tccctggtct ctggggacag caagaaaact 47760
cacccactgg cctggttcta gctttctgtg attctgggaa ttacggctat ggcttccaaa 47820
tatggttgga gaagtgggtt tcttgccaaa aagatcaaaa gtgggagaca aactgaccct 47880

cttgttctaa tcccatccta attcattcac tcagaaaata ttgagcaact actggccggg 47940
cacggtggct cacgcctgta atcccagcac tttgggaggc ctaggtgggt ggatcacctg 48000
aggtacggag ttcgagacca gcttggccaa catggggaaa ctccgtctct actaaaaata 48060
caaatatcag ccggacatgg tcgcgggcgc ctgtaatccc agctacctgg gaggctgaat 48120
aaggagaacc actggaacct aggaggcaga cgttgtagtg agccgagatc gcgccactgc 48180
actcctgcct gggcaacaag agcgagactc catctcaaaa aaaaaaaaaa tttgagcaac 48240
tactatgtgc caggctctat ttgaggcact tgccatacag tgtgagcaat tcaaactatg 48300
ccctcggttt cctgcacttt cattctagag gtggaagaga gacaaaacga tgaaagttag 48360
ataaatgcag tgaactcagg taatgataag cactacaagg aaagataaac tggagagtga 48420
tgcagcgagt aaatggaaac tggcattttc agatagtata gatcggggag gtttcttagg 48480
agtcactgat atcttacctg aactccaata actagcttgc tttgtaccct gggcaaggct 48540
tttgcccctc tctgtggggc agtttcccta tctgtacatg aggacattgg gccagccggg 48600
tttagctttc tgtgattctg ggaattatgg ctatggcttc cagatgtgat tggagaagtg 48660
ggtttcttgc caaaaagatc aaaagtggga gacaaactga ccctcttgtt gatgaatggc 48720
aagatcacat gagaatccat tctccccact acagcaccaa gcccactttg acgggctatt 48780
tgttagactg tgagaatcag gggtctggaa gcctttcctt atctcccacc cacctcctgt 48840
caccctttta cctaccatta cccacccatc cccccattac ttggcacagc gccccataca 48900
tagtgccctc taatatatgc actggaattg taggaccccc atttctataa cctctggagt 48960
aatcagaact ttcttagggg ctgggcctgg tggctcaccc ctgtaaccca gcactttggg 49020
aggccgaggc aggtggatca cctgagatca ggagttcgat accagcctgg ctaacatggt 49080
gaaactctgt ctctactaaa aatacaaaaa attagctggg cgtggtggtg ggcacctgta 49140
atcccagcta ctcaggaggc taagacagga gaatttgcta cgagccaaga tcacagcatt 49200
gcactccagc ctgggcaaca agagtaaaac tctgtctcaa aaaaaaaaga aaagaaaaga 49260
aaagaaagaa agaactttct taggacaatc tggatggggc actgggttcc catgacttag 49320
aacaggaaag atgcctaaaa ttttattagc ccattgattc ccacgctgac atcagagacc 49380
cttgatgata gaaatgggga gagaggcagt agcttgggcc agcatcagca tttcatgaaa 49440
tcttgtggaa atggcacatt cttctcaccc tatcataaga caaccctctt taaatgtcaa 49500
gcaccagcac cgcgagctta gactttaata aattagtgtg gggactctgg ttactccatg 49560
ctgctcatca tgcctttgat gaataaaaac tgagcagatg aattcattac gatttcataa 49620
acaaggtggg ttagatcaac agactccttc cttaaaaagg attcactgtg gggagaacag 49680
gaaagcaagc gagacagtct ttgatgccga aaggttgtgg ctgcgatctg ctccacacac 49740
ttgaccttac ccatggggaa agcaagggag ggagagccaa agggttttgt ccaggtgtcc 49800
ctatgccacc ctgtcttctc ctcccacccc caaaggttag gtgcagagga agtctttgtt 49860

tggctgagag gagggacttg ggcaggaggc cgtgaagaca ggaagtacag cagaggcagg 49920
aaggaggtca ggaagaggct gacaggacca ggccggctca cacccagccc tgccagcctt 49980
caggagagca acatggtgtt tctaaaaata tttacagtga gtctacatta atgagatata 50040
tatcagcact gacaatggga ggatgctggg ccaggccctc cagggtagaa gaccaacgca 50100
attagcccag aggctgcaca aaggcaaggc cagccttgtg ggaaggcctt gtgggaagtc 50160
ttggggaagg ccagccttgc acagctaaag gaagaaatca agccttccac cctggggccc 50220
aaacaggcaa tggagaccac agtgccccag aaacctcccc cggggaacag aaaacatccc 50280
agattcctgg acctgcaacg agcatcttca acctgcatcc ctgaccccaa ttcagtgcag 50340
aaatctcctc taccacaccc actctgagga ccatccaggg gtctcttgaa tacccctgtt 50400
gaccagacac tcaatcctgc ctccagcagc ccattcccat tccagtcttg gcatccctac 50460
ccattcaacc ttcaggagag gagagtggtt cagaacatag aggctggagt caggtagcca 50520
cttacaagtc actttgccat ggagcaccaa ggagtgtgat catttcaagt cttggttttc 50580
tcatctgtaa aatgggagtc attagaatat ctactgcccc agggctggta taaggtttca 50640
gcaaggtaaa ggagacagca tgcaaagaat gccatgtgga cagaaagaca caggctaagg 50700
tgcaaagatg caaaaggaag aatttaaggc tgggtgtggt ggctcacgcc tgtaatccca 50760
gcatttggga ggccaaggcg ggcagatcac aaggtcagga gttcaagact agcctggcca 50820
agatggtgaa accccacctc tactaaaaat acaaaaatta gatgggtgtg gtggcgggcg 50880
cctgtaatcc cagctacttt ggaggctgag gcagagaatt gcttaaacct gggaggcaga 50940
ggttgcagtg agccgagatc gcaccacttc aaccagcctg tgcaacaaag tgagactcca 51000
tctcaaaaaa aaaagaagaa gaaaagaaaa gaaagaaaga atttaaaatg tcctaggact 51060
aaagtgacat aaggctaaga aaattagcaa atgccatttt gctgctgaag agctaacact 51120
gcactgtgca gtgggcgagc cacttgccat gtgtggctat ggagggctgg aaatgtggcc 51180
aggcagatgg tgggacactg caggtgtaga acacacactg ggtttcaaag acttagtatg 51240
aaaaaagaac gtaatatatc tcattagtaa tttttttcta ttgattgcat gtcaaaatga 51300
taacatttgg atatattaaa gtaagtacat tgttaaaatg aagtctactt cttttgtttc 51360
ttttactttt tttttttttt tttttttttt ttgtgagaca gagtcttgct ctgtcaccca 51420
ggctggagtg cagtggcggg atttcagctc attgcaacct ccgtctccca ggttcaagtg 51480
attctcccgc ctcagcctcc cgagtagcta agattacagg tatgtgccat catgcctgga 51540
aaatttttgt atttttggta gagacagggt tttgccatat tgaccaggct ggtctcaaaa 51600
tcctgacatc aagtgatatg cccacatcgg cctcccaaag tgctgggatt acaggagtga 51660
gcctccatgc ccagcctcgt ttactttttt aatgtgtcta ctaggaaaat taaaatcaca 51720
tatgtaactt ctgttatagt cctattggat agtgctgagc tgaaggatca tatctaagcc 51780
ttaggatgct cactctggag gcatctttga ggatgggtta gaatgggtta ggctcgttag 51840
gaaacaggtg cagtttttca gaccaaaggc ccatattaag gagaagactc caattccaat 51900

tttccaacta caggtagttt gtcaccaaca aacaaaaaat aataataata atagtaatgg 51960
ctcttactta ttaagtgctt actccatgtc agcaggcact gttctagtga aggaccatga 52020
ttttaaaaag tgaactctga aagcaaaaat gcctggtttt aaatcctggt tctgctagta 52080
tacgaagcct cagtttcatc aaccttaaaa taggggtaac cgtatatagc attgtcagaa 52140
ctacgagata ctaggcttag ataccagact taggtagtca tctcttacta ttactcccat 52200
agtaacaggt agtgtattgc atcctaatag gtaaagttta ttaccaggca ctgtataagc 52260
acactcaaat gctttttccc aatgtaagtt ttaatgccat cttttacatt atgatcccca 52320
tcttacagat tggtaaactg aggttcacag agttacttca ctgtctcagg accatgtggc 52380
ttctgagtaa gacagagagg atttgaaccc ttggctttga ctctagaagc ctgagagctc 52440
tacagccagg atgtgttttc ccagcacaag gccatgcttc tgcattcaga tatgacctgc 52500
tgttgcttca ttggagggag ggaggagatt tctcagcaga gaaatctcat agatggatcc 52560
cgctctctcc gcctgtctga aggatgatga gattgagtgg agaaaaattc acgtaggctc 52620
acagctatga tgataaaaag ataatttgta aaaccagcat gtttgtaatt actcattgac 52680
ccatagacat aaagatcaag ctatgaataa ctccacaata gcagcaataa caatactagt 52740
gaataatact agtaactatt tacaatagca aagacttgga accaacccaa atgcccatca 52800
atgacagact ggataaagaa aatgtggcac atacacacca tggaatacta tgcagccata 52860
aaaaagaatg agttcatgtc ctttgcaggg acatgaatga agctggaaac catcattctc 52920
agcaaactaa cacaggaaaa gaaaaccaaa cacagcatgt tctcactcat aagtgggagt 52980

tgaacaatga ggacacatgg acagagggag gggaacatca cacactgggg cctggtgggg 53040
ggtggagggc aaggggtggg agagcattag gacaaatacc taatgtatgc agagcttaaa 53100
accttgatga tgggttgata ggtgcagcaa accaccatgt cacatgtata cctatgtaac 53160
aaaccggcat gttcttcaca tgtatcccag aacttaaaac aaaattatta ataataataa 53220
taatggctct tatttattaa gtgcttactc catgtcagca ggcactgttc tagtgaagga 53280
ccatgattta aaaagtaaac cctggaagca aaaatagctg gtttcaaata ctagttctgc 53340
tagcatatga agcatctgtt tcatcaacta taaaatagga gtaaccgtat acagcattgt 53400
cagaattctg agataatagg cttagatacc agacttaggt ccatgcctgg cacagggcga 53460
atgctcagtt aatattttct gttacaatca tctcatttaa tccccacaat aacccaatgg 53520
gtagagagga tcaatagtga taaatgggaa tgataatatt cccagtttac agatgcagaa 53580
actgagatgc gaaacaataa gaaacttgtc taagcccctc catttctcag tattggagat 53640
gggattcaga cctaggaggg ctggtttgag gatctacagt cttagctacc atggtagaca 53700
gttcttaatc ttaaacagtt tgtaaagtca tatttttttc ttattcttct tctttaatga 53760
tactttccat cttagttcct tgaagtgtgg aacgtgcctg ccctgcctgc ccgaggcttt 53820
gacagaagcc agtgggatag tcaacagctt tgtgtgagca cagagagagg tagctgcccc 53880
agatgctgcg gggctctgct gatgaatgac ccatcagtcc cacgggtccc acaggtatcc 53940

agcggcaatc tacccttgcc aagaaggcac aacactagtg ctacagcatc tcccactctt 54000
aggactgctc gggccctaag gatggttggg gaggtggaaa gaagaactgg acatgggtga 54060
gccagctcaa gcacagagct gcctcctgag cctggctgtt tggctgctgt gcctctgatg 54120
gctcggtgat gctgacctct ggtggagatg agctgctact cagctttgac ctccctctga 54180
gcaccaggag tcccagcact ggcttccagc cccgccaccg ggatgcagac ccagcagcct 54240
gagaccccag agggtgcacc taagcccaac ctctctcctt attccagggc cctgtggtat 54300
ctggaattgt ctaccctgga accttatggc tcaggggcta gaagaaccct tagcaaaagg 54360
aacttaccca gctgatgatt ctgacgctga acaggcgcta gaacccagca caggtcccag 54420
tctcttggag aattaaggcc tcggcctctc cacctagttc agtgttttga gctacacatt 54480
ctcttctgac atgtggttac accgcactgt aataatacat gttatgacac tgatatagaa 54540
aaagaaatgt tcttgtctaa ttattttctg tttgggtggt gcagaagtaa aaccaagaga 54600
cgttgaatca tcctcagtat tattcatcac tcttcctcaa gcatgtcact ctgtaaagac 54660
aacatatgaa agggaaaatg ttaccctgtt ttattaagga aaattatgga ctagaaaaag 54720
actgcttttt tttttttttt tttttttaat aagctgacat tgatagagaa aagttgagcc 54780
aaatctctgg aggcaggaat ctcattgctg agcacccagc gccaccttgt ggcttctcgc 54840
aggacagcga ttccagcttc cactcatttc tctacccacc actaaaaaca tttattgagc 54900
atttcttatg ttccctttca cctgtttctc tgctgagcct cttatctgaa tcttttatct 54960
caatgaactt tgcactgaaa tctctttcga tacactcatt aattttagca tgggaaaaag 55020
ggtggctaca cagttgagtt ggcccacata agaaggtgtc tatggagata ttctttatcc 55080
atgagccctc ccagttattc caatgtcagc cctgggaccc tgaagatcac tcacagattt 55140
aattagacaa atctcttaaa atatgtgtgt atattttcct ttccctcttt ctctttctgg 55200
aaatacgttt tcctgtttct caggcagcag ccactctgtt aagacagggg aacttcagca 55260
cagacaggtt gcgggggaca cagctaatta gtgactgagc cagagctggg gcttcagggc 55320
tttgtgactc actcaaaaaa tgtccactaa gtccttcctc tgtagggggc cctatgctaa 55380
gctcaatggt aaatcaaaac cccacccagg attctgccct cgaaagatag agccttgtca 55440
gggacagagg catctactaa agaatgatgt aaaaaagtaa agctgtaact gtgcaaagta 55500
ttacaaggaa agctatgtcc acagacagga tttttagagt ctgactttta gagacttcaa 55560
aggactgaaa atatcatggc cccctccata atctatttta aatgaaaaca aacaatttaa 55620
agataggtgt aaagaaatcc ccaaaagcct tatttttccc ataatgacat ctttgaaagt 55680
attttatgct aaaaggaatc aaatagcatg ttatgaaaaa ttgggagtga ccctgtttct 55740
ctaatgcctg aaagagatgt ttagccagcc tgttggggaa tccaagagtg gatacatata 55800
taataggagt ttgacctagt caggaaaatt tggaaagact ttcctaagaa aatggtaaca 55860
gtactgagag ctacaggata agaagcagag aggtgaaaac catgacgaac aattacagca 55920
agcactgggc agatcgtgta cttcctgcag aagctgggag cttgagacag cagaggctga 55980

aagaaggtgg gagaggctgg aacagaggac aatgaggact gtgttgtaag agaagacaag 56040
gagccagacc acactgggct ttgtgggaca ccttgaggag ttttgcattt atcctccaag 56100
caatggaaag ctgtcgaagg gttttaattg gcagggcatg gtgacatgat ccagttggtg 56160
ttacagaaaa gccacactgg ctgtatgccg aaagtggatt ggcaagagga caagcacctt 56220
agcctactgt cctagtttgt tttgtgttgc tataacagaa tggcatggac tgggtatttt 56280
atatcaatag aaatgtattg gttcatggtt ctggagactg ggaagtccag tgtcaaagtg 56340
gtagcatctg gccagggact ccttgctgtg tcatcccata gcagaaggtg gaagggcaag 56400
agaacacaag agagcaagag aaagagcaag aggacaagag tccaataagg agacattaat 56460
ccccaggaga gatgatagga gcctggatta agagtgtggt agataaaatg gagagaagta 56520
agtacacttg agcaatatgt tggattgggt atgggtgtaa ggaaaaggga gcttttggga 56580
cgtctcaggt ttctcacttg ctggaactaa gtgaacatgg tgtggctgaa ctggtgagca 56640
agccccagta tctactttct cctttttcct ttagtgattg caccggagtc ttcttacatg 56700
ccaacttccg atcttctcag gctcacttac cttttttatc agctctggga gggttctgat 56760
gaacccaaag caggcacagt ctgatagctc cctcccctct ggtccctgct atgcctttct 56820

cacctcccac atgggctttt gctgatttca ctggacttgg cacggtgccc atgttttgca 56880
tggaaactca gaaaagcaaa agagtcggca ctccactgaa aaatgttttg accaatgaga 56940
agagctactg gagatattct tcctgcattc tctcccagag gtctggtcct caggaacagt 57000
tgatacaact tcttggtgtc ttgtttctat agattggtca attaacatgt accctcatat 57060
tagttgtctc tcttttcttc ttcactctcc ttgttagtta ctccgatttg taaactttca 57120
gctccagact cgctttccag gaaacacagt ggtatggttt aaatgtgtcc cgcaaaattt 57180
atgtgttgga aacttaatgc ccgatgctac agtgttgaga agtgggaact ttaagaagtg 57240
attaggtcat tagggctcat gaattgatta atggatggat taataccgtt ggatagacca 57300
atgctaataa atggattaat tccatgacct caaagtgggt tagtcatcat ggcagtgggt 57360
ttccaataaa agagtaagtt cagccccctt cgtttctctt gctctcttgt gttctcttgc 57420
ccttccacct tctgccatgg gatgacacag taagatgtcc ctggccagtt gctaccactt 57480
tgccactgga cttcccagtc tccatagcca tgagccaata aatttctatt catgtaaaat 57540
acccagtcca tgccattctg ttataacaac acaaaacaaa ctaggacact agactaaggt 57600
agtaacataa tatccaagtt ttagctggga acacactacc cagctataga ctagatgttc 57660
cacctcccct tacaactacc tgaagcctta tcaaagtgat atgaacaaag taatgtgtgc 57720
ttcttctggg tcacattctt taaaaaagga aattgcttac tctttatatc ctttgtttct 57780
cctttcttat atggtggaac atggatgtgg gatagcaagc cagctttgac catgcagatg 57840
aagacaacat ccaaagggaa gcagggcaac agagcaacaa gacactgtgt gcaggagagc 57900
tgagccacct gtctacctct ggcttattaa tagaaagaaa aatgactatt tttaaagccc 57960

tgtctctctg cctcatatct taacttgtac cataactcac acttagggaa cagtggaaca 58020
agaccaggct tgaatgcagt ttggggcatg ttgcatttga gaaggttttg agaaatgtaa 58080
gtgaaatatc aattgatcag ttaggcacaa gtccagatat cagaggagag gtctggactg 58140
aaaatacaag tttgtgaata gtctcccttt cacgtgtggt tggtaattaa agtctctgtg 58200
tgtgtgagat ggtctaagga gaaggaagag agtgagaaga gaaggatgcc tgtggtaggc 58260
agcttctaag gtgaccccaa ttatctgcac ctcctggtat ttttgtccat ttgcaatcct 58320
ctccactgga atgtgggctg aacctagcaa ctcatttcaa aaatatataa tatggcaaaa 58380
gtaatgggat gtcacatcca agactagatg acaaaaacac tggcttccat cttgaatgcc 58440
ccctctcagt cacttttttt gagagaagtc aacttcatat tgttagctgc cctggggaca 58500
gatctatgta gcaagggcct gaggaaggct tccacccaac agccagtgag ggattgaggc 58560
ccttagtcca acatcttgta aagaaaggaa ttctgccaac tacctgtgaa agagcttgga 58620
agcagattct acaatccctc tcaagctttc agatgactga gccccagcta acaccttgca 58680
acaccttgac tgcagctgta tgagactcta agtcggaaac atctagctaa gccacagcca 58740
ggctcctgac ccagagaaac tgcaagataa aaagtgcctg ttgttctaag ctactaagtt 58800
tgaggaggat gtgcttctcg gcaattaata actaagacag agcccatgca agagcccgga 58860
ggaccctgtc gtttagtagc tggtagaaga gaatgagcct agaaacttta tacatcaggg 58920
caatccaggt gagtgccaaa tgtctttatc aattttgtgc tgctataaag gaatgcctga 58980
ggctgaatac tttataaaga aaaaggggtt atttgactca tcatttaatg gctggaatgt 59040
ttaagatttg gcctctgcat ctggtgaggg tctcaggctt cttccactca tggcagaagg 59100
tgaaggggag ccagcttgtg cagagatcac ttggcaacag aggaagcaaa agagaaaggg 59160
gtgggatacc aggctctttt tagcaaccag ctcttacggg aattaattga ctgagaagcc 59220
actcgcctct gagaaagggc attaatgtat tcatgagaaa tcttccctca tgaaaaaaac 59280
acatccattg ggccccaact ccaacactgg agatcaaact tcaacatgag tgtcggaggg 59340
gacaaacatc caaatcatag caccaagtca tagaatccaa gagaatagga ctggtcaata 59400
aggagggaat agtcaagagt gttgagatgc tgctgacaga tcaaataaga tgagagaaga 59460
atacttgatg gacattgaga tgcagaagtc actggtgacc ttaggacaga agccaatatc 59520
tcaggactga tagccctgga aaacccaacc atttaaagtc tctagaaaat atcctaaggg 59580
tatatagcaa gtgaaagatt tactcaaaaa ctaaagttca ataaaacagc aagagtctgt 59640
ggaacttgag ctatgacatg tgtgctcact ctctcactca ctctctctct ttctcccttt 59700
ctttttcccc tagtcctaca ttctcagtcc agacagacaa tagaggcctc agtctgaatg 59760
gatgtggtca aaaaatgtga ctccttcttc cctaagttcc cagacaaggg acataatatt 59820
tcactgggaa agacagacca ccagcatttc tcatttcccc aaactccaaa atgcagagga 59880
taaactcctg ggaattgtgg gcaaactatc aggagctcac tttctccagc tagttctcat 59940
tcatggagca gagattcacc ccaggcacag cagactgaga atacgggggt cttgattacc 60000

tttagtttta aggcagaggt tccacactgg aggaggcaaa ctgagaagag gaagagttaa 60060
ccattatcac tcagtgccct gctctagtat ctgggtatca ctctaagaga agtgggtcat 60120
tatccctgtt cccagatcca gagaaaaggt tcaaagactt tggccagtag gagaggcagt 60180
taataagaac aaagagctcc aaagctctct caagatgaac tgactttatt tgaaacagag 60240
tgtagggaag ttcaagccta agggtgctct aaataacagt ggagattttg gtagtaagta 60300
ataataaatg gaggccagta gctccattag ggcagcaaga taaaccaaag tccagatcat 60360
ttaccagaga taattatggt aagagtgaac taagaagagg cttcctggag tcaggtaaaa 60420
tctattgttg agttccttca aagtcttgcc tttaaaacta cctccgcaaa agtcctgcat 60480
ttaatttgat caaactgcaa agcaatttat gttccagtgc attgtttaaa acaatagaga 60540
aatcagatga caattagtga agggtaaaat ctcaatgtta tactagcagt cagacagttt 60600
aacaaagaga tctaagaaag agacagtcaa agacagtcca gctgagacta gtcatgcagg 60660

atgactgtat gcatgcccgt ggctaaactc tctgaggagt gacattagag gcttcatatt 60720
aaaggggaaa taggcttcac taaaatagtt cagccaagtc actagacaaa taaacacaca 60780
aagaacaaca agaacaaacc atggagggaa gacagactca ctctccagca ttcctaaaat 60840
atatgaccta aaatgtctgg gtttcaacaa tgaaaaaatg caaagacatg caaagaaatg 60900
gtaaaatgtg ccccatacct gggggggtag tagacaacag aaaatgcctg tgagatggca 60960
cagatataga ttttaaagat gtcaaagcgg ccatgataaa tgctttcaaa gaactaaagg 61020
gaattatatt taaagaagta aatgaaggta tgatgacaat gtcttatcaa aatgagaata 61080
aaaataaaga gacagtgatt attttaaaaa gaacagaatg gaaatcctga agttaaaaat 61140
gtatagttga tatgaaaaac tcactgaagt aactcaacaa tagattttac ctgacagaag 61200
aaaaaaatca gtgagcttga agatgggtca ataatgatta tgcaatacaa aggacaaaga 61260
taggaaaaaa aatgagaaaa taaacaaaat agaacacaga aatgtgagat accattaaca 61320
gcaaaatatg cataatgaga ttgctagaaa gaaatgacaa aaagaaatga gcagaaaaaa 61380
tactcaaaaa atggtggaaa agttcttatt tatttattta tttatttatt ttcaagaaag 61440
ccttgaactt ctgggttcaa gtgatcttcc cctctcagcc tcccaggtag atgtgcacca 61500
tgatgcccaa ctaatttttt taattttttt cgagatgaag tctcactatg ttgcccaggc 61560
tggtcttgaa cgtgtgacct caagctatcc tcctgtttca gcctcctgag tcactggaat 61620
tacagacatg agccaccaca cccagcaaaa tgtttttaaa tttaatgaaa aaataccatc 61680
taagaagctc aattaactcc aagtaagata aatacacagc aatccacatc caaacacatc 61740
atggtaaaaa tgctgaaagc cagagataaa gataaaattt gaaagcagca aaagaaaaat 61800
gacttgtcag ctagaagaca accctaataa cattaacagt caactattag acacaatagg 61860
taccagaatg cagtaggatt acatacaaag tgctaaaaga aaaaaaaatg gtcaactaag 61920
aatcctatat ccagcaaaaa cagtaaaaaa agactaacaa gccaactttt aaatgggcaa 61980
aggatttgaa aagactttta tccaaagaag atatacaaat agccaataag catgtgaaaa 62040

gaggctcaac atccttagct atcaggaaaa tgcaaagcaa aaccacagtg agatactatt 62100
tcatacccag tgggatgact ataatcagaa ctacagacaa taagaagtgt tgatgaggat 62160
ggagaaaaac gaaccctcat aacactgctc gtggaaacat acaatggtgc agttgctttg 62220
gaaaacaatc tggcagttcc cccaaatgag ttaccatatg acccagcaat ttgtctccta 62280
ggtatatacc caactcctat tatctggatg tttatgtccc ctcaaaattc atatgttgaa 62340
atcctaaccc ctaagatgat agtattagga ggtggggcct ttgggtggtg attagatcat 62400
gaggtaggag ctcttgttaa tgggattagt actcttacaa aataagcccc aaagagctgc 62460
cttgcccctt ccactgtaaa aggacatagc aagtgacacc atctgtgagg aacaagccct 62520
cagcacacac caaatctgcc agttcccttg atcctggact ttcaagcccc cagaactgtg 62580
gcaaataaat gttttctgct tataagccac ctagcctata gtatttttgt tatagcagcc 62640
caacagacta tgacaccaag aaaaatgaaa acatgtgctc agacaaaaat ttgtacattc 62700
ttgttcatag aagcattatt catagtagcc aaaagggtga aacaactcaa aggcctaaca 62760
acaaaaatgt ggtacctcca catagtggaa tattatttgg cagtaacaag gaatgaagta 62820
caaatacctg ctacaacatg gatgatcctt gaaaacattg tgctaggtga aattagccag 62880
tcacaaagaa ccacatattg tatgattcaa tttataggaa atgtccaaaa tagacaaatc 62940
tatttttaaa aagtatatta gtggttgcct agggctgggc aggaagaagg ggaggtgaaa 63000
agggcagtga ctgctaatgg gtatggggtt atttttgcag agtgataaaa atgctccaaa 63060
acttattata gtgatgagtg catacctctg agtattcttt aaaaaaattg aattgtacac 63120
tttacactcc caccaacagt gtataagcat tcccttttct cagcaatctt cccagcatct 63180
gctgtttttt tactttttaa ttctgactgg catgagatga tatctcattg tggttttgat 63240
ttgcatttct ttaatgactg gtgatgctga gcattttttc agccattgtg aaaagcagtg 63300
tgatgacttc tcaaaaaact taaaacagaa ttattgggta tgtacccaag ggaacataaa 63360
tcattctatc ataaagacac gtatattcat tgcagctact cacaatagca aagacatgga 63420
ataaacccaa atgcctttca acagtagtct ggaaaaagaa aatgtggtac atctatacca 63480
tggaatacta tacaaccata aataagaacg agatcatgtc ctttgcagca acatggatgg 63540
agctggaagt cactacccta agcaaactac cacaggaaca gaaaatcaaa taccacatgt 63600
tctcactggt aagtgggagc caaacaagga gaacacatgg atactaggag gggaatgaca 63660
gacactgggt gggaggaggg agaggatcag aaaaaacacc tacctagtac tatgcttatt 63720
agccaggtaa tgaaattatc tgtacgccaa acccccatga catacagttt atctatataa 63780
caaacctgcg cctgtatccc tgagcctaaa ataaaagtta aaaaataggc cgggcatggt 63840
ggctcacgcc tgtaatccca gcactttggg aggccaaggt gggtggatca cttgaggtca 63900
ggagtttgag accagcctgg ccaacatggt gaaactccat ctctactaaa aatacaaaaa 63960
ttagccgggc atggtggcac ccacctatag tcccagctac tcgggaggct gaggcagaat 64020
tgcttgaact cacaggcaga ggttgcagtg agtcgagatc atgccactgc actccagcat 64080

gggtgacagg gcgtgactcc gtctcaaaaa aaaaaacaaa aaaagttaaa aaaaatgaat 64140
aaaaataaat acaattttta aaaagaattg tacactttaa ataggtgaat tgcatggtag 64200
atgagttata tcttaatata gctgttatca ttttaaaatg aaaagaatcg agttaacaat 64260
taggtgaaag caaaaagaga taatgatagc tacccagggg aggaattcaa ggacacgtgg 64320
aagggctagc cttaggcagg aggacggaaa cctcctaccc agggcactgg gatgaatgca 64380
ggtgttatag gtttgaagat ttggatttga gagtatgact gttctcatct gatggctgaa 64440
caggtagaca gagggtagca gccttgaagt ttgaagagaa tgaagaagaa atgaaatagt 64500
tgtggaggtg agatggtggt cgctgggagt cctgcagtac tgatgttgcc tcacactcct 64560
gcccactggc gccctcttcc cacttctccc tttggccctc catgcaattc ataaagggct 64620
tctcaaacaa aagtgctcat tgggggtcgg agtgagggaa atggttgcca ggacaatgag 64680
aaattgataa ccttattcag atctggagaa caatgacaga gagaaagtgc aaaagaggag 64740
gaagtcaaaa tggaagggga gaggagaggg aacctccagc tccaccctgg acttttctaa 64800
atctttcagc agggccatct caggagcacg caccatgtgc cattgcacag aactccatgg 64860
cggaagtggc tccctgtggt ttcacgttct gctttcactg ccttgaaatt cttaacaagt 64920
tgagtttttt gttttgtttt gttttgtttt gttttgacag tgtcttgctc tgttgcccag 64980
gctggagtgc agtggtgcca tctctgctca ctgcagcctt gacctctcag gttcaagcaa 65040
tcctcctgcc tcatcccccc aagtagctgg gactacaggc acatgccacc acacccggct 65100
aatttttttg tatcgagatg gggtttcacc atgttgccca ggctggtctt gatctcctga 65160
gctcaaacaa tctgcccacc tcggcctccc aaagtgctag gattacaagc atgagccacc 65220
atgcccagcc taaaattctt aacaagtttt aaataaggaa gtgcacattt ttatttttca 65280
caggcaatgt aacccatcct gccctataaa gaacacttgt ctatccagaa catggacaag 65340
aatgtcttct ctacagctag gaaaaaaatt acaaagctca gacctggact gacccaagaa 65400
taaaacccca gatcatttgt cttccatagc ctagttactc ttactttcca caaaattcca 65460
gtgaggaact atggtgttta aaggtattcg ttgccactcc ctgaggaggg gggattatgc 65520
ttcctcaccc cactgacatc tggcttggtc gtatgatttg ctttagccaa gaaaatgcct 65580
cacttctaag cagaagcaga ccctgcgcat gtttaccgtg ttttattttc cttctgttac 65640
aaaaccagta atggatagct gggcacgatg gctcacacct gcaatcccag cactttagga 65700
ggccaaagct gggattgctt gagcccagga attcaagacc agcttaggca acatagtgag 65760
accctgcctt tacaaaaaaa tacaaaatta tctaggcatg gtggtgcgca cctgtagccc 65820
cagctagttg ggagactgag ctgggaggat aacttgagcc tggaggtcaa ggttgcagtg 65880
ccatgatggt gtcactgctc tccagcctgg acaacagagc aagactttgt ctcaaaacaa 65940
aacaaaaccc agtaatgccc cacatatagg ctaccgatca gccaagatgt catggggtag 66000
tgctacagct gacctgggtg gagatgcaga gggagcaaga gatacatttg gttgctttaa 66060

gccgctggaa ttcaagagtt atttgtgatt gcaacataat ctaacttatc ccaacttata 66120
cagaatggcc tcattttcca ggatcacatc aacttaaaat cagcaaattt tgagatccta 66180
gatggccctt ctctaacctc tagaatctga ctttacacag aatatatatt tgttaaattc 66240
atagatggga actcctattc cctctgagtc agatccagac tccttcatct gcactcacaa 66300
ccctccatgg ccagtcattt cagccttgcc tcccactacc ccacaaacga ccaatttgct 66360
cactcacctg cacatgcttt tcatttgttc attcattcat tcatgtactc ttttatccca 66420
tatgtactgg tgcctgctct gtgacaggaa gtgtgtgtga tgcagaattg tttacacaga 66480
catggtctcc ctgccctggt ggtttagttc ccagaccttg gtcttgcagt ttctctctgc 66540
caagagcatt gttctgactt ttctctgcct gtctaagctg ctagccctcc ttctacacac 66600
aactcaaacc gtgcctcctt ccactaactc atcaccccac ttactctggc gcaccctctc 66660
cttcctctca actcctacac caatcatttt cttattgatt acgtggcaat tagttacatc 66720
ctgcctgata gtctctctcc tattgtgttt tgaacgacta ttgaaatctc attattaaaa 66780
tatgtatacc ttttctcatg ttcttaaata ggtccataga agcagcacat cccctaatgc 66840
aggctgaact aattaattaa aaccaagtgt agtaccttta tttacattag aagacttgtc 66900
tacattcaac caactttgac ctagaaaaat ggcactttcc caataatcat atgaaaaaaa 66960
gctcaacatc actgatcatt acagaaatgc aaatcaaaac cacaatgaga taccatctca 67020
caccagtcag aatggtgata attaaaaagt caaaaaataa cagatgctgg caaggttgcg 67080
gagaaaagga acacttttat gctgttggtg ggagtgtaaa ttagttcagc cattgtggaa 67140
gacagtgagg tgattcctca aagacctaaa gatacacatg ccattctgaa gaataggacc 67200
tggagccagg aaacctgaga gctgcctaga actaaatcag agacccattc agctatgaca 67260
gaaaatactc tcttcattta catagggcat acacccagta aatgactttg taactttatg 67320
cttttcattt acataaggtg tacacatgac tttgtaactt cacttcatcc tcttggtttt 67380
tttttgtttt ggttttgttt ttgagatgaa gtcttgctct ttcgtccggc tggagtgcgg 67440
tggcatgatc tctgctcatt gcaacctcca cctcccgggt tcaagcgatt ctcctgcctc 67500
agcctcccaa gtagctggga ctacaggtgc atgccaccac gcccagctaa tttttgtatt 67560
ttttagtaga gacggggttt caccatgttg gccaggatgg tctcgatctc ttgaccttgt 67620
gatccacccg cctctgtctc ccaaagtgct gggattacag gcgtgagcca ccatgcccag 67680
cctacttcat ccttgttatt tacatagggc atacaccaag taaccaatgg gaaatctcta 67740
cagggtattg aaaccccaga aaattttgta accaggaccc ttgagccact tgctcagggc 67800
ctgctcccac cctatggagt gtgctttcat ttgcaaaaaa tctctgcctt tgttatttca 67860
ttctttcctt gctttgtttg tgcattttgt ccaattcttt gttcaaaatg ccaagaatct 67920
ggacaccatc caccggtaac atatttttgc cagcaagcca ggaggtaagc ccaaagtttg 67980
ggatttattt ttctcctttc tcttttcctt tctactccat acaggggaat ctcttttttt 68040
tctctctctc tctctctttt tctttccaac tcaggaacct tggtgggcag ttcctaaaca 68100

cagaggtaac tgcaggtttc tggccatggc cactctctgg tgaaactaag gagtttccgt 68160
atggaggctc ctgactgccg tcacctggtt caagtaagag acctgggtct tttccctttt 68220
tttccttttt ctttttcggt ctttcagtgg tcacttccta gcagctcctt ggcaattgag 68280
ggcaactggc cagggccacc ctcgggtgtt gcctgaaggc caaggagtga atggggatag 68340

ttgccctgcc tggagaggga aggactcttt tctatctttt ttggttgtgg tccctgattc 68400
ctacatgtgg ctcagctcat tcaggcaaat tcacatacgt ttcaggacag ttaaaccttc 68460
ttttcttatg ctaaattctt cccttcccct actcaactgg ctagggacaa aagaaaccca 68520
cccagcctcc agttcctaac atgaaagttc atggaacggg aagcatggga aagtgtggcc 68580
atatcaaatt ataaggacgc cggaagtcga ggtcttcatc cagggacaaa agcaaagttc 68640
atagtaggcc atcgcctctg gagggaaaat atgcaaagcg gcaccagtgc ccacctaaag 68700
tcagagatgt ctgaaactct aagattagac accaaagggg ggacccccca ggggatcccc 68760
tggacctcaa gctctccaaa ggggatgccc tcggcagaag ttctgaggcc tagtactaag 68820
ccctccttag aattttctct cacagctgca ataccgtttg gccccaatat tgtttggaat 68880
ctggagtttg ctgttgaatg ggaaagtagg ttggagttgt aatagctagg cttttgtgct 68940
gctgttctaa gcaggatcag gcctggttat tacgtgatgt tctcctgtgg tgctgtctga 69000
ccccagtgtt ctttggagtc tggggaggtt tggcctttaa aaatcaaaca gccatggaaa 69060
ctgctgtacc caaaattttg gttcacaccc ttcattggat tacctattgg ggcaaacaaa 69120
gtaaaactgg tgagtttgta ttgctatctc atggctagag ttccaaaata aaagctattg 69180
gatcttcatt tatgtgtgtt gtacatatgt ctagatgtgc ttatttgtat gcacacttat 69240
tgttgtatat tgtgtctact aaattggctt ataagtaaaa gagcacttat aaattaagtc 69300
taagcaattt tcgagtgcac atgatttaag tataacttta ctaaacaagc tggttttaaa 69360
attattggta aaataaaaat agaaatccct tcagaattgt caacacatgg tcaggtgcag 69420
tggctcatgc ctgtaatccc agcactttgg gaggccgagg caggcagatc acctgaggtc 69480
aggagttcaa gcctgaccaa catggagaaa ccccatctct actaaaaata caaaattagc 69540
caagtgtggt ggcacatgcc tgtaatccca actacgtggg aggctgaggc aggagaatcg 69600
cttaaacttg cggggtggag gttgcagtga gccaagatcg caccattgca ctccagccta 69660
ggcaacaaga gtgaaacttc atctcaaaaa aagaattatc agcatacatt tctgtctgga 69720
ttttatattt gtctctgcta gatattttga ggtgtcaggc tttggcatag aaggttataa 69780
agctataagc caaaacaaaa tgatcttggt ttgcatgcct ttttctgaca aatgagagta 69840
atttaatgtt ggtagctaaa tcttctgagt tattggcaaa aatacatatg tatttaactt 69900
tgaggctttt acttaggttt aggtgagcac ctgatgttcc ctggctattc aaaacagtga 69960
tactgtctaa tatctcagtt tacagaagta atctggataa actattaaaa atgaaagaat 70020
tgagtacagt aaataagata aatattttag gtaaattttt gtgtaaatta aaatcttaaa 70080
attattgttg atgctcattg aatatctggg ttatttccaa ttaagaaggg gttgcgatat 70140

ggggaaatat gtttctaaaa ttggggggaa agtttagata ataaaatatt ccttaaaacc 70200
tcatagagaa taggagacat ttgactaatt aacattttca tagttaaagc tcttaatctt 70260
gattaaagaa aaataagaaa tattgtaaag aaatgtattg gctgtttggc aattcttttt 70320
ttaaatataa ttaagaatgg agctggattt agtgtggaga caaatttcac atacctgctt 70380
gcttcacact attttactgt ttttcatgga tagtgctggc actggaatac ttactggtca 70440
tgtgcctaga gtaaatttct tgattgcaca ggatgtatgt tgatattagt ggacttaagg 70500
atattaaatt gtgtatcagg aataaaatat tcattatgtg ggtttttagg gcatctgggt 70560
aacaatgtag cctccagggt agattgagta ggaaaaattt atggttgatt ttctgtttat 70620
ttgtttttgc ttctaatttt cttttgtttg ctatttattc tcctctgggc tttgcttatg 70680
tgtggagata tataaagcca tgatgctttc tttttgtttt tttttgtttt tgagacagag 70740
tttcactctt gttgcccagg ctggagtgca atagtgcaat ctcgactcac tgcaacctct 70800
gcctcccagg ttcaagtgat tctcctgcct cagcctccct agtagctggg attacaggca 70860
tttgccacca tgcccagcta attttttgta cttttagtag agacatggtt tcactatgtt 70920
gcccaggctg gtctcgaact cctgacctca ggtaatccac ccacctcagc atcccaaagt 70980
gctgggatta caggcatgag ctactgtgcc cagctagcca tgacgctttt ttattttcta 71040
gcagaaggat tttatttggt tctgtgaata gttactttgt ttcctatgca tttctagcaa 71100
gtaatcattt atttcattta tctggaattc ctagactacc tttgtccagc ctgcaagaac 71160
tgatggagca caacagcttt ttcttaaacc ttaaactaac tttttggata ttaggcttcc 71220
tgatacttta agtgcattga gtatacattc atcaatagca ttttagtcat atttctctct 71280
ctgcctaatt tctccaaaat ctgtaaatta tttgtgaata ttcttaattc atggcaatgc 71340
atttgtttgc atacagttga gcagggttgc tagggccact cagggaaaga gaaccctgaa 71400
cctgacatga caccaataag gtaagaattt cttaccagtc agacttctgc cccatctctc 71460
tgtgcaaact ggttgaatga atggtaaaag attataagaa gaaataggaa tgtaaatttt 71520
gtaaaccttt aacatcttta atagacttcc caaaatcaaa cttcagcttt aaaattgtca 71580
tttctgacat ctaactttgg gatgctacag agggcccctg aagcatctga aagagaggta 71640
aacaggatta tctgacatgt ttagttacat gggaagcact gtcaaaataa aaaaaaaatg 71700
tttaatcttt ttcaggttat attttagtga atattaatat atgttacaaa attgtatggg 71760
atttctaaaa ttctaaatat gtctgagtat ataatatcag ttataatgtt tattatgtta 71820
agttactgta gaccacagaa ataatcaaat ttccttgtat aaaactacta acccaaaact 71880
aaatgaatac caagaaaata catttccaga tttgcatgct aaatcagctg atactgaaat 71940
tgtttagata tacaatttga atgaactcca tgggctaagt caaattacct atgataaccc 72000
atgagttatc agtgctatgc atctaaactg gagaaacaaa tggtattcag gaggacgtaa 72060
gttcaacgtt aagcatggac tcatggagaa ccagaatggc tgcctgtcct tcctgagtcc 72120
taaaagctct tgttattaaa ggttctgcat tccatggcct gagaaggact ctgtacttct 72180

atatttgaga acttgtgagc aaactgtaac ctaacttagg aatatgcaca tgtaacaata 72240
gctgaatctt ggccaatccc agcaacccaa aatcaggcca ggagataagg tacttgttaa 72300
cacatggaag gagagatcac ctgctctgga taatagtggc catttttata ttgccccctt 72360
ccaaacagat ggaattactt cctctgtagt aattaagcag aatgtttcaa ttcatctcta 72420
taataaacat tcttgacagc atcggtatcc acccccgata ttcccattcg atcttttaac 72480
caaattcaca tcctcttgcc tagagaccat caagcttcag atgatcatgt gacaaggttt 72540
ccagcaagtt ccaagtgaag gcactacatc ctgccatcaa gaatctaccc tgtctccact 72600
agacagagta gggcaagagt tctatgattc ccaataggta gggactacac cccaagccaa 72660
cacgaagcag ttacagaaga aagaccatca gtccctctgc ctcccataaa gatttatggg 72720
gatcacatct ttcaggtggg aaatgaggta atagaatagg gcctggaggc agggaaccta 72780
agaacttcct agaactaaat caaatggaaa cacttgagct atgacaagaa atatcgtctt 72840
catttacata gggcatacac ccagtaaatg actttgtaac ttaacttcat cctcttcatt 72900
tacatagggc atacaccaag taatcaatgc gaaacctcta gaaggtattg aaaccccaga 72960
aaattctgta actggggccc ttgagccact tgcttgggcc tgctcccacc ccatggagtg 73020
tgttttcatt tgcagtaaat ctctgctttt gttgcttcat tctttccttg ccttgttttt 73080
gtgtttagtc caattctttg ttcaaaacac caagaacctg aacacccttc caccaataac 73140
aattcaaccc agcaatccaa ttactgggta tatactcaaa gaaatataaa tggttctatt 73200
acaaagagac atgcatgtgt atgttcattg cagcactatt cacaatagca aagacatgga 73260
atcaacctac atgcccatca atgatagact ggataaagaa aatgtggtac atatacatcg 73320
tggaatacta tgcagccata aaaaaagaat gagataatgt tctttgcagc aacatggatg 73380
gagctggcag ctattatcct tagcaaacta atgcaggaac agaaaaccaa ataccgtatg 73440
ttctcgcttg taaatgggaa ctagatgatg agaacacatg gacacataga ggggaacaat 73500
gcacactgtg acctttcaga ggatggaggg taagaagagg gagaggacta ggaaaaataa 73560
ctaatggata ctaggcttaa tacctgggtg atgaaataat ctgtacaaca acccccatga 73620
cacaagtttg cctgtgtcac aaacctgcac atcctgcaca cgttcccctg aacgtaaaat 73680
aaatgttaaa aaataaataa ataaaatggt ggggaaaaaa aggtaatttc ctatggttca 73740
acctaataat tctactacaa aaagtaatct gttagctttg gagtcctctc ccttgctagg 73800
ggtttctagg gaagaacaca cacgacctgt cctcacaggg gtcccaccca caggccccaa 73860
cacttgctaa aatctgtgtc ccttagagaa agcgttggca ggcattgggg aaataactgt 73920
ttctggggca tcagcccctt taaacctgct gaccaagtcc tccgctctca ttctgcagta 73980
ggaaagttta caaagataca gcagaaagaa caaagaaaac caaaaaagga gaaggtacta 74040
attgttgagc atttactatg tgccaggccc ttagactaag tgttgcatat atatatgcct 74100
attgatcctc gaagacaatc ttgaaaagtg agcacttccc atttccagaa gaaggaaaca 74160

ggcttacaga catgacgtgt cgtgctcacg gtcactgagc agtgaaagcc agtgtcccct 74220
tatctgtgac attcacacag ctggttattt gggagtattt gatggttttg tgagtatttg 74280
atggttttgt gaagcctgag ctgactcctc ttgtacccat ctgcttgact ggtacaatca 74340
tctccctcac acagatgcag aggtgagccc cgaagacatt gagccttgcc tctcccatca 74400
cacgacaaat gaatggcagg aggattgctt caacagggca tgccagaatc atcccactca 74460
gcctggtagc ttcattggca cggttgagct ggcaacagga cactcatgca gggcaactga 74520
ataatcagaa gcagtgaagc ctgaactcta accactatca ttttaaagat tattatgtgc 74580
agttatttca gctaattaaa ccttgaatgc ctgccagctc agcttccagc tcagagagga 74640
aatgcaggca tgcatggcct cagcttccct tccagcctct gtctgccttt cagggggagc 74700
agagctcagg gcctgggaat ggagggagca aggagagcag cagagcaagg gaggggccct 74760
caaccactgt ggccacttcc ccatgggaac gcagaagttc tgatccttgc cacgggaaaa 74820
atcccctgac ttttgtagcc tctcaggtat gggaagttac agcctgttct ttactggttc 74880
atcaaggaag agtcgggtct ggcttgaagc ctggaatcaa tgccaagagc aaagttgagg 74940
tcccagcttt gtggctggta gctgtgagga cctggacaat cttctgtttt ctcacctgca 75000
tgctctctga aatttagctt ttgcacaaaa atgtgtagac agggtaccta gtgctgtgcc 75060
tatccaatac agtagctaca agccacatgg agcagtttaa atttcaattt caatcaatta 75120
aaattaaatt agattaaaaa ttccatttct ctgtcacact aggcaatttc aagtgctcag 75180
caagcctact gtattggaca gggcagctac agagcatctc catcatcaca ggatgttcca 75240
agggacagca ctggccctgt gattgagggt gtgaaccctg attcgactca aggctgcaat 75300
cccagctcca ccgcttactg gtgaccctgg gcaagttact gaacctcctc atcctcagtt 75360
tcctcatcta taaaatggga aataacagga tccagcccag agtttttgta aagattaaat 75420
gagaaatgtg tgtaagatac ttagaagaat gccaataatg gtaagcacac tataaatatt 75480
agctcttagt aacgtatttt gaggagtatg tataaattca ttctattaca gggctttaca 75540
gttctaggaa gcagtttcaa aatcactatc tcatttgaat ttcacaaggg ttccatgggg 75600
caggcggtgg cacaaggagt cggctgaacc cttttattaa tacaagaagg caaactaaaa 75660
tccataggtt acctgactgc ctgaaagggt ggcagaacta agaactagga cttgaactaa 75720
gggaataatt caagaactaa catctgtaga ctcttttaca cttcatgtcc tatttcagat 75780
ttagcataat ctaaggaggc caggaatgtg cctcccatgt taagagtgag gttctgcgat 75840
gggagtaatc ccaacgctca ctagccctta catagcacag cccttggtgc tcttgcactt 75900
ggcctccctc cctccccatg ttccttgtcc attgctacct tgggcttcct tacagcatgg 75960
gggacccagg gtaagtagag ttttacatgg tggctggctt ccaagaaaga ggtggtgaag 76020

ctgctagtcc tctttggtct taactgtatg ccaggcactg tcctgtaatc tccacaactc 76080
agtagggtag gtacacatat gattcccatt ttagtgatga tgaaggtaaa gcacacagag 76140
ggtaagcaac tttcccaaac tcagccagga ggtggtggag tcaggacatc agagcccagg 76200

aagtctgatt ccagcctggc ttcagagatg tcaccattca cctgcccaca tcccaggaca 76260
ggtgcaggaa gcaggaggac agagctcttt gttcccaggg cctatttctc tccttctctc 76320
acctgtgtcc aaggacagga gagtacagcc cctctcccag cctcagggga ttgctggggg 76380
acacaggtgc ctttctgtgc ctcttgctat caccctgtat tcctttgtta gtgctgccat 76440
aaaaaagcaa cacagaacag agggcttaaa caacgtaaat ttattttctc acagttctgg 76500
aggccagaag tcccagatca aggtgtcagc aaagttggtt ctttctgagg gccatgaggg 76560
aaagatctgt cccaggcccc ttttgttggc ttgtagatgg cctcagtctt cccatgtctt 76620
cacatcctct tccctctgta caggtctata tccaaatctc atcttaggag gaccccaata 76680
gtattggatc agggcccacc ctaatgatat cattttaatt caatgacctc tttaaagact 76740
ctatctctaa atacagtctc agtgtgagat actgggagtt agaacctcaa tatgtgaatt 76800
gggggacata gggaacagtt catcccataa caccctctgt ctgccttcat ccttcttgct 76860
caagtcaatt cttggccacc tggagatgtc agtgggagca aggttctggg ggcagaactg 76920
tgacgagctg atgagtggaa aggagtgttg atgaaagtgg aaactctgga gaccaaatga 76980
cagcctaaac cactgtccac tgaaatgatg gggatgggcc tgtggggaag ggaagcatta 77040
ttaatcattg caattagcct gagcaacata gcaagatcct gtctctacaa aaataaaaat 77100
aaaaattgcc gtgtgtggtg gcgtgtgcct gtagtcctag ctactcagaa ggcggaggca 77160
ggaggatacc ttgagcccaa gaatttgagt ttacagagag ctatgattgt gccactgcac 77220
tccagcctgg gcaacagagc tagaccctgt ctctaaagat ttttttttta attcactgaa 77280
atttactgac taccttctca ttgcatcccc ataacaggcc tgtgataaca aattggtgtt 77340
attatcccat ttgtagttaa gaaaaatgat actcagagtg atttttaaaa catctgggtt 77400
aaggaagcta tggagctaag gatcatgggt cctaacttct caggataaga gccagatcag 77460
gctgggcaca gtgactcatg cctgtaatcc cagcactttg ggaggcaaag gtggatagat 77520
cacttgaggt caggagtttg agaccagcct agccaacatg gtgaaaccct gtctctgcta 77580
aaaatacaaa aaattagctg ggcgtggtgg tgcacacctg taatcccagc tactaaggag 77640
tctgaggcag gagaattgct tgaacccaag agtcagaggt tgcagtgagc ccagatagtg 77700
ccactgcact ccagcctgga taacagagca aggctccatc tcaaaaaaaa aaaaagccag 77760
ataattcatt ctatttgttt ggtgattatg taaaattaat aacgaagctt tactctgagt 77820
atgaaaataa acaaaaacta ttgctatacc caaaaaagtc tctgacacac acacacacac 77880
acatacacac acacacacaa aagggtattt cattcatgct gaacctatgc aaagtgaaat 77940
gaagtaactc atcaaaagtc accacctagc atatggcaga gctggtgctg attcatccat 78000
gttttttcaa cattagcatg cagaaggggt aactggaaaa agatcccact accccagaga 78060
tcttagagca gagttctgga atgagtagcc tcagcagtca cactgcttgg tgggtgtaga 78120
gatggagttt gaaaggttct gggtgtggtc agcagtatat acaaatggtg ccatagattc 78180
cttccctctc tgctcttgct cacaactcct cacatcaaga agttaggtct ttttttcctc 78240

cccttcaact tggggtggcc ttttgatttg ctctagtgac tgaaatatga cagaagtgac 78300
actgtgacca ttctgaaccc agaccaaaga ggactagcag ctctgctacc tctttcttga 78360
aagctagcca ccatgtaaaa gtctagttac cttgggtcca ccatgctgca aggaagccca 78420
aggtagctat ggacagggac atacggagag agagggaagc caagtggaag agtaccaagg 78480
taccagatac atgcatgaag ccttcttagg ctttccatcc caggcccagc ttccagataa 78540
atgcaactga gtgagtgact agagcagaag aaccacccag ctgaaccctg cccaaattcc 78600
tgacccaaag aataatgaga aaccataagt cattgttgtt ttaagccatt aagtctaggg 78660
atagcttatt ttgcatcaag agttagttga aatactattc ggagggagag gagctatgga 78720
ggtatgttag aaaaaacata cgtcatcaac aggcagggag tagggagggc aggtcaagac 78780
aagaaccagc aagagaatat catgagcctt agctgggcaa acaaggtatt ctacagtctg 78840
gccccatgac gatccctggc acttcaagca cacatagcta tggggtgatc tctaagcctc 78900
acacagcttt gcctttgcac atgccatttc ttccttctgg tgtgccattt gctcacttat 78960
ccttccagat ccagtatcaa agacagacca aaaacccata agaaggcaat gggttttaag 79020
gcccttagag tctgactctt gtctatttct ccagcttcat ctcttaccct ctcctctctc 79080
tgtgctccaa acacatgggt cttattataa tagttatttg caccttttgc tccttccctt 79140
ggaccttttg ctccttccct ctacagaacc tttggaggtg ctatttcctc tgctaaggat 79200
gcttccacac atccacactc attccacttg gcctagttaa ttcctgcttc tccttcaact 79260
atcagctgaa gaatttcatc ttcagggaag cctcccatag atcccaaagt caagtctggt 79320
tcccttgtta tttattctca tagcaccata ttcctgccct gcccccaggg ttattctgag 79380
aaatctgatg ctcctgttct catagactgt cagttccttt aggtcaggga ccatatctgt 79440
ctgtgatcac tactatatca ccagtgccta gaacagtttg tggcacatag tagatgctca 79500
gtaaatacct attgaatgaa tgaataaata ttatctcctc taaatagctt taattgacac 79560
tcctctccct ctgagattca ggctttcttt ctcttcttag ttcccaggac acttagtttt 79620
tatctcttat catcttatgc tgaactgttc tggttatgtt tgtcacccct tctaaactgt 79680
gactgtgata aggcccaggt cctgtttgtt tttctatccc aggagcacag ctccgaaact 79740
ggtgcagact aatttgtcca ttgtaaaatg tggaagcttg gaaagataga atgtggatat 79800
gcatgctagt ggtgagattc cccatctcat catattcaga aagccaaagg tgttctttag 79860

aaaaccatgc cagtgtacca gtcagaaatc tcaaataact gaaaaagtca gctgccttca 79920
ggtatggtgg gatccagggt ctcacatgca ggatgtggcc tctgtctttc tctcaacact 79980
atgctttgct gtgttagcct caatctcagg caggctttat ctatacttac taaattccaa 80040
aagctctagg tcatatcctt ctaactatga gtctattaag agagattcta ttccccaata 80100
gttcaaataa aagttcacca atagatgcca aaattattat agtaaaaacg aaaaaaaaaa 80160
aaaaggctta ccaatagaat ctcatgggac cagtttggtc acatgcccac ccctgggcca 80220

atcactgtgg ccttcaagaa tcaacactgt aatttatcaa acatacatca cttgcccatt 80280
cctggagctg gagatagaat cagtctcact gaacctatgg gtttaaagtg ggctcagcaa 80340
ccaggagaca gggcaatgga aacagagcac ataaagtcca ccacattcac tctgcccaga 80400
ctttacagct ctgttcactg gaggtccaaa cacaccgaag agtctgcaag ggatgttgct 80460
gcagaggcca tgacaggctg cgcatggtga catttcccac tcactgtggc tctaagattt 80520
cacttgagcg gagcatagcc tggctttcca tcatcctgct ctccttccac cagcccagaa 80580
gcctggtgga gtgaaagcca gagactccat gactaaggtg ccctgggttg gagattcccc 80640
tcacccactc ctcctccaga gagaacacac accaagaaaa agcacttagc cttgatgctg 80700
gtttcctgtc actgcccttc ctattgagac gtcattggcc ttggctcccc tcctgacttt 80760
tctatttttt tctacctcct gttaaagagg caacaggtcc gtaaaagatt tatcccataa 80820
cctagtgttt caggctctat ctccctgttg gttagactcc ctgacagatt aataaattaa 80880
caatttatta attgttttgt ttgttggatg gatggatgga tagatggatg gatatacgga 80940
cagatggaca gatggacgga tgtatggatg gatggatgga tagatgaatg gatagacaga 81000
tggaggagtg caatgaaagg tggatgaatt ggttctgagt gccctcagaa cactctatct 81060
cctcttctca tatttaccac cctaggctcc agattactgt aaatatagaa agcccttttt 81120
cttgtcctga aatcagtttc ccagccctct gttattataa caatgactga catttaacat 81180
ggttaattct tatccaggca cttaccctga gctaagtact cattcaatct ccacaattcc 81240
attgtctgga tgctgttatt gtctacagtt tacagtgaag gaaaccaagg ctcagagaca 81300
ggaacttgtc caaggcacat agccagtcaa tggcagagcc acaactcaaa gtgtgtgacc 81360
ttagccacta tcttctaggg ccctatgaac acgaacctca gccctgtcta caccaatttt 81420
ctccttctcc cttgaacatg tcatatcttt attcatggaa taaggtcttc ctcctcacct 81480
ctttgtagac tcatatcaag gaatacattc atcaaagtaa cactaagtta cactctgaac 81540
aaataaaccc tcaaatttca gtagcttatc acaaccaaga tttatgtcct actcatgtta 81600
tggactaatg tggggcaagt ggccctcttc catctgatag caggcctcta acaggggaag 81660
ggagaagtgg aagaggagca ctggctcttt gctgcctcag cctaaaggga acacatgtca 81720
ctcctgctca catttcactc acccaactaa ttagtgtgtg gacatttggc gaacatatcc 81780
catctctgcc acaaagaacc atacactcct gccaggggtc atgcagaaga taaaggctca 81840
gccagcccag tcagtacaaa ttcggagggt gggggaggaa aaaagagcca agattcaagc 81900
tggtggctgg ggagtggaga gagatggata ggattccaga attaaccagg tacaaagcac 81960
ccaacacttg ggtggtaccc catgtctatt gtcttgaata ttgctcacaa gtaagccaac 82020
cctaagcttc ttggctaaga ctcagaactc ttgttatcta agtttatggc tgttaattac 82080
aggatttgac ctgaattaac tgaacaatgc agtaagaata acaatccctg acttttcaaa 82140
ttttagattt tccaaatcat acagcaatca tctcatttga ccctcacaat aaccctatga 82200
cgtgggtatg ctatctctac ctctattgtg caataaggaa atttagagaa ggatgatgta 82260

attgaccaaa gtcgctcaaa acctgtgcgt gctgcaatac atctcagtgc cagttttcta 82320
acacatgcta ggggctcttt cttctacagt tcagctgtgt ctcatgcaaa tttctatgga 82380
aatcgacaat gtgaaaatga acatccatag gatttgaatt caaaagcttg ggtgctctgt 82440
gcaccaagag ggagttgttt aagcatacag gtgttgaagt cagatggcct gggttttaat 82500
cccagttctg ccacctgcta gctgggtgat agctggcaag ttaatctacc tgcgctcact 82560
ttccttattt ggaaaataac gatccactgt acctaaccca taagattatg gtgaggatta 82620
atgagacaag gaatataaaa ttcttagcac aatgcctggc tcaacattag aacttaataa 82680
acactgaatg atgctgccaa gatctttctg ggagacaaaa agatgccact attccttcct 82740
tcctccctca gggcatgatc gtgtctcact acatgacact cttggcttca gctggagaac 82800
aaacggacca cctaacccgg cttcacgtgc aactgccagc gagaagagaa tgttcttttc 82860
tttgtctgag attcggcact ggtccctgcc gccaatccca ggtcttcgtc tcagatttgc 82920
tgccacccag aggccatttc atgccatgcc aggatgactc tccaaagtat tccttgggac 82980
agtggttttc aaaagaaagt gggacgtggg ggcagggaag gaaaaggaaa acatatccaa 83040
ctcttggagg caggcgagac tgtgggggtt gaaacaatcc cgcaggggat cctggcgtgc 83100
cctccactct attcacttga ggttgtctga tttcggtgaa gccaaaaaga gagaaaaggg 83160
gaaagggagt caaaaggatc ctgtctgtct tcaccagcac taggaataca tgtcagctgg 83220
tgctttcaag ggtgtcagc tccacctcgt ggaatgagag ccagtgattt gtttctgatg 83280
caattcttca gccagtaaac acaggaacaa ggaagttatg acatgacctt tctaaagatc 83340
cttaaaaata ccccagccaa agggactagt atggagaata tgtaaagaat ccttgcaacc 83400
tggcagagca ctgtggctca tgcctgtaat ctcagtactt tgggaggaca aggtaggagg 83460
atcacttgag cccaggaatt caagacccgc ctgggcaaca tagtgagacc tcatctctac 83520
aaaaaaaaaa aaagaacttt tacaacctaa tcatcaaaag acaaatcagc aaaaggtttt 83580
gtattagtcc attctcacat tgctataaag aaatacctga gacaggataa tttataaaga 83640
aaagaggctt aattggctca tggttctccc ggctacacag gaagcatagc agcttctggg 83700
gaagcctcag ggagctttca atgatggcag aaggcaaagg gggagcaaac aactcatatg 83760
gccagagcag gaggaagaga gagagtgggg aggtgctaca cacatttaaa caagatctcg 83820
cgataactca ctcactatca ggagaacagc accgagggag tggtgctaac ccattcatga 83880
gaatttggcc ccgtgattca attacctccc accaggcccc acctccaaca ctggggatta 83940
caattctata tgacgtttgg tgggagcaca gatccaaacc atatcaggtc cgaatagaca 84000
ttttttaaaa accaataagc acatggaaag gtgatcaaca tgatcagcca tcagggacat 84060
gcaaagcaaa atgataaaga cgtgacctca tacgcactag gtgagctaaa atcaaaaaga 84120
caggtgttgg tgaggatgtg aagaaaccag aaccctcaca cacttccagg gggtggtggg 84180
gagaatggaa aatggatgca acttcttgaa aaacagcctg gaggcttccc aaaagttata 84240
gagttacagt atgactcaac aattccactc ctagttacat atatccaaga gatgaaaaca 84300

tatatccaca caaaaacatg cacaccagtt cccataaaag catcattcgt aacagccaag 84360
aagtgaaaac aacccaaata tgcaccaaat aagatgtcta tagtcggcag ggcaaggtgg 84420
ctcacgcctg taattccagc actttgagag gcggaggcgg gctggtcatt tgaggtcagg 84480
agttcaagac cagcctggcc aacatggtga aactcatctc tactaaaaat acaaaaaaat 84540
taccaggcat ggtggcacaa gcctgtaatc ccagctatca ggaggctgag gcaggagaat 84600
caattgaacc tgggaggcag atgttgcagt gagctgagat tgcaccactg cactccagcc 84660
tgggcaacag aaggagactc tgtctcaaaa aaaaaaaaaa aaaaaaaaaa agatgtctat 84720
agtcatacaa tggaatgtta tttggcaata aaaaggaatg aagaaccaac acatgctata 84780
acatggagga accttgaaaa cattatgcta agtgaaagaa gcagtcacaa agaaccacat 84840
atttcataat tctgcttata tgaaatgaaa tgaaatgtcc agaataggca agtctataga 84900
gacagaaagt agatttgcag ttccctaggg ctggtgcagg tgtgaggctg gggtgggggg 84960
cagacaatta tggctaaggg gtacaaggtg tctttatggg ctgataaaaa tgttcttaag 85020
ttgcttgtga tgatggttgc gcaactctga atatactaaa tgccattgga ttgtacactt 85080
taaatgggtg acttatatgg caggtaaatt atttatcaat aaatctgtta aaaaagtaaa 85140
ataaaccaag ctcttctttg tacatctgct tgaggcaagg gctgtttcca agcccctgta 85200
ttcccaacag tatgaatcac agaaggaaaa caaaatcagg acctctggag gctggaggca 85260
ggagagttgt gttgtactgc gaccctcagt gcccagaggg ctcttcagtc ctggcagcag 85320
ctcattgcta gtggccagca tggccaggcc cattggagct ggaggataca ctgtaaccgg 85380
ctgacaccat gtacaaagga gagtaacaca gctctactac caccaggtat ggctgtaagg 85440
ccgggtgtgg cataaactct gccaggtcac acatgggctc cagacccaca gacacctgct 85500
ctaaagacag agacagaaca gaagcaacat gagctttggc aacagacaga cccaaattgg 85560
caccacgtcc cttctatcta tcagttctat gacctcaggc ctatactcat cttttaaaaa 85620
attcaataat aatgcctatc atgtaagctg ccctcactct tcccttcccc agtttgtttt 85680
cttttgttgg gctaacattt attgaaggct tactaggtac taagcaaatg ctgtacaggc 85740
attaatatct ctgttagtct tcactgcaat cctggacaaa tccagaatga tttcttatta 85800
tgccattttt agatatgaag gaacagaggt tcaaagaagt cagacaactt ctccattgtc 85860
acaaagctaa caagtggcaa agccactatg cccaagtcat cattttaagt atcatactat 85920
actctatcca tatagcaaga gtgacatttc ataagtagat aaagagcttg acacagagtt 85980
gggccctcag aaaagagtcc tgagagccag gtgtatcagt ctgtccccac actgctataa 86040
agaaataccc gagactgggt aatttataaa ggaaagaggt ttaattgact cacaattctg 86100
catgactggg gagacctcag gaaacttaca atcgtggtag aaggtgaagg gaaagcaagg 86160
accttcttca catgcaggca ggagacagtg tgagcaaagg gggaagaacc ccttataaaa 86220
ctatcacatc tcgtgagaac tcactatcac tagaatagta tgggtgaaac cacccccgtg 86280
atccaatcac ctcccaccag gtctctccct agacacatgg ggattacaat tcaagatgag 86340

atttgggtgg aggcacagcc aaaccatatc accagggtac caaaatcatt cccccgagtg 86400
gtgtccctat ccagaggcca cagacaggac acctgccatg tgaaggtgac atggcttgaa 86460
tgtttgttcc ctccaaatct catgttgaaa tgtgacctcc attctgcagt tatacaagca 86520
gtaagaataa aacaaaatga aacaaacaaa aaagaaatgt gacctccaat attggagata 86580
gagcctaaca ggaggcattg caacatgggg atgaatccct catgaatggc ttagtgccat 86640
ccccttggtg atgagtgaat tctctcttgg ttagttcatg tgagatctta tcagggaacg 86700
tgccctgata gtcacgtagg ttcttttcta ttttgcctaa gcgtcagccg gtttgagaaa 86760
taaagggaca gagtacaaaa gagagaaatt ttaaagctgg gtgtccgggg gagacatcac 86820
atgtcagtag gttccatgat gccccacaag ccgcaaaacc agcaagtttt tattagggac 86880
tttcaaaagg ggagggagtg tacgaatagg gtgtgggtca caaagatcac gtacttcaca 86940
aggtaataga atatcacaag gcaaatggag gcagggcgag atcacaggac cacaggatgg 87000
ggcgaaatta aaattgctaa tgaagtttcg ggcaccattg tcattgataa catcttatca 87060
ggagacaggg tttgagagca accggtctga tcaaaattta ttaggtggga atttcctctt 87120
cctaataagc ctgggagcgc tatgggagac tggggtttat ttcatcccta cagtcttgac 87180
catagaagat ggccaaaccc aaggggtcca tttcagagac ccagcctcag gcacatattc 87240
tctttcccag ggatgttcct tgctgagaaa aagaattcag caatatttct cccatttgct 87300
tttgaaagaa gagaaatatg gctctgttct gcccggctca ccagcaatca gagtttaagg 87360
ttatctctct tgttccctaa acattgctgt tatcttgttc ttttttcaag gtgcccagat 87420
ttcatattgt ttaaacacac atgctctaca acttgtgcag ttaacacaat tatcacaggg 87480
tcctgaggcg acatatatcc tcctcggctt acgagatgac aggattaaga gactaaagta 87540

aagacaggca taggaaatca caagggtatt gattggggaa gtgataagtg tccatgaaat 87600
cttcacaatt tagagactgc agtaaagaca ggcataagaa attataaaag tattaatttg 87660
gggaactaat aaatgtccat gaaatcttca caatccacgt tcttctgcca gtccctccat 87720
ttggggtccc tgacttcctg caacaagatc tggttgttta agagtctgag acctccctct 87780
tctctcttac actctcacca tgtgacacac ctgcgccccc tttgccttcc tccatgattg 87840
taagcttcct gaggccctca ccaggagcag agccagtgcc atgcttcctg tacagcctgc 87900
agaactgtga gccaactaaa cctcttgtct ttataaatta cccagtctca aggaatactt 87960
attctttgat agcaacgcaa aaacagacta atacaaaaaa attggtacca aggagtgggg 88020
cattgctata aagatacctg aagatgtgga agtagatttt gaactgggta acagttagag 88080
gttagaagag tttggagagt tcagaagaag acaggaagat gagggaaagt ttgaaacttc 88140
ttagaaactg gttaaagggt tgtgaccaga atgctgatag aaatatggac aatgaagtcc 88200
aggctgatga ggtctcagat aaaaatgagc aacttaatgg agaatgcagg tcaacaaagg 88260
tcacccatgt tacaccttag caaagagctt ggctgctttg tgttcaggcc ctagggatct 88320

gtgggagttt gaacttcaga gtgatgactt agggtatctg gcagaagaaa tttctaagca 88380
gcaaagcatt caagatgtgg tatgtctgct tctaataacc tacaatcaaa tacagagaca 88440
aagaaatgac ttatgtttgg aacttgtatt taaaagggaa gcagagcata aaaatttgga 88500
aaatttgcag cctgcccatg tggtaaagaa agaaaaagca ttttcagagg aggaatacaa 88560
gcaggccgca aagcaaccac ttgctagaga gattagtgtg actaaaaaag gatccaagtg 88620
ctaataagca agacaatggg aaaaagggcc ttgaaggcat tttagaaatc ttctaggtag 88680
cccctcccat cacaggccca gaaacctagg aggaaagaac agtttcagga gccactccca 88740
gggcacctgc tgccctgcac agcctcagga cactgtttcc tgattcaggc ctactctggc 88800
tacggccttg gctcaaaggg gcccaggtac agctcaatct gccactctag agggctcatg 88860
ctgtaagcct tagtggcttc cacataatgt taagcctgta ggtgtgcaga atgcaagagt 88920
gaaggaggct tggtagctcc acctatattt cagagaatgc atggaaaggc ttaggagccc 88980
aggcagaagc ctcctgcagg ggtggacccc accacagaga tgttctacta gggcagtgca 89040
aagggaaagt gcggggttgg agcctccaca cgaagtcccc actagagcac tacccagtgg 89100
agctgtaaga aaggggccac cactcttcag accccacaat ggtacagcca acagcagctt 89160
gcatcctgag cctggaaaag cctcaggcac tcaactccag tccctgagag tggccatggg 89220
ggttgtaccc tgcaaaacca caagggcaga ctttccaaag gccttagagt cccacccttc 89280
cataagtgtg ctctggatgt gggacatggt atcaaaggag actattttgg agctttaaga 89340
tttaataact gatctgctgg gtttcagatt tgtatggggc ctactgcccc tttcttctgg 89400
ctgatttttc ccttttggaa tgagaatatt tacccaatgc tggtaccacc attgtatctt 89460
gaaagtaaat aacttctttt gattttacag gctgataggt agaaggagtg agtctcagat 89520
aaaacttaaa actttggact tgatgctgga acgagttaac actttggaag actgttgcga 89580
aggaatgatt gtatcttgca atgtgagaag aacatgagat ttagagggcc aggggcggaa 89640
tgatatggtt tggatgtttg tcccttccaa atctcatgtt gaaatgtgac ctccaatgtt 89700
ggagatggtg cctggtggga ggcaatttga ttatgggggc gaattcctca tgaatgactc 89760
agcattatcc ccttggtaat gagtgagttc tctctccacc agttcatgcg agatctgatt 89820
tttttttttt ttttttttga gacagagtct tgctctgtca cccagactgg agtgcaatgg 89880
tgcaatctca gctcactgca acctccgcct accagattca agcaattctc tgcctcagcc 89940
tcccgagtag ctgggattac aggtgcccac catcacaccc agctaatttt tgtattttta 90000
gtacagacag gatttcacca tcttggccag gctggtcttg aactcctgac ctcatgatcc 90060
acgcacctcg gcctcccaaa gtgctgggat tacaggggtg agccaccatg ccctgccaga 90120
tctgattgtt tattgtttaa aagagtctac gacttccctc ttctctcgtt cttgctcttg 90180
cttttgctct gtgatgcacc tgctccccct ttgctttcct ccatgattgt aagcttcctg 90240
aggccctcac caggagcaga tgccagtgcc atacttcctc tgcagcctac agaaccatga 90300
gccaattaaa cctcttttct tcataaatta cctagcttca ggtatttctt tttagtgatg 90360

aaggaacaga ctatatggaa gggaaggata atgcctttgg taggcacatc tcttgaggac 90420
tgtgtgcttg ggactgtgtt aagaaattta cttgcacatg ccagaaacta tctgtcctgc 90480
ccaaatattc catgtgccca cctcttcagg agactctgat ggaaaggcaa cagtctacag 90540
gaaaaatcac acccttacta gaagatgcct tttgctttag agggaaatgg ggcagacaat 90600
ttacaactgg cctaaagtga ataagagaag ctctgaagac aggaccagag gatgaggcca 90660
aggcagggct gtacatcagc cagtagactt actgtggaac agccacccag tgagaaggct 90720
aaacttaccc ttcccaagtc cagccacttc tcaccctgtg ttccagttac tactgtgaca 90780
taacaaatta ccccacaatt tagcatctta aaataaccat ttttattatg cacctttatt 90840
ctgtgggtca ggaacttggg cagggcatgg ggtgaagaag atgaccagag ggatgacttg 90900
tatctgttcc ataatctggg gcctcaacca gaaagacccg aaggcaagga atgatttgat 90960
ggctagggtt gtagttatct ggggccattt tgctcatatg atgggagact caaaggctgg 91020
gattaacaac agagcaccta catgtggtct ctccctgcag cttggcttct tcaatgcatg 91080
atggccttag agtactgaga cttcctactt agtggcccag tgctccaagc accagtattc 91140
cagctactaa ggcagaggaa tgtcaaggaa ttaaggaatt tggaggccat gttttaaagc 91200
cttcacaccc tgccttccag gaaggagcca agctcaaggg catacgcaaa agagactgca 91260
gaggatcttc ccctatccgg ggacctattt cctgcagaat ccaatggcag aatctcccag 91320
gcataatgat aagcaaaaga agacaggtgc aaaagagtgc taactgtgtg attccattta 91380
tgcagcattc aaggaaagac aaaactaagt tcagaatagc agttacctgc aggtggggtg 91440
caaaggggca caagggaggg agctttctag gcttctggca tgttctttat attaacacat 91500
ttgttacaca agtgtattca tatgtaaaaa attaatcaac tcctacactt aaaagtcatg 91560
cactggtgac actatggatt ttctacttca ataaaaattt tgtaaaggaa ttatcctcaa 91620
gaggagagga gagggggaac aaaagaatta tcctatccag tcagagcacc tcactctcca 91680
tcccttcccc tagccagatt tacatgccct agaaaagcag actcccaggc ctccccgctc 91740
tggctagtca atggcttgtg gatgcagcat tttaggggag gtgtttccag cccctcctct 91800
gcccttctgc ccagacttcc ttctccgttt tagaatccaa aagtgctgga aggtgccagg 91860
actatcatgg tttcatttca tacccttatt ttatggagga ggaaagcagg aaggggagtg 91920
gtggagggac tggaatgtgc agccagggag gcaggtaagg cccggactcc tctgacggcc 91980
aaggcagaac tacagtctta gggtctttct cttcctctca gtgaggcaga gccctccatt 92040
gcctgtaaga ctgcttagca tgggctctgt gcatccatgt ctatccaaat gggagtttct 92100
ggaaggcagg aaggtttctc acttgataag attccgggac ccacctagct gggtggggat 92160
tttccacagg gcctaggcag agggataatg ctagccactc cttggggcat ggctctgatc 92220
acagaccctg ggggctgtgg gcctgagaga tttcacccct cccagaatta ccagaaacgg 92280
ggtgggaagc tgagatgctt ttgctttcca atgaaactcc caaggctcaa accagaagcc 92340
agcatcttaa gtcctaaaat gtgaagcccc aacaatcaag ccttttcctg atctggcttt 92400

gcccatcccc ctaccacccc ctgtcttttg cagtcagctt ctattatgga ctgaatggtg 92460
ttcccccaaa attcatatgt tgaagtcctg accccccagt gcctcagaat gtgactatat 92520
ttggagactg ggtcattaaa gaggttaagt taaaatgagt cccttagaat ggtccctaat 92580
ccagtctgac tggtgtcctt ataagaagag gaaatttgga cacacggaca caccagggat 92640
gttcagagga ggcagggcaa gaagtcagcc atctgcaagc caaggaaaga gacctcagag 92700
gaaaccagac ctgctgatac cttgctcttg gacgtccagc ctccagaacc ataagaaaat 92760
aaatttctgt tgtttgtgtc tgtcgtattt tgttacagca gccctagcaa actatataac 92820
tcccacttcc taagtgtcac acatttttac acctgcctgg ggtgcctttc cctccctcct 92880
cccttgaccc tctgaaaaac accaccttcc agtccttccc cgccaattgc tccatgacat 92940
gtctcttcaa gccctgagtc agtgaaccgt gctacatggt cttgcattgt acacttgtaa 93000
ccattatcat ccctatgaca ccatatttta gtttatcttc ttgacagacc ttgaccctgg 93060
agacacaggc tattccttac tcatctccgc atccccagcc tgaataacaa atggtgtgat 93120
aaaatgtctg ctggattgaa ctgaactgga gaatttgctg agtgcttcag catgccctgg 93180
cagggtctct gcccctcaga acccacactt ccccttcact gtatccacct tgcaccctgc 93240
acaaatgcca aaaaggcttt aaggtcaaaa gaattagcac tgtcttagaa attgtataga 93300
ggtggtccag atggtcccca acttacagtg gttcagcgta caattttctg actttaagat 93360
ggtgtaaaag caatacacat tcattagaaa ccattcttca aattttgaat tttgatcttt 93420
gcccaggcta gcaataagtg gaaatatgct ctcctgtgat gctgggcagc aggaatgagc 93480
tgcagctccc actgagccac actatcacga gagcaaacaa ctggtactcg acagtatact 93540
gtgttgccag atgattttgc ccaactgtag gctaatgcca gtgtctgagc atgtttaaag 93600
taggctaggc taagctatga tgttcagtaa gttaggtgta gtaaatgcat tttcaactta 93660
caatggattt attgggcact aaccccacat cataagtcaa gaagcaactg tattataaaa 93720
ctaagactgg gggagggcaa tgtttaacct ggagacatgg attactcatg ataacaatgt 93780
aggatgccag ggactgaaca taacaaactc aaggggaggg gaggtccctt ctcagctatc 93840
cagcacccca aagcttgaat caatagctcc ttcccacgct gggtgaaatc agccctgagc 93900
tgtctgtgaa tcagaggaag tgtttgtgtg tgtgtgtgtg tgtgttggag agggtccggg 93960
ggaaggatgt atatgtgcat cctgaggtgg aaaaatccct aaactcactt gtgtggtgaa 94020
gcagggctgg aggcttctag agccctaggg agggcgcagc ctttgacttt cggacagacc 94080
tggttagaag cctcactgct ttgctgccag ctgataaagg agccagatga aagggcccag 94140
catagctcct ggcctttgga gcatgccctt tatccataga atgctactct tctcctgatg 94200
tccatcttcc cctagcgctg agcccaaagc acagaaggca tcctgtttgg agccaggctg 94260
gctggggtta acaagagaaa ggcagctgtt tcccgaaaac aaagggctgg gtcaataaat 94320
ctgccgcagc agccgtggat cagtgagggc aaaggctccc gcggggagca gccagccagt 94380
ttctctgaaa cgtctagaac agagccatcc aggaaagcaa ggctgaggct tgaaaggccc 94440

ttaggtaggc ctgtcctggg gtcaatatcc tcagagcaca gggtccctct cctcaccccc 94500
agcaccttcc aggatcagac tcagagtctc acagaatcac agagctggaa aggaccccaa 94560
gcattctcca atgcagtttt ttatctgggg aacatgttaa gaattcatcc atctatgaac 94620
ttggataggt cagggggaaa aggttcacta atctataact gaagtttagc atttatttct 94680
atcatgaacc taagcaacaa actacagtag aatttggaga acctaagact ttgtcaccaa 94740
gagacatgac aggcattttt atagctcagt acaggcattc cagaattctc aaaacattgt 94800
tcattaatac tacctcaaaa ttgtagtaat gatcagggcc acctctagat ctcgcttaat 94860
gcattaataa ataagcattg ttactgctat atcacaaatt tgttttttta ttttgacaac 94920
tgtattttgg tatcgttagt taactttgtt attccatgta ttttatatta ccagccttca 94980
aaagtgtcca tgacacagaa aggattaaga attcctactg ggctttaacc ttcactatag 95040
atggagaaac caaggtccaa gacaggcagt gcttcttctg gatggatgga tggatggatg 95100
gacgtatgga tggatggatg gatgaaggga tggatggata gtgagtgcac agatggtgaa 95160
tggattaatg aatagaaatg caaatgaaaa aatagcacag taatagcaat aataaccctc 95220

atggactgct taccctgagc caggcattat tttaaactat tcaattctca caacagccct 95280
atgagacaga taatattatt ttctccgttg aacagataag aaaactgaga tataaagtta 95340
tgaagtgact tagacaaggc tacagagcca gtaagaggaa gagttatgta tgactcaaac 95400
ccccaagcct gcactctgaa cctctcttct ctatgactgc tctgaagcag tctggtgaag 95460
cttggtttag tgctgagttc tgggatcata aagcaaggac tgaattccaa ttctggccac 95520
ttgcaagatg gtgactctgg gcaactgatt tcactactgc aaatcacagt tttcatccca 95580
taacatgaag acagtagtat cacctccatc acggggtcat atgtagatta ataagtcagg 95640
gaaagcacgt agcacagtgc ccagtacata gtattgctag ataattttgt ttttaatgaa 95700
ttacaagacc aaggcataga cccatttaga atggtgatat gtgtctctgt gctaaaattg 95760
ccaaaaataa gaaaattaaa gtaacgaggc tagaaaaccc agatcccagc tcctggcaaa 95820
attgaagggt gtcaagaatt aacaagttga gactgggcgc gtggtggctc atggctgtaa 95880
tcccagcact ttgggaggcc gaggtgggca gatcacttga ggtcaagagt tcgagacagg 95940
actggccaac atgatgaaac cctgtctcta ctaaaaatac aaaaattgac cgggcatggt 96000
ggcgggtgcc tgtaatccca gctacgaggg aggctgaggc atgagaatca cacctgggag 96060
atggaggttg cagcgagcca agattgcgcc actgtactcc agcctgggcg atagagagag 96120
tctatatctc aataaaaaaa aaaagaatta agttgacttt caaactgcaa gagtcgaaaa 96180
aaaaaaaaga gagaaaacaa gtgcctagtg aaattttgct ttcaagatgg tgtagacaaa 96240
gggatggatt tagattgaag taaatatatg aaaattctag taattagcga ttccttctga 96300
agagtgtggt atggaaaggg aaggaaagaa taaccttgca gtggagaaat tcaacaagcg 96360
ctactccagc cagctggcca aggttaaagt taccggtgat aaatcacatt gatatgatag 96420

acgtatcata gatagatggc actttacctc taacttccaa aaattcatgt acccagtcta 96480
atcatgagaa aagtatcaga cggactcaaa tggaaggaca tgctacaaaa tacctgacca 96540
aaacgtctca aaaactatca agttcttcaa aaaacaagcc tgagaaactg tcccaatcta 96600
aaggaatcta aggagatagg atgactaaat gtattgtagt atcctgaata ggatcctgga 96660
gcagaaataa gaaatgaggt agaaagtaag gaaatgtgaa taaagtatgg actgtggtca 96720
acaatactgt atcaatattg cttcattcgt tgttgacaaa tgtgccatac taatgtaagc 96780
gattaacatt aggggaaact aggggtgaag tacaggagaa ctctctgtac catcatttca 96840
gcttttctgt aaatctaaat ctattctaaa aaagagtatt ggctttcaaa atgctagtag 96900
ttgatctggt ggtgagatta cagacaattt cttttcttag ttttatttgt ctgtatattt 96960
ttaatttttc ttctttttga gacagggtct gtctgtgttg ccaaggctgg agtgcagtgg 97020
ctattcacag gctcgatcat agcatacaac agcctccaac tcctgggctc aagcaagtaa 97080
actgagtctg tagctgggtc tgcatttttc aaatggtcta tgaggaatac ttactgcttt 97140
tgacatcaga aaaaaaattc aatgaacatt actttttaaa aaaatgaata gatgttaggc 97200
tctttggaga cagtactata tacataaaag tataactaga atcgtccttg gacagcaact 97260
aaaacccatt ttatcggccg ggcgtggtgg ctcacacctg taatcccagc actttgggag 97320
gccaaggcgg gcagatcact tgaggtcaga agttcaagac cagcctgacc aacatagtga 97380
aaccccatct ctactaaaaa tacaaaaatt agctgggcat ggtggtgggt gcctgtaatc 97440
ccagctactc aggaggccga ggcaggagaa tctcgtgaac ccaggaggcg aaggttgcag 97500
tgagccaaga tcgtgccatt gcactccagc ctgggcaaaa ggagtgaaac catgtctcaa 97560
aaaaaaaggc caggcgcagt ggctcatgcc tgtaatccca gcattctggg aggctgaggt 97620
gggaggatca caaggtcagg agattgagac catcctggct aacgcagtaa aaccccatct 97680
ctactaaaaa tacaaaaaat tacaattagc taggcgtggt ggtgggcacc tgtagtccca 97740
gctactcggg aggctgaggc aggagaatgg tgtgaacctg ggaggcggag cttgcagtga 97800
gcagagagca cgccactgca ctccagcctg cgcaacagag ccagactccg tcttaaaaca 97860
aaacaaacaa aaaacaaaaa aacaccttat cgctctgcac ccagggcctg gcactctccc 97920
cgggggaggg cggtgtgctt ctgaacctgc cagcattttt tctatctatg atacacttgc 97980
tgacagaggt caaagggcta tcctgggtaa gcccacactg ctggctcaag aggccccagg 98040
caaatcagcc ccaggaaaat ctcgtccatc agcttctagg ccaagcctca gcctgctctg 98100
tgtcatcagt ctgggaggca ggaagactgc aaagggttct cagttcacca tacgaacaaa 98160
agacaagacg agactcgcca ggaatgtgtg gtttgtccca ggcactttgt cctgcatcca 98220
gacctcaagc agtcagataa agctgattct ttatttttgc acttctttta aagctcaggc 98280
tcagagagag agtccccagc tcacaagagt cagaagcagg atataaactc tgatctactc 98340
actccagagc tgcccgcaag aaggacacct gtctctaccc atctcgggaa tgtgccatga 98400
ccacagcaga tggctggact acgtttagga gacaggatgc tggagaaagg aagtgtcaag 98460

cagtgggcag cacccttcag acctcccctg acaccaacca ccaccacccc aatactgagg 98520
gctccatgga caaaggccag ctcgttcttg ggcccaaaca ccagttgcag ctcctggatc 98580
ctgggaaacg tgggagagca ccagtgactg ctccctgggc tccactgctg ttccattccc 98640
aagggcatcc tccatcatcc tggtgtccaa ttcctaggca gacaggccct ccccttgctg 98700
aggacctcca aagcaggggc caggagagag acggtgctga gacatttctc actgtaattg 98760
cattgggacc agatcctttc tctctctgag agttggcaac aagaaccccc tgggaggaac 98820
aacaactcag ctttctaaca ggtagccttc tttttttttt tttttttttt ttgagatgga 98880
gtctcagtct gtcgcccaac ctggcatgat cttggctcac tgcaacctct gcctcaagca 98940
attctctcac ctcagcctct ggagtagcta ggattacagg cgcacgtcac catgcctagc 99000
taatttttgt atttttagta gagatggggt ttcatcatgt tggccaggct ggtctcgaac 99060

tcctgacctc aactgatcca cccgccttgg cctcccaaag tgcgggtagt acaggtgtga 99120
gccaccgtgc ccggccacag gtagcctctc taaaaggcct tttttttttt ttttttaata 99180
caaacactga gatgttggct tcaaagtggt cccctgaaag gctgtccagt tattctagtg 99240
acttctccag agctcattca actcttcaga aattgccttc agggcaacct ttgaccaaaa 99300
ttggtcaacc tgcttgattg tccaaagtat tccccaatac gggtagccaa agaactttca 99360
gctatttcca aaatccaaat ccaggttcaa aagaccaaga cctgcccctc tgaggctgct 99420
cccgagaacc ctgggcatag tctgagaaag ggctgcatgg cagacatatc attactttct 99480
cagttgtgtt attggtcaga cacctgcctt tagggtcaaa gcttgtgtgt gttggcttcc 99540
tctctccctg actcccaaca aaggctctga accattcccc agatgttggc agagtgtgag 99600
acaccctcct ctgacttccc tagaagcatg gccccctgcc tcttctccct cccagcacag 99660
agcccctgtg ctgggatcag cccaggctcc atttgttcct ctaatgagtc ttaagtattt 99720
ggcttgggct cctgccagtc ctactcctac agagaggagg gagtgggaag aatgggacaa 99780
agctcatgcc agctcagttc tgctgggccc tgggcagact ggcacagggg ctgcctggcc 99840
cagacccacc caggggtgca gccaggaacc catttgaggc ccacagcctt tctttaccct 99900
ccacttcagg ggcttcccac agtgcagatt cctgggcata cttcctgggt tggaatcctg 99960
gggtgaggct caggaatgtg catttttaat aagcacctca ggagattgtg atgcataatg 100020
aagattaaaa accagtgttc cagaaagtca atgacaggga ttcaggctgg ctggtgaaat 100080
tgtgtatatg tgcatttttc tagagtccac agttgtcaaa ttctcacatt aggtcaatag 100140
tccccataac cctttaaaag ttttttaaaa acaagcccga tgtggtagct cacagcctat 100200
aatcccagca ttttgggagg tagagacaga aggatagctt gaggccagaa gttcaagacc 100260
agcctgggca acatagcaag accccatctc tacagataat ttaaaaaaat aataacttag 100320
ctgggcatgg tgacacatac ctgtagtccc agctactcaa gaggctgagg ctcaaggatc 100380
acttgaaccc aggagcttga ggctgctgca gtgagccatg atcatgctac agcactccag 100440
cctgggtgac agtgcaagag gccatctcta aaaaaaatta aaaattaaaa caaaaggcta 100500

agaagcacta gtaaatgaga tgattcctaa gttcagttgt agctctgacc tgccacaact 100560
tggtgaccct gttcaggccc ccatcagggc aagcccccag gatgctctcc ttttaccaaa 100620
tctcagagaa agacaaggct ggccttgaaa aaggctggca agtctggggg aaaccaggat 100680
gacatagaac taggctgagg gacaatagga tggcaatggc catgggcttg ggtgagaaat 100740
gacacaggtt aggggaaatt ctagaatacc tgggctgaaa ggacccttag atatcaatga 100800
aggcaaacct ctggcaagag gggtaaattt ttaccaatgt caggtcctct ctctctggcc 100860
acatagctaa acctcatttc tcactcccct gcagttggcg gaggtaatga gttgaattct 100920
gcccagtgga atgtggatct ctgctatggt ctggatgttt gtgtctcccc aaaatcctag 100980
cccctaaggt gataggttta ggaagtggag ccttttggga ggtaattagg tcatgaaggt 101040
ggaaccctca tgaatgggat tagtgtcctt acaaaagaga ccccagagag ctcccttgcc 101100
ctttccacca tatgacagtg agaaggcact atctatgaac aggatgggtg atatggtttg 101160
gctctgtgtc cccacccaaa tctcatcttg aattgtactc ccataattcc cacatgttgt 101220
gaaagggact cagtgggaag taattgcatc atgggggcag tttctcccat acagttctcg 101280
tggtagtgaa taagtctcac gagatctgat ggtttgataa ggagaaaccc gctttgcttg 101340
attctcattc tcttctcttg tctgcaccat gtgagacatg cctttcacct tctgccatga 101400
ttgtgaaatc tccccagcca catggaacta taagtccaac aaacctttct tttgtaaatt 101460
gctcagtctc aggtatgtct ttatcagcag tgtgagaaca gactaatata atgggttcat 101520
atgaaaaggc cctcaccaga catcaaatct gccagtgcct tgattttgga cctccaagcc 101580
tccagaacta tgggaaataa atgtttgttg tttgtaagcc acccagttta aggtagtttg 101640
ttagagcagc ccaaatggac taacaacaga aaatgtgtat caagaagtag ggtgcggcta 101700
taacaaatat ctaaaatgtg gaagaggctt tggaattggg taatgagtag aagctggaag 101760
aattttgaga tgaatgctag aaaaaaaaat ctacattgcc ataaacagac tattaaaggt 101820
gattctggta aggtctcaga aaaggggtgc tatagagaga gcctcaatct tcttagagat 101880
cacccaagtg gtcatcagaa tgttggtaga aatacagatg gtaagggctg ttctgatggg 101940
gtcttagatg aaaatgagga acatcttatt gaaaactgga ggagagatga cccttgttaa 102000
aaagtggcaa agacatttgg cttttgttca tgtcctactg ttttgtggaa ggtagaactt 102060
gtgagcaatg aaataggata tttggctgaa gaaatttcca agcaaagtaa tgaaggtgca 102120
acttggctct tcttaaatgc ttatagtaaa atgcaagaag acaaactcta aagatggaat 102180
ttttcatcaa aagagaagca gaacttaaag attaggaaac gtctcagcct atttatattg 102240
taaaaaatga gaaagtgtgc tcaggcattt gctacagaga ctagcatgaa tcagacacgc 102300
actattcttc aagacaatag aaaaatggcc ccaaaggcat ttcagagatt ataggggctg 102360
ccccttccat cacaggccta gagtgccagg gcctaaggaa cagaatgatt tcaaaagagg 102420
agccacaggc cctcagtgct cactgtccag catcacctca aggctctgct ccctgcattt 102480
cagtgtagtc ctcctcacca tcccaagttc agctccagtg cccaccctcc tcccacccct 102540

aaaggacata ggtggtaaat tttggtggca tccgcatgat gccatctcag ccagtacaca 102600

gagtatatgt actgtgggga catggttacc ttcacctaga tttcaaagat gctccagaga 102660

gtgtaagggg ccagccaaag aaccaccgca cgggcagggc cactgtagaa agccccaaca 102720

agggcaatgc ccaggggagc cacggaagta gggctacctt acaacccaga ccagtggggc 102780

caccagtgtg tgatcccagc ccaagatagc tacaggtgta caacctaggc acagagcctc 102840

cacagaaaat gggaccacaa gacagaactg ccatggaggc aggaccacca ccccagtggg 102900

tccagaagac aggacttctc tacccctgtg gacttaag 102938

<210> 2
<211> 52030
<212> DNA
<213> Homo sapiens

<400> 2
   ggaatagttc ctggagaaag atctaacatc tgcgcaggtc tatcctgttc tgtggctgtg 60
   tttactaaag tgagttctgc gcttacattc acacactgca aactgcacgt acgatgtaag 120
   tgtacttgtc atgtagagtt ttaccaaaac tatattagat tatactctga agcatgtttt 180
   gcatctgaca ggtcgagctc attcattcag tattaagtcc ctcatgggcc aggctctagc 240
   ctgtgtcctg gtgataagga ggtgaatgtg acagaattcc tattttcaat aaactcactg 300
   tctggagagg aaggtgatca tcaaaataaa ttttagcacg atgtgtaatg attaatgcac 360
   agaggatctt tggaatctcc aggaagaagg cctgacaacc tctggatgaa ggatgggatg 420
   gaagataaag tggacttaga gagatgtatc gtgtggctct gtctttatcc ctggaaaggt 480
   tagcctaagc atggggtagg tgctggtttc ttttgctgag aaatgtctca gagaacctca 540
   gagtagcagg aggcctttgt gatagattgg ccagcagttt ttatttccct gacctttcag 600
   catcaaaaag tgccagttgg accctgggtt ttgccttgga attagtaaat agatagttca 660
   acagcaatgg ttgaaaagcc tcttggagtc tgtcccatga gagtaaggat gttaattctg 720
   aagcctgggc cattttccat cctgggaatt atactctgcc ccttcctaaa ttcccattgt 780
   ggtttcaaat gaagaggcca ttgttcactt tccttcctaa aagaagcaat atatttaact 840
   tctggctctt tgcctgactg atggcgagtt gatctctctg aagccaacac ccgtggcttc 900
   ctatcaagag aaacactctg cctccatctg atttccttct tgggttgaag ttatttagat 960
   cctacactgg ctcagtagga ggcttgcctg ccgttatgtc ctactaggtg gtgacagggc 1020
   ccttgattcc ctccctgtgt ttaatagaaa taaagacaga gtcttagcgc tttagggcag 1080
   ctagttgttc tcccaaagac ctattatagg aatttaaaag caaatgtctg atgtgaaaat 1140
   ctatgctatt ttagggactt tgaagcacaa cacaaaagag tatcactttg ttgaatagtt 1200
   tgggtccagg caggtgtcct agagcagaag ttttcattta tttttaagca acaaaattct 1260
   ttctttccca aatgaaatct cctacagaat ccaaatactg aagggtttct caaccccagc 1320
   atgcttggta ttttgggcca gataattctt gttgtagaag ctgttgtgtg tgttggaaga 1380
   tgcttagcag cattcctggc ttctaccctc cagatgacat aagtggcacc cacattctct 1440
   agttgtgaca acaaaaatgt ctccagacat tgcaaaatgt ccactgggga gcaagatcac 1500
   ctccagttga gaatcaatgc aatataatct agaaaacaga cttttaatta agctcctcct 1560
   ttagaactct gcccagggtc cctgactgtt agtgtttgag acctggtggg gcgttctggg 1620
   ctacacagat gcactatcct agagggttaa gttgtcattt taaaaacctg ggacatagag 1680
   aacttgttat aaacaaagca aaagagctaa aattagacaa atccacaagt actttgaacc 1740
   cacatgtaca ctaaatattt ctgtctatgt attatgtata cattgtgtac ttggatgtgt 1800
   agcataaggg aagagtcgaa gggtggtgtc tgccttactc cttatgtgtt gaggtttggc 1860
   taccacaatt tgccctgttt tgccagagat gccagttgaa ggcctgtgac tgacagccca 1920
   gtgatagagt caccctcact gcacaaaata ctcagcttct gtgggattca gtggccctgg 1980
   ccctgctcat gggcattccg tggtgagtgg gccagcacct gcagcctctt tcaaccagac 2040
   tggttggttg gagcttggac catggttatt ccaaggtgga tttttatcct tctgcccttt 2100
   gtactaaatg ggtcatgtgt gcctctgtac gtgtgtgtgt gcgtgtatgt gtgtgactac 2160
   acgtgtctgt gtctgctctt ctcctttcag aagcccagag ccactctggg cctggccact 2220
   ctgagcattg gttgtgtggg ggatgctggg agcttggcac atgctactcc tcctttttcc 2280
   tcccttagaa tgatgaaagt gttgttggag cctgtcccac agacagaaac tgtacctcat 2340
   ttatgtgggc atcagtataa acaagaacaa taccactatg tgagtcaccc tcacctgcgg 2400
   ggagacgcat aggctcaggg agggaatttg ctgccagagg gtctcagctt cagtctgtct 2460
   ccccggcaga ctcatcctaa cttcattcca cagctcaagt gcagtgtgaa tcagcaggca 2520
   gcctgtgcct agctaaagga ctctctctgt gttttgtttg tttggttgct tgtgaagatc 2580
   tctgggcaca tacagcagtg ttttattttc ccaaaagatc gcatgaccac tacagttagc 2640
   cttgggaaca gattgagtaa gcacaattca tttgatctat ctgcagatct ccagcccttc 2700
   taagaaactc acttccaaaa atgtttcagg agtcctgtag aagtttgctt agagttcctt 2760
   aaagaggcta agcgtcagtt gcaatatctc tctatcaacc ccatttcccc cagtgccact 2820
   gccatgggct ggaggagtgg gtaacttgct taggaacttg ggtaagggac ctgaagctcc 2880
   ccacccactc tggccctaga taggctaaag atttgaaatt agtttgattt atgtaaatct 2940
   ctggtgtgaa ttattgctat cagataaata cacagaccat tgaactgaaa acagagcctc 3000
   aacacaggtt gccattgcta atgttagttt atagtagcca ttatctagta gtgcctttat 3060
   cagtgacccc aggtgtataa ttggtttgtt gtgaaagagg agtggagttc ccaggatgct 3120
   ttttgtcccc catggttccc ttataagtga ctgtatttcc tgagctctct gcacttttgt 3180
   caccttttgt catcaatata tacaatcagt ttcaggcaat attgataggg atgtgaaaac 3240
   taaacactat aagattgaat ggacttaata gctaggagtt gcagaaaata ttcctacatg 3300
   tatacataac ttttataatg tacaaattgt tcttatgtgc atactcttgc ataatcatca 3360
   caagtcagcc ttgtgactta ggctctctct tggccttatc ttgagagaat ttatgaaatc 3420
   tgataatatt gcagccccgt aggaaaactt ttcagtgcct tgctatgatc tctgaaggcc 3480
   tatactcctc cttcccttcc taggctggta ttgacctgct ttttaaatct catctcctac 3540
   cagaccttca tattcattgt agttctagca gtagtgtact acttgtaggt acctgaatgt 3600
   ggcaagctct tttctacttc ttactaattt atggaaacac caatctaccc attcattctt 3660
   tcactgctac ttgtatgcta ggcattggtg atatccccta accccctggt ccttggtcta 3720
   agcaggcata ggtggtgctg tgtggtaaga gctttggtct tggtgctagg ggagctgtaa 3780
   ggagggcacc aagccttgac ttgaagggat tagggaggct tctccagtaa ggcatctcca 3840
   aagtaagtca ttttagtgtg aggtggatgg gagagttagg agaggagagg aagaaagaga 3900
   ttagggcagg tgtggacccc aggctagaca gggatggatg agtgataagg tcttgatagt 3960
   gggtagtgcc agatgcaggt gtatagtttt gaggactttg ccctaaggcc tgaaggcagc 4020
   cattcaatgt ttttgttttt tgtttttttg agacagggtc ttactctgtc acccaggctg 4080
   gagtacagtg gcgcaacaat ggatcactgc aacctcgaac tcctgggctc aagtgatctt 4140
   cccacctcag cctcatgagt agctggggct ataggcatgt actaccatgc ccagctaaat 4200
   attttgtttt tctgtagaga cagggtctct ctatgttgcc caggctggcc tcaaactcct 4260
   ggcctcaagc gatcctcccc tcctgccatg gccttacaaa gtgctgggat tataggcgtg 4320
   agccacagtg cctggccaat taaatgcttt ttaagtagga gcatagcagg gtgcagattg 4380
   ctgcaaagca cctttctaga atatatcctg ttcccctcat tctccctcta gtactttcta 4440
   gtatataccc cgttcccctc atcctccctc tagtactctg ctgcttaggc tttaggttca 4500
   attcaggcat cactgtctct gtatgtgctc cagattcccc gggcaccatc gggggtccct 4560
   ctgccccaca gcaccctgga catgcatact ggagataaag cagcaattgc ttttgtattt 4620
   taaccgtcta gttgtgtgtc cttcctccta gaccactggt tttcaggatt taaaagtgta 4680
   gattcttgtt gcccacccca gatattctta cttactgtgg gatagagtct aggcatcccc 4740
   atttttttta aaagaagcag aaacattgca gtaggctaat cttattgagg gcctggttta 4800
   tacattatgt gtgtgcacac acgtatgcac acacccccac atacttgatc tcattcatgc 4860
   ttatatctgt atttctttta tttttatttt ctttgatcct gtcattcaaa tatccatatt 4920
   tcttaagaat gtccggtata aatttgctat gctacaaatg tttgattgaa tgaatgaatg 4980
   aagttgccca aatttacgtg tgaagatgtg ttggagccta agttaaaatt cataggtgat 5040
   tccgaggcaa ggccctttca cagcatcact cttacaccac agttgctaca ctgagatggc 5100
   ctttgaaaag caggcagtcc cactgaacca cagtatccta gagactaaga aacggggaca 5160
   agagatcttt aacttgcatt ttataatttt cagtggcaca tcaataatct gcagggatac 5220
   actaataaga tgggtattgg acactcatta cgacagaccc tggaggatcc aaaactgtaa 5280
   aatctagctc tgatctccta caaatttttg gtctacttga gggtaaaagg cagaaataca 5340
   cagacagttg aatgactgtg ccagatcgtg acagttacct ggagtaccgt gcacagaagg 5400
   gcaatagaat gatgcagtga ctgagtaaga aagagcatgc cattagctgg agtggtctgc 5460
   agataccttt ggatgaggaa gcacttttat gaggtttggc tttagaaaag tgagaagcca 5520
   ttccaagtgg gaggagtgac atgagtggaa tcctggcagg agatgggcta tgaatggtga 5580
   gggcatcagt ttgacaggaa cataggtttg tttgtgtagg ggagtgatgg ttgacaccta 5640
   aagggagtgc tggttcagga ttgggagggc cttaaatgat aggtggggct ataatacagt 5700
   tggggatgtg tggcacattc agcaatgtaa aagtctgttg tcctcgaccc cagatccatt 5760
   caccttatta atgaaggtca tgaattttta aatataataa ctgatatgga taggttttgt 5820
   gtcctcaccc aaatctcatc ttgaatcata atccccaggt gttgagggag agacctggtg 5880
   ggaggtgatt ggatcatggg agcagtttgc tctttgctgt tcttgtgata gtgagggaat 5940
   tctcacgaga actgatggtt ttataagggg ctcttccccc ttcacttctc ccacatactc 6000
   tctttctcac ctgctgtcat gtaagacata tctgcttccc cttatgccat gattataagt 6060
   ttcctgaggc ctccctagcc atgtggaact atgagtcaat taaacctctt ttctttataa 6120
   attacccagt ttcagtatgt ctttatagca gtgtgagaat ggactaatat agtaaattgg 6180
   tactgataga gtggggtact gctataaaga tacccaaaaa tatggaagca actttggagc 6240
   tggataacag gcagaggttg gaacagtttg gagggctcag aagaatatag gaagatgtgg 6300
   aaaagtttgg aacttcctag agacttattg aatggctttg accaaaatgc tgatagcgac 6360
   atggacaatg gagtccaggg agaagtagct tcagatggag atgaggaact tcttgggaat 6420
   tggagtaaag gtgactcttg ctatgcttta gcaaagagac tgacagcatt ttgcccacac 6480
   cctagagatc tgtggaactt tgaacttgag agagatgatc tgaaattgga acttatttaa 6540
   aagggaagca gagtatagaa gtttggaaaa tttgcagtct gacaatgcag tagaaaagaa 6600
   aaacccattt tctggggaga aattaaagct ggctgcagaa atttgcgtaa gtaacaagga 6660
   gccaaatgtt aatcaccaag acaatgggga aaatgttcct agggcatgtg aaggactttt 6720
   gcagcagccc ctcccattac aggccccgag gcctagaagg gaaaagtggt ttcatggact 6780
   gggcccagga ccctgctgct ctgtgcagcc taggacttag taccctgctt cccagccact 6840
   ccagtcatgg ctaaaatggg caaattttta aaaagtcagc tcaggctgtt tgcttcagag 6900
   ggtgcaagcc ccaatccttg gtggcttcca tgtggtgttg ggcctgcagg tatacaaaag 6960
   tcaagaattg aggtttggga gcctcagctg agatttcaga ggatgtatgg aaatacctgg 7020
   atgtccagtc agaagtctgc tgcaggggca aagccctcat agagaacctc tgctagggca 7080
   gtgcaaaggg aaagtgaggg gttggagccc ccacacagag accccaccgt ggcactgctt 7140
   agttcagctg tgagaaaagg gccaccatcc tccagacccc agcagatggt aaatccatct 7200
   gcttgcactg ggcacctgaa aaagccacaa gcactcaaca ccagcctatg aaagcagcca 7260
   ggagggggat ataccctgca gagccacagt ggtagagctg cccaaggcta tgggagccca 7320
   ccccttgtat ccacatgacc tagatgtgag acatggagtc aaaggagatc attttggagc 7380
   tttaagattt aataactgcc ccactggatt tttgacttgc atcaacctgt agcccctttg 7440
   ttttggccaa tttatcccat ttggaatggg tgttcttatc cagtgcctgt accaccattg 7500
   tatcttggaa ggaactaact tgattttgat tttacaggct tataggcaga agggacttgc 7560
   cttgtctcag atgagacttt ggactgtgga cttttgagtt aatgctgaaa tgagttaaga 7620
   ctttggtgaa ctgttgggaa gccatgattg gttttgaaat gtgaaaagat atgagatttg 7680
   ggaagggcca gggctggaat gatgtggtta ggctttgtgt ccccacctaa acctcatctt 7740
   gaattctaat ccccaagtat tgactagaga cctggtggga ggtgattaga tcatgggggg 7800
   tggtttcgtc catgctgttc ttgtgatatt gagtgagttc tcatgagatc tgttggtttt 7860
   ataaggggct cttctccctt cacttctccc acacactctc tgtctcacct gctgccatgt 7920
   aagacatgcc tgcttcccct tctgccatga ttgtaagttt cttgaggcct ttccagccat 7980
   gtgtaactgt gagtcaatta aacttctttt ctttataaat tacccagtct caggtatgtc 8040
   tttacagcag tgtgagaaca gactaataca ataaccatgt tctcttcttt ctggagatct 8100
   ccagccacat ttgacttcca ggtctgtggg aagcagtcca aggtgcaacc tggaacactt 8160
   tcatttggct tcttcatgta gaggaacatt tacatgtcgc tgcttgttcc ttctacccat 8220
   ggcctacaca cactgccaag ctgtcacctt ccctgttgtg caatgtgttt gccatcacct 8280
   tcaatgaaaa caattttttt aatgtcagaa ttttcttagt tattaatccc aaacacagac 8340
   accagaaaca tgatccagaa taaagttttc agttcacact gagcttgtca ttgctgaagt 8400
   aagactctta gctctttcac aagtatggac aaaattcagt aggaatgttt tcatcacaag 8460
   tcttacttct taataatgct ttgatctagg gtaatttctc tttagtcttg gatggtaatt 8520
   ttgcctgagg aaagtccaag aggtcttatg tgaagatttc tgttttgttt agagtttgcc 8580
   acaaatactg gaaggagaaa gttttctgta atttatacag ctttacatta gaaggctata 8640
   gcttatttta aaatggtttc tgttcaaaaa attttcacct acaattatag tataaagtgc 8700
   cttgtccttg tattcacaga ggaattttct catacaacta caagaaaatg tggaaagttt 8760
   cgtctgagaa aattcaactt tttcatctct cataatttac tgcttcagga atgcaattta 8820
   ccaaagtgga ttagaaactg tttgtaatgg ggaaatacgt gcactctttg aaggcagttg 8880
   tcgacaaaga gtcaaactct aaaatatttg aagaggttta ttctgagcca aatatgagtg 8940
   accacagccc aaggcacagt ctcaagagat cctgagaaca tgtgcccagg gtggttgggt 9000
   tacagcttga ttttatacat ttcagaggga cataagacat caatcagtac atgtgaggta 9060
   tacattggtt tggtctggaa aggcaggaca acttgaagca aggggtgggg agttgtatgg 9120
   gggagggtgc ttataggtta caggtggatt caaagatctg cttattggca gttagttgaa 9180
   aggataaatt agttattatc taaggactta gaatcaatag aaaggagtgt ctgggttaag 9240
   ctaaggggtt gtggaggctg agattcttat tatgtagatg aagtctcata ggtgtcagcc 9300
   cttagagaca atagatggca aatgtttcct atatagacct aggaaaagtg ctagactcaa 9360
   cagttaatct ctttaggatt gggaggacct ggaagaggaa agatctagtt atgttaaaga 9420
   gattctttac ggatgcaaat tttcctccac aaaaagatgc tttgcagggt catttcaaaa 9480
   tatggcagag aaacatattt tgggataaaa tattttgatt ttcttctttg gttgttttgt 9540
   ttgtttgttt ttgagacagg gtctcactct gtctcccagg ctggagtgca gtggcgtgac 9600
   aatggctcat tgcagcttca accttctggg cttaagtaat ccttctgcct cagctgccca 9660
   agtagctggg actacaggca tgcaccacca tgcccagcta atttttgtat ttttgtagag 9720
   acagggtttc atcatgttac ccaggccggt ctcaaactcc tgggctcaag tgatccacct 9780
   gcctcagcct cctggagtgc tgcaattaca ggcatgagcc acttcaccca gctgttacct 9840
   ttatctatca tgtgatgcta gctgagtgtg gtggctcaca cctataatcc tagcactttg 9900
   ggagactgag gcaagtggat cgtttaagac caggaatttg agaccaacct gggcaacatg 9960
   gtgaaacctc atctccacaa atatatatat atagatagat agatagatag atagatagat 10020
   agatacacac acacacacac acacatatat atatacacac atatatatat atatatatat 10080
   gtatctcaca cgatgctata ccagagtcag gttgagttgg tatttttgta gagacagggt 10140
   ttcaccatgt tacccagact ggtctcaaac tcctgggatc aagcagtcta cctgcctcac 10200
   cctccgaaag tgctgggatt ataggtgctc tgcactctag cctttgtaac aataagggtc 10260
   tattctgtcg gttttaggtc tctattttag tgttaatgct ggtcagttga gtctaaactc 10320
   caaaagggag aggatataat gaggcatgtc tgactccctc tttgcgtcat ggctttcact 10380
   agtttttcag gggttttttt taatcccctt ggttgagaag gggtccattc agtcagtagg 10440
   gggagcttaa aattttattt ttggtttaca aagtgaagag ctttcctctt ttaagtcctc 10500
   accatataac cagtctctac cagatgctga gaatagctta aactttctta ctgtcttatt 10560
   tgagcctggg gtggtttgtt gtaggacctt atcaacatag attttttgat aattgatcta 10620
   ctttacttcg atgtctgagt aaagctttaa cctggccctt aaacaccaaa aatgctttag 10680
   tgggagctct tgttttggga aaatgaaaaa tttctgtgtc tatcaatcac ggaaagtatt 10740
   ccccactggt ttgattctga aattcaatca ttgcctatga aagttaaaaa catttttttt 10800
   tccctgtagc tagcttttac tctgtctctt taaaatacct ttttttggag gagaggtgga 10860
   gcaagatatg tgtaactata gtttttcttt tttttgctta gatggtttaa agaattctgc 10920
   atgtcatagt tgacattcca tcattatcct gtgttccaca gtcaaggcca aaggttaaaa 10980
   ataagttctt aaaggaaaca aaattaaagc aagagggaat tacctctgaa ttgttttagg 11040
   gactcctagc attttgcatg agtttctgtt ggtctggaga atagaaggaa gcatgaaagg 11100
   tttcattcct tgcaaatcaa gtgaaactgg ctcctaccct cttcttcaat acaaacatga 11160
   aacagaaaaa gtagactgga agaccagaaa gggcacagat ttttatacca ttttcatcag 11220
   gattactatg ttcatgttat ggaaccaatt tgtaagtttt gggtaacagc ttaaatagaa 11280
   acctatagaa ctgaggcaac cttctgtttc tcaaagacag atggtgaagc aacatttata 11340
   aatgctttac agttagagag catatgctct cgtccactgt tttttgtctt tgtgatcaaa 11400
   gaaggatttg aggaatttct tttaaaaatc agtctgtaca ataactgagt acattacttt 11460
   cagataatgg agaatagtca cacccattgt tttcagggcc tcagggaatc ctagaatgtg 11520
   tgtcttgtgg actgtgtcat agttggctag ttacagtggc agttactaaa atcaaatctg 11580
   tttcaattaa taaagaactt gacaatgaaa ctaacaagac ccattctaaa gacgccagga 11640
   aaataaaata aaaacccata tgtactagga attgtaaata gagaggaagc agaaacaatg 11700
   taatttgaat tatcttatta aaattttaaa atattaaaat ttaactagtt tctcttattc 11760
   agctagagac ggtataaaaa cttggcagaa gtgtgatttt tgtctatctg ctggccagaa 11820
   taatactata tagatgggat tttgtaggtt ttagtctctc taccccgctg tcttccaata 11880
   tgtatgcaga aaaagtaaac atgatcccta acctttctga aaccttcttt accttcagtc 11940
   tacacataca tagtcttggg caaagagtta tcaggtaaat tccatggaat caagagtaga 12000
   ggcatatgtt gcttttctta ataggaaaaa cagtgactgt gtccccctaa ataaagtcat 12060
   gattcaatga aaagacaaac agcccaattt taaaaaaatg agcaaaagat ttgaacagac 12120
   attttctcaa aagaaatatg aatgggcaat acacacatag aaacatgctc agcaatttta 12180
   ggctttaggg atatgcaaat taagaccaca atgagatacc actagaaagg ctaaaatgaa 12240
   aaagaccaac aacactaaat gttggtgaga ctgtggagca ctagaacttt tcatattttt 12300
   agtgggagtt caaaatggta caaccgcttt gaaaaatggt ctggcagttt cttacagaac 12360
   taaacagacc actaccttgt tacccagcag ttcttaggta tttacccaag acaaatgaag 12420
   catatctctg tcaatagaaa agcagatgcc agccatggta tgattttgaa cagccagtgg 12480
   aataaaagaa tagggactat atctgacctt gctgttagag tagttaggca gatatgagcg 12540
   gggcaggaga ggcccccctc ccccacagga atgtcaggca accatcaggt gatggtcagg 12600
   tggttgttaa actgtctcac taacatacta gttggtcaca gcttgcacca gggaaagcag 12660
   actcccagta gatagaaaac accttaagct cttaatcagc agcttccttt tttttttttt 12720
   gagatggagt cttgctctgt tgcccaggct agaatgcggt gtcgtgatct cagctcactg 12780
   caatcgctgc ctcccgggtt caagcgattc tcctgcctca gcctcccgag tagctgggac 12840
   tacaggcacg tgccaccacg cctggctaat tttttatatt tttagtagag atggggtttc 12900
   gccatgttgg ccaggatggt ctggatctcc tgacctcgcg atccgcccgc ctcggcctcc 12960
   caaaatgctg ggattacagg cgtgagccgc ggcacccggc caatcagtag cttcctcata 13020
   ggatctcagg cgttggatga gtgggctcaa acttgcatgc taagagacaa aatggtggag 13080
   tttagctggt gtatgacctt cctctaggaa cactcaattg gtaagggaaa aatgcctcaa 13140
   atgaacatgt gcacagcttc agtaaacaca ctgtacatgc ggcccttccc aagtgctggc 13200
   aggccactgc acatgcggac agcccacccc aaggaaaaac caagggagga gagacacaaa 13260
   cctcagcacc atgccattgt gtaaaaatcc caagtcaagc gtcggacagg gtcctcggat 13320
   ctctcaaatt gcccacttgg ccctcttcca cgtgtacttt gcttcctttc attcctgttc 13380
   tcaaactttt taatatactt taactcctgc tctaaaactt gccttggtct cattctacct 13440
   tatcctctct ggccaaattc tttcctccaa ggaagcaaga atcgagttgc tgcagaccca 13500
   tatggattcg ctgctgctaa tgttgccatt tggatttttt tttcttttta gagtgttcta 13560
   gaatgcatgc ttagagaagg gaatgtaatc ctaggacacc actcagctct gcaacagtat 13620
   agctctgcag taatgagtaa atgctgtttg ttggtttact ttaaagctct gctactgaat 13680
   atatacaaat atactgttat attttcctgg taaaaccttt tattatacaa tcacttcttt 13740
   ttatttctag caattctttc ttttacagtg tattttatct gacgttaatg tggctgcatt 13800
   gcatgtcttt tggctagtat ttgccctgct ttgcatcttc tttaattcat ttactttcta 13860
   ctttttctac ctatgtctta ggtttgtgct ttataaacaa caggtggtta gatttcctat 13920
   ttctagaagt ttattgagat tctccctcta ccactgtctc taccaattcc attttttccc 13980
   acctctattg gtttggaaga tagaatctct ctctcttttt tttttttttt aagtgattat 14040
   acttggaatt ttaacatgtc tgtgtaacta agtctaaagt tagctaccac tttaccctcc 14100
   tactgagcaa tctaaggatg ttagaatgtt tttaactcca gtcactttcc tcctggctta 14160
   tgtgcatttc cttgatattt ttgatatctt tttatttttc ttttgaagct ccattaacta 14220
   gatgttatta gttattccat ccttctttgt ttctatttac ttacatgttt ataatttttt 14280
   aacataccac tttcttgtat agctcaaacc ttccctcgaa catttccctc cttcttcaag 14340
   tagattctct tcaagtacat tctttattta gcgaaggtat ttggtagtga acaatctcag 14400
   ttttctgaaa tatctttatt ttgccctcat tctggaaagc tagtttctta ctttattttt 14460
   ttaatagaca gagggtcttg ctttgttgcc caggctggtc ttaaacttct ggcttcaagc 14520
   agtcctcctg ccttggcctc tcaaagtgct gggattacag gcacgagcca ctgcgcccag 14580
   cagtaacgct agtttcactg ggatcatgga ctcttaagag ctagaaggaa cttcactgat 14640
   tatttgactc agccttttcc atttccagaa aagcctgcca ccaggcacag aaagaataca 14700
   tgacttccag agtccctcgc tatattgtcc agactggttt tgtactcctg gcctcaagtg 14760
   atcctcccac ttcagcctcc caaagtcctg agataatagg cgtgagccac cgtgatcccc 14820
   tttacctttt tctttttttt tttcctttat ttcttcttaa taaaaaaaaa gtggggggga 14880
   tacatgtgca gagcgtacag gtttgttaca taagtatacg tgtaccatgg tggtttgctg 14940
   cacctattga cctgtcctct aagttccctc ccctcacccc ccaacctctt caaaaaaaaa 15000
   aaaaaaaaac tatcaaaaaa tggtggccga ggttcaaagc tctcaccttg gttttgtgat 15060
   aaaggtggtt aatctttggt gttggatttc aaattagtct gtttacagtg agcaacagta 15120
   cagcagtttt aaaaatcatg ttgaatcaga aacatgaaga aggcagcaga gctttctctc 15180
   caccatgcac ttggagaagg aaatgactcg gcagcatact cctcatggct aatatccttt 15240
   aagatttttc caaaatcatg aaaccagaac ttgagagctt aaaagggact ttagaagtct 15300
   accagtggat aagagagaaa aaaaaatcaa aagggaaatg gaataagcca aactaaaggg 15360
   caggattctt ttagttctca gcaggaaaca tatgtttatc tggaaaagat tttggttgga 15420
   caggttgttt gtgtaagaat cttttttgtg aggaaggaag atcaacctgg gcttgaaggc 15480
   tggggtgggg gtagagagaa cagtccctga gcttcaagta aatgagaaaa atacacagat 15540
   tttaaacaga ggagccctgc tttcacgggg atcatatgat cgtgctatca gcttactttt 15600
   aagttgtgat aatgtataca tactttatga aatatgatta aatggtaact ttagtattag 15660
   tggctacaag aacagaaaca gcaactttta aaaagatatg aaaagatgtt ttgggtgaaa 15720
   gtagttggct gcgcaacaca tagagtataa catacatcat taatttaaga ctgtagatgt 15780
   acagtatata tttggcatgt acaccaggat tttcagtgat tattgatgta ataagattta 15840
   tgatttttaa aaagaaactc ttcatgatag ttttctgtac cacttacatt ttccatagtg 15900
   atcatcatat tgtctttata aacaatgaca acaataaatc aaaaagagag atcttacatg 15960
   gtgcctcttc tctgacagct gagaccacat catagctacg ttgttccctt acattgcatt 16020
   tacattacat ttaaaatgtt atcaggagga cagaggctca tgaatgagac atgttctctc 16080
   cagttaaatt tagtaaagaa gaatcagaga aaaatctcca gataactgga aaatgtttac 16140
   attttcaagt atataaaacc aggatacatt atcttgttta agcttggagt tgacaataat 16200
   accattttga gatgtttttc agatagaatg acagtgagag tcatagtggg gttgcacctc 16260
   cacatgatgc aacccaaatg tgatggaaca gaaacatttt tcttcatcat cctgaaaccg 16320
   agcagtgatg atccgctgag gaatgtttaa ttttcatgta ttaaaaaatg aagaatgtct 16380
   gacctactgt taatgagaaa aaaaaacacc aaaaaggaag aaagaagaag tggcagctaa 16440
   aagccttttt atggatgacg tcaatagagg aaaatgaaat attcatagaa tcacagcaag 16500
   agaataggaa ttctgaacat ttttgtagaa ggaaactgta ggattcttgc aattgatagt 16560
   aggacaggag ggaatttcca tccttatgtg tgcagaaagg agtagacaca gtgaaatttg 16620
   aagactattg taaacatttt ctacttcact tcaccttgaa gaagtctaca gaaagcatat 16680
   atggcatagc tgggcagagt ggctcatgcc tgtaatctag cactttgcga gaccaaagag 16740
   agaggatcac ctgagcccag gagtttgaga ccagccaggg caacacagtg agaccccaac 16800
   tctacttttt aaaagatttt ttaaaagcat atatggcagt taaatttcag tgagaaatca 16860
   ggaccagggt aaatgtaaat acattactat agcagataaa taaatatgga tgccccaccc 16920
   atatttcctt tcatcccaca acagaaagct ggaaatgctg aaaaattaat acctcccacc 16980
   cctgaagcag ccatcagcca gtgaggaatg ggagccagag gataaataac ccaacttcct 17040
   cacccgctga gggtgttcaa ccaggtcctt gaaattcccg acaggaatcg agcctcagtt 17100
   gcccaccctg gaaacctgct cagtaatacg cttatcgttg gtttccttcc ctttcctgtc 17160
   tcacttctgt aatcccctgg tgcatctggg ggttccttcc cgaataaact acttgcaact 17220
   taagtcccta tcttgaactc tgctcctggg ggaatccaaa caagaacagg tggagacaga 17280
   gtagaacaag gataagcaaa gcaagagagg agatctgaag acgtctctaa ggttgtttga 17340
   gtgactccaa ggatagtggt acaattagct aggagaaaga gagggtggag gaggccgaga 17400
   agagggttca gcccaacaaa tgccaagttt taggtagctt ggggatacct tgaagagatg 17460
   ctgacaagct acttagaaat aagtttatta gctagaagcc ggagctggcc aggcacagtg 17520
   gtatgcacct gtagtcccag ctactctgga ggctcaggtg ggaggatggc ttgagtccag 17580
   aagctgtttg aggtcagcct gggcaatgta atgagacctc atctcaatat caacaaacct 17640
   tatctcaata taaatgaatg aatgaatgaa tgaatgaatg aatgaatgaa accagagcta 17700
   tagctgtata tttgggaatc ctcagtgtac aggcagttga tgaatgaaca tccagaaaga 17760
   ctagaagcat ggtatgcagg gaaccttgga gcatacacat atttaaaaat tgggtggagg 17820
   agaaaagcca gtgaaggaga ctaagaaaga cgattataga gagaagaacc agagctgtta 17880
   ttggagccaa ggcctctttc ttcaaggact gagaagggga gagtttgtac tttggagttt 17940
   gcacagatca cagacatgaa aaaggaaaga tgatgatgag tggaaaatgg gaggtcagga 18000
   ctctcctccc atcctgatgt ggcaagaggc ttttctcatg gttctggatg gaactttggc 18060
   cctgttgcag atgctcacat gcaacgttct gttctaatca ctgttctgct ggcagaggag 18120
   gctggagtga tgagtgagca tgggctcagc ttgcgtggat gctatgagcc gtaggtccta 18180
   ctcaagagag tcagtctagt aacaaattat tttcctctcc ttcttttgcc atcccattta 18240
   gtttgcagga tggttggtgt ttgtgtgggc tgagattcct cagagggaac caaagccctg 18300
   agaaacatgt gtagcagctg ttgatttggg tctccacagt gagatccggg tctaatacag 18360
   tgagtccaac cactttatct gagtttggaa ataactatga gtacatagtt aagctgtttt 18420
   caaaaaacac agaggattct gtactgctag tgccattgag ggggcttttg ctgccattta 18480
   ttttcaactt atttttttga ggtggagtct cactctgtcg cccaggttgg agtataatgg 18540
   cgtgatctca gctcacagca acctctgcct cccaggttca agcaatcctc ctgcctcagc 18600
   ctcctgagta gctgggatta caggtgcccg ccaccaggcc tggctaattt ttgtattttt 18660
   aatagagatg gggtttcacc atgttggcca gacggtcttg aactcctgac ctcaggtcat 18720
   ctgcccgcct cagccttcca aagtgctggg attacaggca tgagccgcca cacccggcct 18780
   tgctgccatt tttaaatacc aaaacctttt gttgcagtct ttgtcacagg tgctggcaca 18840
   aaattcctgg taccttctcc ctgctctaat tcaccaagtt ctccttcctc ttttccaaag 18900
   actactaaca tggaagtctt ccttaattag ttacaggact atatctgcaa ctcctgtgat 18960
   ttattcgctc agtgcccgtg tcactgtcac ccactggtgg ggtgtgcttg ttgtaaatac 19020
   ccatttaaat gtttacattt tcaagtatat aaaaccagga ttgtaaaata ccgttgtatt 19080
   tccagttgta aatagccgtt tagttaagtt aggcatttgt ttatcacgcg tcctaatctc 19140
   tgtttgtgtc gttactgtct cagagatcat catcatctgt gtttacattt ttatgcatca 19200
   ttctttatgc aagtagttct tattcagtgt ttttgatgat gttaagcaaa attctgttgc 19260
   taccttacct cattaccttc ccgggatata ttttgcagat ataaaccaat aatattttct 19320
   ggttctttct tgactagtag cttttggtta actctaaggt tttctgtttt gtttttctca 19380
   cagttgtttt ctcccacgtg tacaaatgat aaaacaaatt atctcaggaa gaagaaaaaa 19440
   gacatttgat agtatatcca gtagttcaac aatagtcaac ggagtctggt gtcctcactt 19500
   ctgtcattgg ctgcttgacc tcacttaacc ttcctgttag tttctgtttc ctcactagaa 19560
   aatttattac tttctaggga taccaggaga cctggtgtta ataaagggca cagtgcaggg 19620
   tgtgtggaag attttagtgt cagttctagc acaagctagc taaatgtgtc tcggggtaca 19680
   ttaacttctc taggccccaa tttcctaggt attcattctt cttctagaca atagacagtt 19740
   gaattaaaaa atcctttcta tcgaccaggt gcaatggctc acacctgtaa tcccagcagt 19800
   ttgggaggcc gaggcaggag gatcacttga gcccgtgagt ttgagaccag cctagcaaca 19860
   cagcaagacc ctgtctctta aaaaaaaaaa tcctttctat tcctagaact ctagggtagt 19920
   gtgaagtaag ggttttcaca gttacactag gtaagttact acctgtcggg tacttctcta 19980
   attatgctgt gcttattaac ttatcaagtc ctcaaatggc cctatgagga acatatcctt 20040
   tatgttctat agatgaggaa actgaagcat tggatgttaa acaacttgcc cagggccacc 20100
   gagcttataa gggaagaagc cgtctgcaca ccccaggctc cagattccac agcctcaacc 20160
   accaaactct cttgtctgtc gtcatataga aggctgttct gttttctcag tgtagccccc 20220
   ttaaagataa gccacagtaa accacagtgc ttttgatatt aggcaaatta aacttgagtt 20280
   caatcgtatg ttacactgaa tcaaagttat attcgctgtg cattttacgc cagtaattct 20340
   ggaaactaaa aagtgccatg ccagaaattt tctctgattt aatttgctta caatcatttt 20400
   aaaaatgagg agctgtgatc cacgcttttg ccccaggttg gaatatttta gaagtacttt 20460
   caaatatata tttctgctgc tgggatataa aaccataaat tcgagcagat agaacaaaag 20520
   accaaagagg atgatgtatt tactgttttt gagttaaagt tacaacagtt tacagaatca 20580
   cagcaaccga gaataggagt tctcagagac atactcctca tccacagaag cctcacatga 20640
   ccaacacaag tttcagtcca gtgcaacttt ttgcaaatga aaacttaccc tggggttgtt 20700
   tggtgactcc tagtcacatg tgctttctaa agttctcaaa acaagcatgg ggtgacagtc 20760
   cagttttatg aaatataatt ttagttcaga agagatggga ttttagaata gttaatcttc 20820
   attatatttg acataattac tctgcaccct gtggatgggg tggcttttca aaacccactt 20880
   ctggtgaggc agaccagggc tccaggccag cacccctctg gagattggaa tgtgaatttc 20940
   cgtgctggga cacagagcca gcttcctatt tatttagagc cattgggcac ctaaggagcc 21000
   aaacagaagt tgtgatgcaa gttttggctg cagagcatgt tagggttgcc tgacgtgtct 21060
   aactaaaaca tgatctgaga gatttctcaa agccctggtt gatttaagca ccatagtatt 21120
   caatagggtt tttttttagt ccttttaact cagtcctaag taaacaggag gcggtgcaaa 21180
   caatgaggaa gttccctctg cattccgatg gtgaagtggg gttggagtct gtaggcaagt 21240
   gaagagcgtg tgggagagct gtttcctttt gctcatggca gcagtctaga acatggtgac 21300
   agtgaaagtc attggaagcc atgggggccc attttcttta tgtgaccaaa tgtcttgcaa 21360
   agtcccaggt gtgtgtgtag gtgcatgagt gtgtatgttt gttttctaaa cgaagcacac 21420
   gtgcaaaata atgtaaaagg gcagagtgaa gtttgtccat gtccctgtat gaaagtgcat 21480
   gggaagtgcc tggggcagaa gtgaaagaga cacggcccca ggtcgtgaga gaagacattg 21540
   aggtgcactc ccggcctgct gtgggaacag tgggttagca tgtcaccggt gcactcccag 21600
   gctgctgtgg caacagtggg ttagcatgtc acccggcttt aggatttggg ggagtttgct 21660
   caggctctga ggcaggtagg ttaaggctaa tttaactctt aatggttgga aggaaagagg 21720
   gaagggccgt ttaggaaggt gaaaaggcag gagtgaaggt ggcaagaaat gattggcatg 21780
   cagttttttc ctaaatcctc ctctggttat aaggaatcaa ctggaaaatt gcagtggcaa 21840
   tagctgttaa gaaaaaaaat aggattcagt tgatgaaata ttaaacatgc aagtgggctg 21900
   gtcttttaac caatctttgt agtattaagg gattaaaaag ttaattcttg gtaattagat 21960
   ccccagggag aacttaattt gcatctatac atgataagtg tttactatta ataattcaag 22020
   acaagttttg ctttggccgg tattgagctg ggtctcaggc tggtgataac agaggctgga 22080
   tagagagtga taccattagc ctaattttgt catctgcaag caaaggaatg ttaaaatctg 22140
   ttcacttcgt ggtttctaaa tgacttctag aagtatcatg agtggaagag ggcagtgtag 22200
   tgtagtaact tttgttcttc cgcagtcctt agaacctgtc aggattattg gttatgacac 22260
   tgttcccaca gggtggacaa caggggcatt tttatcttct tgagacatat gggaaactta 22320
   agaaggaaag taactaggct gtttgccaaa gattacacct acctccaaac aaaaactgag 22380
   gcaggtataa ttttaactgt gctggaatct ctgaagatgt tggtgtcttg gccaaattca 22440
   tttggactct ttctgcattt cactctttga gatctattgt gtagactcta atttctttga 22500
   caggtattgt cacattggtc tccacaaaaa ttattattga aagccaggca tggtggctca 22560
   cgcctgtaat cccagcactt tgggaggctg aggcgggtgg atcacaaggt caagagatca 22620
   agaccatcct ggccaacatg gtgaaaccct gtctctacta aaaatacgaa aattagctgg 22680
   gcttggtggt gtgcacctgt agtcccagct gcttgggagg ctgaggcagg agaatagctt 22740
   gaacctggga ggtggaggtt gcaatgagct gagaacacgc cactgcactc cagcctggtg 22800
   acagagttaa actctatcca aaaataaaaa taaaaaataa aaaattatta ttgaaataac 22860
   tacatttttt ttcttttttt tttggagaga aagtcttgct ctgttgccca gctgaagtgc 22920
   agaggtgcaa tcatggctca ttatagcctc aacctcctgg gctcaagtga tcctccctag 22980
   tagctgagac cacaggtgca cacttggctt tttttaaaat tttttttggt agagaaggag 23040
   tttcattatg ttgccagact ggtctccaat tcctaggctc aagtgatctc ccacctcagc 23100
   ctcccaaatt tctagaatta aagatgtgag ccactgcact tgccctaaat aactacattt 23160
   ttttgaatgc tgcgatgtgc ctagctttgt gctaagctct ttgaatgtgt tacctgattc 23220
   agtccttggg aaaaccctag gaagaagggg ccacctgatt tctagatggg aaccctgagg 23280
   cttagcaaga aaaagagagc cggtgtggta gggtcaggac tggagcccgg tttatactcc 23340
   tactgacagg atggggatgc ctgtttcacc tcacccttgt taaaaaggac actttagcaa 23400
   tccttttaag tatttgccag cttaagaggt aaaaatgtga tttagcttct ggttcttatg 23460
   cgtccgtctt gggtagcatt ggacatcttg gcatggttat tggctgtttg cacttacagt 23520
   tttaggaatt gcctcttcac atcctctgcc ccatttttac tggattgttc atacttttaa 23580
   aaattgattt ctgtgtatta taaatagtaa ccctttgtca tgtgttacaa gtcctcttct 23640
   ccctcagttt ctttctttca attttctgct ttgtattttt tttttttttt ttgccaaata 23700
   gaactttaaa tttcttaaat tgcaaatctg tgaactttgg atgcctttag agtttattat 23760
   ccctttaaaa aaattttttt aagagatggg gcctcagtat attgtccagg tggtcttgaa 23820
   ctcctggact caagcagtcc tcctgcctca gcctcccaaa gtgttggtat tacagacatg 23880
   agctactgta cccggccaag ttttattatc cttatcttat cctaggagta taatattttt 23940
   ctgtgttttc ttattgttct ataatttcct ttgtaacagg tatctgagat tatctttgaa 24000
   atcttctgtc tcttccaggg tttaaaattt tttagcaaat tattatttca tggtttctta 24060
   aatttccttt gtttcaatga ttaaagcatt attgaggaaa taagctatat tttattaatc 24120
   acaagtctct gcatacacta gaaaaattct agtataatta tttggaagac ataatgatgt 24180
   agttttcaga gtcctcagat tagaagccac tgttttaaaa gcgtgaggtt agaaaaggaa 24240
   ttttgcacct attccaggtt aatttctacc tattccaggt taacagaggt gatcctgtac 24300
   caatgggttt tggacagagc tctcatagtc tgtgctgtgg gcgattccct catttagttt 24360
   gcgaggatac cagaattaaa ggaaaataat gagaaatgct tatagctcat agatagacgc 24420
   gaggtcacag acagccagac tggagctagg agaccatgct gatgtcacat actgccctat 24480
   cagaaccctg ggaaaccctt ggtctgtgct acaagagatt ggcccatgat agccagttat 24540
   aaccaggcag tgggcatctc aggggtttgc ataggtctaa ccttaaaaag ccacatgact 24600
   gggtgcggtg acacacactt gtaatcccag cactttggga ggccaagaca caaggatcac 24660
   ctgaacctgg gagttggaga ccagctctgg catcacagtg agacctccat ctctattaaa 24720
   aaaaaattat ttttaattag ctgggtatgg tggtgcatgc ctgtagtccc agcaccttgg 24780
   gttgctgagg taggagcatc acttgggcct gagagttcaa ggctgcagta agccatgatt 24840
   gagccactgc acttcagctt gggtgacaga gtgaggtcct gtctcaaagt aaagagtcct 24900
   gttaggaagc cataatgatt ttagggggga tggtttagat cttgaagaaa gaaaaaataa 24960
   tttgaggagt gagcacttga actatcaaac tttctcatct attaggtgcg ttaggttcct 25020
   tacatcttat tctttttttc cttcctttta atatatagct actctccaga gagaccggat 25080
   cttcaaacat tttaccagga agcgccaaag ggctatgcga aggcgagtcc accagatcaa 25140
   tggacacaag ttcatggcca cgtatctgag gcagcccacc tactgctctc actgcaggga 25200
   gtttatctgg taaggggttc ccttacttct gtcctcctca gagcttccat ctaataggct 25260
   tcctctggtt atgtatgact tcagcagaat gcatgattat ttaagaattc tttcaggatc 25320
   tgatttttgc ctcttactct agtatgttga cccagtaatt taaatctcca ttgcaaaaac 25380
   atctaaataa tttacagatc caagttttag ataccatttc tttttaaaat catcttggta 25440
   aagatgctaa tgaatcctaa actggtcaac aagcataaat cagagaaaaa ggctaataag 25500
   tcaataaaca atcagtaaat tggataatca gcccctaagg accaaagaat aatcaacagt 25560
   attgctttaa gaaggcatga atcgcataat gacttatgga aattaatggt tgcgagaaaa 25620
   ctaaacacaa agtagtcttc ggcctcttgg tatgtaacag aagataatag ccttcagtga 25680
   tcatatactg taatctaatc cctaggaatt agtaggaact aagataattc aacattgaga 25740
   aattctagag atttggggca atatcttact agacatacaa ctgaagctta agtctcagtc 25800
   tcagtggcaa atgtaaaata aatttaagca gtagttatct ttagtgataa attaagcata 25860
   cttatttgga aagggaagaa agaaagatgc cctcacacac ccatctcaga aggagaaaac 25920
   acagtttggt agcgtgaaag gagatctata tttttgttgt caactttaat tattttgttt 25980
   agtgaaggca gaagcccttg gatcagagca gtttctgaat gttgatcaat tactttgtat 26040
   tttgttttct gtgattattt tggattttat atcttcaaca gtcagaacct agggctagtt 26100
   agtatttctg acatttttaa tcaagaaaca acgattttga gatgctaaac cagctgtaga 26160
   tatgaagcca acaaatgtgt cataacattt attgtattta gggaaaaaat tgtattactt 26220
   ttttttggga aaaaattagc agccctggcg tctttgagtt cacgtttcct ctttgcatga 26280
   ggctgcactc gacttagcca cagccccacc tctctgtggt ttgctcactt gtgacctaag 26340
   ggtgcttgcc tcttgcagtg tatttcatgt cacacttttc tgtttctttt acatgacctg 26400
   ccaggcccct gtaggcatta gtggtgatgg cccctgacct gaactgacat tcataatgga 26460
   aacttatttg atcatttcac tgactttttt ccaccagaga aacattaact gtacccaacc 26520
   agcctttaac gctccccccc tccacaccca tacactgtca ccctcatact ctctctctca 26580
   cacacactta aaatattggt gtatatatta atatatatgg ctaagagtat ggactctgac 26640
   atccagtctc atcaccaact gggtgtgtga cttagtcaag ttgcgtagcc tgtcaatgtc 26700
   tcttcatttg caaaatagac acaacaatct tattttactt gtataataaa ctttcatata 26760
   gtacttaaag ggccaggcac tattgtgagc attttatatt acaactttta atcttcatta 26820
   gagccctgtg cagtaggaac ggttatcatt agtccctttt atggatgagg aaactgtggc 26880
   tcaaagcatc aggtcatctg gcttcagagt ctgtgctctt aaccatgtta ttttgtgtgc 26940
   tgcctcccgt ggctttttgt gaggattaaa tgaaatggta tatatcagct gcttggttca 27000
   gtgcctggca catcctatgg gcttagttac tgtggtcttt gttaatgaga atgagaatac 27060
   tcctaatgtt accatcatta tcatcaccca tagaatatat ttgggagagg caaagatgga 27120
   agtggtgggc ctgaagggca tattgtggca gcaactctgc ccacctgtgg actccagggg 27180
   tgatatggac acagggacaa gggcaggtcc cctgggacag gcagctgcgg tagactcatc 27240
   tgtgcacttc cacagtgtat ctgtgtccgt ttacttcagc atctgaacat ctttatgtga 27300
   gagagaataa ggtgggaact cttcaggcca aggcttaatc acacgtacag atgaggattt 27360
   attgccactg aggacttagg aaattgccat ggaggtagat ggaatgggaa tggtttttac 27420
   ccacccattt gtatcataca gcacattcta atccagcaca tggttttttc tggtgtctca 27480
   tagtaacaaa caatggctca gaaagcacat gatctttgct tcttccctga aattgtgttt 27540
   ttcataaagg aggagaggat gaaatttgtt ttccttaagg actataagag ggttattgct 27600
   gaaatacttg actgatggta gttttcttct cttcaacagg ggagtgtttg ggaaacaggg 27660
   ttatcagtgc caaggtaagg aaacattttt aaaaccatgt ttcattttgt tcctatgtta 27720
   aaagaaatga ttataccaag agaaaacagg gtatcttccc ttaatattgt gataaataac 27780
   tctctgtagg tcaaaggaaa cccctgtgta gatacaactc tttagtttgg tgagatcttg 27840
   gccatatttt agacaatgta aaggggacgt ttttatttct aaagccagga tacctaataa 27900
   attttcattg tttaaagtct tgtctctgca gtaacatgag agtactaaaa ggtaatgtga 27960
   ttttgtgagc atactcttaa attttgaaga taaacttttg ttttttacaa tcctattgat 28020
   aatagatagg gatgaaaagt agcacactgc tgttttctct ttttccccgt ccagggaaca 28080
   taagctatta tccaagttgt tttttgaaat gttatgattt caaaaaaaaa aaaaaaaagt 28140
   gagaaatgtt caaagttaag ctgtcagtga tcttgtaagt tactacatgt tatatttagt 28200
   tttcacaatc taagatgaaa ccaatttact agtgtttcta gcataattgg aaagaaatac 28260
   aatggcagga atggaagaag agccctcagt caggtggttt agccaggccc atcaggcttg 28320
   cctgtcattt gggttccccc ttaacctttg acatgagtat catcacatag tttatacaca 28380
   tggtacctgt ttcactatgt ggatgattga taaaatagca ccacatatcc tagagcagta 28440
   gcctatgtgt ggataagaaa atctcacctt ctcttctgga agatgtgagg taaaacatta 28500
   cttcccttgg atggactgaa taagacatac tcttggatct aattttgact ttactatgta 28560
   tgaacaattc cattgaactt tcaaagttag gaaaaaacaa atatataatc aatatgacag 28620
   caataattga cctgtatgct gttaatctca gtcccaagtg agaagacagt tcacaagcca 28680
   gaatcgctgt cactttgtgg catgggcctg tgagcacagg aatatgccct gcagtccatg 28740
   gtagcccatc ttatgcacac caagataagt tgagaagctc tggataaaca agattatggt 28800
   tttgagttta aaacatggga tttaagatac atctctattg ttattttatt tctttttctc 28860
   tttgtcctat agtgcctgga tatttgatgg cctgattttc agtgggtcac ctgctatgta 28920
   aagagcaggg aagactatta gttaggagac atgcagacac cactttggtg atgtctccct 28980
   aaggcatata aaaccaaatg cctggttgga gagaggtatg ccattctaac actgatcaaa 29040
   agcaaactgg cataggtatt tcagacagag cagacttcag aaaaaggaaa attatcagga 29100
   ataaagaggg gacaacaata aaggggtcag ttttccaaga agacttaaca tttttatttg 29160
   ttttgtttta tttaaagacc agtgtagtag tacataacaa tttttaacat gtatgtatgt 29220
   acctagcaac aggaatcaaa atacatggga caaactgata gaatcacaag gaaaaatgaa 29280
   caaatccact tctatagttg gagacttcaa tatctcagta attgatagat ccagcaggca 29340
   gggaatcagt agggatatag gtgatctaaa cagcattatc acaactaggt gaaattgacg 29400
   tttatagaat acttcatcca acaacagcag ttttcacatt cttctcgggc tcacagggga 29460
   catttaccaa catagaccac attctgggcc ataaaacata ccttaccaaa tttaaaagaa 29520
   gagaaaccat aaacggtatc ttcttagacc acaatggaat taaactagaa atcaattact 29580
   gtaagatagc tggaaaatcc ccaaatattg gaaggttaaa tgtcacattc taagtaacac 29640
   atgggtcaaa gaagaagact caagagaaca taaaaaatat ttagagtcaa gtgaaaatga 29700
   aaatataact tataaaaata tgtgtgatcc agcaaaagca gtgctcagat gggaatgtat 29760
   atatcattaa atgcatacag taggaaagta gaaagatcta aaaccaagat tcgaaatttc 29820
   caccttagga aactagagaa ggaagataaa tttaagcctc aagcgagcat aagaaaagaa 29880
   ataatacaat tagaacagaa atcagtgaag ttgaaaatag gaaaacaaca gagaaaatcc 29940
   atgaaatcaa atgctgattc tttgaaagga ttaattgata agcctttacc aagctaacca 30000
   ggaaaaaacc aaatacctca aaaaataaag tatttctgtg tgaaggatgg ttttgagatc 30060
   ccctcatata aaaagaccag ttctctgggc actttcaatt tggttctcct gagaattgaa 30120
   tctgatgaaa gctagatttg ttttaaggta tgatgctatt tactgacatg tcacctctct 30180
   ttatgtagtt aatcctaaaa ccttccacat gtcttgaaag atcttaagat gatatacatt 30240
   tttatactgc ttcattgcat gtgtttatcc tttgatgaca agctcaacta tctgcagtgt 30300
   atcacttatc tagatgagaa tgaaaaaaat gttattccga aagttctaaa tatttcaaca 30360
   gttgattaag ccattcagca aacatttatt gaatgcctcc tctgttcaag gcatcatgca 30420
   aggaattctg agggcattac aaggctgggt ggaaggtttg aaatgaaggg gcaggctaag 30480
   ggttgtggga gatcagagga gggcaggtgg tttcagtcta gaagtgggga tggggtagag 30540
   tattttcatg gaagagacat tttctgagtt acatcttgtc ctaaacttaa gatttggtta 30600
   gttgataggc aaaaaggata tcccagggag agaaaagggc atcgatagtt tgttggaagg 30660
   gtccaggaag agcaacccat gtagttttta ctggaacaag tagatcccat agaagaaagg 30720
   tgacaggtag cttggagcag cctctggagg gcctggaacc tgggtgggaa atttgaactt 30780
   tgttgatcgg cagtgaatta ccatggaatg ttttcgatta tgggggcagg actagagtgt 30840
   tagaagagtc ctctggtggt gggtgcagga tggaataaag tcaggaagga agaaataggc 30900
   ccaggacttg ataagggata gtttgagcct gagcaaaggt ctgtgttagg atggtggcga 30960
   aggcaatagg aaggatgggc gcaccagctg ctgtcaaggg ggccagacga gtccagtctt 31020
   gttggcacgg tgcagccctg gttgactctg ctgtaactgg ctggactgtg agcttctgat 31080
   aaatgtataa ttgctggatt taatttccta gtgtgcacct gtgtcgtcca taaacgctgc 31140
   catcatctaa ttgttacagc ctgtacttgc caaaacaata ttaacaaagt ggattcaaag 31200
   gtaagaggat agcagtttgc tgattaaatg tgcgtgtgta ttgtgtatgc tgtgtaccgc 31260
   cgtctctctc cctccctccc tcctttcctc cccctctttt ccttctcccc gtccccaaac 31320
   ctctgctttc ctccccctgc cacctcccct ccacattctt tgaagccaag ggttgtatta 31380
   gaatagaaat gtctctctcc ctttttttaa attatggcat ttatatttct gtatttatgt 31440
   ggcatcatcc ctcctttgtg aggaccccat tagtggttgg ctgggtataa gctatatgga 31500
   ttggttctac ttatttggag agacttttgg cagagtgaaa ggagccctga acttggcatc 31560
   tgacagcctg gatttgaatg ctgaatccag cctctgttag ccatcttctc tatgagctgc 31620
   ggcaaggcct acaatctctc ttttcctcag tcttaaaaca ggatcaatgt ttccctcaca 31680
   cagtttttgt aagttaaaca acatagaatt gatgtgatac atatgttaat aactgtaaat 31740
   tatgtttcct aagtaaaaag attgctggct ttctccagcc tcttaagaaa tttcagggaa 31800
   gatgtctctc tctctctctc tctctctctc tctctctctc tctctctctg tgtgtgtgtg 31860
   tgtgtgtgtg tatgtgtata agcttcaaca tcattcttgc cactcatatt gcattcatct 31920
   gcaataggca tacattttaa attctagagg taaaagcaga tggagtgttg ctgacattta 31980
   gcagtagatt tgcagaggca ttggaagaat gactctggaa cacaatggtt ctttatagta 32040
   cacatatagg attgctaaag agtataccta gaggtataaa ccacagcttt tccaagaaag 32100
   aggccataaa agtttcaaga ggagtaatga ttgccctact ccttgtcatg catggatctg 32160
   ttgttatagc atcaaattgt ggtatggact gcagagtaca tgcctagtgg aattatttgc 32220
   tggaaactga gatttaactc agattgtcag attgcaaatg ccaggcaaag aaaggctgga 32280
   agagctgaat aagtagttac tttaatgtca ctttggaata aattccttct gtggttccat 32340
   ggcctctgag tctcttcttt ttatttttct tcccgtgaca caatgtctgg aagaacttac 32400
   agtgatagca agtatatata gtgtataaaa gacccattta atcctgaagt taaaaggttc 32460
   tgggaaatag atgggggtgt taggggattg gcaaggagag aaatatggcc tgaatatgaa 32520
   agatgaagtg aaaatcttaa tttttatttc tcaaaatggc aaatgatcta atgtgtttcc 32580
   cacatttctg tcttgtcctg ttcattagaa aatgtgttta gtctggttcg taagacctga 32640
   gtaattaatc agggctgagt acagtaaaat aatataccta gctcaggtgt catagtgaca 32700
   ctttgcattt gatgggctcc tattacagtt tttacaaagg acattggtat gacattttag 32760
   ctcttgtccc tttatttcct tttttacaga ttgcagaaca gaggttcggg atcaacatcc 32820
   cacacaagtt cagcatccac aactacaaag tgccaacatt ctgcgatcac tgtggctcac 32880
   tgctctgggg aataatgcga caaggacttc agtgtaaaag tgagatgctg aggtgctggg 32940
   tacccacctc ttcatgggaa cactatgccc taagctttca gaattctgtg gactcagaat 33000
   ctgtcctaga actgatggtt ttagatatgt tgtaaatcaa catcttagtc attttaaact 33060
   tacatgtttc ttgtcaggca tgtaaagaga acttcatctg atgccaacat tttctaatca 33120
   taggacatta agtcaaatgg tatattcagt gatctagctc gctagctgac aaatacagcc 33180
   tagacactgt attttaatag gtgtttggaa gagaaaaaga tgggatctta gtcagaaatg 33240
   gaagatggtt gagtcaggtg agacactaaa aagaaaaaaa taaataaaaa tttcaaagaa 33300
   atggaagtta ggaggaggtg aggaggtgag atggccaaat gagaaggttg ggttaagagt 33360
   aagaaatggc agaatagatc tgatgatctc accagaaatc agcaggaatg gagttgtcac 33420
   agcataaaaa gagctgccct gggtcttgtg aaagagattg gagacagggg tagaatgaag 33480
   gaagataaag ccttgattgt aagcttgcat ctcaccagct gtgcccagtg gagtgagagt 33540
   ttggagcttt ggccctagca aaggctctga gtgggtgaga gctgatccct gtcctgttag 33600
   gagttaggga gctgttggcc ttgtgccatc ttgtatggaa atgtaatgat ctgactgccg 33660
   tggggtgcac ctgaagcctt tcttatcaga cacaccccaa gtcgccagtt gaggagaaca 33720
   atggtgaggt agaattggca tgagtatagc cagacaactt ccattgactg cttggactta 33780
   cagagcctgc ttctgtcaac aaaaaggggc cagtcacttg tctttaagaa gggcacattc 33840
   ttctatctct gtagaacact aataattttt ttctcataaa accaaaacag tagccagctc 33900
   atggataact tgtagggcgt tggtccaggt gcagcttgat cttagcgtga cggtctcacc 33960
   tgggtatggg ttcccagtgg gaaatccctt tctgccttgc attgttgctg atgcgcagag 34020
   ccatgcatgg ggctcatctg cattgtcctc ttccctagtg gcctgagggt agcacacctg 34080
   cagcaatccg gctcttgtga tttttgcctt tatggtcact tggtggtttt gaaaggcttc 34140
   tgtaggctcg tgtgtcttac acctgtcttc ctgagagggg tgggtttccc atgaagctta 34200
   agtctcaggg ctcctctctc tcaagggcac cttccaaagc cctctacttg atttggtgtt 34260
   ctttttctta aagaaggcct cctaaattat gtaaattcag gccccacaaa acctggatct 34320
   gtgtgtgctt ccaagatgga ctgactccat aaaacaggaa tgtgttgctt gtcgcttcag 34380
   tttcacctcc catcagcagg gcccatgtca aaaaactgtc tcagaattta ttccagctga 34440
   taacgcctca gatccttcat tgtccccagg cagcttccgg tgcctcagga gcttagcttt 34500
   ctttgctcgc ctcttcaact gccatttgta ctctgacctt cagaaagttt taaaagttgc 34560
   ctttccccac tatgaagcag ttagaaccac aggctgcaaa tgctgagaac cacaggctca 34620
   caggatgccc atctctgtgc tgtgaggcct gcccaaccac aacaatactc agaaagaaag 34680
   ctgcagatag aggtttttct tgtagaaaca ccatttccat cctgtttgaa cagtgatcta 34740
   catgtggcat ttaaactgct accttttatt tcactgttga gagacagaaa aaaatacagt 34800
   tgacatgatt ctttgttatt taattgcctg tgaattgccg ctacgataaa ggttttaatc 34860
   ttagatcata ctaaggcagc aatgcctgtt acatgtgatt tagatgaatg ggtttcatca 34920
   aatcatggtg gaaaaaatgt atttgaaata tattattctt tagtatgctt gcctttctgc 34980
   ttctgcagaa aacagaaata gataaaatgt caggaactat tactagtgtg agcttaattt 35040
   tatgagaaaa tgatactgtt cagccggtat aattcctctg ttataatctt ttaggctatt 35100
   aaagttctct gttgcagcac atgttgaatt ggttcctttc tgaacatgga ttctgttttc 35160
   cttttccctc tagtatgtaa aatgaatgtg catattcgat gtcaagcgaa cgtggcccct 35220
   aactgtgggg taaatgcggt ggaacttgcc aagaccctgg cagggatggg tctccaaccc 35280
   ggaaatattt ctccaacctc ggtgagactt tgcttttttt ccatgtctcg taatttagaa 35340
   gttgctcaga tgtgatgagc ccaaatgaga gcatgtggcc tcatcaaagt tcctaatctt 35400
   agcaaataca ataaacagac ttattgcctt tctttctttc tttctctttc tttctttctt 35460
   ttttcttttc tttctttctc tttctttctt tcttcctttc tttctttctc tctttctccc 35520
   ttttttttct ctctctctcc tctcctttcc tctcctttcc tttttccttc cttcctccct 35580
   tccccttccc cttccccttc cctgtcttca ttcctttttg agatggggtc ttcctctgtc 35640
   acccaggctg gagtgcagtg gcacaattat ggttctctgc agactcgact gcctaggctc 35700
   aagtgatcct cctacctcag tctcccaagt aggtgggact gcaggtgtgc accactatgt 35760
   cagggtaatt ttttattttt ttttttgtag agatggagtc tcactttgtt gcgcaggctg 35820
   gtctcaaact cctgggctca agcgatcctc tcgctttggc ctcccaaagt gttgggattg 35880
   cacaggtgtg agccactgtg tctggctgaa atagcatttt taatagcaat ttgtttcacg 35940
   gataagattg tatatacagt tatatgacaa gaaataatga gcatgagcaa caagtaataa 36000
   acagaaagca aaagaaggac ttttttcctt tctttccttt tttttttctt ttttcttttt 36060
   tctatttgtt ttttgagaca gagtctcgct ttgtcaccca gcctggagtg cagtgatatt 36120
   cgctcactgc aacctccacc tcccaggttc aagcaattct cctgcctcag cctcccgagc 36180
   agctggaatt acaggtgccc actaccgcgc ctggctgatt tttgtatttt tagtaagacg 36240
   gagtttctcc atgttggcca ggctggtctc aaacttctga tctcaagtga tctgcccacc 36300
   ttggcttccc aaagtgctgg aattacaggc atgagctgcc acgcccagcc tttcttttct 36360
   gttttaaaat cagcttcctg tcactgtggt ctggtgaaag cctaaaaccc aggatcctgg 36420
   gttatgcttt tgtctgggga ccaggcagga caggtgacca gtaactatgg gcgtcagcgt 36480
   cctcacctct aaagcggttc aacttgaaga tctcagaggc tctgcacagc cctggagtcc 36540
   agcagttctg aagttttggt ttatggattg tgaatttatc attcacattc agcaggagac 36600
   aggagtctct ccctctggac tctcaggagt gagtggagtt gtgtcagttg cagtgcacat 36660
   gtttttgtca gcactctccc tgaatgacct ccacctgctg tcatccagtt gagccttggc 36720
   tgcttcccaa gcccatgtct tactgttgcc ttcctggtca gcgtctagag atgggaaata 36780
   gccatgtaag atctgaacgg tgtagcttgg tgtccagttc aggaaaatta tcgactcaac 36840
   taattaatct ctgggtgctg aggaagggaa gtgcaattac tgtgagattc ttgataatcc 36900
   tgaaaatgtc agcaccaacc acctcatttc ctttttactg aaggactcct gcctagtaga 36960
   tcagggactt tcattgacaa tttttttttt tttttgagac ggagtctggc tctgtcgccc 37020
   aggctggagt gcagtggcac aatctcagct cactgcaagc tccgcctccc tggttcacgc 37080
   cattctcctg cctcagcctt ccgagtagct gggactatag gcacccgcca ccacgcccag 37140
   ctaatttttt gtattttttt tttttttagt agagatgggg ggtttcaacg tgttacccag 37200
   gatggtctcg atctcctgac cttgtcatcc tcccgtctcg gcctcccaac gtgctgggat 37260
   tacaggcttg agccaccgcg cctggcctat tgacaattta ttgaattgcc actacgataa 37320
   atgttttaat ccttatatta gatcatacta aatcagcaat gactgttaca tgtgatttag 37380
   atgaatgggt ttcatcaaat catggtgaaa aaatgtattt gaaacctatt atgtaaaaaa 37440
   atgtatttga aatccggtct gtctgtcttg tgggtaggcg ggcaggatgc caatattgtg 37500
   cggggattgc taatgcatgg gacagttatc cctgaagagc agagattaaa gatatcagcc 37560
   tgaactaggg cttctgagat acaatgggaa taagataaga ataggaataa agatcatgca 37620
   gcacgatctt taggaatgga cagtgcactt gtcatttgaa tcaagtatcc ttaatccatg 37680
   acatccggtg cctggctgga tgttagactt ttgatctgtt gtctagtggt agcttatctt 37740
   ataaatgtct caaggtgggc cagctgcaaa agtcaccttg cctttaggta gcaacatcct 37800
   caggtactca gtaggatgga actggaaagc caactgaggc aggcacactt accttccatt 37860
   cattgtgttg aaactagaac tctcatcatg ctggtgagag tgtagattgg tcagtcttat 37920
   tgtagacggt aagttggctg tgtatcagaa gtcttcaaaa ttagcacttt ggcccaagga 37980
   attccatttg caggaagtta tcttaagaaa ctgaataatg ataatgacaa tacagcttaa 38040
   gtttattgaa tgtgccaggc accaatctaa gtgctttaca tgcatttgtt catttaggcc 38100
   tcctaatgac tcatgtggca gatgctttaa ccttctcttt gtcagaagag gaaactgagg 38160
   tagggagagg ctgggtaggt aacttgctca gggtcttagg ttagcttgca gagctgttat 38220
   tgaatccagg cagcttgctt gagagctaga cccttaatta ctctgttttg aagatttgca 38280
   tgaagtgtat tcactccagt gttattcact gtggtgaaaa aggggagatg cctcatcctt 38340
   ctaatggcag gggaattaat accttgtgag atatccatga aatggatatt gaatagccgt 38400
   tagaactcat attcttgaaa gaaatttaaa ggcatgaaaa atacccagga caaatttttg 38460
   aatacaaatg tatgttatga aaccataatt tgattatgtt tttaaaaatt aaccaagtat 38520
   aaaaacgtgt gcgtgtgtgt tatttataca tggatataga tatctttggc agagggctgg 38580
   tgagatttta ggtatttaga tttttattct tcatgctttt gatcattgtt ctaatattca 38640
   cattggactt ttatttctcc tatataattt aaaatattga ttaaaaaacg ttgcttcatt 38700
   cgtgtcctgg tgctgaatga tgcttgaatc tctgagtaga catgagaact gctctctgta 38760
   aaaagtggac actaggtggc gctcgtggaa tgggaccttc ccgtggaaag ccaccgcaga 38820
   aggcacccaa gggaccacac aaggcattgg tgtttccatg cacttccact tgagcgctaa 38880
   ctgtaactcc atggcttaga cgtattttta attgaccttt tacacaaagc agtaagcaca 38940
   gattcagaag ctgatactga gggagtttcg agtggcagaa ccgatcatct gtcaaactga 39000
   gttgatcttt cccccacgtt atactggggg tgagactgct cccagccaat aaggtcttct 39060
   tcgtgcatgg gtgctccatg cttctgctgc aatttctgac ttaatgtgtt cttactcttt 39120
   cagaaactcg tttccagatc gaccctaaga cgacagggaa aggagagcag caaagaagga 39180
   aatgggattg gggttaattc ttccaaccga cttggtatcg acaactttga gttcatccga 39240
   gtgttgggga aggggagttt tgggaaggtg agtcttggct ttaactgttt gggttgaagt 39300
   aagtgtgctc tgtgtatggg gggtgtgtgt gtgtgtgcac gcatgcgcac atactcacat 39360
   ttctcatgtg ccattctttc ttcctgttgt gtgcacacac cctaagaccc ccaagaggac 39420
   tccctcatgc tccctccttt tgctttgcca taggtgatgc ttgcaagagt aaaagaaaca 39480
   ggagacctct atgctgtgaa ggtgctgaag aaggacgtga ttctgcagga tgatgatgtg 39540
   gaatgcacca tgaccgagaa aaggatcctg tctctggccc gcaatcaccc cttcctcact 39600
   cagttgttct gctgctttca gacccccgta agtatgaatc acattcactg caccaacagc 39660
   ctcttttctt acagagctga gacagtaaga tactggagat ttcataaagt tgagtatcag 39720
   aaattttggg ggatgggctc ccaaggcagg gtcatatgga ggtattggtg acttctgcca 39780
   acctctggag gaaaaatgga agcttatgaa ttcacccctt ccccttgtgc tgtcctgggg 39840
   aaggagcacc gtgagggttg tgtgtcccag gattgaaaag gtcatcatac ataaatcttc 39900
   tgtaggtgaa gtgaaggtga atgaaagtta cagcagtcta agaacagaga aaatgccatg 39960
   ggctcattgt cttgatgaat ttggctgtga ctctgctcct aaatgctggg ctggttctga 40020
   cctgggcaca ggagtttaga agaggggtct gagaggaccc agagaggtac cttgatttgg 40080
   aacatcccag ggcattgctt ctggccttag caacgaccct ttgcctgaaa ccatggactg 40140
   gtcttaagaa ttgaatctgt ctgccctgta atggcgtgcg tttaggaatg cttctggctg 40200
   cagttaatgg aacacttgac cagcggtgga ttaaacaata aatagaaagg atgagtttgg 40260
   ttcattagta caaacttttt acttgttgtt tcacataatg agaagtctgg tggtaacagt 40320
   gctacggttg gatttgttgc tcagtgacat tggacatgtc tttcccttgg ggtcccaagg 40380
   tatttgctgc agccctagat gccacacctt ggcatgatag catccaaagc agaaagcata 40440
   agggcagggg ctgaagggct tcatggccag tccccttttc tcagggaggg aagatctttc 40500
   ctagacttgg gtcaactgcc ctgtatgtct gttctcacat tgctacaaag aactacctga 40560
   gacggggtaa tttatgaaga aaaaatgttt aattgactca cagttccaca ggccgtacag 40620
   gaggcatggc tggggaggcc tcaagaaact tacagtcata gcggaagggt gaaggagaag 40680
   caagcagggt ttcacgtgac agcaggagag agagagagtg aagggggaaa agctacacac 40740
   tttcaaacag ccagatctca tgagagctca ctatcatgag aagatcaagg ggggaaatct 40800
   gcctcatgat ccaatcacct cccaccaggc ccttccccaa cattagagat tccaattcaa 40860
   catgagatct gggtggggac acacagccaa accatatcat gcccttagca agggaggctg 40920
   gaagagcaaa tatcaggcca agataagctg gcagaccatg attggttcac accaaccttg 40980
   attcagcccc tggagcaggg cattctgctt ttatttagaa tcaggctcaa aattgggctt 41040
   ttggtatcaa tgaagaggga aaatggccct ggagaaggca ccaagaggga gggtctgcca 41100
   cagatgtgtc agaatcttgc tgtcaaaagg cattctggga gagtgaattc tgggcatgtt 41160
   cctggcctgt gccaccatca aggaagtagg ggattccacg agagcgggat aggggcatct 41220
   gactggtcag ggaggctggc aatacacctc tagggaaggt aaagccatgc tgggaccaaa 41280
   cgagtgaatt agaattagtt aggcagagaa tgaaggtgaa tatgtcatac agggtcaaca 41340
   gcacactatg ggtctgctgg tgagacagag catcaggtgt gaaggaggct cagcacacac 41400
   agcatatcga ggttgaacag gggccttgag aactgaggca gccgaggtgg gccagggcca 41460
   ggccatgaag agccttgtag gctgtgttaa ggggtttggg tttagtcctg agagcaatgg 41520
   aaagtcatgg aagaagtgga agcagaggaa tgacgtatca ggttgtatgt tttaattatg 41580
   aaaatctaat ttcttgcgat aggtaatata agtatcctcc aaatattcat atcctttctc 41640
   cgaaagaaac aagtattgct cgttttattt atagcctttg agagaatttt ctgtacataa 41700
   aagcatatat atacagcaca tatatgcata catgtttatc ttttaaaaac acaaataata 41760
   gtatactttg cccatgattc tgtattttta aaatttaatt aatctatttt ttgagacagg 41820
   gtctcacttt gtcaccaggc tggagtgcag tgacggttat ggctcactgc agcctcaagc 41880
   tcccaggcta aagctatcct cccacctcag ccccctgaga aactgggact tacaggcaca 41940
   tgccaccaca cctggctaat ttttgttatt tttttgtaga gatggggttt tgccatgttt 42000
   cccaggctgg tctcaaactc ctgggctaaa gcaatccacc cacctcagtc actcaaagtt 42060
   ctgggattac aggcgtgagc cactgtacct ggccacttta tttttttaag acataatatt 42120
   aatagtatat attggaaatt gttttgtatt tctatatacc aagctgcctc atttttaatg 42180
   gctgcatagt actccatttt agaacatgac ataattaatt taacttttcc tcctctgttg 42240
   ctatgtattg tgttcctagt cttttatttc agtcattgtt gcagtgagtc cccttgtact 42300
   tttgtctttg tgtgcttatg ctggtgtttc tgtatgacac attattagta gtagtattgc 42360
   ttggtcaaag ggtatatgca tttcacattt tgattgagtt caattgcccc ctcccccatt 42420
   gtggttaaag atagtgaacg ttttattgtc catgtaccac acattgttct aagtgcttta 42480
   tgtctgttat ttaatcctta tagtaaccct ctgaaataag ttctgttatt atcaccccat 42540
   ttcacagagg agaaaagtta ggtacaaaga agcttagttc ctaagtggca gagagcgttt 42600
   gaagccaggt ggtctgacgg cagagtccag gcaggctctt cctatttgct gggctgcgtc 42660
   tctaaattca cactcccaaa gcttgagtga gaaggtgcat ttccccgcct tcccatcatg 42720
   gcatgctctc tgtcatttgg atcatctcag agttagaaaa atggtatatt tgaagttcca 42780
   acttgcattc ctctaactgt gagtgaagct gggcgttttc atacatttca gggtcactag 42840
   tattcaaatc tgtgttggga gggccacatg cagcctggag aatgggtgga gcagagaagt 42900
   ctgggagctt ggtgacagga cgcactgaag gagatacatg gcttggactc ccacgccacc 42960
   ccagcgttat caagagaaaa aaaaatgggg aggcagctaa tggcttcgtg ggcctcggct 43020
   cccctgcgcg cctctccctt cccctctgct cctcattggt cctcagtggc tcttgatggc 43080
   cctggagccc atgctgctcc tgcccccatc ctctgctttc cctctgctgg attggtcctt 43140
   ggttcccctg gagctccagt tctccctgcc ccacactgct ccctggagta gccttgaaac 43200
   gacttccttg gctgggcact ttgccaacgt aaagcccctc ccctgccctc acccccttgt 43260
   tgggctcttt tcttggaata gaggagtgga gttacagctt gaggcttgag agggatagct 43320
   ggtagctgat actggttgcc cgccggggtt tcaagcgcca gcctcctgaa caccaccatg 43380
   aacaccatga gtcaagccgc agagccaggt gacctctcag atcttcttcc tagggcctga 43440
   gccaaacatc acatggtttc ctatgacatg aggttagatc atcttcacca ggtggtaacc 43500
   tttgagtcaa gcagtcaact gatatttagt gagcttacct ggtcttgcag cagcaatggt 43560
   tccaagttac tactgctgaa gcaaagaaag aagtatctca tacctaacag gggaatttga 43620
   tctgtaaaag gaaattctgg tgattataag caaagatctc atcaacagat catgttatgg 43680
   tagagtaaaa tgaggaagta acctggaaca gattgggaac ccaatcaata tagcatatgc 43740
   aatcaaaatg ttccaggaaa atgtataaat tctacagtat accaaaattt tagcggccac 43800
   atgagattcc cagtgtttag tgggtgaagc ctttactagc attgaagtct atttttccta 43860
   gtggaagttt tatgtttgac cacaaggtgg cagtcattac cgcaaagtta cttttatttc 43920
   tccaccagag aaaccaaagg catggaactg ccattgcggt ttaagatgtg tgtgttgtag 43980
   tagatgtctc caaagcaaag gataaaggga atgtacccct gctttaggct aaatgataaa 44040
   gaagtagtgg gaacccactt caaagaagca agtgaggctg ggcgcaggca gcactttggg 44100
   aggccgaggt ggggcaatca cttgagccta agtgtttgag accagcctgg ccaacgtggt 44160
   gaaaccccgt ctactaaaaa tacaaaaagt agcttggtgt ggtggtgcat gcctgcaatt 44220
   ccacctactc cagaggctga gacaggagga tcgctggaac ccgggaggca gaggttgcag 44280
   tgagccaaga tcgtgccatt gcactccaac ctgggtgaca gagcacgact ttttctcaaa 44340
   aaagaaaaga aaaaaaagca gcaaaggaga ttttctaatg ctgagataga ggtatacaat 44400
   tttagttttg ctgtcgttgt tgccttcttt gaggtttgca aggtatcgga ctccacagta 44460
   cacatagcat ttgctcgctg tgcattcgtc tgttggaagt ccttatttac tgttgtaaaa 44520
   ctatccccat ttactgttgg aaaactaact ataaagaaca gaaagtgtta cttgctatga 44580
   gaagttacag cagaaagatg cttacttttt agctgcccag tgttgcaggc cctggcttct 44640
   caagtaagat gctaatcagt gtgtaatgct gaactgtgac agtctcccct ggagaattta 44700
   ggagtgagag ggggtgttgg agccactcta cctgcaaagc atttcattgt gagggccttt 44760
   gtaaaaacag cactctcctc atgcagccct gtggaatcag tctctctgaa gcccagagca 44820
   gggcagtaac tagccgagga tcccttagat tgtaagtggc agagacaaag ttcaagttta 44880
   ggtcaggctg actctgactc catatgttgt cttattccgc atttagttgg ttgattcagc 44940
   ctcggcttaa gcacctccta tctgtcttgg ctcccctcca acctgacaat atggaaatgg 45000
   cattgccctt gggaacagaa aacctgaact gtgttttagc tttgttttcc accatctaaa 45060
   tggcttgagt ctgtcctaaa attcctttga cccttaaagt tcttatcatt aaaatgaaaa 45120
   caggatcgct gtgagggtca aatgagagaa tgcagaatat tttgtacagt gcgaagtgct 45180
   gttcaaaagt gagtcagttc aactgtggtg atgaccccct ccatcctcac accaaaggaa 45240
   aaatgtccca gggcttccca atggccagcc cagacaccca catggagggg acagcaaaca 45300
   ttgagaaggt aagtatttta taggtccttt tgttcttggc aaaaaggaaa tagtaagcaa 45360
   atttcaaata tttgataatt tatcaagatt gctacaaagg tttatgaacc catagattat 45420
   ttcccctttt tttttataat catatacact gcttacaagt atataaatgg gcatccttat 45480
   ctgtttctga tagcactgca aatcctaata gtttgtgact aatttgataa cctgtactaa 45540
   aactcacaaa atgtatactc ctggatctgt ttttctgata atctggacta caaaaataat 45600
   ataaagaata aaatgttatt cataaagatg tttaattttc agtgctttgt ttatatatat 45660
   ttttttggtc aacatctctc taatcctcca gcctctggta accactattc tattctgttt 45720
   ctatgagttt ggctttattt gattacacat atcagtgaga ttaagctgca tttgtctttc 45780
   tgtgactggc ttatttcaaa taatacaatg tcctccaggt tgatccatgt tgcaaatgac 45840
   aggatgtctt tctttttaaa agctgaacag tattctattg tgtatatatt ccgttgtttc 45900
   tttgtccatt catccactgg tgaacactta ggttgattcc atatcttggc tattgtgagt 45960
   aatgctgcag tgaacatgag agtgcaggca tcttttcgac atgctgattt catttctttt 46020
   ggatgtatat ccagtattgg aattgctgga gcatatggta gttctatttt aattttttga 46080
   ggagcctcca taccgttttc caaatgagta tactaattta cattcccacc aacagtgtac 46140
   aagggttccc ttttctccac atcctcttca acacctgtta tttttcatct tttcaataat 46200
   agctgttctg acaggtatga cataatatct cattgtggtt ttaatctgca tatccccaat 46260
   gattagtgat attgagcatt ttttctatac ctgttggcca tttgtgtgtg tggttttgtt 46320
   ttgttttttt ttttactttt catttatttt tactttaagt tctgggatac atgtgctgaa 46380
   cttgcaggtt tgttacatag gtgtacatgt gccatggtgg tctgctgcac ctatcaactg 46440
   gtcatccagg ttttaagccc cgaatgcatt aggtatttgt cctaatgctc tccttgccct 46500
   tgccctccca cccaacaggc tctggcatgt gatgttcccc tccctgtgtt ctcattgttc 46560
   aacttatgag tgagaacatg tggtgtttga ttttctgttc ctgtgttagt ttgctgagaa 46620
   tgatggtttc cagcttcatc catgtccctg caaaggacat gagctcattc ttttttatgg 46680
   ctgcatagta ttccatggtg tatatgtgct acattttctt tatccagtct gtcattgatg 46740
   ggcatttgtg ttggttccaa gtctttggta ttgtaaatag tgctgcaata aacatacatg 46800
   tgcatgtgtc tttatagtag aatgatttat taatcctttg ggtatatacc cagtaatggg 46860
   attgctgggt caaatggtat ttctggttct agatccttga ggagtcgcca catggttttc 46920
   cacaatggtt gaactaattt acattcccac cacctgttgg ccatttgtat gtcttctttt 46980
   gagaaatgtc tattcaggtc cattaaccat tttaaaattg ggttgtttct tactattaag 47040
   ttgtttgagt ttcctctgta tttggatatt aactccttat ccagatatgt gttttacaaa 47100
   tattttctcc cattccacag gttgtctctt cactctgttg attgcagaaa ctttgcagaa 47160
   gctttttagt ttgatatagt tccatttgtc tatttttgct tttgttgcct gtgcttttac 47220
   ggtcatattt taaaaagcca ttgcctatgc ctgtgtcaca gatttcttcc cctaagtcat 47280
   catttggtaa ttttacagtt ttaggtctta catttaagtc tttagtccat ttttcagtga 47340
   tttttttatg tgacgtgaga tgagggtctg tttttattct tctgcatgta gatatccagt 47400
   tttttcaaca ccatttattg aagggacagt cctttctttg ttgtgtgtcc ttgggatctt 47460
   tgttgataat taattgattg taaaggtgtg gatttatttc tgagctctgt attctgtccc 47520
   attggtctat atatctgttt ttatgccagt accataatgt tttgattact gtagctttgt 47580
   agtagatttt gaaatcaggt agtataatgc ctccagcttt gttctttttg gtcaagattg 47640
   ctttgatgtc gtgcattatt tatagtagga aaatattaga aacaacttca gcatttagca 47700
   atgggggtac acttcaataa tatactattt gatgagaatt ttcagccagt caagagataa 47760
   ttttatgtta cgtagttaaa ttgtcaggta ccgtcgtaag cacctaacat gtattgttta 47820
   ttgcacgtga ttcctataac agccttataa gataggtgca attattatcc tcattatgtc 47880
   aaatggaaaa gtgagacact gagcagttaa gtcatttgcc taaaatctat cacagaaaat 47940
   ggtaaaaata ggaggttaga atccaggaaa taatacagag gttatgatga tgaacagtct 48000
   gtatccaagt caggttctgc ccaagatagt gaattggcag acccgttccc ctccccagcc 48060
   tcaaaaaaga ctgccaggaa attatgccag taagctatca gggactggat taaggtagtg 48120
   gaattatgaa ggattttttt cctatttacc aaatttggta ataggattac atttcttcaa 48180
   caatgggggt aggaggaata agccattatt aagtcaggga ccagacccct ggtctctcag 48240
   aacctgggat acggcctagt acatgctgtc tccctgactc atttatggcg aaattcacat 48300
   cacactcctt ttatctgaat cagtgagggt tgtgtcagca gctgagtccc tggcatggat 48360
   gtgctggaat ccaggcctcc catcccccat ccccggctcg caggggcctc cccttcggtt 48420
   ctcctcctgg agagagagga acaaagacta gggggtgagg gatggagggg ggctatcaca 48480
   ggaaggggga gcagcaaagt aaatattggt ctgaggcttt gttcggagac ccagtttatg 48540
   gaagcaaagg tttccatttg gaaaacagaa gacacttgtg gccagtgccc gaaacagcct 48600
   ctcactagca ggccctggga ctgggtgaga gggctgagcg aagtactcct cattgttttg 48660
   ggaatgcctt aagtttgagt ttaggatcca ccgctttcag gtgggccaca ggacgctccg 48720
   atgccggtcg cttttgcctc ctcccgctcc tctgcctgct ggggcgtctt tacgatgtca 48780
   gcctgcaagg atggagttcc tggcaggacc atagctttcc gtaagaaacc acttctcaaa 48840
   tggccaacaa agttgaaatc ctggtttttg aaggtcagaa tatcttggaa tcttgtgaaa 48900
   taaatgccat cgctgcatac atgctggcac aaaatgatct cacgtgtgct cagtgtacct 48960
   gcagcttgag tgcaaaggaa cattctttcc tttgtattac tttctccatt gttgttgtat 49020
   taaatattag cgaagaagtg ggagaggcag agagcagcca ggcagcaagg tagtcatgtg 49080
   gccctggaat tcatctttca gaattttccc aggcagggaa ctgcactgtg accttcagca 49140
   caagagctct gagttctaat tctggcttag ctggcccact tgtctttaag aagttcccct 49200
   gccttttatt ttgagataag cctctccacc taaatgtgtg ttccttactt ctggacctgt 49260
   tggccaacgt acctttaggg agtgcccctc cagctggctt caacatgtgg ggtcgtagcg 49320
   gggctgcagc ctgccctgtc cagctctgat gcttcccagc gcctccagct cccctttccc 49380
   ttcctgaggc ctcactgagc atttggctga gcccctgcca gccgctggat gtggggagga 49440
   gaaggcatgc tcagacccta cagtggcatt actgcttgtg ctctgtgttg ctgtgacacc 49500
   cagcgcaagg ggaagaggcc ctgtgacaga gggttagaca aggagaaggg gaagtatagc 49560
   cgaaaggctg ggagactttc ccctttacag gcatgggaat tctgaagaag cctctgttca 49620
   tgccttgggg tagttggttg attcctgggg actgaatggt gcccactttg caggagggga 49680
   tgagcgaaga aggaaagggc agttcatgag accctagcat atgtcctagg tgctcatatt 49740
   cattaccaat gtctatgaaa ctaaactgca cattggcatt ccgtaaatat ccattgaaca 49800
   aaatatctca tttaataccc aaagcatcca gggaagagta tcatcctcat tttaaaggcc 49860
   tagaaactga gatccagaga aattaaacaa cttgccccag atcacacagt aagccagagt 49920
   cagtgcatgg attcctgacc tgactgtcct gccaaataat atacaagcca tttccattat 49980
   atcacaaata gaagttttga gtctctaata tgtacaactg agaatactgt cattagacgg 50040
   gaatagaggc agttttccac aataggtggt ttttggcctt ttgggaaata cctaaggata 50100
   tttcctctga tctgtttgga accaggtttc cccctttccc tgagcagtgg ctgctacaaa 50160
   tacaatctgg tttgctgcgg gtcatttgtt gcagtttgcc tttggctcag atcaaaatag 50220
   aaaatgagcc agaacatggt gtttcctgtc tctgcatgtt cctaccttat tcatagtgtt 50280
   ccatcttttt tttaggttat taattgtgga tttatttcca tagtaagaca ggatctgact 50340
   ttggggccaa catatgttgg acccgcctcc ttctctgctg aagattctct gaagacaaaa 50400
   tgtgcatagg ccttctccag gatctggatt tttgggggat tatttgggac ctttgcttct 50460
   gtcctctccg gctaccaggc tttctgacct tctgtagcta aatagggcca caaaaatgaa 50520
   taggaataag agaacctgaa gttcaagctg tgtctttggc tctgtctctt tggacgcgac 50580
   tgccaagata tttggtgtga caaaaggatt tgaaatagtt ggcctgtttt tttcagtttg 50640
   aggttgttct gttgtaactg gtcttgtttt tggtagccat cattatcact gacaacctag 50700
   agactgagta gaccgccacc tccatgtctg ctaagatagt ataatttaaa tctttgatgt 50760
   aaccagaaca caacccaggt cacactttgc ccctgtgtct catgatgact gaaagctttt 50820
   ggggtgatga ttgcttggtt atggttttcc tggacccaac ttggaaccct taaagcctgc 50880
   caagttatag atactctggc ttcagtaggg ccctgtattg aaacaccaag tgccaggact 50940
   gctcaaaagg tgaaaatgag atgggtgttc tgccaggtgg tttagtgaaa aatcagtcaa 51000
   tccaggatcc cactgactga atgctaggca aaagaaacag ttcttttatt tgttttcatt 51060
   ttttgcagag ttggaggtct tgctatgttg cccacactgg tcttgaactc ctgacctcaa 51120
   gcaatcctac tgccttggcc tcccaaagta ttaggattac aggcatgagc caccgtgcct 51180
   ggctagaaac agttttttaa aaactcattc taattaggaa gagaagacca atacacatgg 51240
   aagaaaagtg tacagtagat agcatgtagt aaatatagtc cttatgctac aaaaggaaag 51300
   gtagttgaaa atgaacattg ggttctaggg gaaactctta agtatgtgcc agtctgtgct 51360
   cagaagtaca acctgattgg ctgtgagact aaagccaact gattgcttta agcagcccct 51420
   cgggtgtggg ctgcagatgg ttccctagga agctgggttg agtcctgctg tgatgagctg 51480
   ggactgccag gtaggacctg aagcggctgc caaggggctg ccaccccacc ctgaagacaa 51540
   aatcatacag tctaaaaacc tgactgcttt ggatcaaaat cataccctgt aatatgttct 51600
   agtttttaaa taacttgggt tttccttaat tacttaatct ctgctgagaa cccagctgca 51660
   tccatctctt ctaggtccgg aattatccac agcatctcat gagcagaaac agccatgtca 51720
   acataaagtt agcagactcg tcgtgtagag agttgtgcac tggcaggtta gggggaaggg 51780
   tgttcattca ttcagcagat atgcactgag tgcttgcaag gcatagggct cgccttcaag 51840
   ccttgaggac gcagcagtga gcaaaacaga aaagagtccc tgccctcatg aaactgacat 51900
   tctttgggaa gcttataagc ctgaagctca tgaacagatt gttcatctta tagtctcagc 51960
   tggtgaccct ccatggcagc tgctccctgt ggcaattcct ggattttcct ttcagacagg 52020
   ctcagagccg 52030

<210> 3
<211> 3847
<212> DNA
<213> Homo sapiens

<220>
<221> CDS

<222>  (436)..(1578)

<400>  3
caaaaccaca gataatgttc agcccagcac agtaggggtc aatttggtcc acttgctcag      60

tgacaaaaag aaaaaaaaag tgggctgtca ctaaagattt tgactcacaa gagaggggct     120

ggtctggagg tgggaggagg gagtgacgag tcaaggagga gacagggacg caggagggtg     180

caaggaagtg tcttaactga gacgggggta aggcaagaga gggtggagga aattctgcag     240

gagacaggct tcctccaggg tctggagaac ccagaggcag ctcctcctga gtgctgggaa     300

ggactctggg catcttcagc ccttcttact ctctgaggct caagccagaa attcaggctg     360

cttgcagagt gggtgacaga gccacggagc tggtgtccct gggaccctct gcccgtcttc     420

tctccactcc ccagc atg gag gaa ggt ggt gat ttt gac aac tac tat ggg     471
                 Met Glu Glu Gly Gly Asp Phe Asp Asn Tyr Tyr Gly
                 1               5                   10

gca gac aac cag tct gag tgt gag tac aca gac tgg aaa tcc tcg ggg     519
Ala Asp Asn Gln Ser Glu Cys Glu Tyr Thr Asp Trp Lys Ser Ser Gly
        15                  20                  25

gcc ctc atc cct gcc atc tac atg ttg gtc ttc ctc ctg ggc acc acg     567
Ala Leu Ile Pro Ala Ile Tyr Met Leu Val Phe Leu Leu Gly Thr Thr
    30                  35                  40

ggc aac ggt ctg gtg ctc tgg acc gtg ttt cgg agc agc cgg gag aag     615
Gly Asn Gly Leu Val Leu Trp Thr Val Phe Arg Ser Ser Arg Glu Lys
45                  50                  55                  60

agg cgc tca gct gat atc ttc att gct agc ctg gcg gtg gct gac ctg     663
Arg Arg Ser Ala Asp Ile Phe Ile Ala Ser Leu Ala Val Ala Asp Leu
                65                  70                  75

acc ttc gtg gtg acg ctg ccc ctg tgg gct acc tac acg tac cgg gac     711
Thr Phe Val Val Thr Leu Pro Leu Trp Ala Thr Tyr Thr Tyr Arg Asp
            80                  85                  90

tat gac tgg ccc ttt ggg acc ttc ttc tgc aag ctc agc agc tac ctc     759
Tyr Asp Trp Pro Phe Gly Thr Phe Phe Cys Lys Leu Ser Ser Tyr Leu
        95                  100                 105

atc ttc gtc aac atg tac gcc agc gtc ttc tgc ctc acc ggc ctc agc     807
Ile Phe Val Asn Met Tyr Ala Ser Val Phe Cys Leu Thr Gly Leu Ser
    110                 115                 120

ttc gac cgc tac ctg gcc atc gtg agg cca gtg gcc aat gct cgg ctg     855
Phe Asp Arg Tyr Leu Ala Ile Val Arg Pro Val Ala Asn Ala Arg Leu
125                 130                 135                 140

agg ctg cgg gtc agc ggg gcc gtg gcc acg gca gtt ctt tgg gtg ctg     903
Arg Leu Arg Val Ser Gly Ala Val Ala Thr Ala Val Leu Trp Val Leu
                145                 150                 155

gcc gcc ctc ctg gcc atg cct gtc atg gtg tta cgc acc acc ggg gac     951
Ala Ala Leu Leu Ala Met Pro Val Met Val Leu Arg Thr Thr Gly Asp
            160                 165                 170

ttg gag aac acc act aag gtg cag tgc tac atg gac tac tcc atg gtg     999
Leu Glu Asn Thr Thr Lys Val Gln Cys Tyr Met Asp Tyr Ser Met Val
        175                 180                 185

gcc act gtg agc tca gag tgg gcc tgg gag gtg ggc ctt ggg gtc tcg    1047
Ala Thr Val Ser Ser Glu Trp Ala Trp Glu Val Gly Leu Gly Val Ser

190                 195                 200

tcc acc acc gtg ggc ttt gtg gtg ccc ttc acc atc atg ctg acc tgt     1095
Ser Thr Thr Val Gly Phe Val Val Pro Phe Thr Ile Met Leu Thr Cys
205                 210                 215                 220

tac ttc ttc atc gcc caa acc atc gct ggc cac ttc cgc aag gaa cgc     1143
Tyr Phe Phe Ile Ala Gln Thr Ile Ala Gly His Phe Arg Lys Glu Arg
                225                 230                 235

atc gag ggc ctg cgg aag cgg cgc cgg ctg ctc agc atc atc gtg gtg     1191
Ile Glu Gly Leu Arg Lys Arg Arg Arg Leu Leu Ser Ile Ile Val Val
            240                 245                 250

ctg gtg gtg acc ttt gcc ctg tgc tgg atg ccc tac cac ctg gtg aag     1239
Leu Val Val Thr Phe Ala Leu Cys Trp Met Pro Tyr His Leu Val Lys
        255                 260                 265

acg ctg tac atg ctg ggc agc ctg ctg cac tgg ccc tgt gac ttt gac     1287
Thr Leu Tyr Met Leu Gly Ser Leu Leu His Trp Pro Cys Asp Phe Asp
    270                 275                 280

ctc ttc ctc atg aac atc ttc ccc tac tgc acc tgc atc agc tac gtc     1335
Leu Phe Leu Met Asn Ile Phe Pro Tyr Cys Thr Cys Ile Ser Tyr Val
285                 290                 295                 300

aac agc tgc ctc aac ccc ttc ctc tat gcc ttt ttc gac ccc cgc ttc     1383
Asn Ser Cys Leu Asn Pro Phe Leu Tyr Ala Phe Phe Asp Pro Arg Phe
                305                 310                 315

cgc cag gcc tgc acc tcc atg ctc tgc tgt ggc cag agc agg tgc gca     1431
Arg Gln Ala Cys Thr Ser Met Leu Cys Cys Gly Gln Ser Arg Cys Ala
            320                 325                 330

ggc acc tcc cac agc agc agt ggg gag aag tca gcc agc tac tct tcg     1479
Gly Thr Ser His Ser Ser Ser Gly Glu Lys Ser Ala Ser Tyr Ser Ser
        335                 340                 345

ggg cac agc cag ggg ccc ggc ccc aac atg ggc aag ggt gga gaa cag     1527
Gly His Ser Gln Gly Pro Gly Pro Asn Met Gly Lys Gly Gly Glu Gln
    350                 355                 360

atg cac gag aaa tcc atc ccc tac agc cag gag acc ctt gtg gtt gac     1575
Met His Glu Lys Ser Ile Pro Tyr Ser Gln Glu Thr Leu Val Val Asp
365                 370                 375                 380

tag ggctgggagc agagagaagc ctggcgccct cggccctccc cggcctttgc     1628

ccttgctttc tgaaaatcag gtagtgtggc tactccttgt cctatgcaca tcctttaact     1688

gtcccctgat tctgccccgc cctgtcctcc tctactgctt tattctttct cagaggtttg     1748

tggtttaggg gaaagagact gggctctaca gacctgaccc tgcacaagcc atttaatctc     1808

actcagcctc agtttctcca ttggtatgaa atgggggaaa gtcatattga tcctaaaatg     1868

ttgaagcctg agtctggacg cagtaaaagc ttgtttccct ctgctgcttt cttagatctg     1928

caatcgtctt tcctcccttc tttccttgta gtttttcccc caccactctc tgcagctgcc     1988

gctccttatc cctgccttct ggcaccaatc ccctcctaca gctcgtcccc ctccctccat     2048

ccatccttct cccctgtcta ctttcttgtt ctgaagggct actaagggtt aaggatccca     2108

aagcttgcag agactgaccc tgtttaagct ttctatcctg ttttctgagt gtgaggcagg     2168

gaatgggctg gggccggggg tgggctgtgt gtcagcagat aattagtgct ccagccctta     2228

gatctgggag ctccagagct tgccctaaaa ttggatcact tccctgtcat tttgggcatt 2288

ggggctagtg tgattcctgc agttccccca tggcaccatg acactgacta gatatgcttt 2348

ctccaaattg tccgcagacc ctttcatcct tcctctattt tctatgagaa ttggaaggca 2408

gcagggctga tgaatggatg tactccttgg tttcattatg tgagtgggga gttgggaagg 2468

gcaactagag agagaggatg gaggggtgtc tgcatttagt ccagacactg cttggctcgc 2528

tccccgagtc ctcctgtttc tgacttcctg cataactgtg agctgaaggg tttcctcatc 2588

tccccatctt accccatcat actgatttct ttcttgggca ctggtgctac ttggtgccaa 2648

gaatcatgtt gtttgggatg gagatgcctg cctcttgtct gtgtgtgttg tacttatatg 2708

tctatatgga tgagcctggc atgaacagca gtgtgcctgg gtcatttgga caaatctcct 2768

cccacccccc aatccactgc aactctgctg ttcacacatt acccttggca gggggtggtg 2828

gggggcaggg acacactgag gcaatgaaaa atgtagaata aaaatgagtc caccccctac 2888

tggatttggg ggctccaacg gctggtccgt gctttaggag cgaagttaat gtttgcacca 2948

ggcttcctgt agggagatcc ctccccaaag cagctggcgc caaggcttgg gggcgtccta 3008

ctgagctggg ttcctgctcc ttcttgggct ccatgaagga agtaagaggc tagttgagag 3068

cctcccttgg cccctttccg gtgcctcccc gcctggcttc aaatttatga gcattgccct 3128

catcgtcctt tcttgttcca gggtcagtgg ccctcttcct aaggaggcct cctgcttgcc 3188

atgggccaaa aggcacgggg tgggtttttt ctctccctac cctcaggatt ggacctcttg 3248

gcttctgctg gattggggat ctgggaatag ggactggagc aagtgtgcag atagcatgat 3308

gtctacactg ccagagagac cgtgaggatg aaattaatag tggggccttt gtgagctaga 3368

ggctgggagt gtctattccg ggttttgttc ttggaggact atgaaagtga aggacaagac 3428

atgagcgatg gagataagaa aagcccagct tgatgtgaat ggacatcttg accctccctg 3488

gaatgacgcc agctctgggg gcagagggag gaggagaggg gaaggggctc ctcacagcct 3548

agtctcccca tcttaagata gcatctttca cagagtcacc tcctctgccc agagctgtcc 3608

tcaaagcatc cagtgaacac tggaagaggc ttctagaagg gaagaaattg tccctctgag 3668

gccgccgtgg gtgacctgca gagacttcct gcctggaact catctgtgaa ctgggacaga 3728

agcagaggag gctgcctgct gtgatacccc cttacctccc ccagtgcctt cttcagaata 3788

tctgcactgt cttctgatcc tgttagtcac tgtggttcat caaataaaac tgtttgtgc 3847

<210> 4
<211> 380
<212> PRT
<213> Homo sapiens

<400> 4

Met Glu Glu Gly Gly Asp Phe Asp Asn Tyr Tyr Gly Ala Asp Asn Gln
1               5                   10                  15

Ser Glu Cys Glu Tyr Thr Asp Trp Lys Ser Ser Gly Ala Leu Ile Pro
            20                  25                  30

Ala Ile Tyr Met Leu Val Phe Leu Leu Gly Thr Thr Gly Asn Gly Leu
        35                  40                  45

Val Leu Trp Thr Val Phe Arg Ser Ser Arg Glu Lys Arg Arg Ser Ala
    50                  55                  60

Asp Ile Phe Ile Ala Ser Leu Ala Val Ala Asp Leu Thr Phe Val Val
65                  70                  75                  80

Thr Leu Pro Leu Trp Ala Thr Tyr Thr Tyr Arg Asp Tyr Asp Trp Pro
                85                  90                  95

Phe Gly Thr Phe Phe Cys Lys Leu Ser Ser Tyr Leu Ile Phe Val Asn
            100                 105                 110

Met Tyr Ala Ser Val Phe Cys Leu Thr Gly Leu Ser Phe Asp Arg Tyr
        115                 120                 125

Leu Ala Ile Val Arg Pro Val Ala Asn Ala Arg Leu Arg Leu Arg Val
    130                 135                 140

Ser Gly Ala Val Ala Thr Ala Val Leu Trp Val Leu Ala Ala Leu Leu
145                 150                 155                 160

Ala Met Pro Val Met Val Leu Arg Thr Thr Gly Asp Leu Glu Asn Thr
                165                 170                 175

Thr Lys Val Gln Cys Tyr Met Asp Tyr Ser Met Val Ala Thr Val Ser
            180                 185                 190

Ser Glu Trp Ala Trp Glu Val Gly Leu Gly Val Ser Ser Thr Thr Val
        195                 200                 205

Gly Phe Val Val Pro Phe Thr Ile Met Leu Thr Cys Tyr Phe Phe Ile
    210                 215                 220

Ala Gln Thr Ile Ala Gly His Phe Arg Lys Glu Arg Ile Glu Gly Leu
225                 230                 235                 240

Arg Lys Arg Arg Arg Leu Leu Ser Ile Ile Val Val Leu Val Val Thr
                245                 250                 255

Phe Ala Leu Cys Trp Met Pro Tyr His Leu Val Lys Thr Leu Tyr Met
            260                 265                 270

Leu Gly Ser Leu Leu His Trp Pro Cys Asp Phe Asp Leu Phe Leu Met
        275                 280                 285

Asn Ile Phe Pro Tyr Cys Thr Cys Ile Ser Tyr Val Asn Ser Cys Leu
    290                 295                 300

Asn Pro Phe Leu Tyr Ala Phe Phe Asp Pro Arg Phe Arg Gln Ala Cys
305                 310                 315                 320

Thr Ser Met Leu Cys Cys Gly Gln Ser Arg Cys Ala Gly Thr Ser His
                325                 330                 335

Ser Ser Ser Gly Glu Lys Ser Ala Ser Tyr Ser Ser Gly His Ser Gln
            340                 345                 350

Gly Pro Gly Pro Asn Met Gly Lys Gly Gly Glu Gln Met His Glu Lys
        355                 360                 365

Ser Ile Pro Tyr Ser Gln Glu Thr Leu Val Val Asp
    370                 375                 380

<210>  5
<211>  3522
<212>  DNA
<213>  Homo sapiens

<220>
<221>  CDS
<222>  (306)..(2357)

<400>  5
aggggcgagt cctgcgcgag tccccgggag gcgccgcgcg cttggaaggg acggtcgggc      60

ttccccggcc cgctgagggc tcggcggcgg gctcccctcc tttccacctc gggagggagg     120

gaaggagggg agggaaaagt cccacggagg aggcagaatg gccagtcgag gggcgcttag     180

gcgctgcctt tccccagggc tgcctcgact cctgcacctg tcccgagggc tggcctgaga     240

cgggactccc ggttctcccg ctgcgaagca gcgcggcccc ccggggccgg ggcagcggcg     300

ccggc atg tcg tct ggc acc atg aag ttc aat ggc tat ttg agg gtc cgc     350
      Met Ser Ser Gly Thr Met Lys Phe Asn Gly Tyr Leu Arg Val Arg
      1               5                   10                  15

atc ggt gag gca gtg ggg ctg cag ccc acc cgc tgg tcc ctg cgc cac       398
Ile Gly Glu Ala Val Gly Leu Gln Pro Thr Arg Trp Ser Leu Arg His
                20                  25                  30

tcg ctc ttc aag aag ggc cac cag ctg ctg gac ccc tat ctg acg gtg       446
Ser Leu Phe Lys Lys Gly His Gln Leu Leu Asp Pro Tyr Leu Thr Val
            35                  40                  45

agc gtg gac cag gtg cgc gtg ggc cag acc agc acc aag cag aag acc       494
Ser Val Asp Gln Val Arg Val Gly Gln Thr Ser Thr Lys Gln Lys Thr
        50                  55                  60

aac aaa ccc acg tac aac gag gag ttt tgc gct aac gtc acc gac ggc       542
Asn Lys Pro Thr Tyr Asn Glu Glu Phe Cys Ala Asn Val Thr Asp Gly
    65                  70                  75

ggc cac ctc gag ttg gcc gtc ttc cac gag acg ccc ctg ggc tac gac       590
Gly His Leu Glu Leu Ala Val Phe His Glu Thr Pro Leu Gly Tyr Asp
80                  85                  90                  95

cac ttc gtg gcc aac tgc acc ctg cag ttc cag gag ctg ctg cgc acg 638
His Phe Val Ala Asn Cys Thr Leu Gln Phe Gln Glu Leu Leu Arg Thr
                100                 105                 110

acc ggc gcc tcg gac acc ttc gag ggt tgg gtg gat ctc gag cca gag 686
Thr Gly Ala Ser Asp Thr Phe Glu Gly Trp Val Asp Leu Glu Pro Glu
            115                 120                 125

ggg aaa gta ttt gtg gta ata acc ctt acc ggg agt ttc act gaa gct 734
Gly Lys Val Phe Val Val Ile Thr Leu Thr Gly Ser Phe Thr Glu Ala
        130                 135                 140

act ctc cag aga gac cgg atc ttc aaa cat ttt acc agg aag cgc caa 782
Thr Leu Gln Arg Asp Arg Ile Phe Lys His Phe Thr Arg Lys Arg Gln
    145                 150                 155

agg gct atg cga agg cga gtc cac cag atc aat gga cac aag ttc atg 830
Arg Ala Met Arg Arg Arg Val His Gln Ile Asn Gly His Lys Phe Met
160                 165                 170                 175

gcc acg tat ctg agg cag ccc acc tac tgc tct cac tgc agg gag ttt 878
Ala Thr Tyr Leu Arg Gln Pro Thr Tyr Cys Ser His Cys Arg Glu Phe
                180                 185                 190

atc tgg gga gtg ttt ggg aaa cag ggt tat cag tgc caa gtg tgc acc 926
Ile Trp Gly Val Phe Gly Lys Gln Gly Tyr Gln Cys Gln Val Cys Thr
            195                 200                 205

tgt gtc gtc cat aaa cgc tgc cat cat cta att gtt aca gcc tgt act 974
Cys Val Val His Lys Arg Cys His His Leu Ile Val Thr Ala Cys Thr
        210                 215                 220

tgc caa aac aat att aac aaa gtg gat tca aag att gca gaa cag agg 1022
Cys Gln Asn Asn Ile Asn Lys Val Asp Ser Lys Ile Ala Glu Gln Arg
    225                 230                 235

ttc ggg atc aac atc cca cac aag ttc agc atc cac aac tac aaa gtg 1070
Phe Gly Ile Asn Ile Pro His Lys Phe Ser Ile His Asn Tyr Lys Val
240                 245                 250                 255

cca aca ttc tgc gat cac tgt ggc tca ctg ctc tgg gga ata atg cga 1118
Pro Thr Phe Cys Asp His Cys Gly Ser Leu Leu Trp Gly Ile Met Arg
                260                 265                 270

caa gga ctt cag tgt aaa ata tgt aaa atg aat gtg cat att cga tgt 1166
Gln Gly Leu Gln Cys Lys Ile Cys Lys Met Asn Val His Ile Arg Cys
            275                 280                 285

caa gcg aac gtg gcc cct aac tgt ggg gta aat gcg gtg gaa ctt gcc 1214
Gln Ala Asn Val Ala Pro Asn Cys Gly Val Asn Ala Val Glu Leu Ala
        290                 295                 300

aag acc ctg gca ggg atg ggt ctc caa ccc gga aat att tct cca acc 1262
Lys Thr Leu Ala Gly Met Gly Leu Gln Pro Gly Asn Ile Ser Pro Thr
    305                 310                 315

tcg aaa ctc gtt tcc aga tcg acc cta aga cga cag gga aag gag agc 1310
Ser Lys Leu Val Ser Arg Ser Thr Leu Arg Arg Gln Gly Lys Glu Ser
320                 325                 330                 335

agc aaa gaa gga aat ggg att ggg gtt aat tct tcc aac cga ctt ggt 1358
Ser Lys Glu Gly Asn Gly Ile Gly Val Asn Ser Ser Asn Arg Leu Gly
                340                 345                 350

atc gac aac ttt gag ttc atc cga gtg ttg ggg aag ggg agt ttt ggg 1406
Ile Asp Asn Phe Glu Phe Ile Arg Val Leu Gly Lys Gly Ser Phe Gly
            355                 360                 365

aag gtg atg ctt gca aga gta aaa gaa aca gga gac ctc tat gct gtg 1454
Lys Val Met Leu Ala Arg Val Lys Glu Thr Gly Asp Leu Tyr Ala Val
        370                 375                 380

aag gtg ctg aag aag gac gtg att ctg cag gat gat gat gtg gaa tgc 1502
Lys Val Leu Lys Lys Asp Val Ile Leu Gln Asp Asp Asp Val Glu Cys
    385                 390                 395

acc atg acc gag aaa agg atc ctg tct ctg gcc cgc aat cac ccc ttc 1550
Thr Met Thr Glu Lys Arg Ile Leu Ser Leu Ala Arg Asn His Pro Phe
400                 405                 410                 415

ctc act cag ttg ttc tgc tgc ttt cag acc ccc gat cgt ctg ttt ttt 1598
Leu Thr Gln Leu Phe Cys Cys Phe Gln Thr Pro Asp Arg Leu Phe Phe
                420                 425                 430

gtg atg gag ttt gtg aat ggg ggt gac ttg atg ttc cac att cag aag 1646
Val Met Glu Phe Val Asn Gly Gly Asp Leu Met Phe His Ile Gln Lys
            435                 440                 445

tct cgt cgt ttt gat gaa gca cga gct cgc ttc tat gct gca gaa atc 1694
Ser Arg Arg Phe Asp Glu Ala Arg Ala Arg Phe Tyr Ala Ala Glu Ile
        450                 455                 460

att tcg gct ctc atg ttc ctc cat gat aaa gga atc atc tat aga gat 1742
Ile Ser Ala Leu Met Phe Leu His Asp Lys Gly Ile Ile Tyr Arg Asp
    465                 470                 475

ctg aaa ctg gac aat gtc ctg ttg gac cac gag ggt cac tgt aaa ctg 1790
Leu Lys Leu Asp Asn Val Leu Leu Asp His Glu Gly His Cys Lys Leu
480                 485                 490                 495

gca gac ttc gga atg tgc aag gag ggg att tgc aat ggt gtc acc acg 1838
Ala Asp Phe Gly Met Cys Lys Glu Gly Ile Cys Asn Gly Val Thr Thr
                500                 505                 510

gcc aca ttc tgt ggc acg cca gac tat atc gct cca gag atc ctc cag 1886
Ala Thr Phe Cys Gly Thr Pro Asp Tyr Ile Ala Pro Glu Ile Leu Gln
            515                 520                 525

gaa atg ctg tac ggg cct gca gta gac tgg tgg gca atg ggc gtg ttg 1934
Glu Met Leu Tyr Gly Pro Ala Val Asp Trp Trp Ala Met Gly Val Leu
        530                 535                 540

ctc tat gag atg ctc tgt ggt cac gcg cct ttt gag gca gag aac gaa 1982
Leu Tyr Glu Met Leu Cys Gly His Ala Pro Phe Glu Ala Glu Asn Glu
    545                 550                 555

gat gac ctc ttt gag gcc ata ctg aat gat gag gtg gtc tac cct acc 2030
Asp Asp Leu Phe Glu Ala Ile Leu Asn Asp Glu Val Val Tyr Pro Thr
560                 565                 570                 575

tgg ctc cat gaa gat gcc aca ggg atc cta aaa tct ttc atg acc aag 2078
Trp Leu His Glu Asp Ala Thr Gly Ile Leu Lys Ser Phe Met Thr Lys
                580                 585                 590

aac ccc acc atg cgc ttg ggc agc ctg act cag gga ggc gag cac gcc 2126
Asn Pro Thr Met Arg Leu Gly Ser Leu Thr Gln Gly Gly Glu His Ala
            595                 600                 605

atc ttg aga cat cct ttt ttt aag gaa atc gac tgg gcc cag ctg aac 2174
Ile Leu Arg His Pro Phe Phe Lys Glu Ile Asp Trp Ala Gln Leu Asn
        610                 615                 620

cat cgc caa ata gaa ccg cct ttc aga ccc aga atc aaa tcc cga gaa 2222
His Arg Gln Ile Glu Pro Pro Phe Arg Pro Arg Ile Lys Ser Arg Glu
    625                 630                 635

gat gtc agt aat ttt gac cct gac ttc ata aag gaa gag cca gtt tta     2270
Asp Val Ser Asn Phe Asp Pro Asp Phe Ile Lys Glu Glu Pro Val Leu
640                 645                 650                 655

act cca att gat gag gga cat ctt cca atg att aac cag gat gag ttt     2318
Thr Pro Ile Asp Glu Gly His Leu Pro Met Ile Asn Gln Asp Glu Phe
                660                 665                 670

aga aac ttt tcc tat gtg tct cca gaa ttg caa cca tag ccttatgggg     2367
Arg Asn Phe Ser Tyr Val Ser Pro Glu Leu Gln Pro
            675                 680

agtgagagag agggcacgag aacccaaagg gaatagagat tctccaggaa tttcctctat     2427

gggaccttcc cagcatcagc cttagaacaa gaaccttacc ttcaaggagc aagtgaagaa     2487

ctctgtgaag gatggaactt tcagatatca actatttaga gtccagaggg agccatggca     2547

ctagaaatag ttgataatga aatgagattt tatgaagtat accgctccac ctatgagcgt     2607

ctgtctctgt gggcttggga tgttaacagg agccaaaagg agggaaagtg tgaagaataa     2667

agtagatctg agaaattctg agccaatcag gcttcttaat tcaagagaca aaccaagacg     2727

ttctgtcaac tgtgctgtgc tcttctttaa gccaatgaac cccaattcct ggcagtctac     2787

aagaagtctc ttaatgctaa tgaagaattt aaaggtcttt ttaaggaaat gaagggcttt     2847

ccaaatagaa tgatttactc tgaagaaaca aacaatggta tctctgaaac tcacaaccta     2907

aagcccaatc ttgaaaatat gttgtgcacc aagacgactg cttcagcttc ttctcttatc     2967

cttactttct ttaatagata tttattaaac tgtccagtga aaaggtgcca caatgcccag     3027

tattgtaaac aacaggtttg cattcatgaa gctttcattc attctggagt ctactaattt     3087

acctgaatgg tgtttgcatt ctgtgaaatg cctctccacg ttgcatatgt cacacttttg     3147

tctgcacata actctttttt cacaagaagg gtcactgcca caacagcaca gtcagcgggt     3207

gaattacagg tgcctgctgc ctgcctacct gggtaatctg atcttgtctg tatcgccgtg     3267

tgctcatcac tgaagaattg caggccactc atgtcagtga ccagatttgt ggcttataaa     3327

cattagcagt ttatttatgt tttaagatgc aaagatgtgt gtttgatatt cactttaata     3387

attagaaatg gatcttgtaa acagggcata tatcaaagat gaccttataa tatgtacccg     3447

aatatacagt tcaagaattt tgtctgactg gaaataaatg cattttgtag caaaaggaaa     3507

aaaaaaaaaa aaaaa                                                      3522

<210>  6
<211>  683
<212>  PRT
<213>  Homo sapiens

<400>  6

Met Ser Ser Gly Thr Met Lys Phe Asn Gly Tyr Leu Arg Val Arg Ile
1               5                   10                  15

Gly Glu Ala Val Gly Leu Gln Pro Thr Arg Trp Ser Leu Arg His Ser
            20                  25                  30

Leu Phe Lys Lys Gly His Gln Leu Leu Asp Pro Tyr Leu Thr Val Ser
            35                      40                      45

Val Asp Gln Val Arg Val Gly Gln Thr Ser Thr Lys Gln Lys Thr Asn
        50                      55                      60

Lys Pro Thr Tyr Asn Glu Glu Phe Cys Ala Asn Val Thr Asp Gly Gly
65                      70                      75                      80

His Leu Glu Leu Ala Val Phe His Glu Thr Pro Leu Gly Tyr Asp His
                    85                      90                      95

Phe Val Ala Asn Cys Thr Leu Gln Phe Gln Glu Leu Leu Arg Thr Thr
                100                     105                     110

Gly Ala Ser Asp Thr Phe Glu Gly Trp Val Asp Leu Glu Pro Glu Gly
            115                     120                     125

Lys Val Phe Val Val Ile Thr Leu Thr Gly Ser Phe Thr Glu Ala Thr
        130                     135                     140

Leu Gln Arg Asp Arg Ile Phe Lys His Phe Thr Arg Lys Arg Gln Arg
145                     150                     155                     160

Ala Met Arg Arg Arg Val His Gln Ile Asn Gly His Lys Phe Met Ala
                165                     170                     175

Thr Tyr Leu Arg Gln Pro Thr Tyr Cys Ser His Cys Arg Glu Phe Ile
                180                     185                     190

Trp Gly Val Phe Gly Lys Gln Gly Tyr Gln Cys Gln Val Cys Thr Cys
            195                     200                     205

Val Val His Lys Arg Cys His His Leu Ile Val Thr Ala Cys Thr Cys
        210                     215                     220

Gln Asn Asn Ile Asn Lys Val Asp Ser Lys Ile Ala Glu Gln Arg Phe
225                     230                     235                     240

Gly Ile Asn Ile Pro His Lys Phe Ser Ile His Asn Tyr Lys Val Pro
                245                     250                     255

Thr Phe Cys Asp His Cys Gly Ser Leu Leu Trp Gly Ile Met Arg Gln
                260                     265                     270

Gly Leu Gln Cys Lys Ile Cys Lys Met Asn Val His Ile Arg Cys Gln
            275                     280                     285

Ala Asn Val Ala Pro Asn Cys Gly Val Asn Ala Val Glu Leu Ala Lys
        290                     295                     300

Thr Leu Ala Gly Met Gly Leu Gln Pro Gly Asn Ile Ser Pro Thr Ser
305                 310                 315                 320

Lys Leu Val Ser Arg Ser Thr Leu Arg Arg Gln Gly Lys Glu Ser Ser
                325                 330                 335

Lys Glu Gly Asn Gly Ile Gly Val Asn Ser Ser Asn Arg Leu Gly Ile
            340                 345                 350

Asp Asn Phe Glu Phe Ile Arg Val Leu Gly Lys Gly Ser Phe Gly Lys
        355                 360                 365

Val Met Leu Ala Arg Val Lys Glu Thr Gly Asp Leu Tyr Ala Val Lys
    370                 375                 380

Val Leu Lys Lys Asp Val Ile Leu Gln Asp Asp Asp Val Glu Cys Thr
385                 390                 395                 400

Met Thr Glu Lys Arg Ile Leu Ser Leu Ala Arg Asn His Pro Phe Leu
                405                 410                 415

Thr Gln Leu Phe Cys Cys Phe Gln Thr Pro Asp Arg Leu Phe Phe Val
            420                 425                 430

Met Glu Phe Val Asn Gly Gly Asp Leu Met Phe His Ile Gln Lys Ser
        435                 440                 445

Arg Arg Phe Asp Glu Ala Arg Ala Arg Phe Tyr Ala Ala Glu Ile Ile
    450                 455                 460

Ser Ala Leu Met Phe Leu His Asp Lys Gly Ile Ile Tyr Arg Asp Leu
465                 470                 475                 480

Lys Leu Asp Asn Val Leu Leu Asp His Glu Gly His Cys Lys Leu Ala
                485                 490                 495

Asp Phe Gly Met Cys Lys Glu Gly Ile Cys Asn Gly Val Thr Thr Ala
            500                 505                 510

Thr Phe Cys Gly Thr Pro Asp Tyr Ile Ala Pro Glu Ile Leu Gln Glu
        515                 520                 525

Met Leu Tyr Gly Pro Ala Val Asp Trp Trp Ala Met Gly Val Leu Leu
    530                 535                 540

Tyr Glu Met Leu Cys Gly His Ala Pro Phe Glu Ala Glu Asn Glu Asp
545                 550                 555                 560

Asp Leu Phe Glu Ala Ile Leu Asn Asp Glu Val Val Tyr Pro Thr Trp
                565                 570                 575

Leu His Glu Asp Ala Thr Gly Ile Leu Lys Ser Phe Met Thr Lys Asn
            580                 585                 590

Pro Thr Met Arg Leu Gly Ser Leu Thr Gln Gly Gly Glu His Ala Ile
        595                 600                 605

Leu Arg His Pro Phe Phe Lys Glu Ile Asp Trp Ala Gln Leu Asn His
    610                 615                 620

Arg Gln Ile Glu Pro Pro Phe Arg Pro Arg Ile Lys Ser Arg Glu Asp
625                 630                 635                 640

Val Ser Asn Phe Asp Pro Asp Phe Ile Lys Glu Glu Pro Val Leu Thr
                645                 650                 655

Pro Ile Asp Glu Gly His Leu Pro Met Ile Asn Gln Asp Glu Phe Arg
            660                 665                 670

Asn Phe Ser Tyr Val Ser Pro Glu Leu Gln Pro
        675                 680

<210>  7
<211>  21
<212>  DNA
<213>  Artificial

<220>
<223>  an artificially synthesized primer sequence

<400>  7
ctgtgggcta cctacacgta c                                                21

<210>  8
<211>  20
<212>  DNA
<213>  Artificial

<220>
<223>  an artificially synthesized primer sequence

<400>  8
taggggatgg atttctcgtg                                                  20

<210>  9
<211>  21
<212>  DNA
<213>  Artificial

<220>
<223>  an artificially synthesized primer sequence

<400>  9
cacccccact gaaaaagatg a                                                21

<210>  10
<211>  19
<212>  DNA

<213>  Artificial

<220>
<223>  an artificially synthesized primer sequence

<400>  10
tacctgtgga gcaacctgc                                                    19


<210>  11
<211>  22
<212>  DNA
<213>  Artificial

<220>
<223>  an artificially synthesized primer sequence

<400>  11
tgccatctac atgttggtct tc                                                22


<210>  12
<211>  20
<212>  DNA
<213>  Artificial

<220>
<223>  an artificially synthesized primer sequence

<400>  12
gtcaccacga aggtcaggtc                                                   20


<210>  13
<211>  20
<212>  DNA
<213>  Artificial

<220>
<223>  an artificially synthesized primer sequence

<400>  13
tctctctttc tggcctggag                                                   20



<210>  14
<211>  20
<212>  DNA
<213>  Artificial

<220>
<223>  an artificially synthesized primer sequence

<400>  14
aatgtcggat ggatgaaacc                                                   20


<210>  15
<211>  60
<212>  PRT
<213>  Homo sapiens

<400>  15

Phe Glu Phe Ile Arg Val Leu Gly Lys Gly Ser Phe Gly Lys Val Met
1               5                   10                  15

Leu Ala Arg Val Lys Glu Thr Gly Asp Leu Tyr Ala Val Lys Val Leu
            20                  25                  30

Lys Lys Asp Val Ile Leu Gln Asp Asp Asp Val Glu Cys Thr Met Thr
        35                  40                  45

Glu Lys Arg Ile Leu Ser Leu Ala Arg Asn His Pro
    50                  55                  60

<210>  16
<211>  60
<212>  PRT
<213>  Homo sapiens

<400>  16

Phe Asn Phe Ile Lys Val Leu Gly Lys Gly Ser Phe Gly Lys Val Met
1               5                   10                  15

Leu Ala Glu Leu Lys Gly Lys Asp Glu Val Tyr Ala Val Lys Val Leu
            20                  25                  30

Lys Lys Asp Val Ile Leu Gln Asp Asp Asp Val Asp Cys Thr Met Thr
        35                  40                  45

Glu Lys Arg Ile Leu Ala Leu Ala Arg Lys His Pro
    50                  55                  60

<210>  17
<211>  60
<212>  PRT
<213>  Homo sapiens

<400>  17

Phe Ile Phe His Lys Val Leu Gly Lys Gly Ser Phe Gly Lys Val Leu
1               5                   10                  15

Leu Gly Glu Leu Lys Gly Arg Gly Glu Tyr Phe Ala Ile Lys Ala Leu
            20                  25                  30

Lys Lys Asp Val Val Leu Ile Asp Asp Asp Val Glu Cys Thr Met Val
        35                  40                  45

Glu Lys Arg Val Leu Thr Leu Ala Ala Glu Asn Pro
    50                  55                  60

<210>  18
<211>  56
<212>  PRT
<213>  Homo sapiens

<400>  18

Lys Met Leu Gly Lys Gly Ser Phe Gly Lys Val Phe Leu Ala Glu Phe
1               5                   10                  15

Lys Lys Thr Asn Gln Phe Phe Ala Ile Lys Ala Leu Lys Lys Asp Val
            20                  25                  30

Val Leu Met Asp Asp Asp Val Glu Cys Thr Met Val Glu Lys Arg Val
        35                  40                  45

Leu Ser Leu Ala Trp Glu His Pro
    50                  55

<210>  19
<211>  60
<212>  PRT
<213>  Homo sapiens

<400>  19

Phe Asn Phe Leu Met Val Leu Gly Lys Gly Ser Phe Gly Lys Val Met
1               5                   10                  15

Leu Ala Asp Arg Lys Gly Thr Glu Glu Leu Tyr Ala Ile Lys Ile Leu
            20                  25                  30

Lys Lys Asp Val Val Ile Gln Asp Asp Asp Val Glu Cys Thr Met Val
        35                  40                  45

Glu Lys Arg Val Leu Ala Leu Leu Asp Lys Pro Pro
    50                  55                  60

<210>  20
<211>  60
<212>  PRT
<213>  Homo sapiens

<400>  20

Phe Asn Phe Leu Met Val Leu Gly Lys Gly Ser Phe Gly Lys Val Met
1               5                   10                  15

Leu Ser Glu Arg Lys Gly Thr Asp Glu Leu Tyr Ala Val Lys Ile Leu
            20                  25                  30

Lys Lys Asp Val Val Ile Gln Asp Asp Asp Val Glu Cys Thr Met Val
        35                  40                  45

Glu Lys Arg Val Leu Ala Leu Pro Gly Lys Pro Pro
    50                  55                  60

<210>  21
<211>  60
<212>  PRT
<213>  Homo sapiens

<400>  21

Phe Asn Phe Leu Met Val Leu Gly Lys Gly Ser Phe Gly Lys Val Met
1               5                   10                  15

Leu Ser Glu Arg Lys Gly Thr Asp Glu Leu Tyr Ala Val Lys Ile Leu
            20                  25                  30

Lys Lys Asp Val Val Ile Gln Asp Asp Asp Val Glu Cys Thr Met Val
        35                  40                  45

Glu Lys Arg Val Leu Ala Leu Pro Gly Lys Pro Pro
    50                  55                  60

<210>  22
<211>  60
<212>  PRT
<213>  Homo sapiens

<400>  22

Phe Ser Phe Leu Met Val Leu Gly Lys Gly Ser Phe Gly Lys Val Met
1               5                   10                  15

Leu Ala Glu Arg Arg Gly Ser Asp Glu Leu Tyr Ala Ile Lys Ile Leu
            20                  25                  30

Lys Lys Asp Val Ile Val Gln Asp Asp Asp Val Asp Cys Thr Leu Val
        35                  40                  45

Glu Lys Arg Val Leu Ala Leu Gly Gly Arg Gly Pro
    50                  55                  60

<210>  23
<211>  60
<212>  PRT
<213>  Homo sapiens

<400>  23

Phe Asp Leu Leu Arg Val Ile Gly Arg Gly Ser Tyr Ala Lys Val Leu
1               5                   10                  15

Leu Val Arg Leu Lys Lys Thr Asp Arg Ile Tyr Ala Met Lys Val Val
            20                  25                  30

Lys Lys Glu Leu Val Asn Asp Asp Glu Asp Ile Asp Trp Val Gln Thr
        35                  40                  45

Glu Lys His Val Phe Glu Gln Ala Ser Asn His Pro
    50                  55                  60

<210>  24
<211>  60
<212>  PRT
<213>  Homo sapiens

<400>  24

Phe Asp Leu Ile Arg Val Ile Gly Arg Gly Ser Tyr Ala Lys Val Leu
1               5                   10                  15

Leu Val Arg Leu Lys Lys Asn Asp Gln Ile Tyr Ala Met Lys Val Val
            20                  25                  30

Lys Lys Glu Leu Val His Asp Asp Glu Asp Ile Asp Trp Val Gln Thr
        35                  40                  45

Glu Lys His Val Phe Glu Gln Ala Ser Ser Asn Pro
    50                  55                  60

<210>  25
<211>  25
<212>  DNA
<213>  Artificial

<220>
<223>  an artificially synthesized probe sequence

<400>  25
aattcttaca ctcgttcttc catct                                          25

<210>  26
<211>  25
<212>  DNA
<213>  Artificial

<220>
<223>  an artificially synthesized probe sequence

<400>  26
gccaggtact gtgttatgtt cgtgt                                          25

<210>  27
<211>  25
<212>  DNA
<213>  Artificial

<220>
<223>  an artificially synthesized probe sequence

<400>  27
tagattggat gggagggggt gagaa                                          25

<210>  28
<211>  25
<212>  DNA
<213>  Artificial

<220>
<223>  an artificially synthesized probe sequence

<400>  28
cttactctct gaggctcaag ccaga                                          25

<210>  29
<211>  25
<212>  DNA

<213>  Artificial

<220>
<223>  an artificially synthesized probe sequence

<400>  29
ggcaccacgg gaaacggtct ggtgc                                          25


<210>  30
<211>  25
<212>  DNA
<213>  Artificial

<220>
<223>  an artificially synthesized probe sequence

<400>  30
tcttgtctgt gttgtactta tatgt                                          25


<210>  31
<211>  25
<212>  DNA
<213>  Artificial

<220>
<223>  an artificially synthesized probe sequence

<400>  31
ctctgctgtt catacattac ccttg                                          25


<210>  32
<211>  25
<212>  DNA
<213>  Artificial

<220>
<223>  an artificially synthesized probe sequence

<400>  32
aggcttgggg gcgtcctact gagct                                          25


<210>  33
<211>  25
<212>  DNA
<213>  Artificial


<220>
<223>  an artificially synthesized probe sequence

<400>  33
tggccccttt ccagtgcctc cccgc                                          25


<210>  34
<211>  25
<212>  DNA
<213>  Artificial

<220>
<223>  an artificially synthesized probe sequence

<400>  34
aattcttaca cttgttcttc catct                                          25

<210>  35
<211>  25
<212>  DNA
<213>  Artificial

<220>
<223>  an artificially synthesized probe sequence

<400>  35
gccaggtact gtattatgtt cgtgt                                          25


<210>  36
<211>  25
<212>  DNA
<213>  Artificial

<220>
<223>  an artificially synthesized probe sequence

<400>  36
   tagattggat ggaagggggt gagaa                                           25


<210>  37
<211>  25
<212>  DNA
<213>  Artificial

<220>
<223>  an artificially synthesized probe sequence

<400>  37
cttactctct gaagctcaag ccaga                                          25


<210>  38
<211>  25
<212>  DNA
<213>  Artificial

<220>
<223>  an artificially synthesized probe sequence

<400>  38
ggcaccacgg gcaacggtct ggtgc                                          25


<210>  39
<211>  25
<212>  DNA
<213>  Artificial

<220>
<223>  an artificially synthesized probe sequence

<400>  39
ttgtctgtgt gtgttgtact tatat                                          25


<210>  40
<211>  25
<212>  DNA
<213>  Artificial

<220>

<223>  an artificially synthesized probe sequence

<400>  40
ctctgctgtt cacacattac ccttg                                          25


<210>  41
<211>  25
<212>  DNA
<213>  Artificial

<220>
<223>  an artificially synthesized probe sequence

<400>  41
aggcttgggg gcatcctact gagct                                          25


<210>  42
<211>  25
<212>  DNA
<213>  Artificial

<220>
<223>  an artificially synthesized probe sequence

<400>  42
tggccccttt ccggtgcctc cccgc                                          25


<210>  43
<211>  25
<212>  DNA
<213>  Artificial

<220>
<223>  an artificially synthesized probe sequence

<400>  43
tagattggat gggagggggt gagaa                                          25


<210>  44
<211>  25
<212>  DNA
<213>  Artificial

<220>
<223>  an artificially synthesized probe sequence


<400>  44
tagattggat ggaagggggt gagaa                                          25


<210>  45
<211>  25
<212>  DNA
<213>  Artificial

<220>
<223>  an artificially synthesized probe sequence

<400>  45
aggcttgggg gcgtcctact gagct                                          25


<210>  46

<211>  25
<212>  DNA
<213>  Artificial

<220>
<223>  an artificially synthesized probe sequence

<400>  46
aggcttgggg gcatcctact gagct                                          25


<210>  47
<211>  22
<212>  DNA
<213>  Artificial

<220>
<223>  an artificially synthesized probe sequence

<400>  47
attcgatcgg ggcggggcga gc                                             22